(19) Europäisches Patentamt
European Patent Office
Office européen des brevets

(11) EP 4 585 592 A1

(12) **EUROPEAN PATENT APPLICATION**
published in accordance with Art. 153(4) EPC

(43) Date of publication:
**16.07.2025 Bulletin 2025/29**

(21) Application number: **23862460.5**

(22) Date of filing: **07.09.2023**

(51) International Patent Classification (IPC):
***C07D 401/04*** (2006.01) ***C07D 401/14*** (2006.01)
***C07D 405/14*** (2006.01) ***C07D 471/04*** (2006.01)
***C07D 491/107*** (2006.01) ***C07D 487/08*** (2006.01)
***C07D 413/14*** (2006.01) ***C07D 471/08*** (2006.01)
***C07D 409/14*** (2006.01) ***C07D 417/14*** (2006.01)
***A61K 31/454*** (2006.01) ***A61K 31/496*** (2006.01)
***A61K 31/541*** (2006.01) ***A61K 31/55*** (2006.01)
***A61K 31/506*** (2006.01) ***A61K 31/517*** (2006.01)
***A61K 31/675*** (2006.01) ***A61P 35/00*** (2006.01)
***A61P 35/02*** (2006.01) ***A61P 7/06*** (2006.01)

(52) Cooperative Patent Classification (CPC):
**A61K 31/454; A61K 31/496; A61K 31/506; A61K 31/517; A61K 31/541; A61K 31/55; A61K 31/675; A61P 7/06; A61P 35/00; A61P 35/02; C07D 401/04; C07D 401/14; C07D 405/14; C07D 409/14; C07D 413/14;** (Cont.)

(86) International application number:
**PCT/CN2023/117399**

(87) International publication number:
**WO 2024/051766 (14.03.2024 Gazette 2024/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.09.2022 CN 202211094076**

(71) Applicant: **Gluetacs Therapeutics (Shanghai) Co., Ltd.**
**Shanghai 201306 (CN)**

(72) Inventors:
- **YANG, Xiaobao**
  **Shanghai 201306 (CN)**
- **SUN, Renhong**
  **Shanghai 201306 (CN)**

(74) Representative: **August Debouzy**
**7, rue de Téhéran**
**75008 Paris (FR)**

(54) **MOLECULAR GLUE COMPOUND BASED ON CEREBLON PROTEIN DESIGN AND USE THEREOF**

(57) The present disclosure relates to a compound of Formula (I) or a salt, enantiomer, diastereomer, isotopically enriched analogue, solvate, prodrug or polymorph thereof, and the use thereof. Further provided in the present disclosure are a pharmaceutical composition comprising, as an active ingredient, the compound of Formula (I) or a salt, enantiomer, diastereomer, isotopically enriched analogue, solvate, prodrug or polymorph thereof, and the use thereof. A series of compounds designed and synthesized in the present disclosure can effectively prevent and/or treat diseases or disorders associated with cereblon protein.

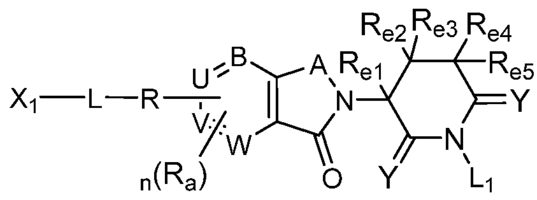

Formula (I)

EP 4 585 592 A1

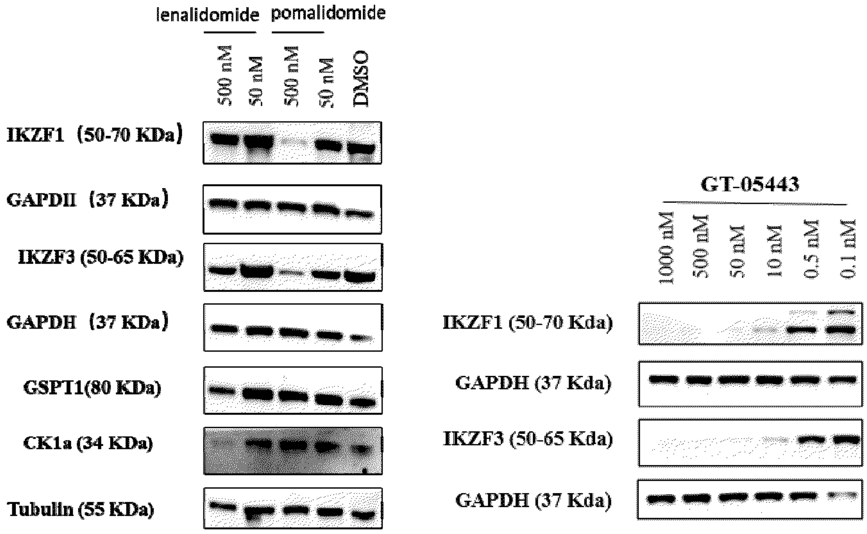


Figure 1

(52) Cooperative Patent Classification (CPC): (Cont.)
**C07D 417/14; C07D 471/04; C07D 471/08; C07D 487/08; C07D 491/107**

## Description

### Technical Field

The present disclosure generally relates to a compound of Formula (I) or salts, enantiomers, diastereoisomer, isotopically enriched analogs, solvates, prodrugs, or polymorphs thereof, and their use in the prevention and/or treatment of diseases or disorders associated with cereblon protein.

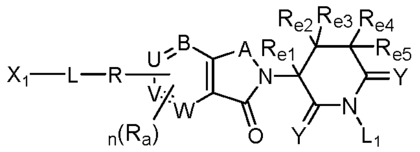

Formula (I)

### Background

Although Thalidomide, lenalidomide and other phthalimide immunomodulatory drugs (IMiDs) have shown significant therapeutic efficacy in the treatment of multiple myeloma and autoimmune diseases, it was not until 2010 that the E3 ubiquitin ligase cereblon (CRBN) was identified as a direct target of IMiDs. Subsequent studies have revealed that these drugs function as molecular glues, recruiting substrate proteins (such as transcription factors IKZF1/3) to CRBN, thereby facilitating protein-protein interactions between CRBN and the substrate proteins and ultimately leading to the ubiquitination of the substrate proteins. The polyubiquitinated substrate proteins are then recognized and degraded by the proteasome, resulting in pharmacological effects including antitumor and immunomodulatory activities. Molecular glue degraders directly target and degrade the target protein, offering potential advantages such as targeting "undruggable" proteins. Moreover, molecular glue degraders typically have low molecular weight and good drug-like properties, making them highly attractive for drug development. Based on the CRBN E3 ubiquitin ligase and molecular glue degradation mechanism, a series of compounds have been developed, including pomalidomide, which has been launched, and CC-122, CC-220, CC-90009, CC-99282, and CC-92480 from Bristol-Myers Squibb (BMS), DKY709 from Novartis and CFT7455 from C4 Therapeutics, which are undergoing clinical trials. The degradation substrates of these molecular glues have expanded from the initially discovered transcription factors IKZF1/3 to include casein kinase 1$\alpha$ (CK1$\alpha$), zinc finger protein 91 (ZFP91) and translation factor GSPT1. Currently, the structural novelty of discovered molecular glue candidates remains limited, making the design of diverse scaffolds and development of novel molecular glues a key research focus in this field.

Therefore, there is an urgent need for a series of novel CRBN E3 ubiquitin ligase ligands that can serve as effective molecular glue degraders for the treatment and/or prevention of diseases or disorders mediated by or associated with degraded proteins.

### Summary of Invention

In view of the above, the objectives of the present disclosure are to provide novel molecular glue protein degraders, their applications and usage methods.

To achieve the above objectives and other related goals, in one aspect, the present disclosure provides a compound of Formula (I):

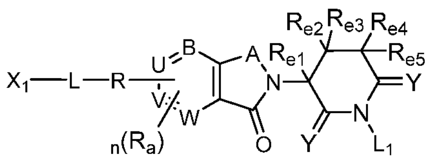

Formula (I)

or salts, enantiomers, diastereoisomer, isotopically enriched analogs, solvates, prodrugs, or polymorphs thereof, wherein

A represents $CH_2$ or C(O);

B, U, V, and W are the same or different and each independently represent CH or N, wherein B, U, V, and W are not N at the same time;

Y represents O or S;

$L_1$ represents hydrogen or $C_{1-3}$ alkyl;

$R_{e1}$, $R_{e2}$, $R_{e3}$, $R_{e4}$, and $R_{e5}$ are the same or different and each independently represent hydrogen or halogen;

$(R_a)_n$ denotes that the ring containing B, U, V, and W is substituted with n $R_a$, where $R_a$ groups are the same or different and each independently represent deuterium, halogen, hydroxy, mercapto, nitro, amino, cyano, optionally deuterated $C_{1-6}$ alkyl, optionally deuterated $C_{1-6}$ alkoxy, halogenated $C_{1-6}$alkyl, optionally substituted $C_{3-6}$ cycloalkyl, $C_{2-6}$ alkenyl, or $C_{2-6}$ alkynyl, and n represents an integer of 1, 2, or 3; and

R represents SH, and L and $X_1$ are absent; or

R represents S, L represents an optionally substituted linear or branched alkyl, and $X_1$ is absent; or

R represents S(O) or $S(O)_2$, L represents an optionally substituted linear or branched alkyl or amino, and $X_1$ is absent; or

R represents S, S(O), $S(O)_2$, O, NH or $CH_2$,

L represents optionally substituted linear or branched alkylene, wherein one or more (e.g., 1-15, 1-10, 1-8, 1-7, 1-6, 1-5, 1-4, 1-3, 1-2 or 1) pairs of adjacent carbon atoms in the backbone carbon chain of the linear or branched alkylene is optionally interrupted by one or more (e.g., 1-30, 1-20, 1-15, 1-10, 1-8, 1-7, 1-6, 1-5, 1-4, 1-3, 1-2 or 1) groups selected from the group consisting of: $R_b$, $R_c$, and any combination thereof, wherein $R_b$ groups are each selected from the group consisting of O, C(O), OC(O), S, S(O), $S(O)_2$, $S(O)_2N(R_1)$, $N(R_1)S(O)_2$, $C(O)N(R_1)$, $N(R_1)C(O)$, $N(R_1)$, and $N(R_1)C(O)N(R_1)$, where $R_1$ groups each independently represent H or $C_{1-3}$ alkyl, and when two or more groups $R_b$ are inserted into the backbone carbon chain of the linear or branched alkylene group, the two or more groups $R_b$ are not directly connected to each other; and wherein $R_c$ groups are each selected from the group consisting of optionally substituted cycloalkylene, optionally substituted arylene, optionally substituted heterocyclylene, optionally substituted heteroarylene, and any combination thereof; and

$X_1$ represents $NR_2R_3$, $C(O)NR_4R_5$, $NHC(O)R_6$, $NHC(O)NR_7R_8$, $OR_9$, $SR_{10}$, quaternary ammonium salt group, linear or branched alkyl optionally substituted with one or more (e.g., 1-30, 1-20, 1-15, 1-10, 1-8, 1-7, 1-6, 1-5, 1-4, 1-3, 1-2 or 1) fluorine, optionally substituted cycloalkyl, optionally substituted aryl, optionally substituted heterocyclyl or optionally substituted heteroaryl, where $R_2$, $R_3$, $R_4$, $R_5$, $R_7$, $R_8$, $R_9$, and $R_{10}$ each independently represent H, optionally substituted linear or branched alkyl, optionally substituted cycloalkyl, optionally substituted aryl, optionally substituted heterocyclyl or optionally substituted heteroaryl, wherein $R_2$ and $R_3$ are not H at the same time, and wherein $R_6$ represents optionally substituted linear or branched alkyl, optionally substituted cycloalkyl, optionally substituted aryl, optionally substituted heteroaryl or optionally substituted heterocyclyl, or wherein $R_4$ and $R_5$ together with the nitrogen atom to which they are attached form an optionally substituted 5- to 8-membered heterocyclyl; or

$X_1$ represents a group of Formula ($G_1$):

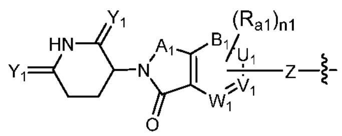

Formula ($G_1$)

wherein $A_1$ represents $CH_2$ or C(O);

$B_1$, $U_1$, $V_1$, and $W_1$ are the same or different and each independently represent CH or N, wherein $B_1$, $U_1$, $V_1$, and $W_1$ are not N at the same time;

$Y_1$ represents O or S; and

Z represents S, S(O), $S(O)_2$, O, NH or $CH_2$; and

$(R_{a1})_{n1}$ denotes that the ring containing $B_1$, $U_1$, $V_1$, and $W_1$ is substituted with n1 $R_{a1}$, where $R_{a1}$ groups are the same or different and each independently represent deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, optionally substituted $C_{3-6}$ cycloalkyl, $C_{2-6}$ alkenyl, or $C_{2-6}$ alkynyl, and n represents an integer of 0, 1, 2, or 3.

In another aspect, the present disclosure provides a pharmaceutical composition comprising the compound of Formula (I) or pharmaceutically acceptable salts, enantiomers, diastereoisomers, isotopically enriched analogs, solvates, prodrugs, or polymorphs thereof, and at least one pharmaceutically acceptable carrier.

In a further aspect, the present disclosure further provides a medicine kit or reagent kit comprising: the compound of Formula (I) or a pharmaceutically acceptable salt, enantiomers, diastereoisomers, isotopically enriched analogs,

solvates, prodrugs, or polymorphs thereof, or the pharmaceutical composition of the present disclosure.

In a further aspect, the present disclosure provides the compound of Formula (I) or pharmaceutically acceptable salts, enantiomers, diastereoisomers, isotopically enriched analogs, solvates, prodrugs, or polymorphs thereof, for use as a medicament.

In a further aspect, the present disclosure provides the compound of Formula (I) or pharmaceutically acceptable salts, enantiomers, diastereoisomers, isotopically enriched analogs, solvates, prodrugs, or polymorphs thereof, or the pharmaceutical composition of the present disclosure for use in the prevention and/or treatment of diseases or disorders associated with cereblon protein.

In a further aspect, the present disclosure provides use of the compound of Formula (I) or pharmaceutically acceptable salts, enantiomers, diastereoisomers, isotopically enriched analogs, solvates, prodrugs, or polymorphs thereof, or the pharmaceutical composition of the present disclosure for the manufacture of a medicament for the prevention and/or treatment of diseases or disorders associated with cereblon protein.

In a further aspect, the present disclosure provides a method for treating or preventing diseases or disorders associated with cereblon protein in a subject, comprising administering to the subject a therapeutically effective amount of the compound of Formula (I) or pharmaceutically acceptable salts, enantiomers, diastereoisomers, isotopically enriched analogs, solvates, prodrugs, or polymorphs thereof, or the pharmaceutical composition.

**Description of Drawings**

Figure 1 illustrates the degradation of substrate proteins, including IKZF1/3 proteins, by the compounds of the present invention in Human peripheral blood mononuclear cells (hPBMC).

**Detailed Description of the Invention**

The following detailed description is provided as exemplary specific embodiments to assist those skilled in the art in understanding and practicing the present disclosure. It should be appreciated, however, that such description is not intended to limit the scope of the present disclosure, and that various modifications and changes may be made to the specific embodiments described in the present disclosure without departing from the spirit and scope of the present disclosure. Such changes and modifications are to be understood as being included within the scope of the present invention as defined by the appended claims.

**I. Compounds**

The present disclosure provides the compound of Formula (I):

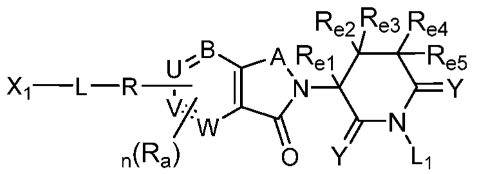

,

Formula (I)

or salts, enantiomers, diastereoisomer, isotopically enriched analogs, solvates, prodrugs, or polymorphs thereof, wherein

A represents $CH_2$ or C(O);

B, U, V, and W are the same or different and each independently represent CH or N, wherein B, U, V, and W are not N at the same time;

Y represents O or S;

$L_1$ represents hydrogen or $C_{1-3}$ alkyl;

$R_{e1}$, $R_{e2}$, $R_{e3}$, $R_{e4}$, and $R_{e5}$ are the same or different and each independently represent hydrogen or halogen (e.g., fluorine, chlorine, bromine, or iodine);

$(R_a)_n$ denotes that the ring containing B, U, V, and W is substituted with n $R_a$, where $R_a$ groups are the same or different and each independently represent deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, optionally substituted $C_{3-6}$ cycloalkyl, $C_{2-6}$ alkenyl, or $C_{2-6}$ alkynyl, and n represents an integer of 1, 2, or 3; and

R represents SH, and L and $X_1$ are absent; or

R represents S, L represents an optionally substituted linear or branched alkyl, and $X_1$ is absent; or
R represents S(O) or $S(O)_2$, L represents an optionally substituted linear or branched alkyl or amino, and $X_1$ is absent; or
R represents S, S(O), $S(O)_2$, O, NH or $CH_2$,
L represents an optionally substituted linear or branched alkylene, wherein one or more (e.g., 1-15, 1-10, 1-8, 1-7, 1-6, 1-5, 1-4, 1-3, 1-2 or 1) pairs of adjacent carbon atoms in the backbone carbon chain of the linear or branched alkylene is optionally interrupted by one or more (e.g., 1-30, 1-20, 1-15, 1-10, 1-8, 1-7, 1-6, 1-5, 1-4, 1-3, 1-2 or 1) groups selected from the group consisting of: $R_b$, $R_c$, and any combination thereof, wherein $R_b$ groups are each selected from the group consisting of O, C(O), OC(O), S, S(O), $S(O)_2$, $S(O)_2N(R_1)$, $N(R_1)S(O)_2$, $C(O)N(R_1)$, $N(R_1)C(O)$, $N(R_1)$, and $N(R_1)C(O)N(R_1)$, where $R_1$ groups each independently represent H or $C_{1-3}$ alkyl, and when two or more groups $R_b$ are inserted into the backbone carbon chain of the linear or branched alkylene group, the two or more groups $R_b$ are not directly connected to each other; and wherein $R_c$ groups are each selected from the group consisting of optionally substituted cycloalkylene, optionally substituted arylene, optionally substituted heterocyclylene, optionally substituted heteroarylene, and any combination thereof; and
$X_1$ represents $NR_2R_3$, $C(O)NR_4R_5$, $NHC(O)R_6$, $NHC(O)NR_7R_8$, $OR_9$, $SR_{10}$, quaternary ammonium salt group, linear or branched alkyl optionally substituted with one or more (e.g., 1-30, 1-20, 1-15, 1-10, 1-8, 1-7, 1-6, 1-5, 1-4, 1-3, 1-2 or 1) fluorine atoms, optionally substituted cycloalkyl, optionally substituted aryl, optionally substituted heterocyclyl or optionally substituted heteroaryl, where $R_2$, $R_3$, $R_4$, $R_5$, $R_7$, $R_8$, $R_9$, and $R_{10}$ each independently represent H, optionally substituted linear or branched alkyl, optionally substituted cycloalkyl, optionally substituted aryl, optionally substituted heterocyclyl or optionally substituted heteroaryl, wherein $R_2$ and $R_3$ are not H at the same time, and wherein $R_6$ represents optionally substituted linear or branched alkyl, optionally substituted cycloalkyl, optionally substituted aryl, optionally substituted heteroaryl or optionally substituted heterocyclyl, or wherein $R_4$ and $R_5$ together with the nitrogen atom to which they are attached form optionally substituted 5- to 8-membered heterocyclyl; or
$X_1$ represents a group of Formula ($G_1$):

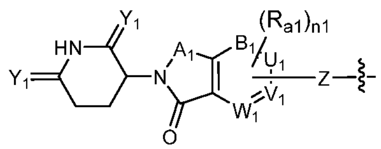

Formula ($G_1$)

wherein $A_1$ represents $CH_2$ or C(O);
$B_1$, $U_1$, $V_1$, and $W_1$ are the same or different and each independently represent CH or N, wherein $B_1$, $U_1$, $V_1$, and $W_1$ are not N at the same time;
$Y_1$ represents O or S; and
Z represents S, S(O), $S(O)_2$, O, NH or $CH_2$; and
$(R_{a1})_{n1}$ denotes that the ring containing $B_1$, $U_1$, $V_1$, and $W_1$ is substituted with n1 $R_{a1}$, where $R_{a1}$ groups are the same or different and each independently represent deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, optionally substituted $C_{3-6}$ cycloalkyl, $C_{2-6}$ alkenyl, or $C_{2-6}$ alkynyl, and n represents an integer of 0, 1, 2, or 3.

The compounds of Formula (I), or salts (including pharmaceutically acceptable salts), stereoisomers (including enantiomers), solvates, isotopically enriched analogs, prodrugs, or polymorphs thereof of the present disclosure, exhibit strong binding affinity to CRBN. The compounds of Formula (I), or salts (including pharmaceutically acceptable salts), stereoisomers (including enantiomers), solvates, isotopically enriched analogs, prodrugs, or polymorphs thereof of the present disclosure are also capable of degrading substrate proteins (e.g., IKZF1/2/3/4 proteins, WEE1 protein, CK1$\alpha$ protein, GSPT1 protein, etc.). The compounds of Formula (I), or salts (including pharmaceutically acceptable salts), stereoisomers (including enantiomers), solvates, isotopically enriched analogs, prodrugs, or polymorphs thereof of the present disclosure can effectively prevent and/or treat cereblon protein-associated diseases or disorders, including tumors, autoimmune diseases, and inflammatory diseases, or exhibit excellent pharmacokinetic properties, and can be used as therapeutic agents for cancer patients or patients with autoimmune diseases.
The present disclosure provides the compound of Formula (I-1):

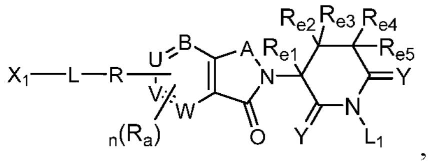

Formula (I-1)

or salts, enantiomers, diastereoisomer, isotopically enriched analogs, solvates, prodrugs, or polymorphs thereof, wherein A, B, U, V, W, Y, $L_1$, $R_{e1}$, $R_{e2}$, $R_{e3}$, $R_{e4}$, $R_{e5}$, and $(R_a)_n$ are as defined in the compounds of Formula (I) above and its various sub-embodiments described herein; and R represents SH, and L and $X_1$ are absent.

The present disclosure provides the compound of Formula (I-2):

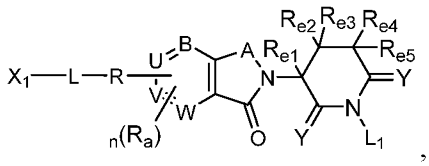

Formula (I-2)

or salts, enantiomers, diastereoisomer, isotopically enriched analogs, solvates, prodrugs, or polymorphs thereof, wherein A, B, U, V, W, Y, $L_1$, $R_e$, and $(R_a)_n$ are as defined in the compounds of Formula (I) above and its various sub-embodiments described herein; and R represents S, L represents an optionally substituted linear or branched alkyl, and $X_1$ is absent.

The present disclosure provides the compound of Formula (I-3):

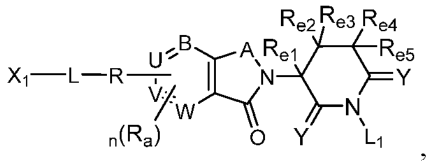

Formula (I-3)

or salts, enantiomers, diastereoisomer, isotopically enriched analogs, solvates, prodrugs, or polymorphs thereof, wherein A, B, U, V, W, Y, $L_1$, $R_e$, and $(R_a)_n$ are as defined in the compounds of Formula (I) above and its various sub-embodiments described herein; and R represents S(O) or $S(O)_2$, L represents an optionally substituted linear or branched alkyl or amino, and $X_1$ is absent.

The present disclosure provides the compound of Formula (I-4):

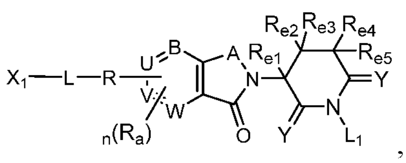

Formula (I-4)

or salts, enantiomers, diastereoisomer, isotopically enriched analogs, solvates, prodrugs, or polymorphs thereof, wherein

A, B, U, V, W, Y, $L_1$, $R_e$, and $(R_a)_n$ are as defined in the compounds of Formula (I) above and its various sub-embodiments described herein; and

R represents S, S(O), $S(O)_2$, O, NH or $CH_2$,

L represents optionally substituted linear or branched alkylene, wherein one or more (e.g., 1-15, 1-10, 1-8, 1-7, 1-6, 1-5, 1-4, 1-3, 1-2 or 1) pairs of adjacent carbon atoms in the backbone carbon chain of the linear or branched alkylene is optionally interrupted by one or more (e.g., 1-30, 1-20, 1-15, 1-10, 1-8, 1-7, 1-6, 1-5, 1-4, 1-3, 1-2 or 1) groups

selected from the group consisting of: $R_b$, $R_c$, and any combination thereof, wherein $R_b$ groups are each selected from the group consisting of O, C(O), OC(O), S, S(O), $S(O)_2$, $S(O)_2N(R_1)$, $N(R_1)S(O)_2$, $C(O)N(R_1)$, $N(R_1)C(O)$, $N(R_1)$, and $N(R_1)C(O)N(R_1)$, where $R_1$ groups each independently represent H or $C_{1-3}$ alkyl, and when two or more groups $R_b$ are inserted into the backbone carbon chain of the linear or branched alkylene group, the two or more groups $R_b$ are not directly connected to each other; and wherein $R_c$ groups are each selected from the group consisting of optionally substituted cycloalkylene, optionally substituted arylene, optionally substituted heterocyclylene, optionally substituted heteroarylene, and any combination thereof; and

$X_1$ represents $NR_2R_3$, $C(O)NR_4R_5$, $NHC(O)R_6$, $NHC(O)NR_7R_8$, $OR_9$, $SR_{10}$, quaternary ammonium salt group, linear or branched alkyl optionally substituted with one or more (e.g., 1-30, 1-20, 1-15, 1-10, 1-8, 1-7, 1-6, 1-5, 1-4, 1-3, 1-2 or 1) fluorine atoms, optionally substituted cycloalkyl, optionally substituted aryl, optionally substituted heterocyclyl or optionally substituted heteroaryl, where $R_2$, $R_3$, $R_4$, $R_5$, $R_7$, $R_8$, $R_9$, and $R_{10}$ each independently represent H, optionally substituted linear or branched alkyl, optionally substituted cycloalkyl, optionally substituted aryl, optionally substituted heterocyclyl or optionally substituted heteroaryl, wherein $R_2$ and $R_3$ are not H at the same time, and wherein $R_6$ represents optionally substituted linear or branched alkyl, optionally substituted cycloalkyl, optionally substituted aryl, optionally substituted heteroaryl or optionally substituted heterocyclyl, or wherein $R_4$ and $R_5$ together with the nitrogen atom to which they are attached form optionally substituted 5- to 8-membered heterocyclyl; or

$X_1$ represents a group of Formula ($G_1$):

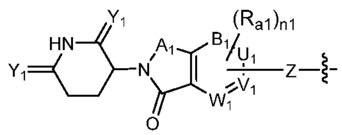

Formula ($G_1$)

wherein $A_1$ represents $CH_2$ or C(O);

$B_1$, $U_1$, $V_1$, and $W_1$ are the same or different and each independently represent CH or N, wherein $B_1$, $U_1$, $V_1$, and $W_1$ are not N at the same time;

$Y_1$ represents O or S; and

Z represents S, S(O), $S(O)_2$, O, NH or $CH_2$; and

$(R_{a1})_{n1}$ denotes that the ring containing $B_1$, $U_1$, $V_1$, and $W_1$ is substituted with n1 $R_{a1}$, where $R_{a1}$ groups are the same or different and each independently represent deuterium, halogen, hydroxy, mercapto, nitro, amino, cyano, optionally deuterated $C_{1-6}$ alkyl, optionally deuterated $C_{1-6}$ alkoxy, halogenated $C_{1-6}$alkyl, optionally substituted $C_{3-6}$ cycloalkyl, $C_{2-6}$ alkenyl, or $C_{2-6}$ alkynyl, and n represents an integer of 0, 1, 2, or 3.

In some embodiments of the compounds of Formula (I), Formula (I-1), Formula (I-2), Formula (I-3), and Formula (I-4) of the present disclosure, B, U, V, and W are identical, each being CH.

In some embodiments of the compounds of Formula (I), Formula (I-1), Formula (I-2), Formula (I-3), and Formula (I-4) of the present disclosure, one of B, U, V, and W is N, and the remaining are CH.

In some embodiments of the compounds of Formula (I), Formula (I-1), Formula (I-2), Formula (I-3), and Formula (I-4) of the present disclosure, two of B, U, V, and W are N, and the remaining are CH.

In some embodiments of the compounds of Formula (I), Formula (I-1), Formula (I-2), Formula (I-3), and Formula (I-4) of the present disclosure, three of B, U, V, and W are N, and the remaining is CH.

In some embodiments of the compounds of Formula (I), Formula (I-1), Formula (I-2), Formula (I-3), and Formula (I-4) of the present disclosure, Y represents O.

In some embodiments of the compounds of Formula (I), Formula (I-1), Formula (I-2), Formula (I-3), and Formula (I-4) of the present disclosure, Y represents S.

In some embodiments of the compounds of Formula (I), Formula (I-1), Formula (I-2), Formula (I-3), and Formula (I-4) of the present disclosure, A represents C(O).

In some embodiments of the compounds of Formula (I), Formula (I-1), Formula (I-2), Formula (I-3), and Formula (I-4) of the present disclosure, A represents $CH_2$.

In some embodiments of the compounds of Formula (I), Formula (I-1), Formula (I-2), Formula (I-3), and Formula (I-4) of the present disclosure, $L_1$ represents hydrogen.

In some embodiments of the compounds of Formula (I), Formula (I-1), Formula (I-2), Formula (I-3), and Formula (I-4) of the present disclosure, $L_1$ represents $C_{1-3}$ alkyl (e.g., methyl).

In some embodiments of the compounds of Formula (I), Formula (I-1), Formula (I-2), Formula (I-3), and Formula (I-4) of the present disclosure, one of $R_{e1}$, $R_{e2}$, $R_{e3}$, $R_{e4}$, and $R_{e5}$ represents halogen (e.g., fluorine), and the remaining

represent hydrogen.

In some embodiments of the compounds of Formula (I), Formula (I-1), Formula (I-2), Formula (I-3), and Formula (I-4) of the present disclosure, two of $R_{e1}$, $R_{e2}$, $R_{e3}$, $R_{e4}$, and $R_{e5}$ represent halogen (e.g., fluorine), and the remaining represent hydrogen.

In some embodiments of the compounds of Formula (I), Formula (I-1), Formula (I-2), Formula (I-3), and Formula (I-4) of the present disclosure, three of $R_{e1}$, $R_{e2}$, $R_{e3}$, $R_{e4}$, and $R_{e5}$ represent halogen (e.g., fluorine), and the remaining represent hydrogen.

In some embodiments of the compounds of Formula (I), Formula (I-1), Formula (I-2), Formula (I-3), and Formula (I-4) of the present disclosure, $(R_a)_n$ denotes that the ring containing B, U, V, and W is substituted with n $R_a$, where $R_a$ groups are the same or different and each independently represent deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, optionally substituted $C_{3-6}$ cycloalkyl, $C_{2-6}$ alkenyl, or $C_{2-6}$ alkynyl, and n represents an integer of 1, 2, or 3.

In some embodiments of the compounds of Formula (I) and Formula (I-2) of the present disclosure, R in Formula (I) represents S, L represents an optionally substituted linear or branched alkyl, and $X_1$ is absent. In a sub-embodiment, R represents S, L represents an optionally substituted linear or branched $C_{1-40}$ alkyl (especially $C_{1-30}$ alkyl), and $X_1$ is absent. In a sub-embodiment, R represents S, L represents the following optionally substituted groups: methyl, ethyl, n-propyl, isopropyl, n-butyl, isobutyl, sec-butyl, tert-butyl, pentyl, isopentyl, neopentyl, tert-amyl, hexyl, heptyl, octyl, nonyl, decyl, undecyl, dodecyl, tridecyl, tetradecyl, or pentadecyl, and $X_1$ is absent. In a sub-embodiment, L represents an optionally substituted linear or branched alkyl, which is optionally substituted by one or more (e.g., 1-30, 1-20, 1-15, 1-10, 1-8, 1-7, 1-6, 1-5, 1-4, 1-3, 1-2 or 1) substituents selected from the group consisting of halogen, cyano, hydroxy, amino, mercapto, or any combination thereof.

In some embodiments of the compounds of Formula (I) and Formula (I-3) of the present disclosure, R in Formula (I) represents S(O) or $S(O)_2$, L represents an optionally substituted linear or branched alkyl or amino, and $X_1$ is absent. In a sub-embodiment, R represents S(O) or $S(O)_2$, L represents an optionally substituted linear or branched $C_{1-40}$ alkyl (especially $C_{1-30}$ alkyl) or amino, and $X_1$ is absent. In a sub-embodiment, R represents S(O) or $S(O)_2$, L represents the following optionally substituted groups: methyl, ethyl, n-propyl, isopropyl, n-butyl, isobutyl, sec-butyl, tert-butyl, pentyl, isopentyl, neopentyl, tert-amyl, hexyl, heptyl, octyl, nonyl, decyl, undecyl, dodecyl, tridecyl, tetradecyl, pentadecyl, or amino, and $X_1$ is absent. In a sub-embodiment, L represents an optionally substituted linear or branched alkyl, which is optionally substituted by one or more (e.g., 1-30, 1-20, 1-15, 1-10, 1-8, 1-7, 1-6, 1-5, 1-4, 1-3, 1-2 or 1) substituents selected from the group consisting of halogen, cyano, hydroxy, amino, mercapto, or any combination thereof.

In some embodiments of the compounds of Formula (I) and Formula (I-1) of the present disclosure, R represents SH, and L and $X_1$ are absent.

In some embodiments of the compounds of Formula (I) and Formula (I-4) of the present disclosure,

R represents S, S(O), $S(O)_2$, O, NH, or $CH_2$;

L represents optionally substituted linear or branched $C_{1-40}$ alkylene, wherein one or more (e.g., 1-15, 1-10, 1-8, 1-7, 1-6, 1-5, 1-4, 1-3, 1-2 or 1) pairs of adjacent carbon atoms in the backbone carbon chain of the linear or branched $C_{1-40}$ alkylene is optionally interrupted by one or more (e.g., 1-30, 1-20, 1-15, 1-10, 1-8, 1-7, 1-6, 1-5, 1-4, 1-3, 1-2 or 1) groups selected from the group consisting of: $R_b$, $R_c$, and any combination thereof, wherein $R_b$ groups are each selected from the group consisting of O, C(O), OC(O), S, S(O), $S(O)_2$, $S(O)_2N(R_1)$, $N(R_1)S(O)_2$, $C(O)N(R_1)$, $N(R_1)C(O)$, $N(R_1)$, and $N(R_1)C(O)N(R_1)$, where $R_1$ groups each independently represent H or $C_{1-3}$alkyl, and when two or more groups $R_b$ are inserted into the backbone carbon chain of the linear or branched $C_{1-40}$ alkylene group, the two or more groups $R_b$ are not directly connected to each other; and wherein $R_c$ groups are each selected from the group consisting of optionally substituted cycloalkylene, optionally substituted arylene, optionally substituted heterocyclylene, optionally substituted heteroarylene, and any combination thereof; and

$X_1$ represents $NR_2R_3$, $C(O)NR_4R_5$, $NHC(O)R_6$, $NHC(O)NR_7R_8$, $OR_9$, $SR_{10}$, quaternary ammonium salt group, linear or branched $C_{1-10}$ alkyl optionally substituted with one or more (e.g., 1-30, 1-20, 1-15, 1-10, 1-8, 1-7, 1-6, 1-5, 1-4, 1-3, 1-2 or 1) fluorine atoms, optionally substituted cycloalkyl, optionally substituted aryl, optionally substituted heterocyclyl or optionally substituted heteroaryl, where $R_2$, $R_3$, $R_4$, $R_5$, $R_7$, $R_8$, $R_9$, and $R_{10}$ each independently represent H, optionally substituted linear or branched $C_{1-10}$ alkyl, optionally substituted cycloalkyl, optionally substituted aryl, optionally substituted heterocyclyl, or optionally substituted heteroaryl, wherein $R_2$ and $R_3$ are not H at the same time, and wherein $R_6$ represents optionally substituted linear or branched $C_{1-8}$ alkyl, optionally substituted cycloalkyl, optionally substituted aryl, optionally substituted heteroaryl, or optionally substituted heterocyclyl, or wherein $R_4$ and $R_5$ together with the nitrogen atom to which they are attached form optionally substituted 5- to 8-membered heterocyclyl; or

$X_1$ represents a group of Formula ($G_1$):

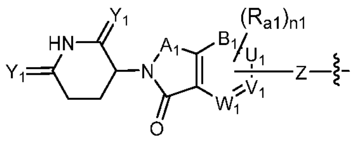

Formula ($G_1$)

wherein $A_1$ represents $CH_2$ or C(O);
$B_1$, $U_1$, $V_1$, and $W_1$ are the same or different and each independently represent CH or N, wherein $B_1$, $U_1$, $V_1$, and $W_1$ are not N at the same time;
$Y_1$ represents O or S; and
Z represents S, S(O) or $S(O)_2$; and
$(R_{a1})_{n1}$ denotes that the ring containing $B_1$, $U_1$, $V_1$, and $W_1$ is substituted with n1 $R_{a1}$, where $R_{a1}$ groups are the same or different and each independently represent deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, optionally substituted $C_{3-6}$ cycloalkyl, $C_{2-6}$ alkenyl, or $C_{2-6}$ alkynyl, and n represents an integer of 0, 1, 2, or 3.

In some embodiments of the compounds of Formula (I) and Formula (I-4) of the present disclosure,

R represents S, S(O), $S(O)_2$, O, NH, or $CH_2$;
L represents linear or branched $C_{1-40}$ alkylene optionally substituted with one or more (e.g., 1-30, 1-20, 1-15, 1-10, 1-8, 1-7, 1-6, 1-5, 1-4, 1-3, 1-2 or 1) substituents selected from the group consisting of halogen, cyano, hydroxy, amino, mercapto, $C_{1-3}$ alkyl, $C_{1-3}$ alkoxy, halogenated $C_{1-3}$alkyl (e.g., trifluoromethyl), $C_{5-10}$ aryl, $C_{5-10}$ heteroaryl, $C_{3-12}$ cycloalkyl, 3- to 12-membered heterocyclyl, or any combination thereof, wherein one or more (e.g., 1-30, 1-20, 1-15, 1-10, 1-8, 1-7, 1-6, 1-5, 1-4, 1-3, 1-2 or 1) pairs of adjacent carbon atoms in the backbone carbon chain of the linear or branched $C_{1-40}$ alkylene is optionally interrupted by one or more (e.g., 1-30, 1-20, 1-15, 1-10, 1-8, 1-7, 1-6, 1-5, 1-4, 1-3, 1-2 or 1) groups selected from the group consisting of: $R_b$, $R_c$, and any combination thereof, wherein $R_b$ groups are each selected from the group consisting of O, C(O), OC(O), S, S(O), $S(O)_2$, $S(O)_2N(R_1)$, $N(R_1)S(O)_2$, $C(O)N(R_1)$, $N(R_1)C(O)$, $N(R_1)$ and $N(R_1)C(O)N(R_1)$, where $R_1$ groups each independently represent H or $C_{1-3}$ alkyl, and when two or more groups $R_b$ are inserted into the backbone carbon chain of the linear or branched alkylene group, the two or more groups $R_b$ are not directly connected to each other; and wherein $R_c$ groups are each selected from the group consisting of cycloalkylene (e.g., $C_{3-20}$ cycloalkylene), arylene (e.g., $C_{5-20}$ arylene), heterocyclylene (e.g., 4- to 20-membered heterocyclylene), heteroarylene (e.g., 5- to 20-membered heteroarylene), and any combination thereof, wherein the cycloalkylene and heterocyclylene are each independently optionally substituted with one or more (e.g., 1-30, 1-20, 1-15, 1-10, 1-8, 1-7, 1-6, 1-5, 1-4, 1-3, 1-2 or 1) substituents selected from the group consisting of halogen, cyano, hydroxy, amino, mercapto, oxo, $C_{1-3}$ alkoxy, halogenated $C_{1-3}$ alkyl, methanesulfonyloxy, trifluoromethanesulfonyloxy, p-toluenesulfonyloxy, optionally deuterated $C_{1-6}$ alkyl, optionally deuterated $C_{3-6}$ cycloalkyl, or any combination thereof; and the arylene and heteroarylene are each independently optionally substituted with one or more (e.g., 1-30, 1-20, 1-15, 1-10, 1-8, 1-7, 1-6, 1-5, 1-4, 1-3, 1-2 or 1) substituents selected from the group consisting of halogen, cyano, hydroxy, amino, mercapto, $C_{1-3}$ alkoxy, halogenated $C_{1-3}$alkyl, methanesulfonyloxy, trifluoromethanesulfonyloxy, p-toluenesulfonyloxy, optionally deuterated $C_{1-6}$ alkyl, optionally deuterated $C_{3-6}$ cycloalkyl, or any combination thereof; and
$X_1$ represents:

$NR_2R_3$, $C(O)NR_4R_5$, $NHC(O)R_6$, $NHC(O)NR_7R_8$, $OR_9$, $SR_{10}$, or quaternary ammonium salt group, where $R_2$, $R_3$, $R_4$, $R_5$, $R_7$, $R_8$, $R_9$, and $R_{10}$ each independently represent H, optionally substituted linear or branched $C_{1-10}$ alkyl, optionally substituted cycloalkyl, optionally substituted aryl, optionally substituted heterocyclyl, or optionally substituted heteroaryl, wherein $R_2$ and $R_3$ are not H at the same time, and wherein $R_6$ represents optionally substituted linear or branched $C_{1-6}$ alkyl, optionally substituted cycloalkyl, optionally substituted aryl, optionally substituted heteroaryl, or optionally substituted heterocyclyl, or wherein $R_4$ and $R_5$ together with the nitrogen atom to which they are attached form optionally substituted 5- to 8-membered heterocyclyl; or
linear or branched $C_{1-10}$ alkyl, optionally substituted with one or more (e.g., 1-30, 1-20, 1-15, 1-10, 1-8, 1-7, 1-6, 1-5, 1-4, 1-3, 1-2 or 1) fluorine; or
$C_3$-$C_{12}$ cycloalkyl, optionally substituted with one or more (e.g., 1-30, 1-20, 1-15, 1-10, 1-8, 1-7, 1-6, 1-5, 1-4, 1-3, 1-2 or 1) substituents selected from the group consisting of: halogen, cyano, amino, hydroxy, mercapto, oxo, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, p-toluenesulfonyloxy, optionally substituted 5- or 6-membered hetero-

cyclyl, optionally substituted $C_5$-$C_{20}$ aryl, optionally substituted 5- or 6-membered heteroaryl, $C_{1-6}$alkyl, $C_{1-3}$ alkoxycarbonyl, halogenated $C_{2-4}$ alkenyl, or any combination thereof, wherein the 5- or 6-membered heterocyclyl is optionally substituted with one or more (e.g., 1-30, 1-20, 1-15, 1-10, 1-8, 1-7, 1-6, 1-5, 1-4, 1-3, 1-2 or 1) substituents selected from the group consisting of: halogen, cyano, amino, hydroxy, mercapto, oxo, $C_{1-6}$ alkyl, halogenated $C_{1-3}$ alkyl, or any combination thereof; and the $C_5$-$C_{20}$ aryl is optionally substituted with one or more (e.g., 1-30, 1-20, 1-15, 1-10, 1-8, 1-7, 1-6, 1-5, 1-4, 1-3, 1-2, or 1) substituents selected from the group consisting of: halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-30, 1-20, 1-15, 1-10, 1-8, 1-7, 1-6, 1-5, 1-4, 1-3, 1-2, or 1) substituents selected from the group consisting of halogen, cyano, amino, hydroxy, mercapto, oxo, $C_{1-6}$ alkyl, halogenated $C_{1-3}$ alkyl, or any combination thereof; or

$C_5$-$C_{14}$ aryl, optionally substituted with one or more (e.g., 1-30, 1-20, 1-15, 1-10, 1-8, 1-7, 1-6, 1-5, 1-4, 1-3, 1-2, or 1) substituents selected from the group consisting of halogen, cyano, amino, hydroxy, mercapto, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, p-toluenesulfonyloxy, optionally substituted 5- or 6-membered heterocyclyl, optionally substituted $C_5$-$C_{20}$ aryl, optionally substituted 5-or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more (e.g., 1-30, 1-20, 1-15, 1-10, 1-8, 1-7, 1-6, 1-5, 1-4, 1-3, 1-2, or 1) substituents selected from the group consisting of halogen, cyano, amino, hydroxy, mercapto, oxo, $C_{1-6}$ alkyl, halogenated $C_{1-3}$ alkyl, or any combination thereof; and the $C_5$-$C_{20}$ aryl is optionally substituted with one or more (e.g., 1-30, 1-20, 1-15, 1-10, 1-8, 1-7, 1-6, 1-5, 1-4, 1-3, 1-2, or 1) substituents selected from the group consisting of halogen, cyano, amino, hydroxy, mercapto, $C_{1-6}$alkyl, halogenated $C_{1-3}$alkyl, or any combination thereof; and the 5- or 6-membered heteroaryl is optionally substituted with one or more (e.g., 1-30, 1-20, 1-15, 1-10, 1-8, 1-7, 1-6, 1-5, 1-4, 1-3, 1-2, or 1) substituents selected from the group consisting of halogen, cyano, amino, hydroxy, mercapto, oxo, $C_{1-6}$alkyl, halogenated $C_{1-3}$ alkyl, or any combination thereof; or

3- to 12-membered heterocyclyl, optionally substituted with one or more (e.g., 1-30, 1-20, 1-15, 1-10, 1-8, 1-7, 1-6, 1-5, 1-4, 1-3, 1-2, or 1) substituents selected from the group consisting of halogen, cyano, amino, hydroxy, mercapto, oxo, $C_{1-3}$ alkoxy, halogenated $C_{1-3}$ alkoxy, halogenated $C_{1-3}$ alkyl, $C_{1-6}$ alkylsulfonyl, bis($C_{1-6}$ alkyl) phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more (e.g., 1-30, 1-20, 1-15, 1-10, 1-8, 1-7, 1-6, 1-5, 1-4, 1-3, 1-2, or 1) substituents selected from the group consisting of halogen, cyano, amino, hydroxy, mercapto, oxo, $C_{1-6}$ alkyl, halogenated $C_{1-3}$ alkyl, or any combination thereof; and the $C_5$-$C_{20}$ aryl is optionally substituted with one or more (e.g., 1-30, 1-20, 1-15, 1-10, 1-8, 1-7, 1-6, 1-5, 1-4, 1-3, 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-30, 1-20, 1-15, 1-10, 1-8, 1-7, 1-6, 1-5, 1-4, 1-3, 1-2, or 1) substituents selected from the group consisting of halogen, cyano, amino, hydroxy, mercapto, oxo, $C_{1-6}$alkyl, halogenated $C_{1-3}$alkyl, or any combination thereof, or

5- to 10-membered heteroaryl, optionally substituted with one or more (e.g., 1-30, 1-20, 1-15, 1-10, 1-8, 1-7, 1-6, 1-5, 1-4, 1-3, 1-2, or 1) substituents selected from the group consisting of halogen, cyano, amino, hydroxy, mercapto, $C_{1-3}$ alkoxy, halogenated $C_{1-3}$ alkoxy, halogenated $C_{1-3}$alkyl, bis($C_{1-6}$ alkyl)phosphono, $C_{1-6}$ alkylsulfonyl, methanesulfonyloxy, trifluoromethanesulfonyloxy, *p*-toluenesulfonyloxy, $C_{1-6}$ alkyl, optionally substituted 5- or 6-membered heterocyclyl, optionally substituted $C_3$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more (e.g., 1-30, 1-20, 1-15, 1-10, 1-8, 1-7, 1-6, 1-5, 1-4, 1-3, 1-2, or 1) substituents selected from the group consisting of $C_{1-6}$ alkyl, cyano, halogenated $C_{1-3}$alkyl, amino, hydroxy, mercapto, halogen, or any combination thereof; and the $C_3$-$C_{20}$ aryl is optionally substituted with one or more (e.g., 1-30, 1-20, 1-15, 1-10, 1-8, 1-7, 1-6, 1-5, 1-4, 1-3, 1-2, or 1) substituents selected from the group consisting of $C_{1-6}$ alkyl, cyano, halogenated $C_{1-3}$ alkyl, amino, hydroxy, mercapto, halogen, or any combination thereof; and the 5- or 6-membered heteroaryl is optionally substituted with one or more (e.g., 1-30, 1-20, 1-15, 1-10, 1-8, 1-7, 1-6, 1-5, 1-4, 1-3, 1-2, or 1) substituents selected from the group consisting of $C_{1-6}$ alkyl, halogenated $C_{1-3}$alkyl, amino, hydroxy, mercapto, halogen, or any combination thereof; or

$X_1$ represents a group of Formula ($G_1$):

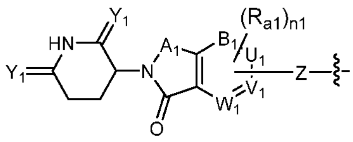

Formula ($G_1$)

wherein $A_1$ represents $CH_2$ or C(O);
$B_1$, $U_1$, $V_1$, and $W_1$ are the same or different and each independently represent CH or N, wherein $B_1$, $U_1$, $V_1$, and $W_1$ are not N at the same time;
$Y_1$ represents O or S; and
Z represents S, S(O), or $S(O)_2$; and
$(R_{a1})_{n1}$ denotes that the ring containing $B_1$, $U_1$, $V_1$, and $W_1$ is substituted with n1 $R_{a1}$, where $R_{a1}$ groups are the same or different and each independently represent deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, optionally substituted $C_{3-6}$ cycloalkyl, $C_{2-6}$ alkenyl, or $C_{2-6}$ alkynyl, and n represents an integer of 0, 1, 2, or 3, wherein the $C_{3-6}$ cycloalkyl is optionally substituted with a substituent selected from the group consisting of: $C_{1-6}$ alkyl, halogenated $C_{1-3}$ alkyl, amino, hydroxy, halogen, or any combination thereof.

In some embodiments of the compounds of Formula (I) and Formula (I-4) of the present disclosure, L represents the structure of the following formula:

linear or branched $C_{1-40}$ alkylene;
$*-(C(R_{a2})(R_{a3}))_{n2}-(R_b-(C(R_{a4})(R_{a5}))_{n3})_{m1}-$;
$*-(C(R_{a2})(R_{a3}))_{n2}-(R_b-(C(R_{a4})(R_{a5}))_{n3})_{m1}-(R_b-(C(R_{a6})(R_{a7}))_{n4})_{m2}-$;
$*-(C(R_{a2})(R_{a3}))_{n2}-(R_b-(C(R_{a4})(R_{a5}))_{n3})_{m1}-(R_b-(C(R_{a6})(R_{a7}))_{n4})_{m2}-(R_b-(C(R_{a8})(R_{a9}))_{n5})_{m3}-$;
$*-(C(R_{a2})(R_{a3}))_{n2}-(R_c-(C(R_{a4})(R_{a5}))_{n3})_{m1}-$;
$*-(C(R_{a2})(R_{a3}))_{n2}-(R_c-(C(R_{a4})(R_{a5}))_{n3})_{m1}-(R_c-(C(R_{a6})(R_{a7}))_{n4})_{m2}-$;
$*-(C(R_{a2})(R_{a3}))_{n2}-(R_c-(C(R_{a4})(R_{a5}))_{n3})_{m1}-(R_c-(C(R_{a6})(R_{a7}))_{n4})_{m2}-(R_c-(C(R_{a8})(R_{a9}))_{n5})_{m3}-$;
$*-(C(R_{a2})(R_{a3}))_{n2}-(R_b-R_c-(C(R_{a4})(R_{a5}))_{n3})_{m1}-$;
$*-(C(R_{a2})(R_{a3}))_{n2}-(R_b-(C(R_{a4})(R_{a5}))_{n3})_{m1}-(R_c-(C(R_{a6})(R_{a7}))_{n4})_{m2}-$;
$*-(C(R_{a2})(R_{a3}))_{n2}-(R_c-R_b-(C(R_{a4})(R_{a5}))_{n3})_{m1}-$; or
$*-(C(R_{a2})(R_{a3}))_{n2}-(R_c-(C(R_{a4})(R_{a5}))_{n3})_{m1}-(R_b-(C(R_{a6})(R_{a7}))_{n4})_{m2}-$;
wherein * indicates the point of attachment to R;
a hydrogen of one or more $CH_2$ groups (e.g., 1-30, 1-20, 1-15, 1-10, 1-8, 1-7, 1-6, 1-5, 1-4, 1-3, 1-2, or 1 $CH_2$) of the linear or branched $C_{1-40}$ alkylene is optionally replaced with a substituent selected from the group consisting of: halogen, cyano, $C_{1-3}$ alkyl, $C_{1-3}$ alkoxy, trifluoromethyl, $C_{5-10}$ aryl, $C_{5-10}$ heteroaryl, $C_{3-12}$ cycloalkyl, 3- to 12-membered heterocyclyl, or any combination thereof;
$R_b$ groups are each independently selected from the group consisting of O, C(O), OC(O), S, S(O), $S(O)_2$, $S(O)_2N(R_1)$, $N(R_1)S(O)_2$, $C(O)N(R_1)$, $N(R_1)C(O)$, $N(R_1)$ and $N(R_1)C(O)N(R_1)$, wherein $R_1$ groups each independently represent H or $C_{1-3}$ alkyl;
$R_c$ groups are each independently selected from the group consisting of optionally substituted cycloalkylene (e.g., $C_{3-20}$ cycloalkylene), optionally substituted arylene (e.g., $C_{5-20}$ arylene), optionally substituted heterocyclylene (e.g., 4- to 20-membered heterocyclylene), optionally substituted heteroarylene (e.g., 5- to 20-membered heteroarylene), and any combination thereof, wherein the cycloalkylene and heterocyclylene are each independently optionally substituted with a substituent selected from the group consisting of halogen, cyano, hydroxy, amino, mercapto, oxo, $C_{1-3}$ alkoxy, halogenated $C_{1-3}$ alkyl, methanesulfonyloxy, trifluoromethanesulfonyloxy, p-toluenesulfonyloxy, optionally deuterated $C_{1-6}$ alkyl, optionally deuterated $C_{3-6}$ cycloalkyl, or any combination thereof; and the arylene and heteroarylene are each independently optionally substituted with a substituent selected from the group consisting of halogen, cyano, hydroxy, amino, mercapto, $C_{1-3}$ alkoxy, halogenated $C_{1-3}$ alkyl, methanesulfonyloxy, trifluoromethanesulfonyloxy, *p*-toluenesulfonyloxy, optionally deuterated $C_{1-6}$ alkyl, optionally deuterated $C_{3-6}$ cycloalkyl, or any combination thereof;
$R_{a2}$, $R_{a3}$, $R_{a4}$, $R_{a5}$, $R_{a6}$, $R_{a7}$, $R_{a8}$, and $R_{a9}$ each independently represent H, halogen, cyano, hydroxy, amino, mercapto, $C_{1-3}$ alkyl, $C_{1-3}$ alkoxy, trifluoromethyl, $C_{5-10}$ aryl, $C_{5-10}$ heteroaryl, $C_{3-12}$ cycloalkyl, or 3- to 12- membered heterocyclyl; and
n2, n3, n4, n5, m1, m2, and m3 each independently represent an integer of 1, 2, 3, 4, 5, 6, 7, 8, 9, 10, 11, 12, 13, 14, or 15.

In some embodiments of the compounds of Formula (I) and Formula (I-4) of the present disclosure, L represents the structure of the following formula:

$*-(C(R_{a2})(R_{a3}))_{n2}-(C_{5-20}$ arylene-$(C(R_{a4})(R_{a5}))_{n3})_{m1}-$;
$*-(C(R_{a2})(R_{a3}))_{n2}-(C_{5-20}$ arylene-$(C(R_{a4})(R_{a5}))_{n3})_{m1}-(C_{5-20}$ arylene-$(C(R_{a6})(R_{a7}))_{n4})_{m2}-$;
$*-(C(R_{a2})(R_{a3}))_{n2}-(C_{5-20}$ arylene-$(C(R_{a4})(R_{a5}))_{n3})_{m1}-(C_{5-20}$ arylene-$(C(R_{a6})(R_{a7}))_{n4})_{m2}-(C_{5-20}$ arylene- $(C(R_{a8})(R_{a9}))_{n5})_{m3}$ -;
$*-(C(R_{a2})(R_{a3}))_{n2}-$(5-to 20-membered heteroarylene-$(C(R_{a4})(R_{a5}))_{n3})_{m1}-$;
$*-(C(R_{a2})(R_{a3}))_{n2}-$(5- to 20-membered heteroarylene-$(C(R_{a4})(R_{a5}))_{n3})_{m1}-$(5- to 20-membered heteroarylene-$(C(R_{a6})(R_{a7}))_{n4})_{m2}-$;
$*-(C(R_{a2})(R_{a3}))_{n2}-$(5- to 20-membered heteroarylene-$(C(R_{a4})(R_{a5}))_{n3})_{m1}-$(5- to 20-membered heteroarylene-$(C(R_{a6})(R_{a7}))_{n4})_{m2}-$(5- to 20-membered heteroarylene-$(C(R_{a8})(R_{a9}))_{n5})_{m3}-$;
$*-(C(R_{a2})(R_{a3}))_{n2}-$(4- to 20-membered heterocyclylene-$(C(R_{a4})(R_{a5}))_{n3})_{m1}-$;
$*-(C(R_{a2})(R_{a3}))_{n2}-$(4- to 20-membered heterocyclylene-$(C(R_{a4})(R_{a5}))_{n3})_{m1}-$(4- to 20-membered heterocyclylene-$(C(R_{a6})(R_{a7}))_{n4})_{m2}-$;
$*-(C(R_{a2})(R_{a3}))_{n2}-(O-(C(R_{a4})(R_{a5}))_{n3})_{m1}-$;
$*-(C(R_{a2})(R_{a3}))_{n2}-(O-(C(R_{a4})(R_{a5}))_{n3})_{m1}-(O-(C(R_{a6})(R_{a7}))_{n4})_{m2}-$;
$*-(C(R_{a2})(R_{a3}))_{n2}-(O-(C(R_{a4})(R_{a5}))_{n3})_{m1}-(O-(C(R_{a6})(R_{a7}))_{n4})_{m2}-(O-(C(R_{a8})(R_{a9}))_{n5})_{m3}-$
$*-(C(R_{a2})(R_{a3}))_{n2}-(S-(C(R_{a4})(R_{a5}))_{n3})_{m1}-$;
$*-(C(R_{a2})(R_{a3}))_{n2}-(S-(C(R_{a4})(R_{a5}))_{n3})_{m1}-(S-(C(R_{a6})(R_{a7}))_{n4})_{m2}-$;
$*-(C(R_{a2})(R_{a3}))_{n2}-(S-(C(R_{a4})(R_{a5}))_{n3})_{m1}-(S-(C(R_{a6})(R_{a7}))_{n4})_{m2}-(S-(C(R_{a8})(R_{a9}))_{n5})_{m3}-$
$*-(C(R_{a2})(R_{a3}))_{n2}-(S(O)-(C(R_{a4})(R_{a5}))_{n3})_{m1}-$;
$*-(C(R_{a2})(R_{a3}))_{n2}-(S(O)_2-(C(R_{a4})(R_{a5}))_{n3})_{m1}-$;
$*-(C(R_{a2})(R_{a3}))_{n2}-(S-(C(R_{a4})(R_{a5}))_{n3})_{m1}-$;
$*-(C(R_{a2})(R_{a3}))_{n2}-(S(O)_2N(R_1)-(C(R_{a4})(R_{a5}))_{n3})_{m1}-$;
$*-(C(R_{a2})(R_{a3}))_{n2}-(N(R_1)S(O)_2-(C(R_{a4})(R_{a5}))_{n3})_{m1}-$;
$*-(C(R_{a2})(R_{a3}))_{n2}-(C(O)N(R_1)-(C(R_{a4})(R_{a5}))_{n3})_{m1}-$;
$*-(C(R_{a2})(R_{a3}))_{n2}-(N(R_1)C(O)-(C(R_{a4})(R_{a5}))_{n3})_{m1}-$;
$*-(C(R_{a2})(R_{a3}))_{n2}-(N(R_1)-(C(R_{a4})(R_{a5}))_{n3})_{m1}-$;
$*-(C(R_{a2})(R_{a3}))_{n2}-(N(R_1)C(O)N(R_1)-(C(R_{a4})(R_{a5}))_{n3})_{m1}-$;
wherein * indicates the point of attachment to R;
$R_1$ groups each independently represent H or $C_{1-3}$alkyl;
$R_{a2}$, $R_{a3}$, $R_{a4}$, $R_{a5}$, $R_{a6}$, $R_{a7}$, $R_{a8}$, and $R_{a9}$ each independently represent H, halogen, cyano, hydroxy, amino, mercapto, $C_{1-3}$ alkyl, $C_{1-3}$ alkoxy, trifluoromethyl, $C_{3-10}$ aryl, $C_{5-10}$ heteroaryl, $C_{3-12}$ cycloalkyl or 3- to 12- membered heterocyclyl;
n2, n3, n4, n5, m1, m2, and m3 each independently represent an integer of 1, 2, 3, 4, 5, 6, 7, 8, 9, 10, 11, 12, 13, 14, or 15; and
the 4- to 20-membered heterocyclylene is optionally substituted with a substituent selected from the group consisting of halogen, cyano, hydroxy, amino, mercapto, oxo, $C_{1-3}$ alkoxy, halogenated $C_{1-3}$ alkyl, methanesulfonyloxy, trifluoromethanesulfonyloxy, p-toluenesulfonyloxy, optionally deuterated $C_{1-6}$ alkyl, optionally deuterated $C_{3-6}$ cycloalkyl, or any combination thereof; and the $C_{5-20}$ arylene and the 5- to 20- membered heteroarylene are each independently optionally substituted with a substituent selected from the group consisting of halogen, cyano, hydroxy, amino, mercapto, $C_{1-3}$ alkoxy, halogenated $C_{1-3}$ alkyl, methanesulfonyloxy, trifluoromethanesulfonyloxy, p-toluenesulfonyloxy, optionally deuterated $C_{1-6}$ alkyl, optionally deuterated $C_{3-6}$ cycloalkyl, or any combination thereof.

In some embodiments of the compounds of Formula (I) and Formula (I-4) of the present disclosure, L represents the structure of the following formula:

linear or branched $C_1$-$C_{40}$ alkylene, $*-(CH_2)_{n2}-(O-(CH_2)_{n3})_{m1}-$, $*-(CH_2)_{n2}-(O-(CH_2)_{n3})_{m1}-(O-(CH_2)_{n4})_{m2}-$, $*-(CH_2)_{n2}-(O-(CH_2)_{n3})_{m1}-(O-(CH_2)_{n4})_{m2}-(O-(CH_2)_{n5})_{m3}-$, $*-(CH_2)_{n2}-S-(CH_2)_{n3}-$, $*-(CH_2)_{n2}-S(O)-(CH_2)_{n3}-$, $*-(CH_2)_{n2}-S(O)_2-(CH_2)_{n3}-$, $*-(CH_2)_{n2}-N(R_1)S(O)_2-(CH_2)_{n3}-$, $*-(CH_2)_{n2}-S(O)_2N(R_1)-(CH_2)_{n3}-$, $*-(CH_2)_{n2}-N(R_1)-(CH_2)_{n3}-$, $*-(CH_2)_{n2}-N(R_1)C(O)-(CH_2)_{n3}-$, $*-(CH_2)_{n2}-C(O)N(R_1)-(CH_2)_{n3}-$, $*-(CH_2)_{n2}-N(R_1)C(O)N(R_1)-(CH_2)_{n3}-$, $*-(CH_2)_{n2}-(N(R_1)C(O)-(CH_2)_{n3})_{m1}-$, $*-(CH_2)_{n2}$-piperazinylene-$(CH_2)_{n3}-$, $*-(CH_2)_{n2}$-phenylene-$(CH_2)_{n3}-$, $*-(CH_2)_{n2}$-phenylene-$(CH_2)_{n3}-N(R_1)-(CH_2)_{n4}-$, $*-(CH_2)_{n2}$-furanylene-$(CH_2)_{n3}-$, $*-(CH_2)_{n2}$-furanylene-$(CH_2)_{n3}-N(R_1)-(CH_2)_{n4}-$, $*-(CH_2)_{n2}$-thiazolylene-$(CH_2)_{n3}-$, $*-(CH_2)_{n2}$-thiazolylene-$C(O)N(R_1)-(CH_2)_{n3}-$, or $*-(CH_2)_{n2}$-thiazolylene-$(CH_2)_{n3}-N(R_1)-(CH_2)_{n4}-$;
wherein a hydrogen of one or more $CH_2$ groups (e.g., 1-30, 1-20, 1-15, 1-10, 1-8, 1-7, 1-6, 1-5, 1-4, 1-3, 1-2, or 1 $CH_2$) of the linear or branched $C_{1-40}$ alkylene is optionally replaced with a substituent selected from the group consisting of:

halogen, cyano, $C_{1-3}$ alkyl, $C_{1-3}$ alkoxy, trifluoromethyl, $C_{3-10}$ aryl, 5- to 10-membered heteroaryl, $C_{3-12}$ cycloalkyl, 3- to 12-membered heterocyclyl, or any combination thereof;

* indicates the point of attachment to R;

$R_1$ groups each independently represent H or $C_{1-3}$ alkyl;

n2, n3, n4, n5, m1, m2, and m3 each independently represent an integer of 1, 2, 3, 4, 5, 6, 7, 8, 9, 10, 11, 12, 13, 14, or 15;

the piperazinylene is optionally substituted with a substituent selected from the group consisting of halogen, cyano, hydroxy, amino, mercapto, oxo, $C_{1-3}$ alkoxy, halogenated $C_{1-3}$ alkyl, methanesulfonyloxy, trifluoromethanesulfonyloxy, *p*-toluenesulfonyloxy, optionally deuterated $C_{1-6}$ alkyl, optionally deuterated $C_{3-6}$ cycloalkyl, or any combination thereof; and the phenylene, the furanylene, and the thiazolylene are each independently optionally substituted with a substituent selected from the group consisting of halogen, cyano, hydroxy, amino, mercapto, $C_{1-3}$ alkoxy, halogenated $C_{1-3}$ alkyl, methanesulfonyloxy, trifluoromethanesulfonyloxy, p-toluenesulfonyloxy, optionally deuterated $C_{1-6}$ alkyl, optionally deuterated $C_{3-6}$ cycloalkyl, or any combination thereof.

In some embodiments of the compounds of Formula (I) and Formula (I-4) of the present disclosure, L represents the following groups:

$-CH_2-$; $-(CH_2)_2-$; $-(CH_2)_3-$; $-(CH_2)_4-$; $-(CH_2)_5-$; $-(CH_2)_6-$; $-(CH_2)_7-$; $-(CH_2)_8-$; $-(CH_2)_9-$; $-(CH_2)_{10}-$; $-(CH_2)_{11}-$; $-(CH_2)_{12}-$; $-(CH_2)_{13}-$; $-(CH_2)_{14}-$; $-(CH_2)_{15}-$; $-(CH_2)_{16}-$; $-(CH_2)_{17}-$; $-(CH_2)_{18}-$; $-(CH_2)_{19}-$; or $-(CH_2)_{20}-$;

$*-CH_2CH_2O(CH_2)_2-$; $*-(CH_2CH_2O)_2-(CH_2)_2-$; $*-(CH_2CH_2O)_3-(CH_2)_2-$; $*-(CH_2CH_2O)_4-(CH_2)_2-$; $*-(CH_2CH_2O)_5-(CH_2)_2-$; $*-(CH_2CH_2O)_6-(CH_2)_2-$; $*-(CH_2CH_2O)_7-(CH_2)_2-$; $*-(CH_2CH_2O)_8-(CH_2)_2-$; $*-(CH_2CH_2O)_9(CH_2)_2-$; $*-(CH_2CH_2O)_{10}(CH_2)_2-$; $*-CH_2CH_2OCH_2-$; $*-(CH_2CH_2O)_2-CH_2-$; $*-(CH_2CH_2O)_3-CH_2-$; $*-(CH_2CH_2O)_4-CH_2-$; $*-(CH_2CH_2O)_5-CH_2-$; $*-(CH_2CH_2O)_6-CH_2-$; $*-(CH_2CH_2O)_7-CH_2-$; $*-(CH_2CH_2O)_8-CH_2-$; $*-(CH_2CH_2O)_9-CH_2-$; $*-(CH_2CH_2O)_{10}-CH_2-$; $*-CH_2CH_2O(CH_2)_3-$; $*-(CH_2CH_2O)_2-(CH_2)_3-$; $*-(CH_2CH_2O)_3-(CH_2)_3-$; $*-(CH_2CH_2O)_4-(CH_2)_3-$; $*-(CH_2CH_2O)_5-(CH_2)_3-$; $*-(CH_2CH_2O)_6-(CH_2)_3-$; $*-(CH_2CH_2O)_7-(CH_2)_3-$; $*-(CH_2CH_2O)_8-(CH_2)_3-$; $*-(CH_2CH_2O)_9(CH_2)_3-$; $*-(CH_2CH_2O)_{10}(CH_2)_3-$; $*-CH_2CH_2OCH_2CH_2CH_2OCH_2-$; $*-CH_2CH_2OCH_2CH_2CH_2O-(CH_2)_2-$; $*-CH_2CH_2OCH_2CH_2CH_2O-(CH_2)_3-$; $*-(CH_2CH_2O)_2(CH_2CH_2CH_2O)(CH_2)_3-$; $*-(CH_2CH_2O)_2(CH_2CH_2CH_2O)_2(CH_2)_3-$; $*-(CH_2)_1O(CH_2)_1-$; $*-(CH_2)_1O(CH_2)_2-$; $*-(CH_2)_2O(CH_2)_2-$; $*-(CH_2)_2O(CH_2)_1-$; $*-(CH_2)_2O(CH_2)_3-$; $*-(CH_2)_2O(CH_2)_4-$; $*-(CH_2)_2O(CH_2)_5-$; $*-(CH_2)_2O(CH_2)_6-$; $*-(CH_2)_3O(CH_2)_1-$; $*-(CH_2)_3O(CH_2)_2-$; $*-(CH_2)_3O(CH_2)_3-$; $*-(CH_2)_4O(CH_2)_1-$; $*-(CH_2)_4O(CH_2)_2-$; $*-(CH_2)_4O(CH_2)_3-$; $*-(CH_2)_5O(CH_2)_1-$; $*-(CH_2)_5O(CH_2)_2-$; $*-(CH_2)_5O(CH_2)_3-$; $*-(CH_2)_5O(CH_2)_4-$; or $*-(CH_2)_5O(CH_2)_5-$;

$*-(CH_2)_1-NH-(CH_2)_1-$; $*-(CH_2)_2-NH-(CH_2)_1-$; $*-(CH_2)_2-NH-(CH_2)_2-$; $*-(CH_2)_2-NH-(CH_2)_3-$; $*-(CH_2)_2-NH-(CH_2)_4-$; $*-(CH_2)_2-NH-(CH_2)_5-$; $*-(CH_2)_2-NH-(CH_2)_6-$; $*-(CH_2)_2-NH-(CH_2)_7-$; $*-(CH_2)_2-NH-(CH_2)_8-$; $*-(CH_2)_2-NH-(CH_2)_9-$; $-(CH_2)_2-NH-(CH_2)_{10}-$; $*-(CH_2)_2-NH-(CH_2)_{11}-$; $*-(CH_2)_2-NH-(CH_2)_{12}-$; $*-(CH_2)_3-NH-(CH_2)_1-$; $*-(CH_2)_3-NH-(CH_2)_2-$; $*-(CH_2)_3-NH-(CH_2)_3-$; $*-(CH_2)_4-NH-(CH_2)_1-$; $*-(CH_2)_4-NH-(CH_2)_2-$; $*-(CH_2)_5-NH-(CH_2)_3-$; $*-(CH_2)_5-NH-(CH_2)_1-$; $*-(CH_2)_5-NH-(CH_2)_2-$; $*-(CH_2)_8-NH-(CH_2)_2-$; $*-(CH_2)_5-NH-(CH_2)_3-$; $*-(CH_2)_5-NH-(CH_2)_4-$; $*-(CH_2)_5-NH-(CH_2)_5-$; $*-(CH_2)_1-N(CH_3)-(CH_2)_8-$; $*-(CH_2)_2-N(CH_3)-(CH_2)_1-$; $*-(CH_2)_3-N(CH_3)-(CH_2)_1-$; $*-(CH_2)_4-N(CH_3)-(CH_2)_1-$; $*-(CH_2)_5-N(CH_3)-(CH_2)_1-$; $*-(CH_2)_6-N(CH_3)-(CH_2)_1-$; $*-(CH_2)_2-N(CH_3)-(CH_2)_2-$; $*-(CH_2)_2-N(CH_3)-(CH_2)_3-$; $*-(CH_2)_2-N(CH_3)-(CH_2)_4-$; $*-(CH_2)_2-N(CH_3)-(CH_2)_5-$; $*-(CH_2)_2-N(CH_3)-(CH_2)_6-$; $*-(CH_2)_2-N(CH_3)-(CH_2)_7-$; $*-(CH_2)_2-N(CH_3)-(CH_2)_8-$; $*-(CH_2)_2-N(CH_3)-(CH_2)_9-$; $*-(CH_2)_2-N(CH_3)-(CH_2)_{10}-$; $*-(CH_2)_2-N(CH_3)-(CH_2)_{11}-$; $*-(CH_2)_2-N(CH_3)-(CH_2)_{12}-$; $*-(CH_2)_2-NHC(O)-CH_2-$; $*-(CH_2)_2-NHC(O)-(CH_2)_2-$; $*-(CH_2)_2-NHC(O)-(CH_2)_3-$; $*-(CH_2)_2-NHC(O)-(CH_2)_4-$; $*-(CH_2)_2-NHC(O)-(CH_2)_5-$; $*-(CH_2)_2-NHC(O)-(CH_2)_6-$; $*-(CH_2)_2-NHC(O)-(CH_2)_7-$; $*-(CH_2)_2-NHC(O)-(CH_2)_8-$; $*-(CH_2)_2-NHC(O)-(CH_2)_9-$; $*-(CH_2)_2-NHC(O)-(CH_2)_{10}-$; $*-(CH_2)_2-NHC(O)-(CH_2)_{11}-$; $*-(CH_2)_2-NHC(O)-(CH_2)_{12}-$; $*-(CH_2)_2-NHC(O)-(CH_2)_{13}-$; $*-(CH_2)_2-NHC(O)-(CH_2)_{14}-$; $*-(CH_2)_2-NHC(O)-(CH_2)_{15}-$; $*-(CH_2)_3-NHC(O)-CH_2-$; $*-(CH_2)_3-NHC(O)-(CH_2)_2-$; $*-(CH_2)_3-NHC(O)-(CH_2)_3-$; $*-(CH_2)_3-NHC(O)-(CH_2)_4-$; $*-(CH_2)_3-NHC(O)-(CH_2)_5-$; $*-(CH_2)_3-NHC(O)-(CH_2)_6-$; $*-(CH_2)_3-NHC(O)-(CH_2)_7-$; $*-(CH_2)_3-NHC(O)-(CH_2)_8-$; $*-(CH_2)_3-NHC(O)-(CH_2)_9-$; $*-(CH_2)_3-NHC(O)-(CH_2)_{10}-$; $*-(CH_2)_3-NHC(O)-(CH_2)_{11}-$; $*-(CH_2)_3-NHC(O)-(CH_2)_{12}-$; $*-(CH_2)_3-NHC(O)-(CH_2)_{13}-$; $*-(CH_2)_3-NHC(O)-(CH_2)_{14}-$; $*-(CH_2)_3-NHC(O)-(CH_2)_{15}-$; $*-(CH_2)_4NHC(O)(CH_2)_1-$; $*-(CH_2)_4NHC(O)(CH_2)_2-$; $*-(CH_2)_4NHC(O)(CH_2)_3-$; $*-(CH_2)_4NHC(O)(CH_2)_4-$; $*-(CH_2)_4NHC(O)(CH_2)_5-$; $*-(CH_2)_4NHC(O)(CH_2)_6-$; $*-(CH_2)_4NHC(O)(CH_2)_7-$; $*-(CH_2)_4NHC(O)(CH_2)_8-$; $*-(CH_2)_4NHC(O)(CH_2)_9-$; $*-(CH_2)_4NHC(O)(CH_2)_{10}-$; $*-(CH_2)_5NHC(O)(CH_2)_1-$; $*-(CH_2)_8NHC(O)(CH_2)_2-$; $*-(CH_2)_2-N(CH_3)C(O)-CH_2-$; $*-(CH_2)_2-N(CH_3)C(O)-(CH_2)_2-$; $*-(CH_2)_2-N(CH_3)C(O)-(CH_2)_3-$; $*-(CH_2)_2-N(CH_3)C(O)-(CH_2)_4-$; $*-(CH_2)_2-N(CH_3)C(O)-(CH_2)_5-$; $*-(CH_2)_2-N(CH_3)C(O)-(CH_2)_6-$; $*-(CH_2)_2-N(CH_3)C(O)-(CH_2)_7-$; $*-(CH_2)_2-N(CH_3)C(O)-(CH_2)_8-$; $*-(CH_2)_2-N(CH_3)C(O)-(CH_2)_9-$; $-(CH_2)_2-N(CH_3)C(O)-(CH_2)_{10}-$; $*-(CH_2)_2-N(CH_3)C(O)-(CH_2)_{11}-$; $*-(CH_2)_2-N(CH_3)C(O)-(CH_2)_{12}-$; $*-(CH_2)_2-N(CH_3)C(O)-(CH_2)_{13}-$; $*-(CH_2)_2-N(CH_3)C(O)-(CH_2)_{14}-$; $*-(CH_2)_2-N(CH_3)C(O)-(CH_2)_{15}-$; $*-(CH_2)_2-C(O)NH-CH_2-$; $*-(CH_2)_2-C(O)NH-(CH_2)_2-$; $*-(CH_2)_2-C(O)NH-(CH_2)_3-$; $*-(CH_2)_2-C(O)NH-(CH_2)_4-$; $*-(CH_2)_2-C(O)NH-(CH_2)_5-$; $*-(CH_2)_2-C(O)NH-(CH_2)_6-$; $*-(CH_2)_2-C(O)NH-(CH_2)_7-$; $*-(CH_2)_2-C(O)NH-(CH_2)_8-$; $*-(CH_2)_2-C(O)NH-(CH_2)_9-$; $*-(CH_2)_2-C(O)NH-(CH_2)_{10}-$; $*-(CH_2)_2-C(O)NH-(CH_2)_{11}-$; $*-(CH_2)_2-C(O)NH-(CH_2)_{12}-$;

*-$(CH_2)_2$-C(O)NH-$(CH_2)_{13}$-; *-$(CH_2)_2$-C(O)NH-$(CH_2)_{14}$-; *-$(CH_2)_2$-C(O)NH-$(CH_2)_{15}$-; *-$(CH_2)_3$-C(O)NH-$CH_2$-; *-$(CH_2)_3$-C(O)NH-$(CH_2)_2$-; *-$(CH_2)_3$-C(O)NH-$(CH_2)_3$-; *-$(CH_2)_3$-C(O)NH-$(CH_2)_4$-; *-$(CH_2)_3$-C(O)NH-$(CH_2)_5$-; *-$(CH_2)_3$-C(O)NH-$(CH_2)_6$-; *-$(CH_2)_3$-C(O)NH-$(CH_2)_7$-; *-$(CH_2)_3$-C(O)NH-$(CH_2)_8$-; *-$(CH_2)_3$-C(O)NH-$(CH_2)_9$-; *-$(CH_2)_3$-C(O)NH-$(CH_2)_{10}$-; *-$(CH_2)_3$-C(O)NH-$(CH_2)_{11}$-; *-$(CH_2)_3$-C(O)NH-$(CH_2)_{12}$-; *-$(CH_2)_3$-C(O)NH-$(CH_2)_{13}$-; *-$(CH_2)_3$-C(O)NH-$(CH_2)_{14}$-; *-$(CH_2)_3$-C(O)NH-$(CH_2)_{15}$-; *-$(CH_2)_4$C(O)NH$(CH_2)_1$-; *-$(CH_2)_4$C(O)NH$(CH_2)_2$-; *-$(CH_2)_4$C(O)NH$(CH_2)_3$-; *-$(CH_2)_4$C(O)NH$(CH_2)_4$-; *-$(CH_2)_4$C(O)NH$(CH_2)_5$-; *-$(CH_2)_4$C(O)NH$(CH_2)_6$-; *-$(CH_2)_4$C(O)NH$(CH_2)_7$-; *-$(CH_2)_4$C(O)NH$(CH_2)_8$-; *-$(CH_2)_4$C(O)NH$(CH_2)_9$-; *-$(CH_2)_4$C(O)NH$(CH_2)_{10}$-; *-$(CH_2)_2$-C(O)N$(CH_3)$-$CH_2$-; *-$(CH_2)_2$-C(O)N$(CH_3)$-$(CH_2)_2$-; *-$(CH_2)_2$-C(O)N$(CH_3)$-$(CH_2)_3$-; *-$(CH_2)_2$-C(O) N$(CH_3)$-$(CH_2)_4$-; *-$(CH_2)_2$-C(O)N$(CH_3)$-$(CH_2)_5$-; *-$(CH_2)_2$-C(O)N$(CH_3)$-$(CH_2)_6$-; *-$(CH_2)_2$-C(O)N$(CH_3)$-$(CH_2)_7$-; *-$(CH_2)_2$-C(O)N$(CH_3)$-$(CH_2)_8$-; *-$(CH_2)_2$-C(O)N$(CH_3)$-$(CH_2)_9$-; *-$(CH_2)_2$-C(O)N$(CH_3)$-$(CH_2)_{10}$-; *-$(CH_2)_2$-C(O) N$(CH_3)$-$(CH_2)_{11}$-; *-$(CH_2)_2$-C(O)N$(CH_3)$-$(CH_2)_{12}$-; *-$(CH_2)_2$-C(O)N$(CH_3)$-$(CH_2)_{13}$-; *-$(CH_2)_2$-C(O)N$(CH_3)$-$(CH_2)_{14}$-; *-$(CH_2)_2$-C(O)N$(CH_3)$-$(CH_2)_{15}$-; *-$(CH_2)_2$-NHC(O)NH-$(CH_2)_4$-; *-$(CH_2)_4$-NHC(O)NH-$(CH_2)_2$-; *-$CH_2$-NHC(O) NH-$(CH_2)_2$-; *-$(CH_2)_2$-NHC(O)NH-$CH_2$-; *-$(CH_2)_2$-NHC(O)NH-$(CH_2)_2$-; *-$(CH_2)_2$-NHC(O)NH-$(CH_2)_3$-; *-$(CH_2)_3$-NHC(O)NH-$(CH_2)_2$-; *-$CH_2$-piperazinylene-$CH_2$-; *-$(CH_2)_2$-piperazinylene-$(CH_2)_2$-; *-$(CH_2)_2$-piperazinylene-$(CH_2)_3$-; *-$(CH_2)_2$-piperazinylene-$(CH_2)_4$-; *-$(CH_2)_2$-piperazinylene-$(CH_2)_5$-; *-$(CH_2)_3$-piperazinylene-$CH_2$-; *-$(CH_2)_3$-piperazinylene-$(CH_2)_2$-; *-$(CH_2)_3$-piperazinylene-$(CH_2)_3$-; *-$(CH_2)_4$-piperazinylene-$CH_2$-; -$(CH_2)_4$-piperazinylene-$(CH_2)_2$-; *-$(CH_2)_4$-piperazinylene-$(CH_2)_3$-; *-$(CH_2)_8$-piperazinylene-$CH_2$-; -$(CH_2)_8$-piperazinylene-$(CH_2)_2$-; *-$(CH_2)_8$-piperazinylene-$(CH_2)_3$-; *-$(CH_2)_8$-piperazinylene-$(CH_2)_4$-; *-$(CH_2)_8$-piperazinylene-$(CH_2)_5$-; *-$(CH_2)_8$-piperazinylene-$(CH_2)_6$-; *-$(CH_2)_8$-piperazinylene-$(CH_2)_7$-; *-$(CH_2)_8$-piperazinylene-$(CH_2)_8$-; *-$CH_2$-piperazinylene-$(CH_2)_8$-; *-$(CH_2)_2$-piperazinylene-$(CH_2)_8$-; *-$(CH_2)_3$-piperazinylene-$(CH_2)_8$-; *-$(CH_2)_4$-piperazinylene-$(CH_2)_8$-; *-$(CH_2)_5$-piperazinylene-$(CH_2)_8$-; *-$(CH_2)_6$-piperazinylene-$(CH_2)_8$-; *-$(CH_2)_7$-piperazinylene-$(CH_2)_8$-; *-$CH_2$-phenylene-$CH_2$-; *-$(CH_2)_2$-phenylene-$(CH_2)_2$-; *-$(CH_2)_2$-phenylene-$(CH_2)_3$-; *-$(CH_2)_2$-phenylene-$(CH_2)_4$-; *-$(CH_2)_2$-phenylene-$(CH_2)_5$-; *-$(CH_2)_3$-phenylene-$CH_2$-; *-$(CH_2)_3$-phenylene-$(CH_2)_2$-; *-$(CH_2)_3$-phenylene-$(CH_2)_3$-; *-$(CH_2)_4$-phenylene-$CH_2$-; *-$(CH_2)_4$-phenylene-$(CH_2)_2$-; *-$(CH_2)_4$-phenylene-$(CH_2)_3$-; *-$(CH_2)_5$-phenylene-$(CH_2)_3$-; *-$(CH_2)_6$-phenylene-$(CH_2)_3$-; *-$(CH_2)_7$-phenylene-$(CH_2)_3$-; *-$(CH_2)_8$-phenylene-$CH_2$-; *-$(CH_2)_8$-phenylene-$(CH_2)_2$-; *-$(CH_2)_8$-phenylene-$(CH_2)_3$-; *-$(CH_2)_8$-phenylene-$(CH_2)_4$-; *-$(CH_2)_8$-phenylene-$(CH_2)_5$-; *-$(CH_2)_8$-phenylene-$(CH_2)_6$-; *-$(CH_2)_8$-phenylene-$(CH_2)_7$-; *-$(CH_2)_8$-phenylene-$(CH_2)_8$-; *-$CH_2$-phenylene-$(CH_2)_8$-; *-$(CH_2)_2$-phenylene-$(CH_2)_8$-; *-$(CH_2)_3$-phenylene-$(CH_2)_8$-; *-$(CH_2)_4$-phenylene-$(CH_2)_8$-; *-$(CH_2)_5$-phenylene-$(CH_2)_8$-; *-$(CH_2)_6$-phenylene-$(CH_2)_8$-; *-$(CH_2)_7$-phenylene-$(CH_2)_8$-; *-$(CH_2)_1S(CH_2)_1$-; *-$(CH_2)_2S(CH_2)_2$-; *-$(CH_2)_2S(CH_2)_1$-; *-$(CH_2)_1S(CH_2)_2$-; *-$(CH_2)_1S(CH_2)_3$-; *-$(CH_2)_1S(CH_2)_4$-; *-$(CH_2)_2S(CH_2)_3$-; *-$(CH_2)_2S(CH_2)_4$-; *-$(CH_2)_2S(CH_2)_5$-; *-$(CH_2)_3S(CH_2)_1$-; *-$(CH_2)_3S(CH_2)_2$-; *-$(CH_2)_3S(CH_2)_3$-; *-$(CH_2)_4S(CH_2)_1$-; -$(CH_2)_4S(CH_2)_2$-; *-$(CH_2)_4S(CH_2)_3$-; *-$(CH_2)_5S(CH_2)_1$-; *-$(CH_2)_5S(CH_2)_2$-; *-$(CH_2)_5S(CH_2)_3$-; -$(CH_2)_6S(CH_2)_1$-; *-$(CH_2)_6S(CH_2)_2$-; *-$(CH_2)_6S(CH_2)_3$-; *-$(CH_2)_7S(CH_2)_1$-; *-$(CH_2)_7S(CH_2)_2$-; *-$(CH_2)_7S(CH_2)_3$-; *-$(CH_2)_8S(CH_2)_1$-; *-$(CH_2)_8S(CH_2)_2$-; *-$(CH_2)_8S(CH_2)_3$-; *-$(CH_2)_9S(CH_2)_1$-; *-$(CH_2)_9S(CH_2)_2$-; *-$(CH_2)_9S(CH_2)_3$-; *-$(CH_2)_1$S(O) $(CH_2)_1$-; *-$(CH_2)_2$S(O)$(CH_2)_2$-; *-$(CH_2)_2$S(O)$(CH_2)_1$-; *-$(CH_2)_1$S(O)$(CH_2)_2$-; *-$(CH_2)_1$S(O)$(CH_2)_3$-; *-$(CH_2)_1$S(O)$(CH_2)_4$-; *-$(CH_2)_2$S(O)$(CH_2)_3$-; *-$(CH_2)_2$S(O)$(CH_2)_4$-; *-$(CH_2)_2$S(O)$(CH_2)_5$-; *-$(CH_2)_3$S(O)$(CH_2)_1$-; *-$(CH_2)_3$S(O)$(CH_2)_2$-; *-$(CH_2)_3$S(O)$(CH_2)_3$-; *-$(CH_2)_4$S(O)$(CH_2)_1$-; *-$(CH_2)_4$S(O)$(CH_2)_2$-; * -$(CH_2)_4$S(O)$(CH_2)_3$-; *-$(CH_2)_4$S(O)$(CH_2)_1$-; *-$(CH_2)_5$S(O)$(CH_2)_2$-; *-$(CH_2)_5$S(O)$(CH_2)_3$-; *-$(CH_2)_6$S(O)$(CH_2)_1$-; *-$(CH_2)_6$S(O)$(CH_2)_2$-; *-$(CH_2)_6$S(O)$(CH_2)_3$-; *-$(CH_2)_7$S(O)$(CH_2)_1$-; *-$(CH_2)_7$S(O)$(CH_2)_2$-; *-$(CH_2)_7$S(O)$(CH_2)_3$-; *-$(CH_2)_8$S(O)$(CH_2)_1$-; *-$(CH_2)_8$S(O)$(CH_2)_2$-; *-$(CH_2)_8$S(O)$(CH_2)_3$-; *-$(CH_2)_9$S(O)$(CH_2)_1$-; *-$(CH_2)_9$S(O)$(CH_2)_2$-; -$(CH_2)_9$S(O)$(CH_2)_3$-; -$(CH_2)_1$S$(O)_2(CH_2)_1$-; *-$(CH_2)_2$S$(O)_2(CH_2)_2$-; *-$(CH_2)_2$S$(O)_2(CH_2)_1$-; *-$(CH_2)_1$S$(O)_2(CH_2)_2$-; *-$(CH_2)_1$S$(O)_2(CH_2)_3$-; *-$(CH_2)_1$S$(O)_2(CH_2)_4$-; *-$(CH_2)_2$S$(O)_2(CH_2)_3$-; -$(CH_2)_2$S$(O)_2(CH_2)_4$-; *-$(CH_2)_2$S$(O)_2(CH_2)_5$-; *-$(CH_2)_3$S$(O)_2(CH_2)_1$-; *-$(CH_2)_3$S$(O)_2(CH_2)_2$-; *-$(CH_2)_3$S$(O)_2(CH_2)_3$-; *-$(CH_2)_4$S$(O)_2(CH_2)_1$-; *-$(CH_2)_4$S$(O)_2(CH_2)_2$-; *-$(CH_2)_4$S$(O)_2(CH_2)_3$-; *-$(CH_2)_5$S$(O)_2(CH_2)_1$-; *-$(CH_2)_5$S$(O)_2(CH_2)_2$-; *-$(CH_2)_5$S(O)$(CH_2)_3$-; *-$(CH_2)_6$S$(O)_2(CH_2)_1$-; *-$(CH_2)_6$S$(O)_2(CH_2)_2$-; *-$(CH_2)_6$S$(O)_2(CH_2)_3$-; *-$(CH_2)_7$S$(O)_2(CH_2)_1$-; *-$(CH_2)_7$S$(O)_2(CH_2)_2$-; *-$(CH_2)_7$S$(O)_2(CH_2)_3$-; *-$(CH_2)_8$S$(O)_2(CH_2)_1$-; *-$(CH_2)_8$S$(O)_2(CH_2)_2$-; *-$(CH_2)_8$S$(O)_2(CH_2)_3$-; *-$(CH_2)_9$S$(O)_2(CH_2)_1$-; *-$(CH_2)_9$S$(O)_2(CH_2)_2$-; or *-$(CH_2)_9$S$(O)_2(CH_2)_3$-;

*-$(CH_2)_6$-NH-$(CH_2)_1$-; *-$(CH_2)_6$-NH-$(CH_2)_3$-; *-$(CH_2)_6$-NH-$(CH_2)_4$-; *-$(CH_2)_6$-NH-$(CH_2)_5$-; *-$(CH_2)_7$-NH-$(CH_2)_1$-; *-$(CH_2)_7$-NH-$(CH_2)_2$-; *-$(CH_2)_7$-NH-$(CH_2)_3$-; *-$(CH_2)_7$-NH-$(CH_2)_4$-; *-$(CH_2)_6$-NH-CH$(CH_3)$-; *-$(CH_2)_5$-NH-CH$(CH_3)$-; *-$(CH_2)_4$-NH-CH$(CH_3)$-; *-$(CH_2)_3$-NH-CH$(CH_3)$-; *-$(CH_2)_2$-NH-CH$(CH_3)$-; *-$(CH_2)_7$-NH-CH$(CH_3)$-; *-$(CH_2)_8$-NH-CH$(CH_3)$-; *-$(CH_2)_7$-NH-CH$(CF_3)$-; *-$(CH_2)_6$-NH-CH$(CF_3)$-; *-$(CH_2)_5$-NH-CH$(CF_3)$-; *-$(CH_2)_4$-NH-CH$(CF_3)$-; *-$(CH_2)_3$-NH-CH$(CF_3)$-; *-$(CH_2)_2$-NH-CH$(CF_3)$-; *-$(CH_2)_8$-NH-CH$(CF_3)$-; *-$CH_2$-C(O)NH-$(CH_2)_4$-; *-$CH_2$-C(O) NH-$(CH_2)_2$-; *-$CH_2$-C(O)NH-$(CH_2)_3$-; *-$CH_2$-C(O)NH-$(CH_2)_5$-; *-$(CH_2)_2$-phenylene-$(CH_2)_1$-; *-$(CH_2)_1$-phenylene-$(CH_2)_2$-; *-$(CH_2)_1$-phenylene-$(CH_2)_3$-; *-$(CH_2)_3$-phenylene-$(CH_2)_1$-; *-$(CH_2)_4$-phenylene-$(CH_2)_1$-; *-$(CH_2)_1$-phenylene-$(CH_2)_4$-; *-$(CH_2)_2$-phenylene-$(CH_2)_2$-; *-$CH_2$-phenylene-$CH_2$-NH-CH$(CH_3)$-; *-$CH_2$-phenylene-$(CH_2)_2$-NH-CH$(CH_3)$-; *-$CH_2$-phenylene-$CH_2$-NH-$CH_2$-; *-$CH_2$-phenylene-$(CH_2)_2$-NH-$CH_2$-; *-$(CH_2)_1$-C(O)NH-$(CH_2)_4$-; *-$(CH_2)_1$-furanylene-$(CH_2)_1$-; *-$(CH_2)_1$-furanylene-$(CH_2)_2$-; *-$(CH_2)_1$-furanylene-$(CH_2)_3$-; *-$(CH_2)_2$-furanylene-$(CH_2)_1$-; *-$(CH_2)_2$-furanylene-$(CH_2)_2$-; *-$(CH_2)_3$-furanylene-$(CH_2)_1$-; *-$(CH_2)_3$-furanylene-$(CH_2)_2$-; *-$(CH_2)_1$-thiazolylene-$(CH_2)_1$-; *-$(CH_2)_1$-thiazolylene-$(CH_2)_2$-; *-$(CH_2)_1$-thiazolylene-$(CH_2)_3$-; *-$(CH_2)_2$-thiazolylene-$(CH_2)_1$-;

*-$(CH_2)_2$-thiazolylene-$(CH_2)_2$-; *-$(CH_2)_3$-thiazolylene-$(CH_2)_1$-; *-$(CH_2)_3$-thiazolylene-$(CH_2)_2$-; or *-$CH_2$-thiazolylene-$CH_2$-NH-$CH_2$-;

wherein a hydrogen of one or more $CH_2$ (e.g., 1-30, 1-20, 1-15, 1-10, 1-8, 1-7, 1-6, 1-5, 1-4, 1-3, 1-2, or 1 $CH_2$) of the groups is optionally replaced with a substituent selected from the group consisting of: halogen, cyano, hydroxy, amino, mercapto, $C_{1-3}$alkyl, $C_{1-3}$ alkoxy, trifluoromethyl, $C_{3-10}$ aryl, 5- to 10-membered heteroaryl, $C_{3-12}$cycloalkyl, 3- to 12-membered heterocyclyl, or any combination thereof;

the piperazinylene is optionally substituted with a substituent selected from the group consisting of halogen, cyano, hydroxy, amino, mercapto, oxo, $C_{1-3}$ alkoxy, halogenated $C_{1-3}$alkyl, methanesulfonyloxy, trifluoromethanesulfonyloxy, p-toluenesulfonyloxy, optionally deuterated $C_{1-6}$ alkyl, optionally deuterated $C_{3-6}$ cycloalkyl, or any combination thereof,

the phenylene, the furanylene, and the thiazolylene are each independently optionally substituted with a substituent selected from the group consisting of halogen, cyano, hydroxy, amino, mercapto, $C_{1-3}$ alkoxy, halogenated $C_{1-3}$alkyl, methanesulfonyloxy, trifluoromethanesulfonyloxy, p-toluenesulfonyloxy, optionally deuterated $C_{1-6}$ alkyl, optionally deuterated $C_{3-6}$ cycloalkyl, or any combination thereof; and

* indicates the point of attachment to R.

In some embodiments of the compounds of Formula (I) and Formula (I-4) of the present disclosure, L represents the following optionally substituted groups:

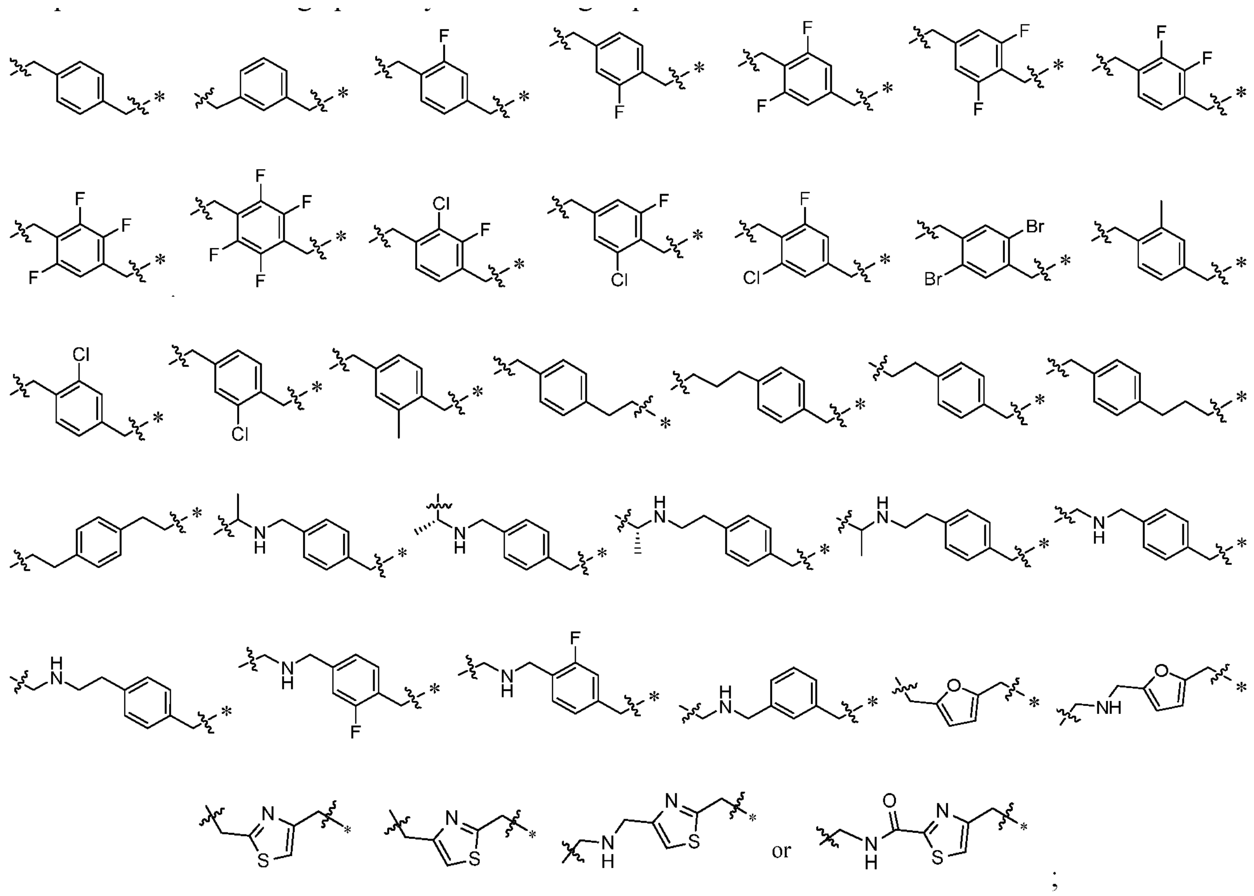

or

;

wherein * indicates the point of attachment to R.

In some embodiments of the compounds of Formula (I) and Formula (I-4) of the present disclosure, $X_1$ represents linear or branched $C_{1-10}$ alkyl (e.g., $C_{1-10}$ alkyl, $C_{1-6}$ alkyl, $C_{1-3}$ alkyl, fluorinated $C_{1-10}$ alkyl, fluorinated $C_{1-6}$ alkyl, or fluorinated $C_{1-3}$ alkyl, such as $F_3C$-, $FCH_2$-, $F_2CH$-, $CF_3CF_2$-, $CF_3CHF$-, $CHF_2CF_2$-, $CHF_2CHF$-, or $CF_3CH_2$-) optionally substituted with one or more (e.g., 1-15, 1-10, 1-9, 1-8, 1-7, 1-6, 1-5, 1-4, 1-3, or 1-2, or 1) fluorine.

In embodiments of the present disclosure, the number of substituents is not limited in principle, or is automatically limited by the size of the building units.

In some embodiments of the compounds of Formula (I) and Formula (I-4) of the present disclosure, $X_1$ represents $C_3$-$C_{12}$ cycloalkyl, which is optionally substituted with one or more (e.g., 1-10, 1-8, 1-7, 1-6, 1-5, 1-4, 1-3, 1-2, or 1)

substituents selected from the group consisting of halogen, cyano, amino, hydroxy, mercapto, oxo, $C_{1-3}$ alkoxy, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more (e.g., 1-6, 1-5, 1-4, 1-3, 1-2, or 1) substituents selected from the group consisting of halogen, cyano, amino, hydroxy, mercapto, oxo, $C_{1-6}$ alkyl, halogenated $C_{1-3}$ alkyl, or any combination thereof; and the $C_5$-$C_{20}$ aryl is optionally substituted with one or more (e.g., 1-6, 1-5, 1-4, 1-3, 1-2, or 1) substituents selected from the group consisting of halogen, cyano, amino, hydroxy, mercapto, $C_{1-6}$alkyl, halogenated $C_{1-3}$ alkyl, or any combination thereof; and the 5- or 6-membered heteroaryl is optionally substituted with one or more (e.g., 1-6, 1-5, 1-4, 1-3, 1-2, or 1) substituents selected from the group consisting of halogen, cyano, amino, hydroxy, mercapto, oxo, $C_{1-6}$ alkyl, halogenated $C_{1-3}$ alkyl, or any combination thereof.

In some embodiments of the compounds of Formula (I) and Formula (I-4) of the present disclosure, $X_1$ represents cyclopropyl, cyclobutyl, 3,3-difluorocyclobutyl, cyclopentyl, cyclopentenyl, cyclohexyl, cyclohexenyl, cycloheptyl, cyclooctyl, decalinyl, octahydropentalenyl, octahydro-1H-indenyl, 2,3-dihydro-1H-indenyl, 4-cyano-2,3-dihydro-1H-indenyl, spiro[3.3]heptanyl, spiro[2.5]octyl, spiro[3.5]nonyl, spiro[4.4]nonyl, spiro[4.5]decyl, spiro[5.5]undecyl, *p*-menthanyl, *m*-menthanyl, quinuclidinyl, adamantanyl, noradamantanyl, bornyl, 3,5-dimethyladamantan-1-yl, 1,7,7-trimethylbicyclo[2.2.1]heptan-2-yl, 7,7-dimethylbicyclo[2.2.1]heptan-1-yl, 7,7-dimethyl-2-oxobicyclo[2.2.1]heptan-1-yl, bicyclo[2.2.1]heptanyl, 2-oxobicyclo[2.2.1]heptyl, or bicyclo[2.2.1]heptenyl, with each group being optionally substituted with one or more substituents selected from the group consisting of halogen, cyano, amino, hydroxy, mercapto, oxo, $C_{1-3}$ alkoxy, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more (e.g., 1-6, 1-5, 1-4, 1-3, 1-2, or 1) substituents selected from the group consisting of: halogen, cyano, amino, hydroxy, mercapto, oxo, $C_{1-6}$alkyl, halogenated $C_{1-3}$alkyl, or any combination thereof; and the $C_5$-$C_{20}$ aryl is optionally substituted with one or more (e.g., 1-6, 1-5, 1-4, 1-3, 1-2, or 1) substituents selected from the group consisting of: halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-6, 1-5, 1-4, 1-3, 1-2, or 1) substituents selected from the group consisting of: halogen, cyano, amino, hydroxy, mercapto, oxo, $C_{1-6}$ alkyl, halogenated $C_{1-3}$ alkyl, or any combination thereof.

In some embodiments of the compounds of Formula (I) and Formula (I-4) of the present disclosure, $X_1$ represents adamantanyl, noradamantanyl, bornyl, or norbornyl, with each group being optionally substituted with one or more (e.g., 1-6, 1-5, 1-4, 1-3, 1-2, or 1) substituents selected from the group consisting of halogen, cyano, amino, hydroxy, mercapto, oxo, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more substituents selected from the group consisting of: halogen, cyano, amino, hydroxy, mercapto, oxo, $C_{1-6}$ alkyl, halogenated $C_{1-3}$ alkyl, or any combination thereof; and the $C_5$-$C_{20}$ aryl is optionally substituted with one or more (e.g., 1-6, 1-5, 1-4, 1-3, 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-6, 1-5, 1-4, 1-3, 1-2, or 1) substituents selected from the group consisting of halogen, cyano, amino, hydroxy, mercapto, oxo, $C_{1-6}$ alkyl, halogenated $C_{1-3}$ alkyl, or any combination thereof.

In some embodiments of the compounds of Formula (I) and Formula (I-4) of the present disclosure, $X_1$ represents $C_5$-$C_{14}$ aryl, which is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-3}$ alkoxy, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$alkyl, halogenated $C_{1-3}$ alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$ alkyl, halogenated $C_{1-3}$ alkyl, or any combination thereof.

In some embodiments of the compounds of Formula (I) and Formula (I-4) of the present disclosure, $X_1$ represents phenyl or naphthyl, with each group being optionally substituted with one or more (e.g., 1-5, 1-4, 1-3, or 1-2, or 1) substituents selected from the group consisting of halogen, cyano, amino, hydroxy, mercapto, $C_{1-3}$ alkoxy, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more (e.g., 1-10, 1-9, 1-8, 1-7, 1-6, 1-5, 1-4, 1-3 or 1-2, or 1) substituents selected from the group consisting of halogen, cyano, amino, hydroxy, mercapto, oxo, $C_{1-6}$alkyl, halogenated $C_{1-3}$alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$ alkyl, halogenated $C_{1-3}$ alkyl, or any combination thereof.

In some embodiments of the compounds of Formula (I) and Formula (I-4) of the present disclosure, $X_1$ represents phenyl, 4-fluorophenyl, 4-chlorophenyl, 3,5-difluorophenyl, 4-(bromomethyl)-2-fluorophenyl, 3,4,5-trifluorophenyl, or (2-bromovinyl)phenyl.

In some embodiments of the compounds of Formula (I) and Formula (I-4) of the present disclosure, $X_1$ represents 3- to 12-membered heterocyclyl, which is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$ alkyl, halogenated $C_{1-3}$ alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$alkyl, halogenated $C_{1-3}$ alkyl, or any combination thereof.

In some embodiments of the compounds of Formula (I) and Formula (I-4) of the present disclosure, $X_1$ represents azetidinyl, oxetanyl, pyrrolidinyl, imidazolidinyl, pyrazolidinyl, oxazolidinyl, thiazolidinyl, piperidinyl, piperazinyl, morpholinyl, thiomorpholinyl, azacycloheptyl, azacyclooctyl, diazacycloheptanyl, diazacyclooctyl, 3-azabicyclo[3.1.0]hexyl, 3-azabicyclo[3.1.1]heptanyl, 3-azabicyclo[4.1.0]heptanyl, 2-azabicyclo[2.2.1]heptanyl, 6-azabicyclo[3.1.1]heptanyl, 2-azabicyclo[2.2.2]octyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, 2,6-diazaspiro[3.3]heptanyl, 2,7-diazaspiro[3.5]nonyl, 3-azaspiro[5.5]undecyl, 5-azaspiro[2.4]heptanyl, 6-azaspiro[2.5]octyl, 2-oxa-7-azaspiro[3.5]nonyl, 7-azaspiro[3.5]nonyl, hexahydro-1H-isoindol-2(3H)-yl, (3aR,7aS)-octahydro-2H-isoindol-2-yl, octahydropyrrolo[3,4-c]pyrrolyl, or 5,7-dihydro-6H-pyrrolo[3,4-b]pyridyl, with each group being optionally substituted with one or more (e.g., 1-10, 1-9, 1-8, 1-7, 1-6, 1-5, 1-4, 1-3 or 1-2, or 1) substituents selected from the group consisting of halogen, cyano, amino, hydroxy, mercapto, oxo, $C_{1-3}$ alkoxy, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more (e.g., 1-10, 1-9, 1-8, 1-7, 1-6, 1-5, 1-4, 1-3 or 1-2, or 1) substituents selected from the group consisting of halogen, cyano, amino, hydroxy, mercapto, oxo, $C_{1-6}$ alkyl, halogenated $C_{1-3}$ alkyl, or any combination thereof; and the $C_5$-$C_{20}$ aryl is optionally substituted with one or more (e.g., 1-10, 1-9, 1-8, 1-7, 1-6, 1-5, 1-4, 1-3 or 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3 or 1-2, or 1) substituents selected from the group consisting of halogen, cyano, amino, hydroxy, mercapto, oxo, $C_{1-6}$ alkyl, halogenated $C_{1-3}$ alkyl, or any combination thereof.

In some embodiments of the compounds of Formula (I) and Formula (I-4) of the present disclosure, $X_1$ represents morpholinyl, piperidinyl, difluoropiperidinyl, 4,4-dimethylpiperidin-1-yl, 3,5-dimethylpiperidin-1-yl, piperazinyl, 3-hydroxy-2-methylazetidin-1-yl, trifluoromethoxyazetidinyl, azetidinyl, cyanoazetidinyl, hydroxyazetidinyl, 3-hydroxy-3-methylazetidin-1-yl, 3-fluoro-4-hydroxypyrrolidin-1-yl, 3-methyl-4-(trifluoromethyl)pyrrolidin-1-yl, 7-fluoro-5-azaspiro[2.4]heptan-5-yl, 3-azabicyclo[3.1.0]hexan-3-yl, 6-(difluoromethyl)-3-azabicyclo[4.1.0]heptan-3-yl, 7-(3-fluoro-5,7-dihydro-6H-pyrrolo[3,4-b]pyridin-6-yl, 1,1-difluoro-5-azaspiro[2.4]heptan-5-yl, 1,1-difluoro-6-azaspiro[2.5]octan-6-yl, 2-oxa-7-azaspiro[3.5]nonan-7-yl, 4-(methylsulfonyl)piperazin-1-yl, 4-(ethylsulfonyl)piperazin-1-yl, 1,4-diazepan-1-yl, 3,8-diazabicyclo[3.2.1]octan-3-yl, 2-azabicyclo[3.2.1]octanyl, 1,4-diazabicyclo[3.2.1]octanyl, 2,5-diazabicyclo[2.2.2]octan-2-yl, or pyrrolidinyl.

In some embodiments of the compounds of Formula (I) and Formula (I-4) of the present disclosure, $X_1$ represents: morpholinyl, piperidinyl, 2-oxopiperidin-1-yl, piperazinyl, N-methylpiperazinyl, 4-(methylsulfonyl)piperazin-1-yl, 4-(ethylsulfonyl)piperazin-1-yl, (N-methylpiperazinyl)piperidinyl, 3,5-dimethylpiperazinyl, 1,4-diazepan-1-yl, 4-methyl-1,4-diazepan-1-yl, 1-methyl-1,4-diazepan-1-yl, 8-methyl-3,8-diazabicyclo[3.2.1]octan-3-yl, 1,4-diazabicyclo[3.2.1]octan-4-yl, 5-methyl-2,5-diazabicyclo[2.2.2]octan-2-yl, 8,8-difluoro-2-azabicyclo[3.2.1]octan-2-yl, 4-(4-fluorophenyl)piperazin-1-yl, 4-(3,4-difluorophenyl)piperazin-1-yl, 2-oxopiperidin-1-yl, 2-oxopyrrolidin-1-yl, 3-fluoro-4-hydroxypyrrolidin-1-yl, 3-methyl-4-(trifluoromethyl)pyrrolidin-1-yl, 2,6-dioxopiperidin-3-yl, 4,4-dimethylpiperidin-1-yl, 3,5-dimethylpiperidin-1-yl, 4,4-difluoropiperidin-1-yl, 4-methylpiperazin-1-yl, tetrahydro-2H-pyran-2-yl, pyrrolidinyl, pyrrolidin-1-yl, 3-cyanoazetidinyl, 3-hydroxyazetidinyl, 3-hydroxy-3-methylazetidinyl, 3-hydroxy-2-methylazetidin-1-yl, 3-(trifluoromethoxy)azetidin-1-yl, azepan-1-yl, azacyclooctan-1-yl, 7-fluoro-5-azaspiro[2.4]heptan-5-yl, 1,1-difluoro-5-azaspiro[2.4]heptan-5-yl, 1,1-difluoro-6-azaspiro[2.5]octan-6-yl, 6-azaspiro[2.5]octan-6-yl, 2-oxa-7-azaspiro[3.5]nonan-7-yl, 3-azabicyclo[3.1.0]hexan-3-yl, 6-(difluoromethyl)-3-azabicyclo[4.1.0]heptan-3-yl, thiomorpholino, [1,4'-dipiperidin]-1'-yl,

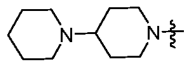,

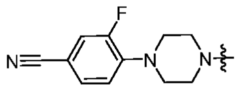, 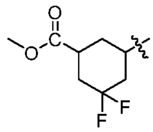,

or 4,4-difluoropiperidinyl.

In some embodiments of the compounds of Formula (I) and Formula (I-4) of the present disclosure, $X_1$ represents 5- to 10-membered heteroaryl, which is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-3}$ alkoxy, halogenated $C_{1-3}$ alkoxy, halogenated $C_{1-3}$ alkyl, bis($C_{1-6}$ alkyl)phosphono, $C_{1-6}$ alkylsulfonyl, methanesulfonyloxy, trifluoromethanesulfonyloxy, *p*-toluenesulfonyloxy, $C_{1-6}$ alkyl, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of $C_{1-6}$ alkyl, cyano, halogenated $C_{1-3}$ alkyl, amino, hydroxy, mercapto, halogen, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of $C_{1-6}$ alkyl, cyano, halogenated $C_{1-3}$ alkyl, amino, hydroxy, mercapto, halogen, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of $C_{1-6}$ alkyl, halogenated $C_{1-3}$ alkyl, amino, hydroxy, mercapto, halogen, or any combination thereof.

In some embodiments of the compounds of Formula (I) and Formula (I-4) of the present disclosure, $X_1$ represents furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, tetrahydro-2H-pyran-2-yl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl, or imidazo[2,1-b]thiazolyl, with each group being optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-3}$ alkoxy, halogenated $C_{1-3}$ alkoxy, halogenated $C_{1-3}$ alkyl, bis($C_{1-6}$ alkyl)phosphono, $C_{1-6}$ alkylsulfonyl, methanesulfonyloxy, trifluoromethanesulfonyloxy, *p*-toluenesulfonyloxy, $C_{1-6}$ alkyl, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more (e.g., 1-10, 1-9, 1-8, 1-7, 1-6, 1-5, 1-4, 1-3 or 1-2, or 1) substituents selected from the group consisting of $C_{1-6}$ alkyl, cyano, halogenated $C_{1-3}$ alkyl, amino, hydroxy, mercapto, halogen, or any combination thereof; and the $C_5$-$C_{20}$ aryl is optionally substituted with one or more (e.g., 1-10, 1-9, 1-8, 1-7, 1-6, 1-5, 1-4, 1-3 or 1-2, or 1) substituents selected from the group consisting of: $C_{1-6}$ alkyl, cyano, halogenated $C_{1-3}$alkyl, amino, hydroxy, mercapto, halogen, or any combination thereof; and the 5- or 6-membered heteroaryl is optionally substituted with one or more (e.g., 1-10, 1-9, 1-8,

1-7, 1-6, 1-5, 1-4, 1-3 or 1-2, or 1) substituents selected from the group consisting of: $C_{1-6}$ alkyl, halogenated $C_{1-3}$ alkyl, amino, hydroxy, mercapto, halogen, or any combination thereof.

In some embodiments of the compounds of Formula (I) and Formula (I-4) of the present disclosure, $X_1$ represents pyridyl, pyrimidinyl, thiazolyl, quinolyl, 6,7-difluoroquinazolin-4-yl, 3-cyano-quinolyl, indolyl, 1-methyl-1H-indol-7-yl, benzothienyl, benzofuranyl, quinazolinyl, 6,7-difluoroquinazolin-4-yl, 4-(pyridin-3-yl)pyrimidin-2-yl, 3-fluoro-5,7-dihydro-6H-pyrrolo[3,4-b]pyridin-6-yl, 3-(trifluoromethyl)-5,7-dihydro-6H-pyrrolo[3,4-b]pyridin-6-yl, or isoindol-2-yl.

In some embodiments of the compounds of Formula (I) and Formula (I-4) of the present disclosure, $X_1$ represents $NR_2R_3$, wherein $R_2$ and $R_3$ each independently represent H, optionally substituted linear or branched $C_{1-10}$ alkyl, optionally substituted cycloalkyl, optionally substituted aryl, optionally substituted heterocyclyl or optionally substituted heteroaryl, wherein $R_2$ and $R_3$ are not H at the same time.

In some embodiments of the compounds of Formula (I) and Formula (I-4) of the present disclosure, $X_1$ represents $NR_2R_3$, wherein

$R_2$ and $R_3$ each independently represent H, optionally substituted linear or branched $C_{1-10}$ alkyl, optionally substituted $C_3$-$C_{20}$ cycloalkyl, optionally substituted 3- to 20-membered heterocyclyl, optionally substituted $C_5$-$C_{14}$ aryl, or optionally substituted 5- to 20-membered heteroaryl, wherein $R_2$ and $R_3$ are not H at the same time,

wherein the optionally substituted linear or branched $C_{1-10}$ alkyl is optionally substituted with one or more (e.g., 1-10, 1-9, 1-8, 1-7, 1-6, 1-5, 1-4, 1-3, or 1-2, or 1) substituents selected from the group consisting of halogen, cyano, hydroxy, amino, mercapto, $C_{1-3}$ alkyl, $C_{1-3}$ alkoxy, trifluoromethyl, optionally substituted $C_{5-10}$ aryl, optionally substituted 5- to 10-membered heteroaryl, optionally substituted $C_{3-12}$ cycloalkyl, optionally substituted 3- to 12-membered heterocyclyl, or any combination thereof, wherein the optionally substituted $C_{5-10}$ aryl, optionally substituted 5- to 10-membered heteroaryl, optionally substituted $C_{3-12}$ cycloalkyl and optionally substituted 3- to 12-membered heterocyclyl are each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, $C_{1-3}$ alkoxy, or any combination thereof;

the optionally substituted $C_3$-$C_{20}$cycloalkyl and the optionally substituted 3- to 20-membered heterocyclyl are each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$ alkyl, $C_{1-3}$ alkoxy, $C_{1-3}$ alkoxycarbonyl, halogenated $C_{2-4}$ alkenyl, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, or any combination thereof, wherein the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$ alkyl, halogenated $C_{1-3}$ alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and

wherein the optionally substituted $C_5$-$C_{14}$ aryl or optionally substituted 5- to 20-membered heteroaryl is each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, $C(O)NHR_d$, or any combination thereof, wherein $R_a$ represents $C_{1-5}$ alkyl or optionally substituted phenyl, and the optionally substituted phenyl is optionally substituted with one or more (e.g., 1-5, 1-4, 1-3 or 1-2, or 1) substituents selected from the group consisting of halogen, cyano, amino, hydroxy, mercapto, oxo, and $C_{1-6}$ alkyl, or any combination thereof; and the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$ alkyl, halogenated $C_{1-3}$ alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$ alkyl, halogenated $C_{1-3}$ alkyl, or any combination thereof.

In some embodiments of the compounds of Formula (I) and Formula (I-4) of the present disclosure, $X_1$ represents $NR_2R_3$, wherein

$R_2$ and $R_3$ each independently represent H or optionally substituted linear or branched $C_{1-10}$ alkyl, wherein $R_2$ and $R_3$ are not H at the same time, and the optionally substituted linear or branched $C_{1-10}$ alkyl is optionally substituted with one or more (e.g., 1-10, 1-9, 1-8, 1-7, 1-6, 1-5, 1-4, 1-3, or 1-2, or 1) substituents selected from the group consisting of halogen, cyano, hydroxy, amino, mercapto, $C_{1-3}$ alkyl, $C_{1-3}$ alkoxy, trifluoromethyl, optionally substituted $C_{5-10}$ aryl, optionally substituted 5- to 10-membered heteroaryl, optionally substituted $C_{3-12}$ cycloalkyl, optionally substituted 3- to 12-membered heterocyclyl, or any combination thereof, wherein the optionally substituted $C_{5-10}$ aryl, optionally substituted 5- to 10-membered heteroaryl, optionally substituted $C_{3-12}$ cycloalkyl and optionally substituted 3- to 12-membered heterocyclyl are each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, $C_{1-3}$ alkoxy, or any combination thereof.

In some embodiments of the compounds of Formula (I) and Formula (I-4) of the present disclosure, $X_1$ represents $NR_2R_3$, wherein

$R_2$ represents H or $C_{1-3}$ alkyl, and $R_3$ represents optionally substituted $C_3$-$C_{20}$ cycloalkyl, optionally substituted $C_5$-$C_{14}$ aryl, optionally substituted 3- to 20-membered heterocyclyl, or optionally substituted 5- to 20-membered heteroaryl, wherein the $C_3$-$C_{20}$ cycloalkyl and 3- to 20-membered heterocyclyl are each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$ alkyl, $C_{1-3}$ alkoxy, $C_{1-3}$ alkoxycarbonyl, halogenated $C_{2-4}$ alkenyl, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, or any combination thereof, wherein the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$ alkyl, halogenated $C_{1-3}$ alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and the $C_5$-$C_{14}$ aryl and 5- to 20-membered heteroaryl are each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-3}$ alkoxy, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, -C(O)NHR$_d$, or any combination thereof, wherein $R_d$ represents $C_{1-5}$ alkyl or optionally substituted phenyl, and the optionally substituted phenyl is optionally substituted with one or more (e.g., 1-5, 1-4, 1-3, or 1-2, or 1) substituents selected from the group consisting of halogen, cyano, amino, hydroxy, mercapto, oxo, and $C_{1-6}$ alkyl, or any combination thereof; and the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$alkyl, halogenated $C_{1-3}$alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$ alkyl, halogenated $C_{1-3}$ alkyl, or any combination thereof.

In some embodiments of the compounds of Formula (I) and Formula (I-4) of the present disclosure, $X_1$ represents adamantanyl-NH-, noradamantanyl-NH-, cyclohexyl-NH-, adamantanyl-N($CH_3$)-, noradamantanyl-N($CH_3$)-, bornyl-NH-, norbornyl-NH-, spiro[3.3]heptyl-NH-, or $N(CH_2CH_3)_2$.

In some embodiments of the compounds of Formula (I) and Formula (I-4) of the present disclosure, $X_1$ represents $NR_2R_3$, wherein $R_2$ represents H, and $R_3$ represents ethyl substituted with 2,6-dichloro-3-fluorophenyl.

In some embodiments of the compounds of Formula (I) and Formula (I-4) of the present disclosure, $X_1$ represents $NR_2R_3$, wherein:

$R_2$ represents H, methyl, ethyl, isopropyl, or cyclohexyl, and
$R_3$ represents the following optionally substituted groups: adamantanyl, noradamantanyl, norbornyl, cyclohexyl, cyclopentyl, cyclopropyl, cyclobutyl, fluorocyclobutyl, difluorocyclobutyl, difluorocyclopentyl, hydroxycyclohexyl, ethyl, isopropyl, *tert*-butyl, methyl, 2,4-dimethylpent-3-yl, dicyclopropylmethyl, spiro[3.3]heptyl, 2-methoxycyclopropyl, hexahydro-1H-isoindol-2(3H)-yl, bicyclo[1.1.1]pentyl, bicyclo[2.2.2]octyl, 4-hydroxybicyclo[2.2.2]octan-1-yl, 4,4-dimethylcyclohexyl, oxetanyl, oxazolyl, 2,3-dihydro-1H-indenyl, quinuclidinyl, 1,7,7-trimethylbicyclo[2.2.1]heptyl, 7,7-dimethylbicyclo[2.2.1]heptyl, bicyclo[2.2.1]heptyl, 1-cyclopropylethyl,

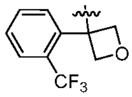 or 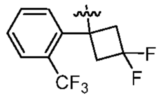 .

In some embodiments of the compounds of Formula (I) and Formula (I-4) of the present disclosure, $X_1$ represents $NR_2R_3$, wherein $R_2$ represents H, and $R_3$ represents -thiazolyl-C(O)NHR$_d$, where $R_d$ represents $C_{1-5}$ alkyl or 2-chloro-6-methylphenyl.

In some embodiments of the compounds of Formula (I) and Formula (I-4) of the present disclosure, $X_1$ represents $NR_2R_3$, wherein $R_2$ represents H, and $R_3$ represents pyrimidin-4-yl, pyridin-4-yl, or quinazolin-4-yl.

In some embodiments of the compounds of Formula (I) and Formula (I-4) of the present disclosure, $X_1$ represents $NR_2R_3$, wherein $R_2$ and $R_3$ represents phenyl.

In some embodiments of the compounds of Formula (I) and Formula (I-4) of the present disclosure, $X_1$ represents $NHCH_3$, $N(CH_3)_2$, $N(CH_2CH_3)_2$ or $N(CH(CH_3)_2)_2$.

In some embodiments of the compounds of Formula (I) and Formula (I-4) of the present disclosure, $X_1$ represents $NR_2R_3$, wherein $R_2$ represents H or methyl, and $R_3$ represents pyrimidinyl, pyridyl, quinazolinyl, 6,7-difluoroquinazolin-4-yl, phenyl, 3-chloro-4-methylphenyl, 4-fluorophenyl, 3,5-difluorophenyl, 3,4,5-trifluorophenyl, 2-(dimethylphosphono) phenyl, 2-(isopropylsulfonyl)phenyl, 4-(pyridin-3-yl)pyrimidin-2-yl, indolyl, 1-methyl-1H-indol-7-yl, benzothienyl, benzo [b]thien-7-yl, benzofuran-7-yl, benzofuranyl, 3-cyano-quinol-4-yl, quinolyl, thiazolyl, adamantanyl, adamantan-1-yl, adamantan-2-yl, 3,5-dimethyladamantan-1-yl, 1,7,7-trimethylbicyclo[2.2.1]heptan-2-yl, 4-(4-methylpiperazin-1-yl)piperidin-1-yl, cyclohexyl, dimethylcyclohexyl, 4,4-dimethylcyclohexyl, spiro-cycloalkyl, spiro[5.5]undec-3-yl, spiro[3.3]heptyl, spiro[3.3]hept-2-yl, pyrrolidinyl, azepanyl, azacyclooctanyl, piperidinyl, dimethylpiperidinyl, or azaspirocycloalkyl.

In some embodiments of the compounds of Formula (I) and Formula (I-4) of the present disclosure, $X_1$ represents $NR_2R_3$, wherein

$R_2$ represents H, methyl, ethyl, isopropyl, or cyclohexyl, and

$R_3$ represents bicyclo[1.1.1]pent-1-yl, 4,4-dimethylcyclohexyl, 2-methoxycyclopropyl, 3-fluorocyclobutyl, 3,3-difluorocyclobutyl, 3,3-difluoro-1-(2-(trifluoromethyl)phenyl)cyclobutyl, 3-(2-(trifluoromethyl)phenyl)oxetan-3-yl, 3,3-difluorocyclopentyl, 3-hydroxycyclohexyl, 3-hydroxycyclohexyl, 3-cyano-bicyclo[1.1.1]pent-1-yl, bicyclo[2.2.2]octan-1-yl, 4-hydroxybicyclo[2.2.2]octan-1-yl, 2-isopropyl-5-methylcyclohexyl, 5-methyloxazol-2-yl, 4-cyano-2,3-dihydro-1H-inden-1-yl, 2,4-dimethylpentan-3-yl, dicyclopropylmethyl, quinuclidin-3-yl, (S)-quinuclidin-3-yl, (R)-quinuclidin-3-yl, adamantan-1-yl, 3-hydroxyadamantanyl, 3-hydroxyadamantan-1-yl, 3-chloroadamantan-1-yl, 4-chloroadamantan-1-yl, 2-chloroadamantan-1-yl, adamantan-2-yl, 1,7,7-trimethylbicyclo[2.2.1]heptan-2-yl, 7,7-dimethylbicyclo[2.2.1]heptan-1-yl, 7,7-dimethyl-2-oxobicyclo[2.2.1]heptan-1-yl, 3,5-dimethyladamantanyl, 3,5-dimethyladamantan-1-yl, hexahydro-2,5-methanopentalen-3a(1H)-yl, bicyclo[2.2.1]heptyl, bicyclo[2.2.1]heptan-2-yl, 1-cyclopropylethyl,

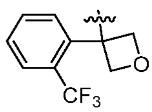 , 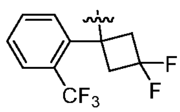 , or 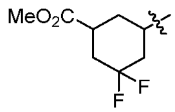 .

In some embodiments of the compounds of Formula (I) and Formula (I-4) of the present disclosure, $X_1$ represents $C(O)NR_4R_5$, wherein $R_4$ and $R_5$ each independently represent H, optionally substituted linear or branched $C_{1-10}$ alkyl, optionally substituted cycloalkyl, optionally substituted aryl, optionally substituted heterocyclyl, or optionally substituted heteroaryl; or wherein $R_4$ and $R_5$ together with the nitrogen atom to which they are attached form optionally substituted 5- to 8-membered heterocyclyl.

In some embodiments of the compounds of Formula (I) and Formula (I-4) of the present disclosure, $X_1$ represents $C(O)NR_4R_5$, wherein

$R_4$ and $R_5$ each independently represent H, optionally substituted linear or branched $C_{1-10}$ alkyl, optionally substituted $C_3$-$C_{20}$ cycloalkyl, optionally substituted $C_5$-$C_{14}$ aryl, optionally substituted 3- to 20-membered heterocyclyl, or optionally substituted 5- to 20-membered heteroaryl;

wherein the optionally substituted linear or branched $C_{1-10}$ alkyl is optionally substituted with one or more (e.g., 1-10, 1-9, 1-8, 1-7, 1-6, 1-5, 1-4, 1-3, or 1-2, or 1) substituents selected from the group consisting of halogen, cyano, hydroxy, amino, mercapto, $C_{1-3}$ alkyl, $C_{1-3}$ alkoxy, trifluoromethyl, $C_{5-10}$ aryl, 5- to 10-membered heteroaryl, $C_{3-12}$ cycloalkyl, 3-

to 12-membered heterocyclyl, or any combination thereof;

the optionally substituted $C_3$-$C_{20}$ cycloalkyl and the optionally substituted 3- to 20-membered heterocyclyl are each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$ alkyl, $C_{1-3}$ alkoxy, $C_{1-3}$ alkoxycarbonyl, halogenated $C_{2-4}$ alkenyl, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesulfonyloxy, p-toluenesulfonyloxy, optionally substituted 5- or 6-membered heterocyclyl, optionally substituted $C_5$-$C_{20}$ aryl, optionally substituted 5- or 6-membered heteroaryl, or any combination thereof, wherein the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$ alkyl, halogenated $C_{1-3}$ alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof;

the optionally substituted $C_5$-$C_{14}$ aryl or optionally substituted 5- to 20-membered heteroaryl is each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-3}$ alkoxy, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$ alkyl, halogenated $C_{1-3}$ alkyl, or any combination thereof; and the $C_5$-$C_{20}$ aryl is optionally substituted with one or more (e.g., 1-10, 1-9, 1-8, 1-7, 1-6, 1-5, 1-4, 1-3 or 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3 or 1-2, or 1) substituents selected from the group consisting of halogen, cyano, amino, hydroxy, oxo, mercapto, $C_{1-6}$ alkyl, halogenated $C_{1-3}$ alkyl, or any combination thereof.

In some embodiments of the compounds of Formula (I) and Formula (I-4) of the present disclosure, $X_1$ represents $C(O)NR_4R_5$, wherein $R_4$ and $R_5$ together with the nitrogen atom to which they are attached form optionally substituted 5- to 8-membered heterocyclyl;

the optionally substituted 5- to 8-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$alkyl, $C_{1-3}$ alkoxycarbonyl, halogenated $C_{2-4}$ alkenyl, or any combination thereof, wherein the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$alkyl, halogenated $C_{1-3}$alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$alkyl, halogenated $C_{1-3}$alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$alkyl, halogenated $C_{1-3}$ alkyl, or any combination thereof.

In some embodiments of the compounds of Formula (I) and Formula (I-4) of the present disclosure, $X_1$ represents piperidinyl-C(O)-, difluoropiperidinyl-C(O)-, $-C(O)N(CH(CH_3)_2)_2$, $C(O)NH_2$, N,N-diisopropylcarbamoyl, adamantanyl-NHC(O)-, 3,5-dimethyladamantan-1-yl-NHC(O)-, or adamantan-2-yl-NHC(O)-.

In some embodiments of the compounds of Formula (I) and Formula (I-4) of the present disclosure, $X_1$ represents $NHC(O)R_6$, wherein $R_6$ represents optionally substituted linear or branched $C_{1-6}$alkyl, optionally substituted cycloalkyl, optionally substituted aryl, optionally substituted heteroaryl or optionally substituted heterocyclyl.

In some embodiments of the compounds of Formula (I) and Formula (I-4) of the present disclosure, $X_1$ represents $NHC(O)R_6$, wherein $R_6$ represents optionally substituted linear or branched $C_{1-10}$alkyl, optionally substituted $C_3$-$C_{20}$ cycloalkyl, optionally substituted $C_5$-$C_{14}$ aryl, optionally substituted 5- to 20-membered heteroaryl, or optionally substituted 3- to 20-membered heterocyclyl;

wherein the optionally substituted linear or branched $C_{1-10}$ alkyl is optionally substituted with one or more (e.g., 1-10, 1-9, 1-8, 1-7, 1-6, 1-5, 1-4, 1-3, or 1-2, or 1) substituents selected from the group consisting of halogen, cyano, hydroxy, amino, mercapto, $C_{1-3}$alkyl, $C_{1-3}$ alkoxy, trifluoromethyl, $C_{5-10}$ aryl, 5- to 10-membered heteroaryl, $C_{3-12}$ cycloalkyl, 3- to 12-membered heterocyclyl, or any combination thereof;

the optionally substituted $C_3$-$C_{20}$ cycloalkyl and the optionally substituted 3- to 20-membered heterocyclyl are each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$alkyl, $C_{1-3}$ alkoxy, $C_{1-3}$ alkoxycarbonyl, halogenated $C_{2-4}$ alkenyl, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, p-toluenesulfonyloxy, optionally substituted 5- or 6-membered heterocyclyl, optionally substituted $C_5$-$C_{20}$ aryl, optionally substituted 5- or 6-membered heteroaryl, or any combination thereof, wherein the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$alkyl, halogenated $C_{1-3}$alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$alkyl, halogenated $C_{1-3}$alkyl, or any combination thereof; and

the optionally substituted $C_5$-$C_{14}$ aryl or optionally substituted 5- to 20-membered heteroaryl is each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$ alkyl, halogenated $C_{1-3}$ alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$ alkyl, halogenated $C_{1-3}$ alkyl, or any combination thereof; or

In some embodiments of the compounds of Formula (I) and Formula (I-4) of the present disclosure, $X_1$ represents $NHC(O)R_6$, wherein $R_6$ represents adamantanyl-C(O)NH-, adamantan-1-yl-C(O)NH-, 3-hydroxyadamantanyl-C(O)NH-, 3-hydroxyadamantan-1-yl-C(O)NH-, 3-chloroadamantan-1-yl-C(O)NH-, 4-chloroadamantan-1-yl-C(O)NH-, 2-chloroadamantan-1-yl-C(O)NH-, adamantan-2-yl-C(0)NH- or - $NHC(O)CH_3$.

In some embodiments of the compounds of Formula (I) and Formula (I-4) of the present disclosure, $X_1$ represents $NHC(O)NR_7R_8$, wherein $R_7$ and $R_8$ each independently represent H, optionally substituted linear or branched $C_{1-10}$ alkyl, optionally substituted cycloalkyl, optionally substituted aryl, optionally substituted heterocyclyl or optionally substituted heteroaryl.

In some embodiments of the compounds of Formula (I) and Formula (I-4) of the present disclosure, $X_1$ represents $NHC(O)NR_7R_8$, wherein:

$R_7$ and $R_8$ each independently represent H, optionally substituted linear or branched $C_{1-10}$ alkyl, optionally substituted $C_3$-$C_{20}$ cycloalkyl, optionally substituted 3- to 20-membered heterocyclyl, optionally substituted $C_5$-$C_{14}$ aryl or optionally substituted 5- to 20-membered heteroaryl,

wherein the optionally substituted linear or branched $C_{1-10}$ alkyl is optionally substituted with one or more (e.g., 1-10, 1-9, 1-8, 1-7, 1-6, 1-5, 1-4, 1-3, or 1-2, or 1) substituents selected from the group consisting of halogen, cyano, hydroxy, amino, mercapto, $C_{1-3}$ alkyl, $C_{1-3}$ alkoxy, trifluoromethyl, optionally substituted $C_{5-10}$ aryl, optionally substituted 5- to 10-membered heteroaryl, optionally substituted $C_{3-12}$ cycloalkyl, optionally substituted 3- to 12-membered heterocyclyl, or any combination thereof, wherein the optionally substituted $C_{5-10}$ aryl, optionally substituted 5- to 10-membered heteroaryl, optionally substituted $C_{3-12}$ cycloalkyl, and optionally substituted 3- to 12-membered heterocyclyl are each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, $C_{1-3}$ alkoxy, or any combination thereof;

the optionally substituted $C_3$-$C_{20}$ cycloalkyl and the optionally substituted 3- to 20-membered heterocyclyl are each independently optionally substituted with one or more (e.g., 1-10, 1-9, 1-8, 1-7, 1-6, 1-5, 1-4, 1-3, or 1-2, or 1)

substituents selected from the group consisting of halogen, cyano, amino, hydroxy, mercapto, oxo, $C_{1-6}$ alkyl, $C_{1-3}$ alkoxy, $C_{1-3}$ alkoxycarbonyl, halogenated $C_{2-4}$ alkenyl, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, or any combination thereof, wherein the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$ alkyl, halogenated $C_{1-3}$ alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and wherein the optionally substituted $C_5$-$C_{14}$ aryl or optionally substituted 5- to 20-membered heteroaryl is each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-3}$ alkoxy, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, C(O)NHR$_d$, or any combination thereof, wherein $R_d$ represents $C_{1-5}$alkyl or optionally substituted phenyl, and the optionally substituted phenyl is optionally substituted with one or more (e.g., 1-5, 1-4, 1-3, or 1-2, or 1) substituents selected from the group consisting of halogen, cyano, amino, hydroxy, mercapto, oxo, and $C_{1-6}$ alkyl, or any combination thereof; and the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$ alkyl, halogenated $C_{1-3}$ alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$ alkyl, halogenated $C_{1-3}$ alkyl, or any combination thereof.

In some embodiments of the compounds of Formula (I) and Formula (I-4) of the present disclosure, $X_1$ represents NHC(O)NR$_7$R$_8$, wherein $R_7$ represents H, and $R_8$ represents adamantanyl, adamantan-1-yl, adamantan-2-yl, 3,5-dimethyladamantan-1-yl, phenyl, or 3-chloro-4-methylphenyl.

In some embodiments of the compounds of Formula (I) and Formula (I-4) of the present disclosure, $X_1$ represents OR$_9$, wherein $R_9$ represents H, optionally substituted linear or branched $C_{1-10}$ alkyl, optionally substituted cycloalkyl, optionally substituted aryl, optionally substituted heterocyclyl or optionally substituted heteroaryl.

In some embodiments of the compounds of Formula (I) and Formula (I-4) of the present disclosure, $X_1$ represents OR$_9$, wherein $R_9$ represents H, optionally substituted linear or branched $C_{1-10}$ alkyl, optionally substituted $C_3$-$C_{20}$ cycloalkyl, optionally substituted 3- to 20-membered heterocyclyl, optionally substituted $C_5$-$C_{14}$ aryl or optionally substituted 5- to 20-membered heteroaryl,

wherein the optionally substituted linear or branched $C_{1-10}$ alkyl is optionally substituted with one or more (e.g., 1-10, 1-9, 1-8, 1-7, 1-6, 1-5, 1-4, 1-3, or 1-2, or 1) substituents selected from the group consisting of halogen, cyano, hydroxy, amino, mercapto, $C_{1-3}$ alkyl, $C_{1-3}$ alkoxy, trifluoromethyl, optionally substituted $C_{5-10}$ aryl, optionally substituted 5- to 10-membered heteroaryl, optionally substituted $C_{3-12}$ cycloalkyl, optionally substituted 3- to 12-membered heterocyclyl, or any combination thereof, wherein the optionally substituted $C_{5-10}$ aryl, optionally substituted 5- to 10-membered heteroaryl, optionally substituted $C_{3-12}$ cycloalkyl and optionally substituted 3- to 12-membered heterocyclyl are each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, $C_{1-3}$ alkoxy, or any combination thereof;

the optionally substituted $C_3$-$C_{20}$ cycloalkyl and the optionally substituted 3- to 20-membered heterocyclyl are each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$ alkyl, $C_{1-3}$ alkoxy, $C_{1-3}$ alkoxycarbonyl, halogenated $C_{2-4}$ alkenyl, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, or any combination thereof, wherein the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the

group consisting of halogen, cyano, amino, hydroxy, oxo, mercapto, $C_{1-6}$ alkyl, halogenated $C_{1-3}$ alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof;

wherein the optionally substituted $C_5$-$C_{14}$ aryl or optionally substituted 5- to 20-membered heteroaryl is each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-3}$ alkoxy, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesulfonyloxy, p-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, C(O)NHR$_d$, or any combination thereof, wherein $R_d$ represents $C_{1-5}$ alkyl or optionally substituted phenyl, and the optionally substituted phenyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, and $C_{1-6}$ alkyl, or any combination thereof; and the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$ alkyl, halogenated $C_{1-3}$ alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$ alkyl, halogenated $C_{1-3}$ alkyl, or any combination thereof.

In some embodiments of the compounds of Formula (I) and Formula (I-4) of the present disclosure, $X_1$ represents OH, adamantanyl-O-, adamantan-1-yl-O-, adamantan-2-yl-O-, norbornyl-O-, 1,7,7-trimethyl-bicyclo[2.2.1]heptanyl-O-, or 2-isopropyl-5-methylcyclohexyl-O-.

In some embodiments of the compounds of Formula (I) and Formula (I-4) of the present disclosure, $X_1$ represents $SR_{10}$, wherein $R_{10}$ represents H, optionally substituted linear or branched $C_{1-10}$ alkyl, optionally substituted cycloalkyl, optionally substituted aryl, optionally substituted heterocyclyl or optionally substituted heteroaryl.

In some embodiments of the compounds of Formula (I) and Formula (I-4) of the present disclosure, $X_1$ represents $SR_{10}$, wherein $R_{10}$ represents H, optionally substituted linear or branched $C_{1-10}$ alkyl, optionally substituted $C_3$-$C_{20}$ cycloalkyl, optionally substituted 3- to 20-membered heterocyclyl, optionally substituted $C_5$-$C_{14}$ aryl or optionally substituted 5- to 20-membered heteroaryl,

wherein the optionally substituted linear or branched $C_{1-10}$ alkyl is optionally substituted with one or more (e.g., 1-10, 1-9, 1-8, 1-7, 1-6, 1-5, 1-4, 1-3, or 1-2, or 1) substituents selected from the group consisting of halogen, cyano, hydroxy, amino, mercapto, $C_{1-3}$ alkyl, $C_{1-3}$ alkoxy, trifluoromethyl, optionally substituted $C_{5-10}$ aryl, optionally substituted 5- to 10-membered heteroaryl, optionally substituted $C_{3-12}$ cycloalkyl, optionally substituted 3- to 12-membered heterocyclyl, or any combination thereof, wherein the optionally substituted $C_{5-10}$ aryl, optionally substituted 5- to 10-membered heteroaryl, optionally substituted $C_{3-12}$cycloalkyl and optionally substituted 3- to 12-membered heterocyclyl are each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, $C_{1-3}$ alkoxy, or any combination thereof;

the optionally substituted $C_3$-$C_{20}$ cycloalkyl and the optionally substituted 3- to 20-membered heterocyclyl are each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$ alkyl, $C_{1-3}$ alkoxy, $C_{1-3}$ alkoxycarbonyl, halogenated $C_{2-4}$ alkenyl, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesulfonyloxy, p-toluenesulfonyloxy, optionally substituted 5- or 6-membered heterocyclyl, optionally substituted $C_5$-$C_{20}$ aryl, optionally substituted 5- or 6-membered heteroaryl, or any combination thereof, wherein the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$ alkyl, halogenated $C_{1-3}$ alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the

group consisting of halogen, cyano, amino, hydroxy, mercapto, $C_{1-6}$ alkyl, halogenated $C_{1-3}$ alkyl, or any combination thereof;

wherein the optionally substituted $C_5$-$C_{14}$ aryl or optionally substituted 5- to 20-membered heteroaryl is each independently optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, C(O)NH$R_d$, or any combination thereof, wherein $R_d$ represents $C_{1-5}$ alkyl or optionally substituted phenyl, and the optionally substituted phenyl is optionally substituted with one or more (e.g., 1-5, 1-4, 1-3, or 1-2, or 1) substituents selected from the group consisting of halogen, cyano, amino, hydroxy, mercapto, oxo, and $C_{1-6}$ alkyl, or any combination thereof; and the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$ alkyl, halogenated $C_{1-3}$ alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, oxo, mercapto, $C_{1-6}$ alkyl, halogenated $C_{1-3}$ alkyl, or any combination thereof.

In some embodiments of the compounds of Formula (I) and Formula (I-4) of the present disclosure, $X_1$ represents -SH, adamantanyl-S-, adamantan-1-yl-S-, 3-hydroxyadamantanyl-S-, 3-hydroxyadamantan-1-yl-S-, 3-chloroadamantan-1-yl-S-, 4-chloroadamantan-1-yl-S-, 2-chloroadamantan-1-yl-S-, or adamantan-2-yl-S-.

In some embodiments of the compounds of Formula (I) and Formula (I-4) of the present disclosure, $X_1$ represents a quaternary ammonium salt group.

In some embodiments of the compounds of Formula (I) and Formula (I-4) of the present disclosure, $X_1$ represents a quaternary ammonium salt group of Formula $N^+R_aR_bR_cX^-$, wherein $R_a$, $R_b$, $R_c$ each independently represent $C_{1-20}$ alkyl, and $X^-$ represents $F^-$, $Cl^-$, $Br^-$, or $I^-$.

In some embodiments of the compounds of Formula (I) and Formula (I-4) of the present disclosure, $X_1$ represents the quaternary ammonium salt group of Formula $N^+R_aR_bR_cX^-$, wherein $R_a$, $R_b$, $R_c$ each independently represent $C_{1-10}$ alkyl, and $X^-$ represents $Cl^-$.

In some embodiments of the compounds of Formula (I) and Formula (I-4) of the present disclosure, $X_1$ represents the quaternary ammonium salt group of Formula $N^+R_aR_bR_cX^-$, wherein $R_a$ and $R_b$ each independently represent methyl, ethyl, propyl, isopropyl or butyl; $R_c$ represents ethyl; and $X^-$ represents $Cl^-$.

In some embodiments of the compounds of Formula (I) and Formula (I-4) of the present disclosure, $X_1$ represents the quaternary ammonium salt group of Formula $N^+R_aR_bR_cX^-$, wherein $R_a$, $R_b$, and $R_c$ represent ethyl, and $X^-$ represents $Cl^-$.

In some embodiments of the compounds of Formula (I) and Formula (I-4) of the present disclosure, $X_1$ represents the quaternary ammonium salt group of Formula $N^+R_aR_bR_cX^-$, wherein $R_a$, $R_b$, and $R_c$ represent methyl, and $X^-$ represents $Cl^-$.

In some embodiments of the compounds of Formula (I) and Formula (I-4) of the present disclosure, $X_1$ represents the quaternary ammonium salt group of Formula $N^+R_aR_bR_cX^-$, wherein $X^-$ represents $F^-$, $Cl^-$, $Br^-$, or $I^-$; $R_c$ represents $C_{1-20}$ alkyl or hydrogen; and $R_a$ and $R_b$ together with the nitrogen atom to which they are attached form a 5- to 8-membered heterocyclyl, wherein the ring atoms of the 5- to 8-membered heterocyclyl optionally further comprise a heteroatom selected from oxygen, nitrogen, and sulfu, and the 5-to 8-membered heterocyclyl is optionally substituted with one or more (e.g., 1-10, 1-9, 1-8, 1-7, 1-6, 1-5, 1-4, 1-3 or 1-2, or 1) substituents selected from the group consisting of: halogen, cyano, amino, hydroxy, mercapto, $C_{1-3}$ alkoxy, halogenated $C_{1-3}$ alkoxy, halogenated $C_{1-3}$ alkyl, $C_{1-6}$ alkylsulfonyl, bis($C_{1-6}$ alkyl) phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$alkyl, $C_{1-3}$ alkoxycarbonyl, halogenated $C_{2-4}$ alkenyl, or any combination thereof, wherein the 5- or 6-membered heterocyclyl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$ alkyl, halogenated $C_{1-3}$alkyl, or any combination thereof; and the $C_5$-$C_{20}$ aryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more (e.g., 1-10, 1-9, 1-8, 1-7, 1-6, 1-5, 1-4, 1-3, or 1-2, or 1) substituents selected from the group consisting of halogen, cyano, amino, hydroxy, mercapto, oxo, $C_{1-6}$ alkyl, halogenated $C_{1-3}$ alkyl, or any combination thereof.

In some embodiments of the compounds of Formula (I) and Formula (I-4) of the present disclosure, $X_1$ represents

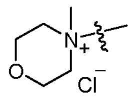 or 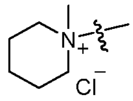 .

In some embodiments of the compounds of Formula (I) and Formula (I-4) of the present disclosure, wherein $X_1$ represents a group of Formula ($G_1$):

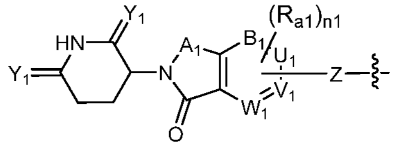

Formula ($G_1$)

wherein $A_1$ represents $CH_2$ or C(O);
$B_1$, $U_1$, $V_1$, and $W_1$ are the same or different and each independently represent CH or N, wherein $B_1$, $U_1$, $V_1$, and $W_1$ are not N at the same time;
$Y_1$ represents O or S; and
Z represents S, S(O) or $S(O)_2$; and
$(R_{a1})_{n1}$ denotes that the ring containing $B_1$, $U_1$, $V_1$, and $W_1$ is substituted with n1 $R_{a1}$, where $R_{a1}$ groups are the same or different and each independently represent deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, optionally substituted $C_{3-6}$ cycloalkyl, $C_{2-6}$ alkenyl, or $C_{2-6}$ alkynyl, and n represents an integer of 0, 1, 2, or 3, wherein the $C_{3-6}$ cycloalkyl is optionally substituted with one or more (e.g., 1-10, 1-9, 1-8, 1-7, 1-6, 1-5, 1-4, 1-3 or 1-2, or 1) substituents selected from the group consisting of $C_{1-6}$ alkyl, halogenated $C_{1-3}$ alkyl, amino, hydroxy, halogen, or any combination thereof.

In some embodiments of the compounds of Formula (I) and Formula (I-4) of the present disclosure, in Formula ($G_1$), $B_1$, $U_1$, $V_1$, and $W_1$ are identical, each being CH.

In some embodiments of the compounds of Formula (I) and Formula (I-4) of the present disclosure, in Formula ($G_1$), one of $B_1$, $U_1$, $V_1$, and $W_1$ is N, and the remaining are CH.

In some embodiments of the compounds of Formula (I) and Formula (I-4) of the present disclosure, in Formula ($G_1$), two of $B_1$, $U_1$, $V_1$, and $W_1$ are N, and the remaining are CH.

In some embodiments of the compounds of Formula (I) and Formula (I-4) of the present disclosure, in Formula ($G_1$), three of $B_1$, $U_1$, $V_1$, and $W_1$ are N, and the remaining is CH.

In some embodiments of the compounds of Formula (I) and Formula (I-4) of the present disclosure, wherein $X_1$ represents the group of Formula ($G_2$):

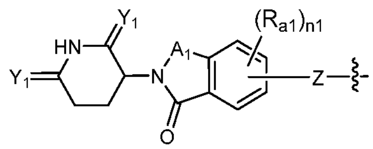

Formula ($G_2$)

wherein $A_1$ represents $CH_2$ or C(O), $Y_1$ represents O or S, and Z represents S, S(O), $S(O)_2$, O, NH or $CH_2$, and $(R_{a1})_{n1}$ denotes that the benzene ring is substituted with n1 $R_{a1}$, where $R_{a1}$ groups are the same or different and each independently represent deuterium, halogen, hydroxy, mercapto, nitro, amino, cyano, optionally deuterated $C_{1-6}$ alkyl, optionally deuterated $C_{1-6}$ alkoxy, halogenated $C_{1-6}$alkyl, optionally substituted $C_{3-6}$ cycloalkyl, $C_{2-6}$ alkenyl, or $C_{2-6}$ alkynyl, and n represents an integer of 0, 1, 2, or 3.

In some embodiments of the compounds of Formula (I) and Formula (I-4) of the present disclosure, wherein $X_1$ represents the group of Formula ($G_3$):

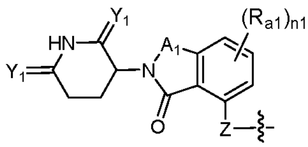

Formula ($G_3$)

wherein $A_1$, $Y_1$, Z, and $(R_{a1})_{n1}$ are as defined in the compounds of Formula ($G_1$) above and its various sub-embodiments described herein.

In some embodiments of the compounds of Formula (I) and Formula (I-4) of the present disclosure, wherein $X_1$ represents the group of Formula ($G_4$):

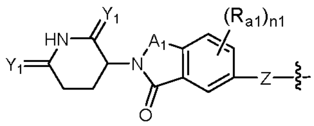

Formula ($G_4$)

wherein $A_1$, $Y_1$, Z, and $(R_{a1})_{n1}$ are as defined in the compounds of Formula ($G_1$) above and its various sub-embodiments described herein.

In some embodiments of the compounds of Formula (I) and Formula (I-4) of the present disclosure, wherein $X_1$ represents the group of Formula ($G_5$):

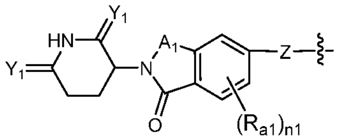

Formula ($G_5$)

wherein $A_1$, $Y_1$, Z, and $(R_{a1})_{n1}$ are as defined in the compounds of Formula ($G_1$) above and its various sub-embodiments described herein.

In some embodiments of the compounds of Formula (I) and Formula (I-4) of the present disclosure, wherein $X_1$ represents the group of Formula ($G_6$):

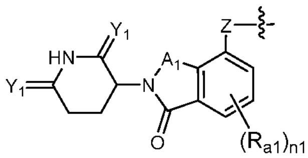

Formula ($G_6$)

wherein $A_1$, $Y_1$, Z, and $(R_{a1})_{n1}$ are as defined in the compounds of Formula ($G_1$) above and its various sub-embodiments described herein.

In some embodiments of the compounds of Formula (I) and Formula (I-4) of the present disclosure, wherein $A_1$ represents $CH_2$ or C(O), $Y_1$ represents O, and Z represents S, S(O) or $S(O)_2$.

In some embodiments of the present disclosure, the compounds of Formula (I), Formula (I-1), Formula (I-2), Formula (I-3), and Formula (I-4) are also of the compounds of Formula (Ia), Formula (Ib), Formula (Ic), Formula (Id), or Formula (Ie):

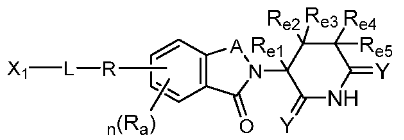

(Ia)

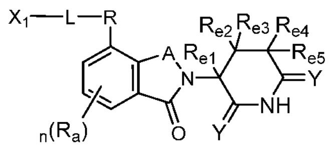

(Ib)

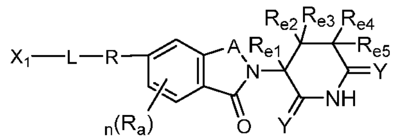

(Ic)

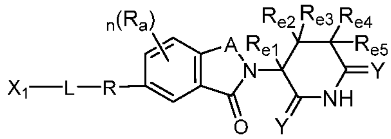

(Id)

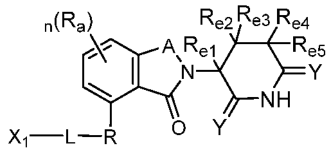

(Ie)

wherein A, $(R_a)_n$, $R_{e1}$, $R_{e2}$, $R_{e3}$, $R_{e4}$, $R_{e5}$, R, L, $X_1$, and Y are as defined in the compounds of Formula (I), Formula (I-1), Formula (I-2), Formula (I-3), and Formula (I-4) and their various embodiments described herein.

Preferably the compounds of the present invention and their salts (especially pharmaceutically acceptable salts, such as hydrochloride, etc.), enantiomers, diastereomers, isotopically enriched analogs, solvates, prodrugs, or polymorphs thereof in Table 1 below are provided:

Table 1. The compounds of the present invention

| Compound No. | Structure of the compounds | The compounds' name | Chinese translation of the compounds' name |
|---|---|---|---|
| | 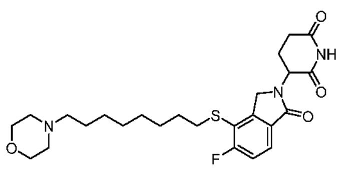 | 3-(5-fluoro-4-((8-morpholinooctyl)thio)-1-oxoisoindolin-2-yl)piperidine-2,6-dione | 3-(5-fluoro-4-((8-morpholinooctyl)thio)-1-oxoisoindolin-2-yl)piperidine-2,6-dione |
| GT-05341 | 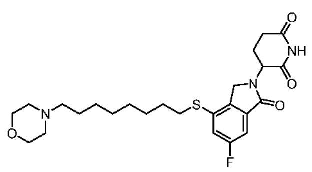 | 3-(6-fluoro-4-((8-morpholinooctyl)thio)-1-oxoisoindolin-2-yl)piperidine-2,6-dione | 3-(6-fluoro-4-((8-morpholinooctyl)thio)-1-oxoisoindolin-2-yl)piperidine-2,6-dione |
| | 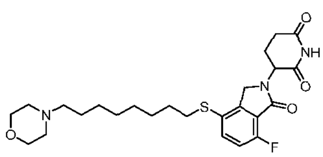 | 3-(7-fluoro-4-((8-morpholinooctyl)thio)-1-oxoisoindolin-2-yl)piperidine-2,6-dione | 3-(7-fluoro-4-((8-morpholinooctyl)thio)-1-oxoisoindolin-2-yl)piperidine-2,6-dione |
| | 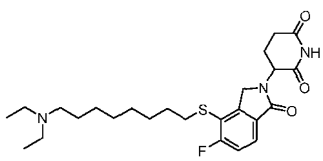 | 3-(4-((8-(diethylamino)octyl)thio)-5-fluoro-1-oxoisoindolin-2-yl)piperidine-2,6-dione | 3-(4-((8-(diethylamino)octyl)thio) -5-fluoro-1-oxoisoindolin-2-yl)piperidine-2,6-dione |
| GT-05342 | 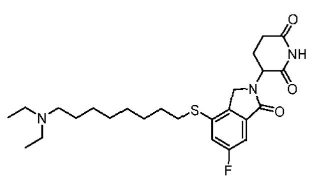 | 3-(4-((8-(diethylamino)octyl)thio)-6-fluoro-1-oxoisoindolin-2-yl)piperidine-2,6-dione | 3-(4-((8-(diethylamino)octyl)thio) -6-fluoro-1-oxoisoindolin-2-yl)piperidine-2,6-dione |

(continued)

| Compound No. | Structure of the compounds | The compounds' name | Chinese translation of the compounds' name |
|---|---|---|---|
| | 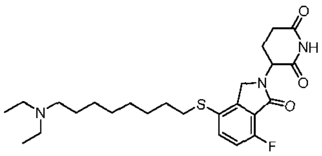 | 3-(4-((8-(diethylamino)octyl)thio)-7-fluoro-1-oxoisoindolin-2-yl)piperidine-2,6-dione | 3-(4-((8-(diethylamino)octyl)thio) -7-fluoro-1-oxoisoindolin-2-yl)piperidine-2,6-dione |
| | 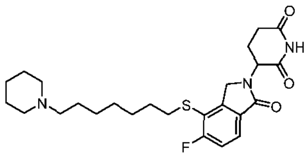 | 3-(5-fluoro-1-oxo-4-((7-(piperidin-1-yl)heptyl)thio)isoindolin-2-yl)piperidine-2,6-dione | 3-(5-fluoro-1-oxo-4-((7-(piperidin-1-yl)heptyl)thio)isoindolin-2-yl)piperidine-2,6-dione |
| GT-05344 | 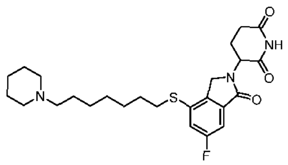 | 3-(6-fluoro-1-oxo-4-((7-(piperidin-1-yl)heptyl)thio)isoindolin-2-yl)piperidine-2,6-dione | 3-(6-fluoro-1-oxo-4-((7-(piperidin-1-yl)heptyl)thio)isoindolin-2-yl)piperidine-2,6-dione |
| | 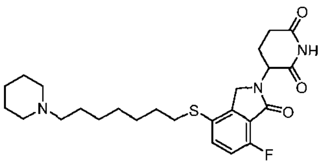 | 3-(7-fluoro-1-oxo-4-((7-(piperidin-1-yl)heptyl)thio)isoindolin-2-yl)piperidine-2,6-dione | 3-(7-fluoro-1-oxo-4-((7-(piperidin-1-yl)heptyl)thio)isoindolin-2-yl)piperidine-2,6-dione |
| | 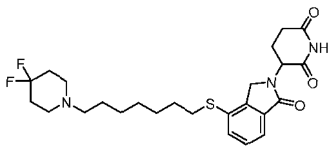 | 3-(4-((7-(4,4-difluoropiperidin-1-yl)heptyl)thio)-1-oxoisoindolin-2-yl)piperidine-2,6-dione | 3-(4-((7-(4,4-difluoropiperidin-1-yl)heptyl)thio)-1-oxoisoindolin-2-yl)piperidine-2,6-dione |
| | 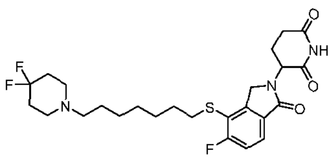 | 3-(4-((7-(4,4-difluoropiperidin-1-yl)heptyl)thio)-5-fluoro-1-oxoisoindolin-2-yl)piperidine-2,6-dione | 3-(4-((7-(4,4-difluoropiperidin-1-yl)heptyl)thio)-5-fluoro-1-oxoisoindolin-2-yl)piperidine-2,6-dione |
| GT-05408 | 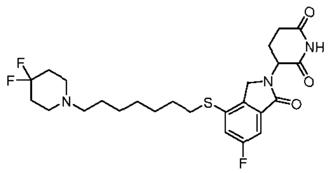 | 3-(4-((7-(4,4-difluoropiperidin-1-yl)heptyl)thio)-6-fluoro-1-oxoisoindolin-2-yl)piperidine-2,6-dione | 3-(4-((7-(4,4-difluoropiperidin-1-yl)heptyl)thio)-6-fluoro-1-oxoisoindolin-2-yl)piperidine-2,6-dione |
| | 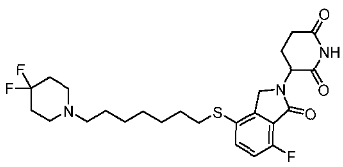 | 3-(4-((7-(4,4-difluoropiperidin-1-yl)heptyl)thio)-7-fluoro-1-oxoisoindolin-2-yl)piperidine-2,6-dione | 3-(4-((7-(4,4-difluoropiperidin-1-yl)heptyl)thio)-7-fluoro-1-oxoisoindolin-2-yl)piperidine-2,6-dione |
| | 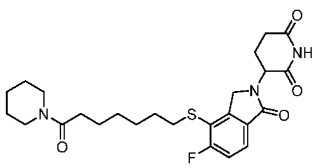 | 3-(5-fluoro-1-oxo-4-((7-oxo-7-(piperidin-1-yl)heptyl)thio)isoindolin-2-yl)piperidine-2,6-dione | 3-(5-fluoro-1-oxo-4-((7-oxo-7-(piperidin-1-yl)heptyl)thio)isoindolin-2-yl)piperidine-2,6-dione |

(continued)

| Compound No. | Structure of the compounds | The compounds' name | Chinese translation of the compounds' name |
|---|---|---|---|
| GT-05343 | 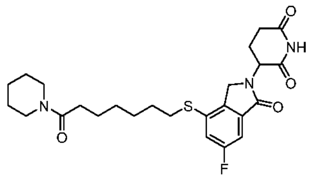 | 3-(6-fluoro-1-oxo-4-((7-oxo-7-(piperidin-1-yl)heptyl)thio)isoindolin-2-yl)piperidine-2,6-dione | 3-(6-fluoro-1-oxo-4-((7-oxo-7-(piperidin-1-yl)heptyl)thio)isoindolin-2-yl)piperidine-2,6-dione |
| | 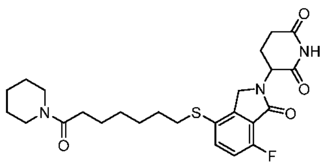 | 3-(7-fluoro-1-oxo-4-((7-oxo-7-(piperidin-1-yl)heptyl)thio)isoindolin-2-yl)piperidine-2,6-dione | 3-(7-fluoro-1-oxo-4-((7-oxo-7-(piperidin-1-yl)heptyl)thio)isoindolin-2-yl)piperidine-2,6-dione |
| | 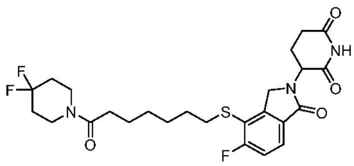 | 3-(4-((7-(4,4-difluoropiperidin-1-yl)-7-oxoheptyl)thio)-5-fluoro-1-oxoisoindolin-2-yl)piperidine-2,6-dione | 3-(4-((7-(4,4-difluoropiperidin-1-yl)-7-oxoheptyl)thio)-5-fluoro-1-oxoisoindolin-2-yl)piperidine-2,6-dione |
| GT-05407 | 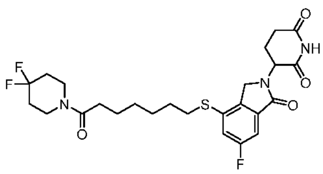 | 3-(4-((7-(4,4-difluoropiperidin-1-yl)-7-oxoheptyl)thio)-6-fluoro-1-oxoisoindolin-2-yl)piperidine-2,6-dione | 3-(4-((7-(4,4-difluoropiperidin-1-yl)-7-oxoheptyl)thio)-6-fluoro-1-oxoisoindolin-2-yl)piperidine-2,6-dione |
| | 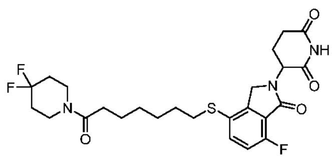 | 3-(4-((7-(4,4-difluoropiperidin-1-yl)-7-oxoheptyl)thio)-7-fluoro-1-oxoisoindolin-2-yl)piperidine-2,6-dione | 3-(4-((7-(4,4-difluoropiperidin-1-yl)-7-oxoheptyl)thio)-7-fluoro-1-oxoisoindolin-2-yl)piperidine-2,6-dione |
| | 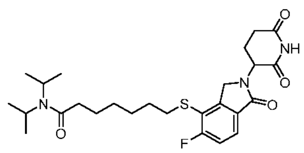 | 7-((2-(2,6-dioxopiperidin-3-yl)-5-fluoro-1-oxoisoindolin-4-yl)thio)-N,N-diisopropylheptanamide | 7-((2-(2,6-dioxopiperidin-3-yl)-5-fluoro-1-oxoisoindolin-4-yl)thio)-N,N-diisopropylheptanamide |
| | 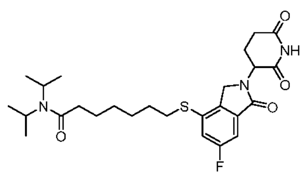 | 7-((2-(2,6-dioxopiperidin-3-yl)-6-fluoro-1-oxoisoindolin-4-yl)thio)-N,N-diisopropylheptanamide | 7-((2-(2,6-dioxopiperidin-3-yl)-6-fluoro-1-oxoisoindolin-4-yl)thio)-N,N-diisopropylheptanamide |
| | 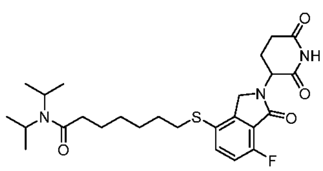 | 7-((2-(2,6-dioxopiperidin-3-yl)-7-fluoro-1-oxoisoindolin-4-yl)thio)-N,N-diisopropylheptanamide | 7-((2-(2,6-dioxopiperidin-3-yl)-7-fluoro-1-oxoisoindolin-4-yl)thio)-N,N-diisopropylheptanamide |
| | 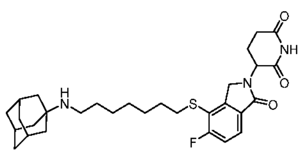 | 3-(4-((7-((adamantan-1-yl)amino)heptyl)thio)-5-fluoro-1-oxoisoindolin-2-yl)piperidine-2,6-dione | 3-(4-((7-((adamantan-1-yl)amino)heptyl)thio)-5-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-04243 | 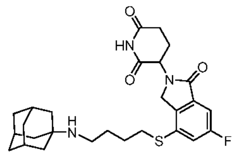 | 3-(4-((4-((adamantan-1-yl)amino)butyl)thio)-6-fluoro-1-oxoisoindolin-2-yl)piperidine-2,6-dione | 3-(4-((4-((adamantan-1-yl)amino)butyl)thio)-6-fluoro-1-oxoisoindolin-2-yl)piperidine-2,6-dione |
| | 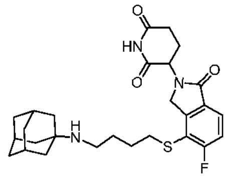 | 3-(4-((4-((adamantan-1-yl)amino)butyl)thio)-5-fluoro-1-oxoisoindolin-2-yl)piperidine-2,6-dione | 3-(4-((4-((adamantan-1-yl)amino)butyl)thio)-5-fluoro-1-oxoisoindolin-2-yl)piperidine-2,6-dione |
| | 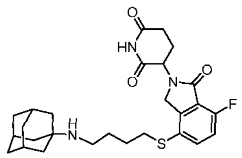 | 3-(4-((4-((adamantan-1-yl)amino)butyl)thio)-7-fluoro-1-oxoisoindolin-2-yl)piperidine-2,6-dione | 3-(4-((4-((adamantan-1-yl)amino)butyl)thio)-7-fluoro-1-oxoisoindolin-2-yl)piperidine-2,6-dione |
| GT-04333 | 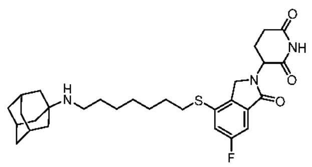 | 3-(4-((7-((adamantan-1-yl)amino)heptyl)thio)-6-fluoro-1-oxoisoindolin-2-yl)piperidine-2,6-dione | 3-(4-((7-((adamantan-1-yl)amino)heptyl)thio)-6-fluoro-1-oxoisoindolin-2-yl)piperidine-2,6-dione |
| | 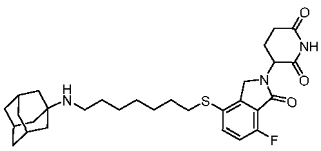 | 3-(4-((7-((adamantan-1-yl)amino)heptyl)thio)-7-fluoro-1-oxoisoindolin-2-yl)piperidine-2,6-dione | 3-(4-((7-((adamantan-1-yl)amino)heptyl)thio)-7-fluoro-1-oxoisoindolin-2-yl)piperidine-2,6-dione |
| | 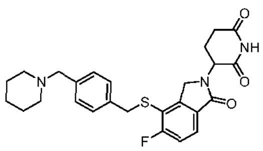 | 3-(5-fluoro-1-oxo-4-((4-(piperidin-1-ylmethyl)benzyl)thio)isoindolin-2-yl)piperidine-2,6-dione | 3-(5-fluoro-1-oxo-4-((4-(piperidin-1-ylmethyl)benzyl)thio)iso indolin-2-yl)piperidine-2,6-dione |
| GT-04117 | 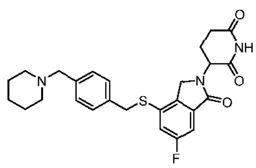 | 3-(6-fluoro-1-oxo-4-((4-(piperidin-1-ylmethyl)benzyl)thio)isoindolin-2-yl)piperidine-2,6-dione | 3-(6-fluoro-1-oxo-4-((4-(piperidin-1-ylmethyl)benzyl)thio)iso indolin-2-yl)piperidine-2,6-dione |
| | 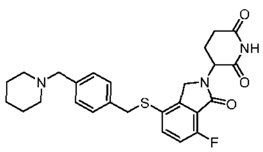 | 3-(7-fluoro-1-oxo-4-((4-(piperidin-1-ylmethyl)benzyl)thio)isoindolin-2-yl)piperidine-2,6-dione | 3-(7-fluoro-1-oxo-4-((4-(piperidin-1-ylmethyl)benzyl)thio)iso indolin-2-yl)piperidine-2,6-dione |
| | 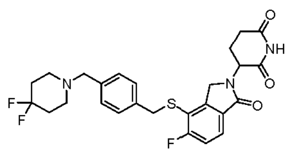 | 3-(4-((4-((4,4-difluoropiperidin-1-yl)methyl)benzyl)thio)-5-fluoro-1-oxoisoindolin-2-yl)piperidine-2,6-dione | 3-(4-((4-((4,4-difluoropiperidin-1-yl)methyl)benzyl)thio)-5-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-04024 | 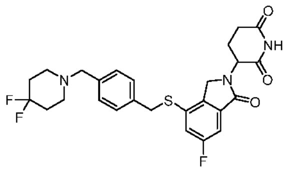 | 3-(4-((4-((4,4-difluoropiperidin-1-yl)methyl)benzyl)thio)-6-fluoro-1-oxoisoindolin-2-yl)piperidine-2,6-dione | 3-(4-((4-((4,4-difluoropiperidin-1-yl)methyl)benzyl)thio)-6-fluoro-1-oxoisoindolin-2-yl)piperidine-2,6-dione |
| | 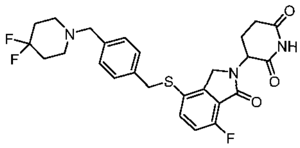 | 3-(4-((4-((4,4-difluoropiperidin-1-yl)methyl)benzyl)thio)-7-fluoro-1-oxoisoindolin-2-yl)piperidine-2,6-dione | 3-(4-((4-((4,4-difluoropiperidin-1-yl)methyl)benzyl)thio)-7-fluoro-1-oxoisoindolin-2-yl)piperidine-2,6-dione |
| | 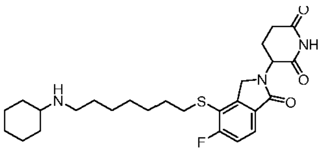 | 3-(4-((7-(cyclohexylamino) heptyl)thio)-5-fluoro-1-oxoisoindolin-2-yl)piperidine-2,6-dione | 3-(4-((7-(cyclohexylamino) heptyl )thio)-5-fluoro-1-oxoisoindolin-2-yl)piperidine-2,6-dione |
| GT-05409 | 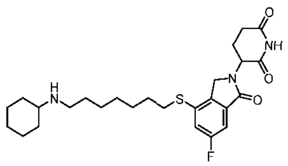 | 3-(4-((7-(cyclohexylamino) heptyl)thio)-6-fluoro-1-oxoisoindolin-2-yl)piperidine-2,6-dione | 3-(4-((7-(cyclohexylamino) heptyl )thio)-6-fluoro-1-oxoisoindolin-2-yl)piperidine-2,6-dione |
| | 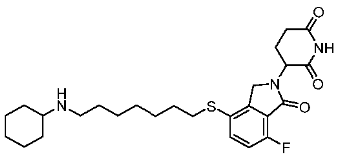 | 3-(4-((7-(cyclohexylamino) heptyl)thio)-7-fluoro-1-oxoisoindolin-2-yl)piperidine-2,6-dione | 3-(4-((7-(cyclohexylamino) heptyl )thio)-7-fluoro-1-oxoisoindolin-2-yl)piperidine-2,6-dione |
| | 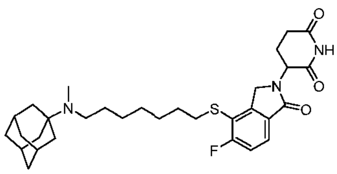 | 3-(4-((7-((adamantan-1-yl) (methyl)amino)heptyl)thio)-5-fluoro-1-oxoisoindolin-2-yl)piperidine-2,6-dione | 3-(4-((7-((adamantan-1-yl) (methyl)amino)heptyl )thio)-5-fluoro-1-oxoisoindolin-2-yl)piperidine-2,6-dione |
| GT-05421 | 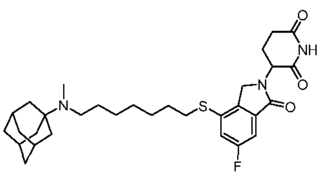 | 3-(4-((7-((adamantan-1-yl) (methyl)amino)heptyl)thio)-6-fluoro-1-oxoisoindolin-2-yl)piperidine-2,6-dione | 3-(4-((7-((adamantan-1-yl) (methyl)amino)heptyl )thio)-6-fluoro-1-oxoisoindolin-2-yl)piperidine-2,6-dione |
| | 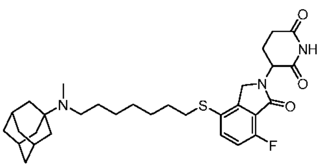 | 3-(4-((7-((adamantan-1-yl) (methyl)amino)heptyl)thio)-7-fluoro-1-oxoisoindolin-2-yl)piperidine-2,6-dione | 3-(4-((7-((adamantan-1-yl) (methyl)amino)heptyl )thio)-7-fluoro-1-oxoisoindolin-2-yl)piperidine-2,6-dione |
| | 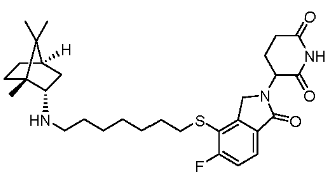 | 3-(5-fluoro-1-oxo-4-((7-(((1R,2S,4R)-1,7,7-trimethylbicyclo[2.2.1]heptan-2-yl)amino)heptyl)thio)isoindolin-2-yl)piperidine-2,6-dione | 3-(5-fluoro-1-oxo-4-((7-(((1R,2S,4R)-1,7,7-trimethylbicyclo[2.2.1]h eptan-2-yl)amino)heptyl)thio)isoi ndolin-2-yl)piperidine-2,6-dione |

(continued)

| Compo und No. | Structure of the compounds | The compounds' name | Chinese translation of the compounds' name |
|---|---|---|---|
| GT-05425 | 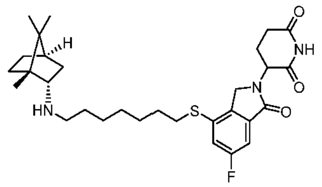 | 3-(6-fluoro-1-oxo-4-((7-(((1R,2S,4R)-1,7,7-trimethylbicyclo[2.2.1]heptan-2-yl)amino)heptyl)thio)isoindolin-2-yl)piperidine-2,6-dione | 3-(6-fluoro-1-oxo-4-((7-(((1R,2S,4R)-1,7,7-trimethylbicyclo[2.2.1]h eptan-2-yl)amino)heptyl)thio)isoi ndolin-2-yl)piperidine-2,6-dione |
| | 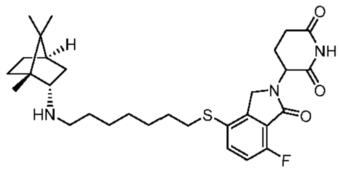 | 3-(7-fluoro-1-oxo-4-((7-(((1R,2S,4R)-1,7,7-trimethylbicyclo[2.2.1]heptan-2-yl)amino)heptyl)thio)isoindolin-2-yl)piperidine-2,6-dione | 3-(7-fluoro-1-oxo-4-((7-(((1R,2S,4R)-1,7,7-trimethylbicyclo[2.2.1]h eptan-2-yl)amino)heptyl)thio)isoi ndolin-2-yl)piperidine-2,6-dione |
| | 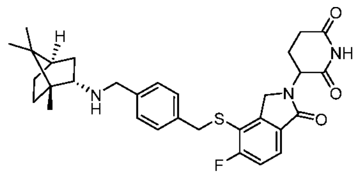 | 3-(5-fluoro-1-oxo-4-((4-((((1R,2S,4R)-1,7,7-trimethylbicyclo[2.2.1]heptan-2-yl)amino)methyl)benzyl)thio) isoi ndolin-2-yl)piperidine-2,6-dione | 3-(5-fluoro-1-oxo-4-((4-((((1R,2S,4R)-1,7,7-trimethylbicyclo[2.2.1]h eptan-2-yl)amino)methyl)benzyl) thio) isoindolin-2-yl)piperidine-2,6-dione |
| GT-05424 | 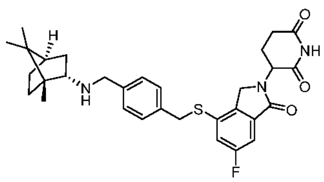 | 3-(6-fluoro-1-oxo-4-((4-((((1R,2S,4R)-1,7,7-trimethylbicyclo[2.2.1]heptan-2-yl)amino)methyl)benzyl)thio) isoi ndolin-2-yl)piperidine-2,6-dione | 3-(6-fluoro-1-oxo-4-((4-((((1R,2S,4R)-1,7,7-trimethylbicyclo[2.2.1]h eptan-2-yl)amino)methyl)benzyl) thio) isoindolin-2-yl)piperidine-2,6-dione |
| | 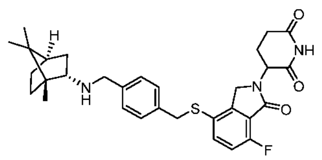 | 3-(7-fluoro-1-oxo-4-((4-((((1R,2S,4R)-1,7,7-trimethylbicyclo[2.2.1]heptan-2-yl)amino)methyl)benzyl)thio) isoi ndolin-2-yl)piperidine-2,6-dione | 3-(7-fluoro-1-oxo-4-((4-((((1R,2S,4R)-1,7,7-trimethylbicyclo[2.2.1]h eptan-2-yl)amino)methyl)benzyl) thio) isoindolin-2-yl)piperidine-2,6-dione |
| | 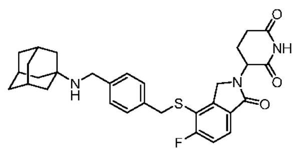 | 3-(4-((4-(((adamantan-1-yl) amino)methyl)benzyl)thio)-5-fluoro-1-oxoisoindolin-2-yl)piperidine-2,6-dione | 3-(4-((4-(((adamantan-1-yl) amino)methyl)benzyl) thio)-5-fluoro-1-oxoisoindolin-2-yl)piperidine-2,6-dione |
| | 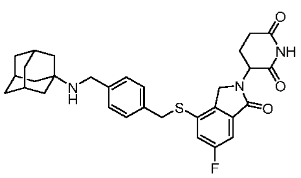 | 3-(4-((4-(((adamantan-1-yl) amino)methyl)benzyl)thio)-6-fluoro-1-oxoisoindolin-2-yl)piperidine-2,6-dione | 3-(4-((4-(((adamantan-1-yl) amino)methyl)benzyl) thio)-6-fluoro-1-oxoisoindolin-2-yl)piperidine-2,6-dione |
| | 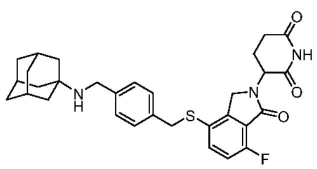 | 3-(4-((4-(((adamantan-1-yl) amino)methyl)benzyl)thio)-7-fluoro-1-oxoisoindolin-2-yl)piperidine-2,6-dione | 3-(4-((4-(((adamantan-1-yl) amino)methyl)benzyl) thio)-7-fluoro-1-oxoisoindolin-2-yl)piperidine-2,6-dione |

(continued)

| Compound No. | Structure of the compounds | The compounds' name | Chinese translation of the compounds' name |
|---|---|---|---|
| | 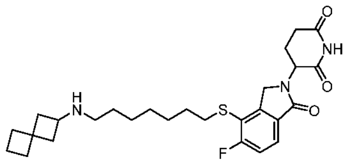 | 3-(5-fluoro-1-oxo-4-((7-(spiro[3.3]heptan-2-ylamino)heptyl)thio)isoindolin-2-yl)piperidine-2,6-dione | 3-(5-fluoro-1-oxo-4-((7-(spiro[3.3]heptan-2-ylamino)heptyl)thio)isoi ndolin-2-yl)piperidine-2,6-dione |
| GT-05422 | 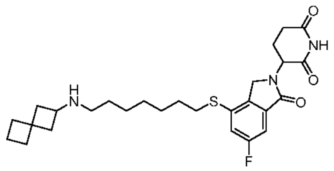 | 3-(6-fluoro-1-oxo-4-((7-(spiro[3.3]heptan-2-ylamino)heptyl)thio)isoindolin-2-yl)piperidine-2,6-dione | 3-(6-fluoro-1-oxo-4-((7-(spiro[3.3]heptan-2-ylamino)heptyl)thio)isoi ndolin-2-yl)piperidine-2,6-dione |
| | 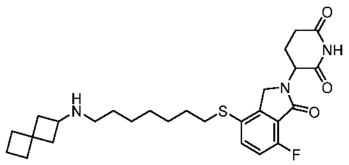 | 3-(7-fluoro-1-oxo-4-((7-(spiro[3.3]heptan-2-ylamino)heptyl)thio)isoindolin-2-yl)piperidine-2,6-dione | 3-(7-fluoro-1-oxo-4-((7-(spiro[3.3]heptan-2-ylamino)heptyl)thio)isoi ndolin-2-yl)piperidine-2,6-dione |
| | 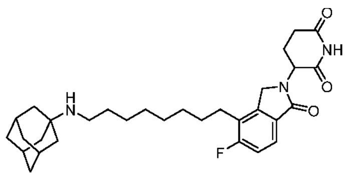 | 3-(4-(8-((adamantan-1-yl)amino)octyl)-5-fluoro-1-oxoisoindolin-2-yl)piperidine-2,6-dione | 3-(4-(8-((adamantan-1-yl)amino)octyl)-5-fluoro-1-oxoisoindolin-2-yl)piperidine-2,6-dione |
| GT-05617 | 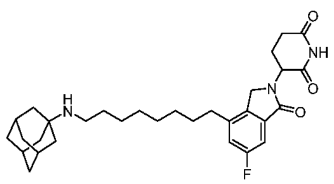 | 3-(4-(8-((adamantan-1-yl)amino)octyl)-6-fluoro-1-oxoisoindolin-2-yl)piperidine-2,6-dione | 3-(4-(8-((adamantan-1-yl)amino)octyl)-6-fluoro-1-oxoisoindolin-2-yl)piperidine-2,6-dione |
| | 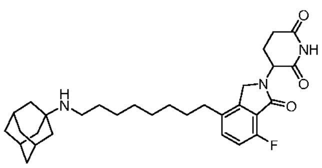 | 3-(4-(8-((adamantan-1-yl)amino)octyl)-7-fluoro-1-oxoisoindolin-2-yl)piperidine-2,6-dione | 3-(4-(8-((adamantan-1-yl)amino)octyl)-7-fluoro-1-oxoisoindolin-2-yl)piperidine-2,6-dione |
| | 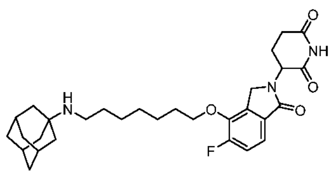 | 3-(4-((7-((yl)amino)heptyl)oxy)-5-fluoro-1-oxoisoindolin-2-yl)piperidine-2,6-dione | 3-(4-((7-((yl)amino)heptyl)oxy)-5-fluoro-1-oxoisoindolin-2-yl)piperidine-2,6-dione |
| | 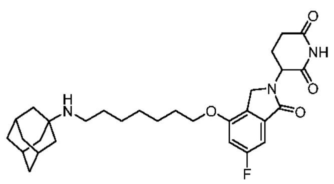 | 3-(4-((7-((adamantan-1-yl)amino)heptyl)oxy)-6-fluoro-1-oxoisoindolin-2-yl)piperidine-2,6-dione | 3-(4-((7-((adamantan-1-yl)amino)heptyl)oxy)-6-fluoro-1-oxoisoindolin-2-yl)piperidine-2,6-dione |

(continued)

| Compound No. | Structure of the compounds | The compounds' name | Chinese translation of the compounds' name |
|---|---|---|---|
| | 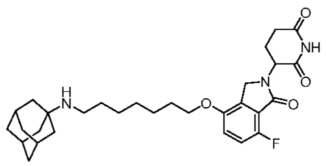 | 3-(4-((7-((adamantan-1-yl)amino)heptyl)oxy)-7-fluoro-1-oxoisoindolin-2-yl)piperidine-2,6-dione | 3-(4-((7-((adamantan-1-yl)amino)heptyl)oxy)-7-fluoro-1-oxoisoindolin-2-yl)piperidine-2,6-dione |
| | 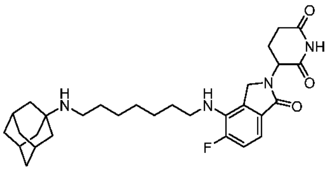 | 3-(4-((7-((adamantan-1-yl)amino)heptyl)amino)-5-fluoro-1-oxoisoindolin-2-yl)piperidine-2,6-dione | 3-(4-((7-((adamantan-1-yl)amino)heptyl)amino)-5-fluoro-1-oxoisoindolin-2-yl)piperidine-2,6-dione |
| GT-05879 | 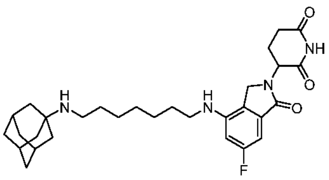 | 3-(4-((7-((adamantan-1-yl)amino)heptyl)amino)-6-fluoro-1-oxoisoindolin-2-yl)piperidine-2,6-dione | 3-(4-((7-((adamantan-1-yl)amino)heptyl)amino)-6-fluoro-1-oxoisoindolin-2-yl)piperidine-2,6-dione |
| | 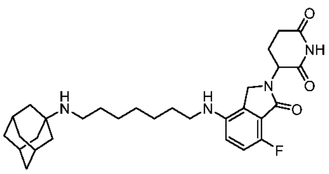 | 3-(4-((7-((adamantan-1-yl)amino)heptyl)amino)-7-fluoro-1-oxoisoindolin-2-yl)piperidine-2,6-dione | 3-(4-((7-((adamantan-1-yl)amino)heptyl)amino)-7-fluoro-1-oxoisoindolin-2-yl)piperidine-2,6-dione |
| | 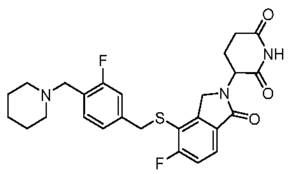 | 3-(5-fluoro-4-((3-fluoro-4-(piperidin-1-ylmethyl)benzyl)thio)-1-oxoisoindolin-2-yl)piperidine-2,6-dione | 3-(5-fluoro-4-((3-fluoro-4-(piperidin-1-ylmethyl)benzyl)thio)-1-oxoisoindolin-2-yl)piperidine-2,6-dione |
| GT-05443 | 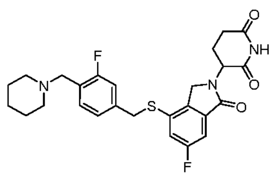 | 3-(6-fluoro-4-((3-fluoro-4-(piperidin-1-ylmethyl)benzyl)thio)-1-oxoisoindolin-2-yl)piperidine-2,6-dione | 3-(6-fluoro-4-((3-fluoro-4-(piperidin-1-ylmethyl)benzyl)thio)-1-oxoisoindolin-2-yl)piperidine-2,6-dione |
| | 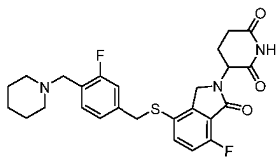 | 3-(7-fluoro-4-((3-fluoro-4-(piperidin-1-ylmethyl)benzyl)thio)-1-oxoisoindolin-2-yl)piperidine-2,6-dione | 3-(7-fluoro-4-((3-fluoro-4-(piperidin-1-ylmethyl)benzyl)thio)-1-oxoisoindolin-2-yl)piperidine-2,6-dione |
| | 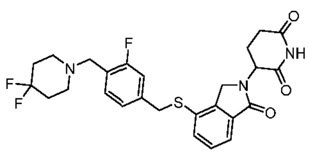 | 3-(4-((4-((4,4-difluoropiperidin-1-yl)methyl)-3-fluorobenzyl)thio)-1-oxoisoindolin-2-yl)piperidine-2,6-dione | 3-(4-((4-((4,4-difluoropiperidin-1-yl)methyl)-3-fluorobenzyl)thio)-1-oxoisoindolin-2-yl)piperidine-2,6-dione |
| | 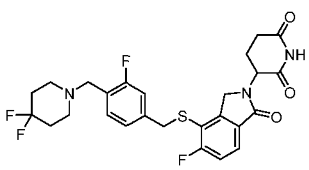 | 3-(4-((4-((4,4-difluoropiperidin-1-yl)methyl)-3-fluorobenzyl)thio)-5-fluoro-1-oxoisoindolin-2-yl)piperidine-2,6-dione | 3-(4-((4-((4,4-difluoropiperidin-1-yl)methyl)-3-fluorobenzyl)thio)-5-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-05444 | 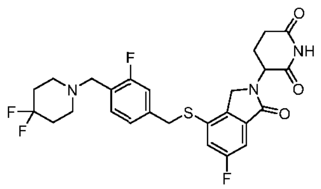 | 3-(4-((4-((4,4-difluoropiperidin-1-yl)methyl)-3-fluorobenzyl)thio)-6-fluoro-1-oxoisoindolin-2-yl)piperidine-2,6-dione | 3-(4-((4-((4,4-difluoropiperidin-1-yl)methyl)-3-fluorobenzyl)thio)-6-fluoro-1-oxoisoindolin-2-yl)piperidine-2,6-dione |
| | 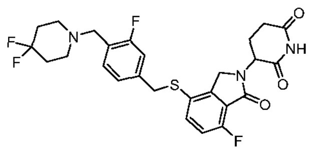 | 3-(4-((4-((4,4-difluoropiperidin-1-yl)methyl)-3-fluorobenzyl)thio)-7-fluoro-1-oxoisoindolin-2-yl)piperidine-2,6-dione | 3-(4-((4-((4,4-difluoropiperidin-1-yl)methyl)-3-fluorobenzyl)thio)-7-fluoro-1-oxoisoindolin-2-yl)piperidine-2,6-dione |
| | 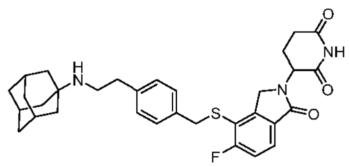 | 3-(4-((4-(2-((adamantan-1-yl)amino)ethyl)benzyl)thio)-5-fluoro-1-oxoisoindolin-2-yl)piperidine-2,6-dione | 3-(4-((4-(2-((adamantan-1-yl)amino)ethyl)benzyl)th io)-5-fluoro-1-oxoisoindolin-2-yl)piperidine-2,6-dione |
| GT-05431 | 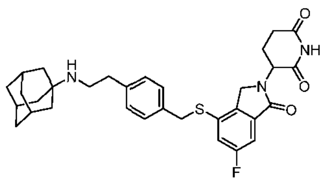 | 3-(4-((4-(2-((adamantan-1-yl)amino)ethyl)benzyl)thio)-6-fluoro-1-oxoisoindolin-2-yl)piperidine-2,6-dione | 3-(4-((4-(2-((adamantan-1-yl)amino)ethyl)benzyl)th io)-6-fluoro-1-oxoisoindolin-2-yl)piperidine-2,6-dione |
| | 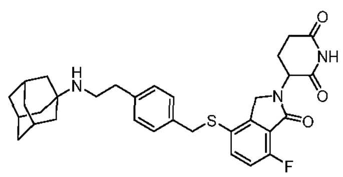 | 3-(4-((4-(2-((adamantan-1-yl)amino)ethyl)benzyl)thio)-7-fluoro-1-oxoisoindolin-2-yl)piperidine-2,6-dione | 3-(4-((4-(2-((adamantan-1-yl)amino)ethyl)benzyl)th io)-7-fluoro-1-oxoisoindolin-2-yl)piperidine-2,6-dione |
| | 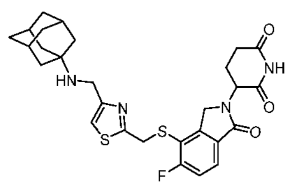 | 3-(4-(((4-(((adamantan-1-yl)amino)methyl)thiazol-2-yl)methyl)thio)-5-fluoro-1-oxoisoindolin-2-yl)piperidine-2,6-dione | 3-(4-(((4-(((adamantan-1-yl)amino)methyl)thiazol-2-yl)methyl)thio)-5-fluoro-1-oxoisoindolin-2-yl)piperidine-2,6-dione |
| | 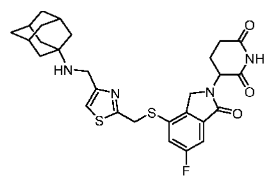 | 3-(4-(((4-(((adamantan-1-yl)amino)methyl)thiazol-2-yl)methyl)thio)-6-fluoro-1-oxoisoindolin-2-yl)piperidine-2,6-dione | 3-(4-(((4-(((adamantan-1-yl)amino)methyl)thiazol-2-yl)methyl)thio)-6-fluoro-1-oxoisoindolin-2-yl)piperidine-2,6-dione |
| | 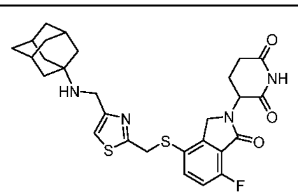 | 3-(4-(((4-(((adamantan-1-yl)amino)methyl)thiazol-2-yl)methyl)thio)-7-fluoro-1-oxoisoindolin-2-yl)piperidine-2,6-dione | 3-(4-(((4-(((adamantan-1-yl)amino)methyl)thiazol-2-yl)methyl)thio)-7-fluoro-1-oxoisoindolin-2-yl)piperidine-2,6-dione |

(continued)

| Compound No. | Structure of the compounds | The compounds' name | Chinese translation of the compounds' name |
|---|---|---|---|
| | 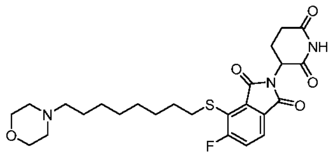 | 2-(2,6-dioxopiperidin-3-yl)-5-fluoro-4-((8-morpholinooctyl)thio)isoindoline-1,3-dione | 2-(2,6-dioxopiperidin-3-yl)-5-fluoro-4-((8-morpholinooctyl)thio)iso indoline-1,3-dione |
| | 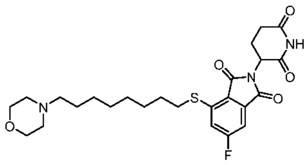 | 2-(2,6-dioxopiperidin-3-yl)-6-fluoro-4-((8-morpholinooctyl)thio)isoindoline-1,3-dione | 2-(2,6-dioxopiperidin-3-yl)-6-fluoro-4-((8-morpholinooctyl)thio)iso indoline-1,3-dione |
| | 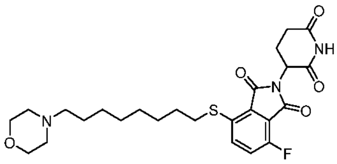 | 2-(2,6-dioxopiperidin-3-yl)-4-fluoro-7-((8-morpholinooctyl)thio)isoindoline-1,3-dione | 2-(2,6-dioxopiperidin-3-yl)-4-fluoro-7-((8-morpholinooctyl)thio)iso indoline-1,3-dione |
| | 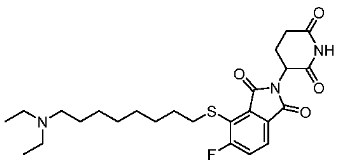 | 4-((8-(diethylamino)octyl)thio)-2-(2,6-dioxopiperidin-3-yl)-5-fluoroisoindoline-1,3-dione | 4-((8-(diethylamino)octyl)thio)-2-(2,6-dioxopiperidin-3-yl)-5-fluoroisoindoline-1,3-dione |
| | 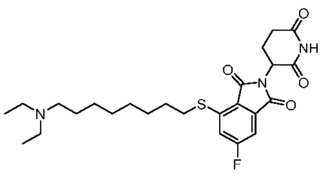 | 4-((8-(diethylamino)octyl)thio)-2-(2,6-dioxopiperidin-3-yl)-6-fluoroisoindoline-1,3-dione | 4-((8-(diethylamino)octyl)thio)-2-(2,6-dioxopiperidin-3-yl)-6-fluoroisoindoline-1,3-dione |
| | 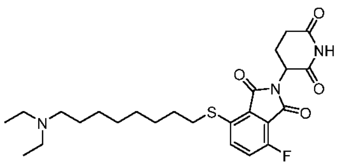 | 4-((8-(diethylamino)octyl)thio)-2-(2,6-dioxopiperidin-3-yl)-7-fluoroisoindoline-1,3-dione | 4-((8-(diethylamino)octyl)thio)-2-(2,6-dioxopiperidin-3-yl)-7-fluoroisoindoline-1,3-dione |
| | 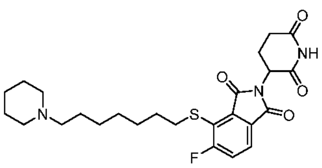 | 2-(2,6-dioxopiperidin-3-yl)-5-fluoro-4-((7-(piperidin-1-yl)heptyl)thio)isoindoline-1,3-dione | 2-(2,6-dioxopiperidin-3-yl)-5-fluoro-4-((7-(piperidin-1-yl)heptyl)thio)isoindolin e-1,3-dione |
| | 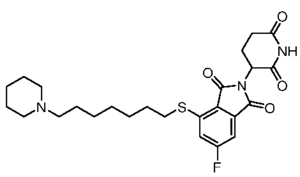 | 2-(2,6-dioxopiperidin-3-yl)-6-fluoro-4-((7-(piperidin-1-yl)heptyl)thio)isoindoline-1,3-dione | 2-(2,6-dioxopiperidin-3-yl)-6-fluoro-4-((7-(piperidin-1-yl)heptyl)thio)isoindolin e-1,3-dione |
| | 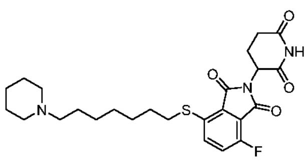 | 2-(2,6-dioxopiperidin-3-yl)-4-fluoro-7-((7-(piperidin-1-yl)heptyl)thio)isoindoline-1,3-dione | 2-(2,6-dioxopiperidin-3-yl)-4-fluoro-7-((7-(piperidin-1-yl)heptyl)thio)isoindolin e-1,3-dione |

(continued)

| Compound No. | Structure of the compounds | The compounds' name | Chinese translation of the compounds' name |
|---|---|---|---|
| | 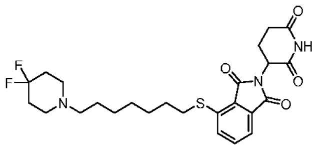 | 4-((7-(4,4-difluoropiperidin-1-yl)heptyl)thio)-2-(2,6-dioxopiperidin-3-yl)isoindoline-1,3-dione | 4-((7-(4,4-difluoropiperidin-1-yl)heptyl)thio)-2-(2,6-dioxopiperidin-3-yl)isoindoline-1,3-dione |
| | 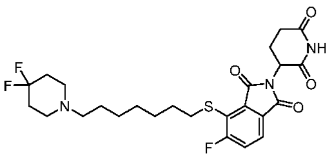 | 4-((7-(4,4-difluoropiperidin-1-yl)heptyl)thio)-2-(2,6-dioxopiperidin-3-yl)-5-fluoroisoindoline-1,3-dione | 4-((7-(4,4-difluoropiperidin-1-yl)heptyl)thio)-2-(2,6-dioxopiperidin-3-yl)-5-fluoroisoindoline-1,3-dione |
| | 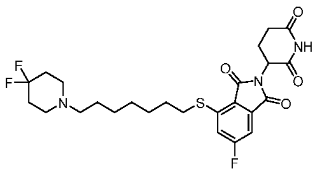 | 4-((7-(4,4-difluoropiperidin-1-yl)heptyl)thio)-2-(2,6-dioxopiperidin-3-yl)-6-fluoroisoindoline-1,3-dione | 4-((7-(4,4-difluoropiperidin-1-yl)heptyl)thio)-2-(2,6-dioxopiperidin-3-yl)-6-fluoroisoindoline-1,3-dione |
| | 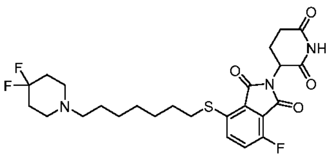 | 4-((7-(4,4-difluoropiperidin-1-yl)heptyl)thio)-2-(2,6-dioxopiperidin-3-yl)-7-fluoroisoindoline-1,3-dione | 4-((7-(4,4-difluoropiperidin-1-yl)heptyl)thio)-2-(2,6-dioxopiperidin-3-yl)-7-fluoroisoindoline-1,3-dione |
| | 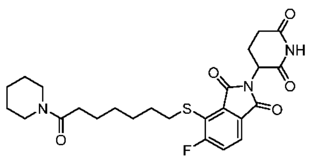 | 2-(2,6-dioxopiperidin-3-yl)-5-fluoro-4-((7-oxo-7-(piperidin-1-yl)heptyl)thio)isoindoline-1,3-dione | 2-(2,6-dioxopiperidin-3-yl)-5-fluoro-4-((7-oxo-7-(piperidin-1-yl)heptyl)thio)isoindolin e-1,3-dione |
| | 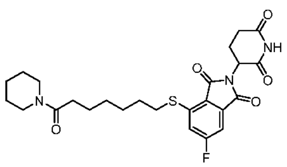 | 2-(2,6-dioxopiperidin-3-yl)-6-fluoro-4-((7-oxo-7-(piperidin-1-yl)heptyl)thio)isoindoline-1,3-dione | 2-(2,6-dioxopiperidin-3-yl)-6-fluoro-4-((7-oxo-7-(piperidin-1-yl)heptyl)thio)isoindolin e-1,3-dione |
| | 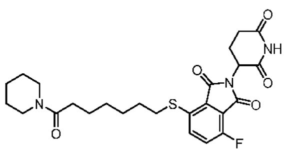 | 2-(2,6-dioxopiperidin-3-yl)-4-fluoro-7-((7-oxo-7-(piperidin-1-yl)heptyl)thio)isoindoline-1,3-dione | 2-(2,6-dioxopiperidin-3-yl)-4-fluoro-7-((7-oxo-7-(piperidin-1-yl)heptyl)thio)isoindolin e-1,3-dione |
| | 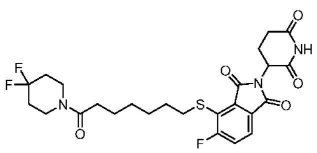 | 4-((7-(4,4-difluoropiperidin-1-yl)-7-oxoheptyl)thio)-2-(2,6-dioxopiperidin-3-yl)-5-fluoroisoindoline-1,3-dione | 4-((7-(4,4-difluoropiperidin-1-yl)-7-oxoheptyl)thio)-2-(2,6-dioxopiperidin-3-yl)-5-fluoroisoindoline-1,3-dione |
| | 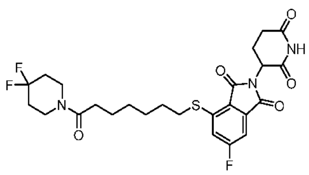 | 4-((7-(4,4-difluoropiperidin-1-yl)-7-oxoheptyl)thio)-2-(2,6-dioxopiperidin-3-yl)-6-fluoroisoindoline-1,3-dione | 4-((7-(4,4-difluoropiperidin-1-yl)-7-oxoheptyl)thio)-2-(2,6-dioxopiperidin-3-yl)-6-fluoroisoindoline-1,3-dione |

(continued)

| Compo und No. | Structure of the compounds | The compounds' name | Chinese translation of the compounds' name |
|---|---|---|---|
| | 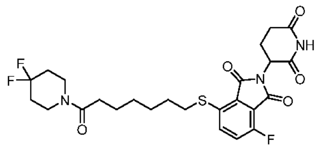 | 4-((7-(4,4-difluoropiperidin-1-yl)-7-oxoheptyl)thio)-2-(2,6-dioxopiperidin-3-yl)-7-fluoroisoindoline-1,3-dione | 4-((7-(4,4-difluoropiperidin-1-yl)-7-oxoheptyl)thio)-2-(2,6-dioxopiperidin-3-yl)-7-fluoroisoindoline-1,3-dione |
| | 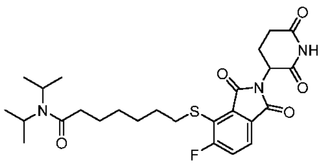 | 7-((2-(2,6-dioxopiperidin-3-yl)-5-fluoro-1,3-dioxoisoindolin-4-yl)thio)-N,N-diisopropylheptanamide | 7-((2-(2,6-dioxopiperidin-3-yl)-5-fluoro-1,3-dioxoisoindolin-4-yl)thio)-N,N-diisopropylheptanamide |
| | 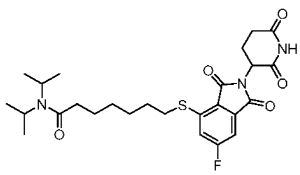 | 7-((2-(2,6-dioxopiperidin-3-yl)-6-fluoro-1,3-dioxoisoindolin-4-yl)thio)-N,N-diisopropylheptanamide | 7-((2-(2,6-dioxopiperidin-3-yl)-6-fluoro-1,3-dioxoisoindolin-4-yl)thio)-N,N-diisopropylheptanamide |
| | 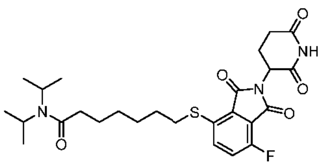 | 7-((2-(2,6-dioxopiperidin-3-yl)-7-fluoro-1,3-dioxoisoindolin-4-yl)thio)-N,N-diisopropylheptanamide | 7-((2-(2,6-dioxopiperidin-3-yl)-7-fluoro-1,3-dioxoisoindolin-4-yl)thio)-N,N-diisopropylheptanamide |
| | 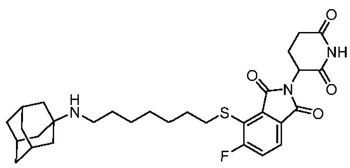 | 4-((7-((adamantan-1-yl)amino) heptyl)thio)-2-(2,6-dioxopiperidin-3-yl)-5-fluoroisoindoline-1,3-dione | 4-((7-((adamantan-1-yl)amino) heptyl)thio)-2-(2,6-dioxopiperidin-3-yl)-5-fluoroisoindoline-1,3-dione |
| | 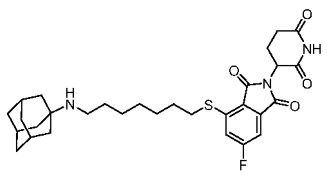 | 4-((7-((adamantan-1-yl)amino) heptyl)thio)-2-(2,6-dioxopiperidin-3-yl)-6-fluoroisoindoline-1,3-dione | 4-((7-((adamantan-1-yl)amino) heptyl)thio)-2-(2,6-dioxopiperidin-3-yl)-6-fluoroisoindoline-1,3-dione |
| | 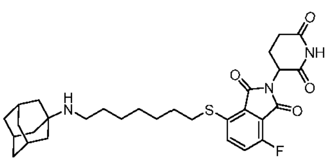 | 4-((7-((adamantan-1-yl)amino) heptyl)thio)-2-(2,6-dioxopiperidin-3-yl)-7-fluoroisoindoline-1,3-dione | 4-((7-((adamantan-1-yl)amino) heptyl)thio)-2-(2,6-dioxopiperidin-3-yl)-7-fluoroisoindoline-1,3-dione |
| | 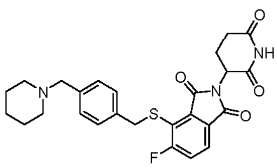 | 2-(2,6-dioxopiperidin-3-yl)-5-fluoro-4-((4-(piperidin-1-ylmethyl)benzyl)thio)isoindoline-1,3-dione | 2-(2,6-dioxopiperidin-3-yl)-5-fluoro-4-((4-(piperidin-1-ylmethyl)benzyl)thio)iso indoline-1,3-dione |
| | 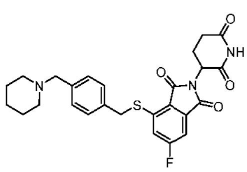 | 2-(2,6-dioxopiperidin-3-yl)-6-fluoro-4-((4-(piperidin-1-ylmethyl)benzyl)thio)isoindoline-1,3-dione | 2-(2,6-dioxopiperidin-3-yl)-6-fluoro-4-((4-(piperidin-1-ylmethyl)benzyl)thio)iso indoline-1,3-dione |

(continued)

| Compound No. | Structure of the compounds | The compounds' name | Chinese translation of the compounds' name |
|---|---|---|---|
| | 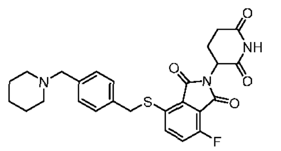 | 2-(2,6-dioxopiperidin-3-yl)-4-fluoro-7-((4-(piperidin-1-yl-methyl)benzyl)thio)isoindo-line-1,3-dione | 2-(2,6-dioxopiperidin-3-yl)-4-fluoro-7-((4-(piperidin-1-yl-methyl)benzyl)thio)iso indo-line-1,3-dione |
| | 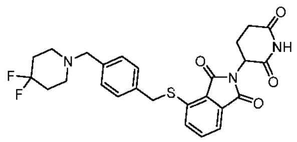 | 4-((4-((4,4-difluoropiperidin-1-yl)methyl)benzyl)thio)-2-(2,6-dioxopiperidin-3-yl)isoindoline-1,3-dione | 4-((4-((4,4-difluoropiperidin-1-yl)methyl)benzyl)thio)-2-(2,6-dioxopiperidin-3-yl)isoindo-line-1,3-dione |
| | 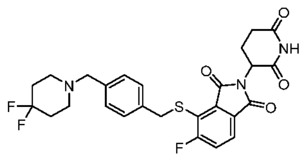 | 4-((4-((4,4-difluoropiperidin-1-yl)methyl)benzyl)thio)-2-(2,6-dioxopiperidin-3-yl)-5-fluoroisoindoline-1,3-dione | 4-((4-((4,4-difluoropiperidin-1-yl)methyl)benzyl)thio)-2-(2,6-dioxopiperidin-3-yl)-5-fluoroi-soindoline-1,3-dione |
| | 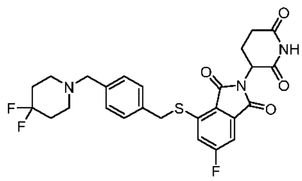 | 4-((4-((4,4-difluoropiperidin-1-yl)methyl)benzyl)thio)-2-(2,6-dioxopiperidin-3-yl)-6-fluoroisoindoline-1,3-dione | 4-((4-((4,4-difluoropiperidin-1-yl)methyl)benzyl)thio)-2-(2,6-dioxopiperidin-3-yl)-6-fluoroi-soindoline-1,3-dione |
| | 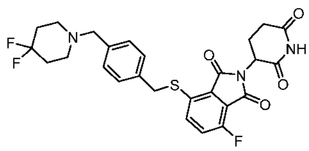 | 4-((4-((4,4-difluoropiperidin-1-yl)methyl)benzyl)thio)-2-(2,6-dioxopiperidin-3-yl)-7-fluoroisoindoline-1,3-dione | 4-((4-((4,4-difluoropiperidin-1-yl)methyl)benzyl)thio)-2-(2,6-dioxopiperidin-3-yl)-7-fluoroi-soindoline-1,3-dione |
| | 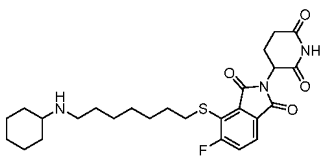 | 4-((7-(cyclohexylamino)heptyl) thio)-2-(2,6-dioxopiperidin-3-yl)-5-fluoroisoindoline-1,3-dione | 4-((7-(cyclohexylamino)heptyl ) thio)-2-(2,6-dioxopiperidin-3-yl)-5-fluoroisoindoline-1,3-dione |
| | 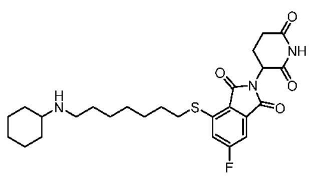 | 4-((7-(cyclohexylamino)heptyl) thio)-2-(2,6-dioxopiperidin-3-yl)-6-fluoroisoindoline-1,3-dione | 4-((7-(cyclohexylamino)heptyl ) thio)-2-(2,6-dioxopiperidin-3-yl)-6-fluoroisoindoline-1,3-dione |
| | 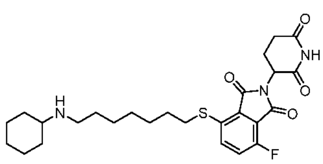 | 4-((7-(cyclohexylamino)heptyl) thio)-2-(2,6-dioxopiperidin-3-yl)-7-fluoroisoindoline-1,3-dione | 4-((7-(cyclohexylamino)heptyl ) thio)-2-(2,6-dioxopiperidin-3-yl)-7-fluoroisoindoline-1,3-dione |
| | 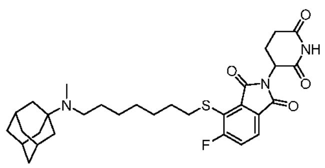 | 4-((7-((adamantan-1-yl) (methyl)amino)heptyl) thio)-2-(2,6-dioxopiperidin-3-yl)-5-fluoroisoindoline-1,3-dione | 4-((7-((adamantan-1-yl) (methyl)amino)heptyl ) thio)-2-(2,6-dioxopiperidin-3-yl)-5-fluoroisoindoline-1,3-dione |

(continued)

| Compo und No. | Structure of the compounds | The compounds' name | Chinese translation of the compounds' name |
|---|---|---|---|
| | 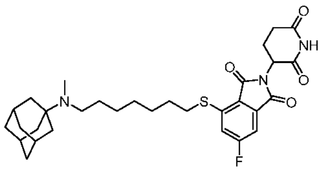 | 4-((7-((adamantan-1-yl)(methyl)amino)heptyl)thio)-2-(2,6-dioxopiperidin-3-yl)-6-fluoroisoindoline-1,3-dione | 4-((7-((adamantan-1-yl)(methyl)amino)heptyl )thio)-2-(2,6-dioxopiperidin-3-yl)-6-fluoroisoindoline-1,3-dione |
| | 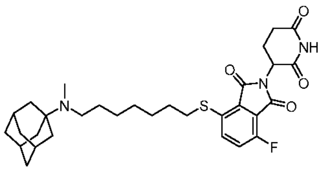 | 4-((7-((adamantan-1-yl)(methyl)amino)heptyl)thio)-2-(2,6-dioxopiperidin-3-yl)-7-fluoroisoindoline-1,3-dione | 4-((7-((adamantan-1-yl)(methyl)amino)heptyl )thio)-2-(2,6-dioxopiperidin-3-yl)-7-fluoroisoindoline-1,3-dione |
| | 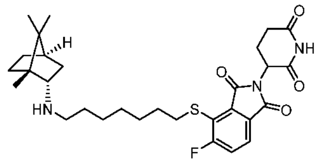 | 2-(2,6-dioxopiperidin-3-yl)-5-fluoro-4-((7-(((1R,2S,4R)-1,7,7-trimethylbicyclo[2.2.1]heptan-2-yl)amino)heptyl)thio)isoindoline-1,3-dione | 2-(2,6-dioxopiperidin-3-yl)-5-fluoro-4-((7-(((1R,2S,4R)-1,7,7-trimethylbicyclo[2.2.1]h eptan-2-yl)amino)heptyl)thio)isoi ndoline-1,3-dione |
| | 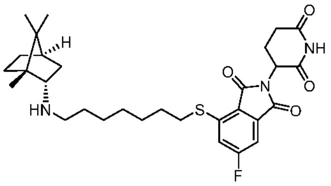 | 2-(2,6-dioxopiperidin-3-yl)-6-fluoro-4-((7-(((1R,2S,4R)-1,7,7-trimethylbicyclo[2.2.1]heptan-2-yl)amino)heptyl)thio)isoindoline-1,3-dione | 2-(2,6-dioxopiperidin-3-yl)-6-fluoro-4-((7-(((1R,2S,4R)-1,7,7-trimethylbicyclo[2.2.1]h eptan-2-yl)amino)heptyl)thio)isoi ndoline-1,3-dione |
| | 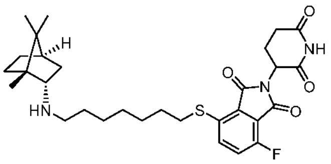 | 2-(2,6-dioxopiperidin-3-yl)-4-fluoro-7-((7-(((1R,2S,4R)-1,7,7-trimethylbicyclo[2.2.1]heptan-2-yl)amino)heptyl)thio)isoindoline-1,3-dione | 2-(2,6-dioxopiperidin-3-yl)-4-fluoro-7-((7-(((1R,2S,4R)-1,7,7-trimethylbicyclo[2.2.1]h eptan-2-yl)amino)heptyl)thio)isoi ndoline-1,3-dione |
| | 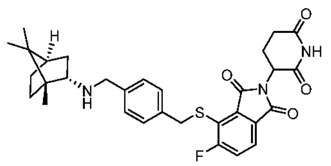 | 2-(2,6-dioxopiperidin-3-yl)-5-fluoro-4-((4-((((1R,2S,4R)-1,7,7-trimethylbicyclo[2.2.1]heptan-2-yl)amino)methyl)benzyl)thio)isoi ndoline-1,3-dione | 2-(2,6-dioxopiperidin-3-yl)-5-fluoro-4-((4-((((1R,2S,4R)-1,7,7-trimethylbicyclo[2.2.1]h eptan-2-yl)amino)methyl)benzyl) thio)isoindoline-1,3-dione |
| | 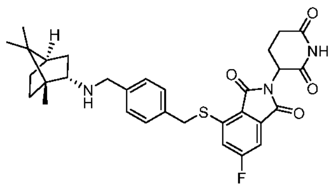 | 2-(2,6-dioxopiperidin-3-yl)-6-fluoro-4-((4-((((1R,2S,4R)-1,7,7-trimethylbicyclo[2.2.1]heptan-2-yl)amino)methyl)benzyl)thio)isoi ndoline-1,3-dione | 2-(2,6-dioxopiperidin-3-yl)-6-fluoro-4-((4-((((1R,2S,4R)-1,7,7-trimethylbicyclo[2.2.1]h eptan-2-yl)amino)methyl)benzyl) thio)isoindoline-1,3-dione |
| | 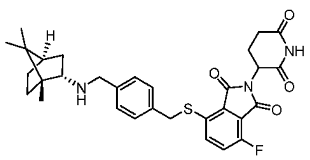 | 2-(2,6-dioxopiperidin-3-yl)-4-fluoro-7-((4-((((1R,2S,4R)-1,7,7-trimethylbicyclo[2.2.1]heptan-2-yl)amino)methyl)benzyl)thio)isoi ndoline-1,3-dione | 2-(2,6-dioxopiperidin-3-yl)-4-fluoro-7-((4-((((1R,2S,4R)-1,7,7-trimethylbicyclo[2.2.1]h eptan-2-yl)amino)methyl)benzyl) thio)isoindoline-1,3-dione |

(continued)

| Compound No. | Structure of the compounds | The compounds' name | Chinese translation of the compounds' name |
|---|---|---|---|
| | 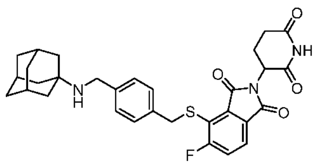 | 4-((4-(((adamantan-1-yl)amino)methyl)benzyl)thio)-2-(2,6-dioxopiperidin-3-yl)-5-fluoroisoindoline-1,3-dione | 4-((4-(((adamantan-1-yl)amino)methyl)benzyl) thio)-2-(2,6-dioxopiperidin-3-yl)-5-fluoroisoindoline-1,3-dione |
| | 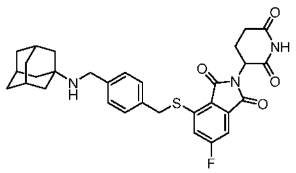 | 4-((4-(((adamantan-1-yl)amino)methyl)benzyl)thio)-2-(2,6-dioxopiperidin-3-yl)-6-fluoroisoindoline-1,3-dione | 4-((4-(((adamantan-1-yl)amino)methyl)benzyl) thio)-2-(2,6-dioxopiperidin-3-yl)-6-fluoroisoindoline-1,3-dione |
| | 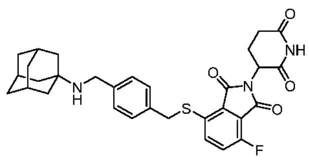 | 4-((4-(((adamantan-1-yl)amino)methyl)benzyl)thio)-2-(2,6-dioxopiperidin-3-yl)-7-fluoroisoindoline-1,3-dione | 4-((4-(((adamantan-1-yl)amino)methyl)benzyl) thio)-2-(2,6-dioxopiperidin-3-yl)-7-fluoroisoindoline-1,3-dione |
| | 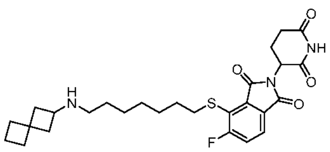 | 2-(2,6-dioxopiperidin-3-yl)-5-fluoro-4-((7-(spiro[3.3]heptan-2-ylamino)heptyl)thio)isoindoline-1,3-dione | 2-(2,6-dioxopiperidin-3-yl)-5-fluoro-4-((7-(spiro[3.3]heptan-2-ylamino)heptyl)thio)isoi ndoline-1,3-dione |
| | 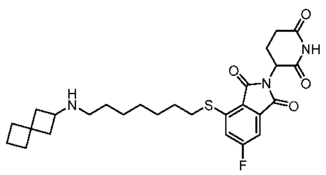 | 2-(2,6-dioxopiperidin-3-yl)-6-fluoro-4-((7-(spiro[3.3]heptan-2-ylamino)heptyl)thio)isoindoline-1,3-dione | 2-(2,6-dioxopiperidin-3-yl)-6-fluoro-4-((7-(spiro[3.3]heptan-2-ylamino)heptyl)thio)isoi ndoline-1,3-dione |
| | 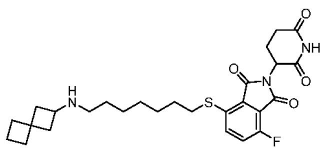 | 2-(2,6-dioxopiperidin-3-yl)-4-fluoro-7-((7-(spiro[3.3]heptan-2-ylamino)heptyl)thio)isoindoline-1,3-dione | 2-(2,6-dioxopiperidin-3-yl)-4-fluoro-7-((7-(spiro[3.3]heptan-2-ylamino)heptyl)thio)isoi ndoline-1,3-dione |
| | 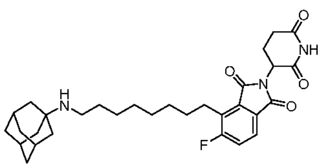 | 4-(8-((adamantan-1-yl)amino) octyl)-2-(2,6-dioxopiperidin-3-yl)-5-fluoroisoindoline-1,3-dione | 4-(8-((adamantan-1-yl)amino) octyl)-2-(2,6-dioxopiperidin-3-yl)-5-fluoroisoindoline-1,3-dione |
| | 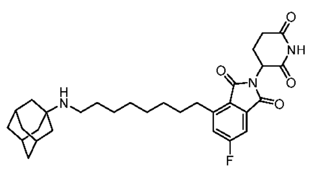 | 4-(8-((adamantan-1-yl)amino) octyl)-2-(2,6-dioxopiperidin-3-yl)-6-fluoroisoindoline-1,3-dione | 4-(8-((adamantan-1-yl)amino) octyl)-2-(2,6-dioxopiperidin-3-yl)-6-fluoroisoindoline-1,3-dione |
| | 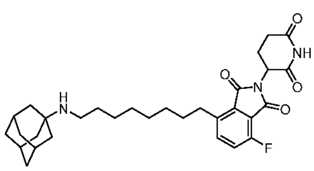 | 4-(8-((adamantan-1-yl)amino) octyl)-2-(2,6-dioxopiperidin-3-yl)-7-fluoroisoindoline-1,3-dione | 4-(8-((adamantan-1-yl)amino) octyl)-2-(2,6-dioxopiperidin-3-yl)-7-fluoroisoindoline-1,3-dione |

(continued)

| Compound No. | Structure of the compounds | The compounds' name | Chinese translation of the compounds' name |
|---|---|---|---|
| | 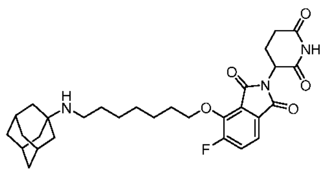 | 4-((7-((adamantan-1-yl)amino)heptyl)oxy)-2-(2,6-dioxopiperidin-3-yl)-5-fluoroisoindoline-1,3-dione | 4-((7-((adamantan-1-yl)amino)heptyl)oxy)-2-(2,6-dioxopiperidin-3-yl)-5-fluoroisoindoline-1,3-dione |
| | 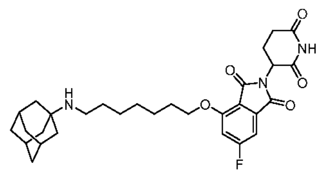 | 4-((7-((adamantan-1-yl)amino)heptyl)oxy)-2-(2,6-dioxopiperidin-3-yl)-6-fluoroisoindoline-1,3-dione | 4-((7-((adamantan-1-yl)amino)heptyl)oxy)-2-(2,6-dioxopiperidin-3-yl)-6-fluoroisoindoline-1,3-dione |
| | 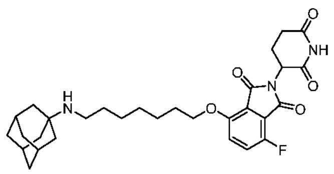 | 4-((7-((adamantan-1-yl)amino)heptyl)oxy)-2-(2,6-dioxopiperidin-3-yl)-7-fluoroisoindoline-1,3-dione | 4-((7-((adamantan-1-yl)amino)heptyl)oxy)-2-(2,6-dioxopiperidin-3-yl)-7-fluoroisoindoline-1,3-dione |
| | 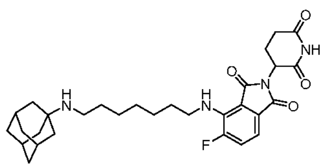 | 4-((7-((adamantan-1-yl)amino)heptyl)amino)-2-(2,6-dioxopiperidin-3-yl)-5-fluoroisoindoline-1,3-dione | 4-((7-((adamantan-1-yl)amino)heptyl)amino)-2-(2,6-dioxopiperidin-3-yl)-5-fluoroisoindoline-1,3-dione |
| | 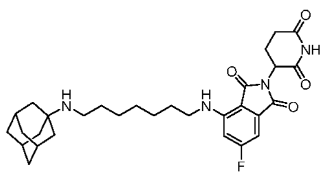 | 4-((7-((adamantan-1-yl)amino)heptyl)amino)-2-(2,6-dioxopiperidin-3-yl)-6-fluoroisoindoline-1,3-dione | 4-((7-((adamantan-1-yl)amino)heptyl)amino)-2-(2,6-dioxopiperidin-3-yl)-6-fluoroisoindoline-1,3-dione |
| | 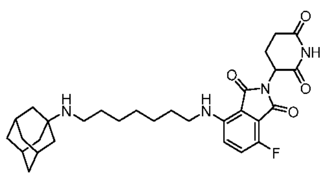 | 4-((7-((adamantan-1-yl)amino)heptyl)amino)-2-(2,6-dioxopiperidin-3-yl)-7-fluoroisoindoline-1,3-dione | 4-((7-((adamantan-1-yl)amino)heptyl)amino)-2-(2,6-dioxopiperidin-3-yl)-7-fluoroisoindoline-1,3-dione |
| | 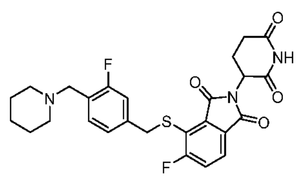 | 2-(2,6-dioxopiperidin-3-yl)-5-fluoro-4-((3-fluoro-4-(piperidin-1-ylmethyl)benzyl)thio)isoindoline-1,3-dione | 2-(2,6-dioxopiperidin-3-yl)-5-fluoro-4-((3-fluoro-4-(piperidin-1-ylmethyl)benzyl)thio)iso indoline-1,3-dione |
| | 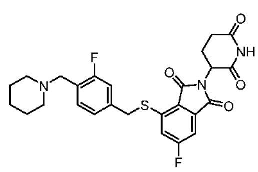 | 2-(2,6-dioxopiperidin-3-yl)-6-fluoro-4-((3-fluoro-4-(piperidin-1-ylmethyl)benzyl)thio)isoindoline-1,3-dione | 2-(2,6-dioxopiperidin-3-yl)-6-fluoro-4-((3-fluoro-4-(piperidin-1-ylmethyl)benzyl)thio)iso indoline-1,3-dione |

(continued)

| Compo und No. | Structure of the compounds | The compounds' name | Chinese translation of the compounds' name |
|---|---|---|---|
| | 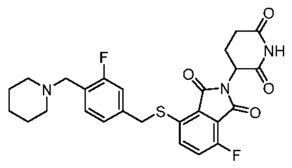 | 2-(2,6-dioxopiperidin-3-yl)-4-fluoro-7-((3-fluoro-4-(piperidin-1-ylmethyl)benzyl)thio)isoindoline-1,3-dione | 2-(2,6-dioxopiperidin-3-yl)-4-fluoro-7-((3-fluoro-4-(piperidin-1-ylmethyl)benzyl)thio)iso indoline-1,3-dione |
| | 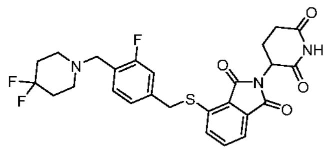 | 4-((4-((4,4-difluoropiperidin-1-yl)methyl)-3-fluorobenzyl) thio)-2-(2,6-dioxopiperidin-3-yl)isoindoline-1,3-dione | 4-((4-((4,4-difluoropiperidin-1-yl)methyl)-3-fluorobenzyl) thio)-2-(2,6-dioxopiperidin-3-yl)isoindoline-1,3-dione |
| | 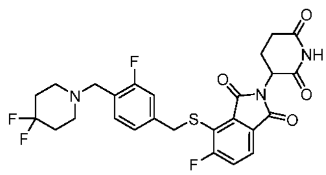 | 4-((4-((4,4-difluoropiperidin-1-yl)methyl)-3-fluorobenzyl) thio)-2-(2,6-dioxopiperidin-3-yl)-5-fluoroisoindoline-1,3-dione | 4-((4-((4,4-difluoropiperidin-1-yl)methyl)-3-fluorobenzyl) thio)-2-(2,6-dioxopiperidin-3-yl)-5-fluoroisoindoline-1,3-dione |
| | 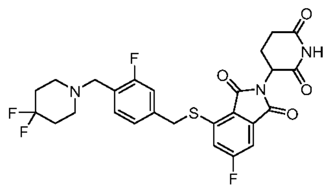 | 4-((4-((4,4-difluoropiperidin-1-yl)methyl)-3-fluorobenzyl) thio)-2-(2,6-dioxopiperidin-3-yl)-6-fluoroisoindoline-1,3-dione | 4-((4-((4,4-difluoropiperidin-1-yl)methyl)-3-fluorobenzyl) thio)-2-(2,6-dioxopiperidin-3-yl)-6-fluoroisoindoline-1,3-dione |
| | 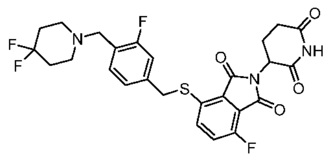 | 4-((4-((4,4-difluoropiperidin-1-yl)methyl)-3-fluorobenzyl) thio)-2-(2,6-dioxopiperidin-3-yl)-7-fluoroisoindoline-1,3-dione | 4-((4-((4,4-difluoropiperidin-1-yl)methyl)-3-fluorobenzyl) thio)-2-(2,6-dioxopiperidin-3-yl)-7-fluoroisoindoline-1,3-dione |
| | 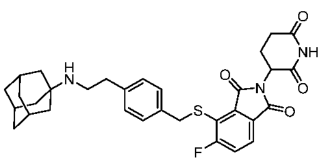 | 4-((4-(2-((adamantan-1-yl) amino)ethyl)benzyl) thio)-2-(2,6-dioxopiperidin-3-yl)-5-fluoroisoindoline-1,3-dione | 4-((4-(2-((adamantan-1-yl)amino)ethyl)benzyl)th io)-2-(2,6-dioxopiperidin-3-yl)-5-fluoroi-soindoline-1,3-dione |
| | 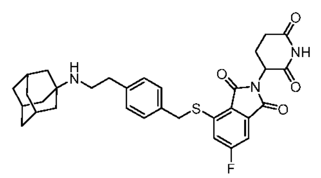 | 4-((4-(2-((adamantan-1-yl) amino)ethyl)benzyl) thio)-2-(2,6-dioxopiperidin-3-yl)-6-fluoroisoindoline-1,3-dione | 4-((4-(2-((adamantan-1-yl)amino)ethyl)benzyl)th io)-2-(2,6-dioxopiperidin-3-yl)-6-fluoroi-soindoline-1,3-dione |
| | 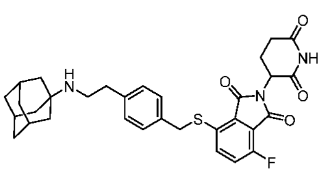 | 4-((4-(2-((adamantan-1-yl) amino)ethyl)benzyl) thio)-2-(2,6-dioxopiperidin-3-yl)-7-fluoroisoindoline-1,3-dione | 4-((4-(2-((adamantan-1-yl)amino)ethyl)benzyl)th io)-2-(2,6-dioxopiperidin-3-yl)-7-fluoroi-soindoline-1,3-dione |
| | 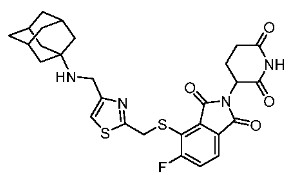 | 4-(((4-(((adamantan-1-yl)amino)methyl)thiazol-2-yl)methyl) thio)-2-(2,6-dioxopiperidin-3-yl)-5-fluoroisoindoline-1,3-dione | 4-(((4-(((adamantan-1-yl)amino)methyl)thiazol-2-yl)methyl) thio)-2-(2,6-dioxopiperidin-3-yl)-5-fluoroisoindoline-1,3-dione |

(continued)

| Compound No. | Structure of the compounds | The compounds' name | Chinese translation of the compounds' name |
|---|---|---|---|
| | 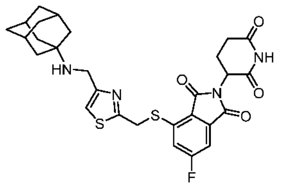 | 4-(((4-(((adamantan-1-yl)amino)methyl)thiazol-2-yl)methyl)thio)-2-(2,6-dioxopiperidin-3-yl)-6-fluoroisoindoline-1,3-dione | 4-(((4-(((adamantan-1-yl)amino)methyl)thiazol-2-yl)methyl)thio)-2-(2,6-dioxopiperidin-3-yl)-6-fluoroisoindoline-1,3-dione |
| | 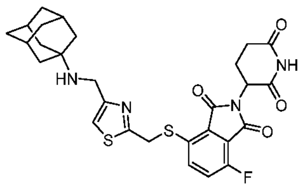 | 4-(((4-(((adamantan-1-yl)amino)methyl)thiazol-2-yl)methyl)thio)-2-(2,6-dioxopiperidin-3-yl)-7-fluoroisoindoline-1,3-dione | 4-(((4-(((adamantan-1-yl)amino)methyl)thiazol-2-yl)methyl)thio)-2-(2,6-dioxopiperidin-3-yl)-7-fluoroisoindoline-1,3-dione |

**II. Other Forms of Compounds** (including salts, enantiomers, stereoisomers, solvates, isotopically enriched analogs, prodrugs, or polymorphs of the compounds)

The compounds of the present disclosure have the structures of any one of Formula (I), Formula (I-1), Formula (I-2), Formula (I-3), Formula (I-4), Formula (Ia), Formula (Ib), Formula (Ic), Formula (Id), or Formula (Ie). Unless otherwise specified, all references to the compounds of the present disclosure also include compounds of any one of Formula (I), Formula (I-1), Formula (I-2), Formula (I-3), Formula (I-4), Formula (Ia), Formula (Ib), Formula (Ic), Formula (Id), or Formula (Ie) and specific compounds within the scope of these general formulae.

It should be recognized that compounds of the present disclosure (including compounds of Formula (I), Formula (I-1), Formula (I-2), Formula (I-3), Formula (I-4), Formula (Ia), Formula (Ib), Formula (Ic), Formula (Id), or Formula (Ie)) may have a stereo-configuration and thus can exist in more than one stereoisomeric form. The present disclosure also relates to optically enriched compounds having a stereo-configuration, e.g., greater than about 90% enantiomeric/diastereomeric excess ("ee"), such as about 95% ee or 97% ee, or greater than 99% ee, and mixtures thereof, including racemic mixtures. As used herein, "optically enriched" means that a mixture of enantiomers consists of a significantly greater proportion of one enantiomer, and can be described by enantiomeric excess (ee%). Purification of isomers and separation of mixtures of isomers can be accomplished by standard techniques known in the art (e.g., column chromatography, preparative TLC, preparative HPLC, asymmetric synthesis (e.g., by using chiral intermediates) and/or or chiral resolution, etc.).

In some embodiments, polymorph forms or salts of the compounds of the present disclosure are also provided. Salts of the compounds of the present disclosure can be pharmaceutically acceptable salts including, but not limited to, hydrochlorides, sulfates, citrates, maleates, sulfonates, citrates, lactates, tartrates, fumarates, phosphates, dihydrophosphates, pyrophosphates, metaphosphates, oxalates, malonates, benzoates, mandelates, succinates, trifluoroacetates, hydroxyacetates, or *p*-toluenesulfonates, etc. The compounds of the present disclosure can exist as non-solvated or solvated forms in pharmaceutically acceptable solvents such as water, ethanol, and the like. In some embodiments, the compounds of the present disclosure can be prepared as prodrugs or precursor drugs. Prodrugs can be converted into parent drugs in the body to play their role. In some embodiments, isotopically-labeled compounds of the present disclosure are also provided, examples of which include deuterium (D or $^2$H).

**III. Compositions/Formulations**

In some embodiments, the present disclosure provides a pharmaceutical composition comprising as an active ingredient the compound of the present disclosure or a pharmaceutically acceptable salt, solvate, isotopically enriched analog, polymorph, prodrug, stereoisomer (including enantiomer), or mixture of stereoisomers thereof, and at least one pharmaceutically acceptable carrier.

In some embodiments, pharmaceutically acceptable carriers include, but are not limited to, fillers, stabilizers, dispersants, suspending agents, diluents, excipients, thickeners, colorants, solvents, or encapsulating materials. Each carrier must be "acceptable" in the sense of being compatible with the other ingredients of the formulation (including the compounds useful in the present disclosure) and not injurious to the patient. Some examples of materials that can be used as pharmaceutically acceptable carriers include: sugars such as lactose, glucose, and sucrose; starches such as corn starch and potato starch; cellulose and its derivatives such as sodium carboxymethyl cellulose, ethyl cellulose and

cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; polyethylene oxide, polyvinylpyrrolidone, polyacrylamide, poloxamer; and other common non-toxic compatible substances used in pharmaceutical formulations.

The pharmaceutical composition of the present disclosure can further comprise at least one second therapeutic agent, e.g., an anticancer agent. The second therapeutic agent may be used in combination with the compounds of Formula (I) of the present disclosure to treat the diseases or disorders as disclosed herein. The second therapeutic agent includes, but is not limited to, chemotherapeutic agents, immunotherapeutic agents, gene therapeutic agents, anti-angiogenic agents, immunomodulators, and the like.

The pharmaceutical composition of the present disclosure comprising, as an active ingredient, the compounds of Formula (I) of the present disclosure or a pharmaceutically acceptable salt thereof can be formulated into any suitable formulations such as sprays, patches, tablets (such as conventional tablets, dispersible tablets, orally disintegrating tablets), capsules (such as soft capsules, hard capsules, enteric-coated capsules), dragees, troches, powders, granules, powder injections, suppositories, or liquid formulations (such as suspensions (e.g., aqueous or oily suspensions), solutions, emulsions, or syrups), or conventional injection dosage forms such as injectable solutions (e.g., sterile injectable solutions formulated according to methods known in the art using water, Ringer's solution, or isotonic sodium chloride solution or the like as a vehicle or solvent) or lyophilized injectable formulation and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). Those skilled in the art can also formulate the compounds of Formula (I) of the present disclosure into conventional, dispersible, chewable, orally disintegrating or rapidly dissolving formulations, or sustained-release capsules or controlled-release capsules as needed.

The compounds of Formula (I) of the present disclosure, as an active ingredient, is contained in the pharmaceutically acceptable carrier or diluent in an amount sufficient to deliver to a subject a therapeutically effective amount for the indication to be treated, without causing serious toxic effects in the subject treated. A dosage of the active compound for all diseases or disorders mentioned herein ranges, for example, from about 5ng/kg to 500mg/kg, from about 10ng/kg to 300mg/kg per day, such as from 0.1 to 100 mg/kg, or from 0.5 to about 25mg per kilogram body weight of the subject per day.

The compounds of Formula (I) of the present disclosure or pharmaceutically acceptable salts thereof can be conveniently administered in any suitable unit dosage form. Suitable unit dosage form specifications include, but are not limited to, less than 1mg, 1mg to 3000mg, 5mg to 1000mg, for example, 5 to 500mg, 25 to 250mg of active ingredient per unit dosage form.

## IV. Kits/Packaged Products

The compounds of Formula (I) or a pharmaceutically acceptable salt, solvate, isotopically enriched analog, polymorph, prodrug, stereoisomer (including enantiomer), or mixture of stereoisomers thereof is used as a medicament. The medicament of the present disclosure or the pharmaceutical composition of the present disclosure may be presented in a kit/packaged product. The kit/packaged product may include a package or container including, but not limited to, ampoules, blister packs, pharmaceutical plastic bottles, vials, pharmaceutical glass bottles, containers, syringes, laminated flexible packaging, co-extruded film infusion containers, test tubes and dispensing devices, and the like. The kit/packaged product may contain instructions for use of the product.

## V. Methods and Uses

The compounds of Formula (I) or a pharmaceutically acceptable salt, solvate, isotopically enriched analog, polymorph, prodrug, stereoisomer (including enantiomer), or mixture of stereoisomers thereof can be used as a medicament. Especially the compounds of Formula (I) or a pharmaceutically acceptable salt, solvate, isotopically enriched analog, polymorph, prodrug, stereoisomer (including enantiomer), or mixture of stereoisomers thereof can be used for the manufacture of a medicament for the prevention and/or treatment of diseases or disorders associated with cereblon protein. The diseases or disorders associated with cereblon protein comprise: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes. In some embodiments, the diseases or disorders associated with cereblon protein include,

but are not limited to, tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes. In some embodiments, the diseases or disorders associated with cereblon protein include, but are not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; Burkitt's lymphoma; thyroid cancer; melanoma; lung cancer, including lung adenocarcinoma, lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; neuroglioma; Peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

The present disclosure provides a method for preventing and/or treating diseases or disorders associated with cereblon protein, comprising administering to a subject a therapeutically effective amount of the compounds of Formula (I) of the present disclosure or a pharmaceutically acceptable salt thereof, or the pharmaceutical composition of the present disclosure comprising as an active ingredient the compounds of Formula (I) of the present disclosure or a pharmaceutically acceptable salt thereof. In some embodiments, the diseases or disorders associated with cereblon protein comprise: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes. In some embodiments, the diseases or disorders associated with cereblon protein include, but are not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; Burkitt's lymphoma; thyroid cancer; melanoma; lung cancer, including lung adenocarcinoma, lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; neuroglioma; Peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell

carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

In the method for preventing and/or treating diseases or disorders associated with cereblon protein, the compound of Formula (I) of the present disclosure or the pharmaceutical composition comprising as an active ingredient the compound of Formula (I) of the present disclosure is administered to the subject through at least one mode of administration selected from the group consisting of nasal administration, inhalation administration, topical administration, oral administration, oral mucosal administration, rectal administration, pleural cavity administration, peritoneal administration, vaginal administration, intramuscular administration, subcutaneous, transdermal, epidural, intrathecal, and intravenous administration.

The term "treatment" or "treating" refers to the administration of the compound of Formula (I) or a pharmaceutically acceptable salt thereof according to the present disclosure, or the pharmaceutical composition containing, as an active ingredient, the compound of Formula (I) or a pharmaceutically acceptable salt thereof, to a subject to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or alleviating the condition, and alleviating the disease.

A "therapeutic effective amount" of the compound of the present disclosure depends on a variety of factors, including the activity of the specific compound used, the metabolic stability of the compound and the duration of its action, the age, sex and weight of the patient, the patient's current medical condition, the route and duration of administration, the excretion rate, the combined administration of additional drugs, and the progression of the diseases or conditions of the patient being treated. Those skilled in the art will be able to determine appropriate dosages based on these and other factors.

It is to be understood that the choice of using one or more active compounds and/or compositions and their dosage depends on the basic situations of the individual (which should generally render the individual situation to achieve the best effect). Dosing and dosing regimens should be within the ability of those skilled in the art, and the appropriate dosage depends on many factors including the knowledge and ability of the physicians, veterinarians or researchers (see e.g., Jun Li (chief editor), "Clinical Pharmacology", 4th edition, People's Public Health Press, 2008).

As used herein, the term "patient" or "subject" to be treated refers to animal, for example mammal, including but not limited to primate (such as human being), cow, sheep, goat, horse, dog, cat, rabbit, guinea pig, rat, mice, etc.

### VI. Definitions

Unless otherwise specified, the following words, phrases and symbols used herein generally have the meanings as described below.

In general, the nomenclature used herein (including the IUPAC nomenclature) and the laboratory procedures described below (including those used in cell culture, organic chemistry, analytical chemistry, and pharmacology, etc.) are those well-known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or a noun in the claims and/or the specification may mean "one", but it is also consistent with the meaning of "one or more", "at least one", and "one or more than one". Similarly, the terms "another" or "other" can mean at least a second or more.

It should be understood that whenever the term "comprise" or "include" is used herein to describe various aspects, other similar aspects described by "consisting of" and/or "consisting essentially of" are also provided.

As used herein, the term "about" used alone or in combination refers to approximately, roughly, nearly, or around. When the term "about" is used in conjunction with a numerical range, it modifies that range by extending the boundaries

above and below the stated numerical value. In general, the term "about" can modify a numerical value above and below the stated value by an upward or downward (increasing or decreasing) variation, e.g., 10%, 5%, 2%, or 1%.

As used herein, the term "optionally substituted" used alone or in combination means that the referenced group may be unsubstituted or substituted with one or more substituents as defined here. Herein, the wording "optionally substituted with..." and "unsubstituted or substituted" can be used interchangeably. The term "substituted" generally indicates that one or more hydrogens in the referenced structure are replaced by the same or different specific substituents. The number of substituents is not theoretically limited in any way, or is automatically limited by the size of the building units (i.e., the total number of replaceable hydrogen atoms in the building units), or as clearly defined herein.

As used herein, the expression "a hydrogen of one or more $CH_2$ of the linear or branched $C_{x-y}$ alkylene is replaced with......", used alone or in combination, means that a hydrogen atom of any one or more $CH_2$ of the linear or branched $C_{x-y}$ alkylene is replaced by a substituent(s) as defined herein. Herein, the term "one or more" of "a hydrogen atom of one or more $CH_2$ of groups $-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$, $-(CH_2)_{15}-$, $-(CH_2)_{16}-$, $-(CH_2)_{17}-$, $-(CH_2)_{18}-$, $-(CH_2)_{19}-$, $-(CH_2)_{20}-$, $-(CH_2)_{21}-$, $-(CH_2)_{22}-$, $-(CH_2)_{25}-$, $-(CH_2)_{30}-$, $-(CH_2)_{35}-$, $-(CH_2)_{40}-$, $-(CH_2)_{45}-$, $-(CH_2)_{50}-$, $-(CH_2)_{55}-$, or $-(CH_2)_{60}-$" may refer to part or all hydrogen atoms of each referenced alkylene group, including but not limited to 1-60 hydrogens. In some embodiments, the expression "a hydrogen atom of one or more $CH_2$" may refer to part or all of the hydrogen atoms of the referenced alkylene group, including but not limited to 1-30, such as 1-25, 1-20, 1-15, 1-10, 1-5, 1-4, 1-3, 1-2 or 1 hydrogen atoms. In some embodiments, the expression "a hydrogen atom of one or more $CH_2$" may include 1-3 of the plurality of hydrogen atoms of the referenced alkylene group. The number of hydrogens to be replaced is in principle not limited in any way, or is automatically limited by the size of the building unit.

As used herein, the term "oxo", used alone or in combination, refers to = O.

As used herein, the term "C(O)" or "C(=O)" or "C=O", used alone or in combination, refers to carbonyl.

As used herein, the term "interrupted" of the expression "one or more pairs of adjacent carbon atoms in the backbone carbon chain of the linear or branched alkylene is optionally interrupted by one or more groups selected from the group consisting of: $R_b$, $R_c$, and any combination thereof", used alone or in combination, has a known definition in the art, which can mean that one or more groups $R_b$, one or more groups $R_c$, or any combination of one or more groups $R_b$ and $R_c$ are optionally inserted between one or more pairs of adjacent carbon atoms in the backbone carbon chain of the linear or branched alkylene group. Herein, examples of the above-mentioned expression "one or more pairs of adjacent carbon atoms in the backbone carbon chain...... is optionally interrupted by one or more groups selected from the group consisting of ... " may include, but are not limited to, that one or more (e.g., 1-30, 1-20, or 1-15, 1-10, 1-9, 1-8, 1-7, 1-6, 1-5, 1-4, 1-3, or 1-2, or 1) groups $R_b$ as defined herein and/or one or more (e.g., 1-30, 1-20, or 1-15, 1-10, 1-9, 1-8, 1-7, 1-6, 1-5, 1-4, 1-3, or 1-2, or 1) groups $R_c$ as defined herein and/or one or more (e.g., 1-30, 1-20, or 1-15, 1-10, 1-9, 1-8, 1-7, 1-6, 1-5, 1-4, 1-3, or 1-2, or 1) combinations of $R_b$ with $R_c$ are inserted into the backbone carbon chain, and the resulting backbone chain group conforms to the covalent bond theory. When two or more groups $R_b$ are inserted into the backbone carbon chain of the linear or branched alkylene group, the two or more groups $R_b$ are not directly connected to each other. For example, the expression "one or more pairs of adjacent carbon atoms in the backbone carbon chain of the linear or branched $C_{2-40}$ alkylene is optionally interrupted by one or more groups selected from the group consisting of: $R_b$, $R_c$, and any combination thereof" refers to that one or more (e.g., 1-30, 1-20, or 1-15, 1-10, 1-9, 1-8, 1-7, 1-6, 1-5, 1-4, 1-3, or 1-2, or 1) groups $R_b$ as defined herein and/or one or more (e.g., 1-30, 1-20, or 1-15, 1-10, 1-9, 1-8, 1-7, 1-6, 1-5, 1-4, 1-3, or 1-2, or 1) groups $R_c$ as defined herein and/or one or more (e.g., 1-30, 1-20, or 1-15, 1-10, 1-9, 1-8, 1-7, 1-6, 1-5, 1-4, 1-3, or 1-2, or 1) combinations of $R_b$ with $R_c$ are inserted between one or more (e.g., 1-30, 1-20, 1-15, 1-10, 1-8, 1-7, 1-6, 1-5, 1-4, 1-3, 1-2 or 1) pairs of any two adjacent carbon atoms of the backbone carbon chain of the linear or branched $C_{2-40}$ alkylene group, resulting in the formation of a backbone chain group containing one or more (e.g., 1-30, 1-20, 1-15, 1-10, 1-8, 1-7, 1-6, 1-5, 1-4, 1-3, 1-2 or 1) fragments "$-CH_2-R_b-CH_2-$" and/or one or more (e.g., 1-30, 1-20, 1-15, 1-10, 1-8, 1-7, 1-6, 1-5, 1-4, 1-3, 1-2 or 1) fragments "$-CH_2-(R_c)_{n6}-CH_2-$" and/or one or more (e.g., 1-30, 1-20, 1-15, 1-10, 1-8, 1-7, 1-6, 1-5, 1-4, 1-3, 1-2 or 1) fragments "$-CH_2-(R_3-(R_c)_{n6})_{n7}-CH_2-$", where each $R_b$ group is the same or different, each $R_c$ group is the same or different, and is as defined herein; and n6 represents e.g., an integer of 1, 2, 3, 4, 5, 6, 7, 8, 9, or 10, and n7 represents e.g., an integer of 1, 2, 3, 4, 5, 6, 7, 8, 9, or 10.

Herein, a bond interrupted by a wavy line shows the point of attachment of the depicted group to the rest of the molecule. For example, the group of Formula ($G_1$) represented by $X_1$ depicted below

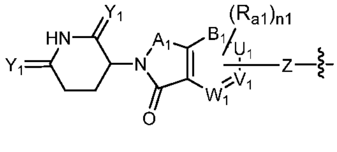

Formula ($G_1$)

shows the point of attachment of Z in the group of Formula ($G_1$) to L of the compound of Formula (I).

Herein, the following structural diagram indicates that the group "-R-L-$X_1$" and n substituents "$R_a$" are connected to any position of the isoindoline ring, meaning that the connection sites of the group "-R-L-$X_1$" and n substituents "$R_a$" on the isoindoline ring are variable.

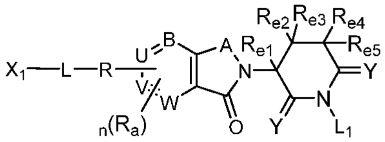

Formula (I)

In other words, the structural diagram of the compound of Formula (I) indicates that the structure of the compound has multiple positional isomers. In the compound of Formula (I), the group "-R-L-$X_1$" and n substituents "$R_a$" are connected to at least two of B, U, V, and W, meaning that at least two of B, U, V, and W represent C, and the remaining represent CH or N. Here, n represents an integer of 1, 2, or 3.

Although the symbol "*" in the group L in the present disclosure indicates the point of attachment of the group L to the group R, the present invention also encompasses embodiments wherein the symbol "*" in group L indicates the point of attachment of the group L to the group $X_1$.

As used herein, the term "halogen atom" or "halogen", used alone or in combination, refers to fluorine (F), chlorine (Cl), bromine (Br), or iodine (I).

As used herein, the term "alkyl", used alone or in combination, refers to a linear or branched alkyl group. The term "$C_x$-$C_y$ alkyl" or "$C_{x-y}$ alkyl" (x and y each being an integer) refers to a linear or branched alkyl group containing from x to y carbon atoms. The term "$C_{1-6}$ alkyl" used alone or in combination in the present disclosure refers to a linear or branched alkyl group containing from 1 to 6 carbon atoms, examples of which include $C_1$-$C_5$ alkyl, $C_1$-$C_4$ alkyl, and $C_1$-$C_3$ alkyl and the like. The term "$C_{1-4}$ alkyl" used alone or in combination in the present disclosure refers to a linear or branched alkyl group containing from 1 to 4 carbon atoms. Examples of the $C_{1-6}$ alkyl of the present disclosure may include a $C_1$-$C_5$ alkyl, $C_1$-$C_4$ alkyl, $C_1$-$C_3$ alkyl, $C_1$-$C_2$ alkyl and methyl. Representative examples of the term "$C_{1-6}$ alkyl" include methyl, ethyl, n-propyl, isopropyl, n-butyl, isobutyl, sec-butyl, tert-butyl, pentyl, isopentyl, neopentyl, tert-amyl, and hexyl. The term "$C_{1-4}$ alkyl" used alone or in combination in the present disclosure refers to a linear or branched alkyl group containing from 1 to 4 carbon atoms. Examples of the $C_{1-4}$ alkyl of the present disclosure may include $C_1$-$C_4$ alkyl, $C_1$-$C_3$ alkyl, $C_1$-$C_2$ alkyl and methyl. Representative examples of the term "$C_{1-4}$ alkyl" include methyl, ethyl, n-propyl, isopropyl, n-butyl, isobutyl, sec-butyl, and tert-butyl. The term "$C_{1-3}$ alkyl" or "$C_1$-$C_3$ alkyl" in the present disclosure refers to an alkyl group containing from 1 to 3 carbon atoms, and representative examples of which include methyl, ethyl, n-propyl, and isopropyl. In the present disclosure, the "alkyl" is optionally substituted with one or more substituents optionally selected from the group consisting of D (i.e., deuterium), halogen, hydroxy, cyano, $C_{1-3}$ alkyl, $C_{1-3}$ alkoxy, trifluoromethyl, $C_{5-10}$ aryl, $C_{5-10}$ heteroaryl, $C_{3-12}$ cycloalkyl, heterocyclyl (e.g., 3- to 12-membered heterocyclyl), or a combination thereof.

As used herein, the term "halogenated alkyl" or "haloalkyl", used alone or in combination, refers to a linear or branched alkyl group substituted with one or more halogens, wherein one or more hydrogen atom(s) of the alkyl group are replaced with one or more halogens. The term "halogenated $C_{x-Cy}$ alkyl" or "halogenated $C_{x-y}$ alkyl" (x and y are each an integer) refers to a linear or branched alkyl containing from x to y carbon atoms substituted with one or more halogens. The term "halogenated $C_{1-6}$ alkyl" used alone or in combination in the present invention refers to a linear or branched alkyl group containing from 1 to 6 carbon atoms substituted with one or more halogens. Examples of the halogenated $C_{1-6}$ alkyl group of the present disclosure include halogenated $C_{1-6}$alkyl, halogenated $C_{1-5}$alkyl, halogenated $C_{1-4}$alkyl, halogenated $C_{1-3}$alkyl, halogenated $C_{1-2}$alkyl, and halogenated methyl. Representative examples of "halogenated $C_{1-6}$ alkyl" include halomethyl, haloethyl, halo-n-propyl, haloisopropyl, halo-n-butyl, haloisobutyl, halo-sec-butyl, halo-tert-butyl, halopentyl, haloisoamyl, haloneopentyl, halo-tert-amyl, and halohexyl, particularly including, for example, $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- and $CH_2ClCH_2$-. The term "halogenated $C_{1-3}$alkyl" or "halo-$C_1$-$C_3$ alkyl" of the present disclosure refers to an alkyl group containing from 1 to 3 carbon atoms substituted with one or more halogens, and its representative examples include halomethyl, haloethyl, halo-n-propyl and haloisopropyl, particularly including, for example, $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-, and $CH_2ClCH_2$-.

As used herein, the term "deuterated", used alone or in combination, means that one or more hydrogen atom(s) of the referenced group are replaced with deuterium atoms (i.e., D).

As used herein, the term "deuterated $C_{x-Cy}$ alkyl" or "deuterated $C_{x-y}$ alkyl" (x and y are each an integer) refers to a linear or branched alkyl containing from x to y carbon atoms substituted with one or more deuterium atoms. The term "deuterated $C_{1-6}$ alkyl" used alone or in combination in the present disclosure refers to a linear or branched alkyl group

containing from 1 to 6 carbon atoms substituted with one or more deuterium atoms. Examples of the deuterated $C_{1-6}$ alkyl group of the present disclosure include deuterated $C_{1-5}$ alkyl group, e.g., deuterated $C_{1-4}$ alkyl group, deuterated $C_{2-5}$ alkyl group, deuterated $C_{1-3}$ alkyl group, deuterated $C_{1-2}$ alkyl, and deuterated methyl. Representative examples of the deuterated $C_{1-6}$ alkyl group include perdeuterated methyl ($CD_3$), perdeuterated ethyl ($CD_3CD_2$), perdeuterated n-propyl, perdeuterated isopropyl, perdeuterated n-butyl, perdeuterated isobutyl, perdeuterated sec-butyl, perdeuterated tert-butyl, perdeuterated pentyl, perdeuterated isopentyl, perdeuterated neopentyl, perdeuterated tert-amyl, and perdeuterated hexyl. The term "deuterated $C_{1-3}$ alkyl" or "deuterated $C_1$-$C_3$ alkyl" of the present disclosure refers to an alkyl group containing from 1 to 3 carbon atoms substituted with one or more deuterium atoms, and its representative examples include perdeuterated methyl ($CD_3$) and perdeuterated ethyl ($CD_3CD_2$).

As used herein, the term "alkylene" (which is used interchangeably with "alkylene chain"), used alone or in combination, refers to a linear or branched divalent saturated hydrocarbon group composed of carbon and hydrogen atoms. The term "$C_x$-$C_y$ alkylene" or "$C_{x-y}$ alkylene" (x and y each being an integer) refers to a linear or branched alkylene group containing from x to y carbon atoms. The term "$C_1$-$C_{30}$ alkylene" or "$C_{1-30}$ alkylene" refers to a linear or branched alkylene group containing from 1 to 30 carbon atoms, examples of which include, but are not limited to $C_1$-$C_{29}$alkylene, $C_1$-$C_{28}$alkylene, $C_1$-$C_{27}$alkylene, $C_1$-$C_{26}$alkylene, $C_1$-$C_{25}$alkylene, $C_1$-$C_{24}$alkylene, $C_1$-$C_{23}$alkylene, $C_1$-$C_{22}$alkylene, $C_1$-$C_{21}$alkylene, $C_1$-$C_{20}$alkylene, $C_1$-$C_{19}$alkylene, $C_1$-$C_{18}$alkylene, $C_1$-$C_{17}$alkylene, $C_1$-$C_{16}$alkylene, $C_1$-$C_{15}$alkylene, $C_1$-$C_{14}$alkylene, $C_1$-$C_{13}$alkylene, $C_1$-$C_{12}$alkylene, $C_1$-$C_{11}$alkylene, $C_1$-$C_{10}$alkylene, $C_1$-$C_9$alkylene, $C_1$-$C_8$alkylene, $C_1$-$C_7$alkylene, $C_1$-$C_6$alkylene, $C_1$-$C_5$alkylene, $C_1$-$C_4$alkylene, $C_1$-$C_3$alkylene, or $C_1$-$C_2$alkylene. Representative examples include, but are not limited to, methylene, ethylene, propylene, isopropylene, butylene, isobutylene, sec-butylene, tert-butylene, n-pentylene, isopentylene, neopentylidene, tert-amylene, hexylene, heptylene, octylene, nonylene, decylene, undecylene, dodecylene, tridecylene, tetradecylene, pentadecylene, hexadecylene, heptadecylene, octadecylene, nonadecylene, eicosylene, heneicosylene, docosylene, tricosylene, tetracosylene, pentacosylene, hexacosylene, heptacosylene, octacosylene, nonacosylene, and triacontylene. In the present disclosure, the "alkylene" is optionally substituted with one or more substituents optionally selected from the group consisting of: halogen, cyano, $C_{1-3}$alkyl, $C_{1-3}$ alkoxy, trifluoromethyl, $C_{5-10}$ aryl, $C_{5-10}$ heteroaryl, $C_{3-12}$cycloalkyl, 3- to 12-membered heterocyclyl, or any combination thereof.

As used herein, the term "alkoxy", used alone or in combination, refers to a linear or branched alkoxy group having structural formula of alkyl-O-. Optionally, the alkyl portion of the alkoxy group may contain 1-10 carbon atoms. Representative examples of "alkoxy" include, but are not limited to, methoxy, ethoxy, propoxy, isopropoxy, n-butoxy, isobutoxy, tert-butoxy, pentyloxy, 2-pentyloxy, isopentyloxy, neopentyloxy, hexyloxy, 2-hexyloxy, 3-hexyloxy, 3-methylpentyloxy, etc. The term "$C_1$-$C_4$ alkoxy" or "$C_{1-4}$ alkoxy" refers to a linear or branched alkoxy group containing from 1 to 4 carbon atoms. Representative examples of $C_{1-4}$ alkoxy include, but are not limited to, methoxy, ethoxy, n-propoxy, isopropoxy, n-butoxy, isobutoxy, and tert-butoxy.

As used herein, the term "halogenated alkoxy", used alone or in combination, refers to a alkoxy group substituted with one or more halogen atoms. Optionally, the alkyl portion of the alkoxy group may contain 1-10 carbon atoms. Examples of "halogenated alkoxy" include, but are not limited to, halogenated $C_{1-6}$ alkoxy or halogenated $C_{1-4}$ alkoxy. Representative examples include, but are not limited to, $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, and $CH_2ClCH_2$-O-.

As used herein, the term "alkyl-NH-", used alone or in combination, refers to a linear or branched alkyl-NH-, wherein alkyl is as defined above. Optionally, the alkyl portion of the alkyl-NH- group may contain 1-6 carbon atoms, i.e., $C_{1-6}$ alkyl-NH-. Representative examples of "alkyl-NH-" include, but are not limited to, methyl-NH-, ethyl-NH-, n-propyl-NH-, isopropyl-NH-, n-butyl-NH-, isobutyl-NH-, tert-butyl-NH-, pentyl-NH-, and hexyl-NH-, etc. The term "$C_1$-$C_4$ alkyl-NH-" or "$C_{1-4}$ alkyl-NH-" refers to a linear or branched alkyl-NH- group containing from 1 to 4 carbon atoms. Representative examples of $C_{1-4}$alkyl-NH-include, but are not limited to, methyl-NH-, ethyl-NH-, n-propyl-NH-, isopropyl-NH-, n-butyl-NH-, isobutyl-NH-, and tert-butyl-NH-.

As used herein, the term "alkyl-NHC(O)-", used alone or in combination, refers to a linear or branched alkyl-NHC(O)- group, wherein alkyl is as defined above. Optionally, the alkyl portion of the alkyl-NHC(O)- group may contain 1-6 carbon atoms. The term "$C_{1-4}$ alkyl-NHC(O)-" or "$C_1$-$C_4$ alkyl-NHC(O)-" refers to a linear or branched alkyl-NHC(O)- group containing from 1 to 4 carbon atoms. Representative examples of $C_{1-4}$ alkyl-NHC(O)- include, but are not limited to, $CH_3$-NHC(O)-, $CH_3CH_2$-NHC(O)-, and $CH_3CH_2CH_2$-NHC(O)-.

As used herein, the term "alkyl-C(O)NH-", used alone or in combination, refers to a linear or branched alkyl-C(O) NH- group, wherein alkyl is as defined above. Optionally, the alkyl portion of the alkyl-C(O)NH- group may contain 1-6 carbon atoms. The term "$C_{1-4}$ alkyl-C(O)NH-" or "$C_1$-$C_4$ alkyl-C(O)NH-" refers to a linear or branched alkyl-C(O)NH- group containing from 1 to 4 carbon atoms. Representative examples of $C_{1-4}$ alkyl-C(O)NH- include, but are not limited to, $CH_3$-C(O)NH-, $CH_3CH_2$-C(O)NH-, and $CH_3CH_2CH_2$-C(O)NH-.

As used herein, the term "aryl", used alone or in combination, refers to a monovalent aromatic hydrocarbon group containing from 5 to 14 carbon atoms and optionally one or more fused rings, such as phenyl group, naphthyl group, or fluorenyl group. As used herein, the "aryl" is optionally substituted. The aryl group is optionally substituted one or more

times (e.g., 1-4, 1-3, or 1-2 times) with a substituent(s). For example, aryl is mono-, di-, or tri-substituted with a substituent(s) as defined above or optionally selected from e.g., optionally deuterated $C_1$-$C_6$alkyl, optionally deuterated $C_{3-6}$cycloalkyl, hydroxy, amino, mercapto, halogen, cyano, oxo, optionally deuterated $C_1$-$C_6$ alkoxy, $C_1$-$C_4$alkylamino, halogenated $C_1$-$C_6$alkyl, amino-substituted $C_{1-6}$alkylene, $C_{1-4}$alkyl-NHC(O)-, $C_{1-4}$alkyl-C(O)NH-, and any combination thereof.

As used herein, the term "arylene", used alone or in combination, refers to a divalent aromatic hydrocarbon group containing from 5 to 14 carbon atoms and optionally one or more fused rings, such as phenylene, naphthylene, or fluorenylene. As used herein, the "arylene" is optionally substituted. The arylene group is optionally substituted one or more times (e.g., 1-4, 1-3, or 1-2 times) with a substituent(s). For example, arylene is mono-, di-, or tri-substituted with a substituent(s) as defined above or optionally selected from e.g., deuterium, optionally deuterated $C_1$-$C_6$ alkyl, optionally deuterated $C_{3-6}$ cycloalkyl, hydroxy, amino, mercapto, halogen, cyano, oxo, optionally deuterated $C_1$-$C_6$ alkoxy, $C_1$-$C_4$ alkylamino, halogenated $C_1$-$C_6$ alkyl, amino-substituted $C_{1-6}$ alkylene, $C_{1-4}$ alkyl-NHC(O)-, $C_{1-4}$ alkyl-C(O)NH- and any combination thereof.

As used herein, the term "heteroaryl", used alone or in combination, refers to a 5- to 20-membered (e.g., 5- to 15-membered, 5- to 12-membered, 5- to 11-membered, 5- to 10-membered, 5- to 9-membered, 5-to 8-membered, 5- to 7-membered, 5- to 6-membered, 6- to 15-membered, or 6- to 9-membered) monocyclic, bicyclic, or polycyclic cyclic radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroaryl groups include bicyclic, tricyclic or tetracyclic heteroaryl groups, which contain one aromatic ring having one or more heteroatoms independently selected from O, S and N, and the remaining rings which may be a saturated, partially unsaturated or aromatic ring and can be carbocyclic ring or contain one or more heteroatoms independently selected from O, S and N. Examples of monocyclic heteroaryl groups include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, tetrazolyl, and triazinyl. Examples of bicyclic heteroaryl groups include, but are not limited to, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, oxazolopyridyl, furopyridyl, pteridyl, purinyl, pyridopyridyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl and imidazo[2,1-b]thiazolyl. Examples of tricyclic or polycyclic heteroaryl groups include, but are not limited to, acridinyl, benzindolyl, carbazolyl, dibenzofuranyl, and xanthyl. The heteroaryl group may be unsubstituted or substituted. A substituted heteroaryl refers to heteroaryl substituted one or more times (e.g., 1-4, 1-3, or 1-2 times) by a substituent(s) as defined above or optionally selected from the group consisting of $C_1$-$C_3$alkyl, $C_{3-6}$cycloalkyl, hydroxy, amino, mercapto, halogen, $C_1$-$C_3$ alkoxy, $C_1$-$C_3$alkylamino, halogenated $C_1$-$C_3$alkyl, amino $C_{1-3}$alkylene, $C_{1-3}$alkyl-NHC(O)-, $C_{1-3}$alkyl-C(O)NH-, cyano, or any combination thereof.

As used herein, the term "heteroarylene", used alone or in combination, refers to a 5- to 20-membered (e.g., 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) bivalent monocyclic, bicyclic, or polycyclic cyclic aromatic ring radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroarylene groups include bicyclic, tricyclic or tetracyclic heteroarylene groups, which contain one aromatic ring having one or more heteroatoms independently selected from O, S and N, and the remaining ring(s) which may be a saturated, partially unsaturated or aromatic ring and can be carbocyclic ring or contain one or more heteroatoms independently selected from O, S and N. Examples of monocyclic heteroarylene groups include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, tetrazolylene, and triazinylene. Examples of bicyclic heteroarylene groups include, but are not limited to, indolylene, isoindolylene, isoindolinylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, oxazolopyridylene, furopyridylene, pteridylene, purinylene, pyridopyridylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene. Examples of tricyclic or polycyclic heteroarylene groups include, but are not limited to, acridinylene, benzindolylene, carbazolylene, dibenzofuranylene, and xanthylene. The heteroarylene group may be unsubstituted or substituted. A substituted heteroarylene refers to heteroarylene substituted one or more times (e.g., 1-4, 1-3, or 1-2 times) by a substituent(s) as defined above or optionally selected from the group consisting of $C_1$-$C_3$alkyl, $C_{3-6}$cycloalkyl, hydroxy, amino, mercapto, halogen, $C_1$-$C_3$ alkoxy, $C_1$-$C_3$alkylamino, halogenated $C_1$-$C_3$alkyl, amino-substituted $C_{1-3}$alkylene, $C_{1-3}$alkyl-NHC(O)-, $C_{1-3}$alkyl-C(O)NH-, cyano, or any combination thereof.

As used herein, the term "cycloalkyl", used alone or in combination, refers to a saturated or partially unsaturated (i.e., containing one or more double bonds, but not having a fully conjugated π-electron system) monocyclic or bicyclic or polycyclic cyclic hydrocarbon radical, which in some embodiments contains from 3 to 20 carbon atoms (i.e., $C_{3-20}$ cycloalkyl), or from 3 to 15 carbon atoms (i.e., $C_{3-15}$ cycloalkyl), or from 3 to 12 carbon atoms (i.e., $C_{3-12}$ cycloalkyl), or from 3 to 11 carbon atoms (i.e., $C_{3-11}$ cycloalkyl), or from 3 to 10 carbon atoms (i.e., $C_{3-10}$ cycloalkyl), or from 3 to 8 carbon atoms ( i.e., $C_{3-8}$ cycloalkyl), or from 3 to 7 carbon atoms (i.e., $C_{3-7}$ cycloalkyl), or from 3 to 6 carbon atoms (i.e., $C_{3-6}$ cycloalkyl). The term "cycloalkyl" includes monocyclic, bicyclic, tricyclic or polycyclic cyclic hydrocarbon radical having from 3 to 20 carbon atoms. Representative examples of monocyclic cycloalkyl groups include, but are not limited to, cyclopropyl, cyclobutyl, cyclopentyl, cyclopentenyl, cyclohexyl, cyclohexenyl, cycloheptyl, and cyclooctyl. Bicyclic, tricyclic and polycyclic cycloalkyl groups include bridged cycloalkyl (e.g., $C_{5-20}$ bridged cycloalkyl, $C_{5-15}$ bridged cycloalkyl and $C_{7-15}$ bridged cycloalkyl), fused cycloalkyl (e.g., $C_{5-20}$ fused cycloalkyl, $C_{5-15}$ fused cycloalkyl, $C_{6-20}$ fused cycloalkyl, $C_{6-15}$ fused cycloalkyl, $C_{7-20}$ fused cycloalkyl, $C_{7-15}$ fused cycloalkyl and $C_{8-15}$ fused cycloalkyl) and spiro-cycloalkyl groups (e.g., $C_{5-20}$ spiro-cycloalkyl, $C_{5-15}$ spiro-cycloalkyl, $C_{6-20}$ spiro-cycloalkyl, $C_{6-15}$ spiro-cycloalkyl, $C_{7-20}$ spiro-cycloalkyl, $C_{7-15}$ spiro-cycloalkyl and $C_{8-15}$ spiro-cycloalkyl), representative examples of which include, but not limited to, decalinyl, octahydropentalenyl, octahydro-1H-indenyl, $C_{5-20}$ spiro-cycloalkyl, $C_{5-15}$ spiro-cycloalkyl, adamantanyl, nor-adamantanyl, bornyl, norbornyl (also named as bicyclo[2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted cyclohexyl. The substituents of the substituted "cycloalkyl" can be optionally one or more (e.g., 1-5, 1-4, 1-3, 1-2, or 1) substituents selected from the group consisting of deuterium, optionally deuterated $C_1$-$C_6$ alkyl, optionally deuterated $C_{3-6}$ cycloalkyl, hydroxy, amino, mercapto, halogen, cyano, oxo, optionally deuterated $C_1$-$C_6$ alkoxy, $C_1$-$C_4$ alkylamino, halogenated $C_1$-$C_6$ alkyl, amino-substituted $C_{1-6}$ alkylene, $C_{1-4}$ alkyl-NHC(O)-, $C_{1-4}$ alkyl-C(O)NH- and any combination thereof. Examples of "$C_{3-6}$ cycloalkyl" include, but are not limited to, cyclopropyl, cyclobutyl, cyclopentyl, cyclopentenyl, cyclohexenyl, and cyclohexyl.

As used herein, the term "cycloalkylene", used alone or in combination, refers to a saturated or partially unsaturated (i.e., containing one or more double bonds, but not having a fully conjugated π-electron system) monocyclic, bicyclic, or polycyclic divalent cyclic hydrocarbon radical, which in some embodiments contains from 3 to 20 carbon atoms (i.e., $C_{3-20}$ cycloalkylene), or from 3 to 15 carbon atoms (i.e., $C_{3-15}$ cycloalkylene), or from 3 to 12 carbon atoms (i.e., $C_{3-12}$ cycloalkylene), or from 3 to 11 carbon atoms (i.e., $C_{3-11}$ cycloalkylene), or from 3 to 10 carbon atoms (i.e., $C_{3-10}$ cycloalkylene), or from 3 to 8 carbon atoms ( i.e., $C_{3-8}$ cycloalkylene), or from 3 to 7 carbon atoms (i.e., $C_{3-7}$ cycloalkylene), or from 3 to 6 carbon atoms (i.e., $C_{3-6}$ cycloalkylene). The term "cycloalkylene" includes monocyclic, bicyclic, tricyclic or polycyclic cycloalkylene having from 3 to 20 carbon atoms. Representative examples of monocyclic cycloalkylene groups include, but are not limited to, cyclopropylene, cyclobutylene, cyclopentylene, cyclopentenylene, cyclohexylene, cyclohexenylene, cycloheptylene, and cyclooctylene. Bicyclic, tricyclic and polycyclic cycloalkylene groups include bridged cycloalkylene (e.g., $C_{5-20}$ bridged cycloalkylene, $C_{5-15}$ bridged cycloalkylene and $C_{7-15}$ bridged cycloalkylene), fused cycloalkylene (e.g., $C_{5-20}$ fused cycloalkylene, $C_{5-15}$ fused cycloalkylene, $C_{6-20}$ fused cycloalkylene, $C_{6-15}$ fused cycloalk-ylene, $C_{7-20}$ fused cycloalkylene, $C_{7-15}$ fused cycloalkylene and $C_{8-15}$ fused cycloalkylene) and spiro-cycloalkylene groups (e.g., $C_{5-20}$ spiro-cycloalkylene, $C_{5-15}$ spiro-cycloalkylene, $C_{6-20}$ spiro-cycloalkylene, $C_{6-15}$ spiro-cycloalkylene, $C_{7-20}$ spiro-cycloalkylene, $C_{7-15}$ spiro-cycloalkylene and $C_{8-15}$ spiro-cycloalkylene), representative examples of which include, but not limited to, decalinylene, octahydropentalenylene, octahydro-1H-indenylene, 2,3-dihydro-1H-indenylene, $C_{5-20}$ spiro-cycloalkylene, $C_{5-15}$ spiro-cycloalkylene, adamantanylene, noradamantanylene, and norbomylene (also named as bicyclo[2.2.1]heptylene by the IUPAC system). As used herein, the "cycloalkylene" is optionally mono- or poly-substituted, such as, but not limited to, 2,2-, 2,3-, 2,4-, 2,5-, or 2,6-disubstituted cyclohexylene. The substituents of the substituted "cycloalkylene" can be optionally one or more substituents selected from the group consisting of deuterium, optionally deuterated $C_1$-$C_6$ alkyl, optionally deuterated $C_{3-6}$ cycloalkyl, hydroxy, amino, mercapto, halogen, cyano, oxo, optionally deuterated $C_1$-$C_6$ alkoxy, $C_1$-$C_4$ alkylamino, halogenated $C_1$-$C_6$ alkyl, amino-substituted $C_{1-6}$ alkylene, $C_{1-4}$ alkyl-NHC(O)-, $C_{1-4}$ alkyl-C(O)NH- and any combination thereof. Examples of "$C_{3-6}$ cycloalkylene" include, but are not limited to, cyclopropylene, cyclobutylene, cyclopentylene, cyclopentenylene, cyclohexenylene, and cyclohexylene.

As used herein, the term "$C_{x-y}$ spiro-cycloalkyl" (x and y each being an integer), used alone or in combination, refers to a spiro-cycloalkyl group containing from x to y carbon atoms. As used herein, the term "$C_{7-12}$ spiro-cycloalkyl", used alone or in combination, refers to a spiro-cycloalkyl group containing from 7 to 12 carbon atoms. The term "$C_{5-20}$ spiro-cycloalkyl" includes "$C_{5-15}$ spiro-cycloalkyl", "$C_{7-15}$ spiro-cycloalkyl" and "$C_{7-20}$ spiro-cycloalkyl". Representative examples of "$C_{7-11}$ spiro-cycloalkyl" include, but are not limited to, spiro[3.3]heptyl, spiro[2.5]octyl, spiro[3.5]nonyl, spiro [4.4]nonyl, spiro[4.5]decyl, or spiro[5.5]undecyl. The term "$C_{7-12}$ spiro-cycloalkyl" is optionally substituted with one or more substituents selected from the group consisting of deuterium, optionally deuterated $C_1$-$C_6$ alkyl, optionally deuterated $C_{3-6}$ cycloalkyl, hydroxy, amino, mercapto, halogen, cyano, oxo, optionally deuterated $C_1$-$C_6$ alkoxy, $C_1$-$C_4$ alkylamino, halogenated $C_1$-$C_6$ alkyl (e.g., trifluoromethyl), amino-substituted $C_{1-6}$ alkylene, $C_{1-4}$ alkyl-NHC(O)-, $C_{1-4}$ alkyl-C(O) NH- and any combination thereof.

As used herein, the term "$C_{x-y}$ spiro-cycloalkylene" (x and y each being an integer), used alone or in combination,

refers to a spiro-cycloalkylene group containing from x to y carbon atoms. As used herein, the term "$C_{7-12}$ spiro-cycloalkylene", used alone or in combination, refers to a spiro-cycloalkylene group containing from 7 to 12 carbon atoms (e.g., 7-10, 7-9 carbon atoms). The term "$C_{5-20}$ spiro-cycloalkylene" includes "$C_{5-15}$ spiro-cycloalkylene", "$C_{7-15}$ spiro-cycloalkylene" and "$C_{7-20}$ spiro-cycloalkylene". Representative examples of "$C_{7-12}$ spiro-cycloalkylene" include, but are not limited to, spiro[3.3]heptylene, spiro[2.5]octylene, spiro[3.5]nonylene, spiro[4.4]nonylene, spiro[4.5]decylene, or spiro [5.5]undecylene. The term "$C_{7-12}$ spiro-cycloalkylene" is optionally substituted with one or more substituents selected from the group consisting of deuterium, optionally deuterated $C_1$-$C_6$ alkyl, optionally deuterated $C_{3-6}$ cycloalkyl, hydroxy, amino, mercapto, halogen, cyano, oxo, optionally deuterated $C_1$-$C_6$ alkoxy, $C_1$-$C_4$ alkylamino, halogenated $C_1$-$C_6$ alkyl (e.g., trifluoromethyl), amino-substituted $C_{1-6}$ alkylene, $C_{1-4}$ alkyl-NHC(O)-, $C_{1-4}$ alkyl-C(O)NH- and any combination thereof.

As used herein, the term "heterocyclyl" or "heterocyclic group", used alone or in combination, refers to a 3- to 20-membered saturated or partially unsaturated (i.e., containing one or more double bonds, but not having a fully conjugated $\pi$-electron system) monocyclic, bicyclic, tricyclic or polycyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclyl" may refer to a 3- to 15-membered (e.g., 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated $\pi$-electron system) monocyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclyl groups include, but not limited to, monocyclic heterocyclyl (e.g., 4- to 20-membered monocyclic heterocyclyl, and 4- to 15-membered monocyclic heterocyclyl), bridged heterocyclyl (e.g., 5- to 20-membered bridged heterocyclyl, 5- to 15-membered bridged heterocyclyl, 7- to 20-membered bridged heterocyclyl, and 7- to 15-membered bridged heterocyclyl), fused heterocyclyl (e.g., 5- to 20-membered fused heterocyclyl, and 5- to 15-membered fused heterocyclyl), spiro-heterocyclyl groups (e.g., 5- to 20-membered spiro-heterocyclyl, and 5- to 15-membered spiro-heterocyclyl), cyclic ester group (i.e., (Lactone), such as 4- to 15-membered, 4- to 14-membered, 4- to 13-membered, 4- to 12-membered, 4- to 11-membered, 4- to 10-membered, 4- to 9-membered, 4- to 8-membered, 5- to 15-membered, 5- to 10-membered, 5- to 8-membered, 6- to 15-membered, or 6- to 10-membered cyclic ester group), and cyclic amide groups (i.e. lactams group, such as 4- to 15-membered, 4- to 14-membered, 4- to 13-membered, 4- to 12-membered, 4- to 11-membered, 4- to 10-membered, 4- to 9-membered, 4- to 8-membered, 5- to 15-membered, 5- to 10-membered, 5- to 8-membered, 6- to 15-membered, or 6- to 10-membered cyclic amide groups). Representative examples of the monocyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl) and diazacyclooctyl. Bicyclic, tricyclic and polycyclic heterocyclyl groups include bridged heterocyclyl, fused heterocyclyl and spiro-heterocyclyl groups, representative examples of which include, but not limited to, 6-azabicyclo[3.1.1] heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, and azaspirocycloalkyl (e.g., 5- to 20-membered azaspirocycloalkyl, such as 3-azaspiro[5.5]undecan-3-yl). The heterocyclyl may be unsubstituted or substituted as explicitly defined (e.g., mono-, di-, tri-, or poly-substituted) by a substituent(s) as defined above or optionally selected from the group consisting of deuterium, hydroxy, amino, mercapto, nitro, halogen, cyano, oxo, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, $C_{1-4}$ alkyl-NH-, $NH_2$-$C_{1-6}$ alkylene, $C_{1-4}$ alkyl-NHC(O)-, $C_{1-4}$ alkyl-C(O)NH- and any combination thereof.

As used herein, the term "heterocyclylene", used alone or in combination, refers to a 3- to 20-membered saturated or partially unsaturated (i.e., containing one or more double bonds, but not having a fully conjugated $\pi$-electron system) monocyclic, bicyclic, tricyclic or polycyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclylene" may refer to e.g., a 3- to 15-membered (optionally 3- to 14-membered, 3- to 12-membered, 3- to 11-membered, 3-to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated $\pi$-electron system) monocyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclylene groups include, but not limited to, monocyclic heterocyclylene (e.g., 4- to 20-membered monocyclic heterocyclylene, and 4-to 15-membered monocyclic heterocyclylene), bridged heterocyclylene (e.g., 5- to 20-membered bridged heterocyclylene, 5- to 15-membered bridged heterocyclylene, 7- to 20-membered bridged heterocyclylene, and 7- to 15-membered bridged heterocyclylene), fused heterocyclylene (e.g., 5- to 20-membered fused heterocyclylene, and 5- to 15-membered fused heterocyclylene), spiro-heterocyclylene groups (e.g., 5- to 20-membered spiro-heterocyclylene, and 5- to 15-membered spiro- heterocyclylene), bivalent cyclic ester group (i.e., (Lactone), such as 4- to 15-membered, 4- to 14-membered, 4- to 13-membered, 4- to 12-membered, 4-to 11-membered, 4- to 10-membered, 4- to 9-membered, 4- to 8-membered, 5- to 15-membered, 5- to 10-membered, 5- to 8-membered, 6-

to 15-membered, or 6- to 10-membered bivalent cyclic ester group), and bivalent cyclic amide groups (i.e. bivalent lactams group, such as 4- to 15-membered, 4- to 14-membered, 4-to 13-membered, 4- to 12-membered, 4- to 11-membered, 4- to 10-membered, 4- to 9-membered, 4- to 8-membered, 5- to 15-membered, 5- to 10-membered, 5- to 8-membered, 6- to 15-membered, or 6- to 10-membered bivalent cyclic amide groups). Representative examples of the monocyclic heterocyclylene include, but are not limited to, azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, and diazacycloheptanylene (e.g., 1,4-diazacycloheptanylene, 4,5-diazacycloheptanylene, 1,3-diazacycloheptanylene). Bicyclic, tricyclic and polycyclic heterocyclylene groups include bridged heterocyclylene, fused heterocyclylene and spiro-heterocyclylene groups, representative examples of which include, but not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 5- to 20-membered azaspirocycloalkylene, such as 3-azaspiro[5.5]undecanylene). The heterocyclylene may be unsubstituted or substituted as explicitly defined (e.g., mono-, di-, tri-, or poly-substituted) by a substituent(s) as defined above or optionally selected from the group consisting of deuterium, hydroxy, amino, mercapto, nitro, halogen, cyano, oxo, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, $C_{1-4}$ alkyl-NH-, $NH_2$-$C_{1-6}$ alkylene, $C_{1-4}$ alkyl-NHC(O)-, $C_{1-4}$ alkyl-C(O)NH- and any combination thereof.

As used herein, the term "alkynyl", used alone or in combination, refers to a linear or branched monovalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of alkynyl include, but are not limited to, ethynyl, 1-propynyl, 1-butynyl, and 1,3-diynyl.

As used herein, the term "alkenyl", used alone or in combination, refers to a linear or branched monovalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of alkenyl group include, but are not limited to, vinyl (e.g., -CH=CH-), 1-propenyl, allyl, 1-butenyl, 2-butenyl, 3-butenyl, isobutenyl, pentenyl, n-pent-2,4-dienyl, 1-methyl-but-1-enyl, 2-methyl-but-1-enyl, 3-methyl-but-1-enyl, 1-methyl-but-2-enyl, 2-methyl-but-2-enyl, 3-methyl-but-2-enyl, 1-methyl-but-3-enyl, 2-methyl-but-3-enyl, 3-methyl-but-3-enyl, and hexenyl.

As used herein, the term "quaternary ammonium salt group" used alone or in combination refers to a quaternary ammonium salt group having the Formula $N^+ R_aR_bR_cX^-$, where $R_a$, $R_b$, and $R_c$ each independently represent $C_{1-20}$ alkyl group, and $X^-$ represents $F^-$, $Cl^-$, $Br^-$, or $I^-$; or where $R_a$ and $R_b$ together with the N atom to which they are attached form a 4- to 8-membered heterocyclyl (especially a 5- to 8-membered heterocyclyl, or a 5- to 6-membered heterocyclyl), and the ring atoms of the 5- to 8-membered heterocyclyl optionally further include a heteroatom selected from oxygen, nitrogen, and sulfur, and $R_c$ represents a $C_{1-20}$ alkyl group, and $X^-$ represents $F^-$, $Cl^-$, $Br^-$, or $I^-$. The heterocyclyl in the 5- to 8-membered heterocyclyl may be as defined above, and the heteroatom(s) of the heterocyclyl optionally further includes an oxygen atom, a nitrogen atom, and/or a sulfur atom. Exemplary heterocyclyl includes, but is not limited to, piperidinyl, morpholinyl, imidazolyl, triazolyl, piperazinyl. Exemplary heterocyclyl quaternary ammonium salt groups include, but are not limited to

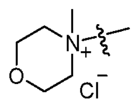 or 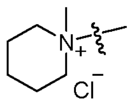 .

As used herein, "bornylane" or "bornane" (also known as 1,7,7-trimethylbicyclo[2.2.1]heptane; camphane; bomylane) has a definition known to those skilled in the art. As used herein, "camphanyl" or "bornyl" refers to a monovalent group of bornane, i.e., the group remaining after any one of the hydrogens in bornane is removed. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl,

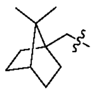 , or 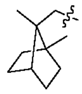 .

As used herein, "bicyclo[2.2.1]heptane" also known as "norbornane", has a definition known to those skilled in the art. As used herein, "bicyclo[2.2.1]heptyl" or "norbornyl" refers to a monovalent group of bicyclo[2.2.1]heptane, i.e., the group remaining after any hydrogen in bicyclo[2.2.1]heptane is removed. Representative examples of "bicyclo[2.2.1]heptyl" include, but are not limited to, bicyclo[2.2.1]heptan-2-yl, bicyclo[2.2.1]heptan-3-yl, bicyclo[2.2.1]heptan-4-yl, bicyclo[2.2.1]heptan-5-yl, or bicyclo[2.2.1]heptan-6-yl.

As used herein, the term "bicyclo[2.2.1]heptene" has a definition known to those skilled in the art. As used herein, "bicyclo[2.2.1]heptenyl" refers to a monovalent group of bicyclo[2.2.1]heptene, i.e., the group remaining after any hydrogen in bicyclo[2.2.1]heptene is removed. Representative examples of "bicyclo[2.2.1]heptenyl" include, but are not limited to, bicyclo[2.2.1]hept-5-en-2-yl, bicyclo[2.2.1]hept-5-en-3-yl, or bicyclo[2.2.1]hept-5-en-7-yl.

As used herein, "adamantane" (also known as tricyclo[3.3.1.1$^{3,7}$]decane) has a definition known to those skilled in the art, and its structural formula is e.g., as follows:

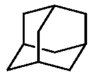.

As used herein, "adamantanyl" refers to a monovalent group of adamantane, that is, the group remaining after any hydrogen in adamantane is removed. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, "noradamantane" (also known as octahydro-2,5-methanopentalen) has a definition known to those skilled in the art, and its structural formula is e.g., as follows:

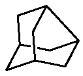 or 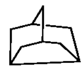.

As used herein, "noradamantanyl" refers to a monovalent group of noradamantane, that is, the group remaining after any hydrogen in noradamantane is removed. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl or 9-noradamantanyl.

In all embodiments of the present disclosure, the salts or pharmaceutically acceptable salts of the compounds of Formula (I), Formula (II) or Formula (III) refer to non-toxic inorganic or organic acid and/or base addition salts. Examples include: sulfates, hydrohalides (including hydrochlorides and hydrobromates), maleates, sulfonates, citrates, lactates, lactobionates, L-tartrates, fumarates, L-malates, L-lactates, α-ketoglutarates, hippurates, D-glucuronates, D-gluconates, α-D-glucoheptates, glycolates, mucates, L-ascorbates, orotates, picrates, glycinates, alaninates, argininates, cinnamates, laurates, pamoates, sebacates, benzenesulfonates, methanesulfonates, ethanesulfonates, ethanedisulfonates, formates, acetates, 2,2-dichloroacetates, trimethylacetates, propionates, valerates, palmitates, triphenylacetates, 2-ethyl-butanedioates, iodates, nicotiniates, L-pyroglutamates, L-prolinates, ferulates, 2-hydroxyethanesulfonates, nitrates, gentisates, cholates, salicylate, terephthalates, glutarates, adipates, stearates, oleoates, undecylenates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, pyruvates, p-chlorobenzenesulfonates, 1,5-naphthalenedisulfonates, 3-hydroxy-2-naphthoates, 1-hydroxy-2-naphthoates, 2-naphthalenesulfonates, glycolates, or *p*-toluenesulfonates, etc.

"Pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

As used herein, the term "room temperature" refers to the ambient temperature, such as 20-30°C.

As used herein, "stereoisomer" refers to a compound with the same chemical structural formula, but a different arrangement of atoms or groups in space. Stereoisomers include enantiomers, diastereomers, conformational isomers (rotational isomers), geometric isomers (cis/trans isomers), atropisomers, and so on.

As used herein, the term "solvate" refers to an association or complex formed by the interaction between one or more solvent molecules and compounds of the present invention. Examples of solvents include water, isopropanol, ethanol, methanol, DMSO, ethyl acetate, acetic acid and ethanolamine. The term "hydrate" means that a complex formed

with water.

As used herein, the term "chiral" refers to a molecule that is nonsuperimposable on its mirror image; whereas "achiral" refers to a molecule that can be superimposed on its mirror image.

As used herein, the term "enantiomers" refers to two isomers of a compound that are nonsuperimposable mirror images.

As used herein, the term "diastereomers" refers to stereoisomers that have two or more chiral centers but are not mirror images of each other. The diastereomers have different physical properties, such as melting point, boiling point, spectral properties and reactivity. The diastereomeric mixture can be separated by high-resolution analytical operations such as electrophoresis and chromatography, such as HPLC.

As used herein, the term "oxo", used alone or in combination, refers to = O.

As used herein, the term "C(O)" or "C(=O)" or "C=O", used alone or in combination, refers to carbonyl.

As used herein, the term "$C_{x-y}$ spiro-cycloalkyl" (x and y each being an integer), used alone or in combination, refers to a spiro-cycloalkyl group containing from x to y carbon atoms. As used herein, the term "$C_{7-11}$ spiro-cycloalkyl", used alone or in combination, refers to a spiro-cycloalkyl group containing from 7 to 11 carbon atoms. The term "$C_{5-15}$ spiro-cycloalkyl" includes "$C_{7-11}$ spiro-cycloalkyl", representative examples of which include, but are not limited to, spiro[3.3]heptyl, spiro[2.5]octyl, spiro[3.5]nonyl, spiro[4.4]nonyl, spiro[4.5]decyl, or spiro[5.5]undecyl. The term "$C_{7-11}$ spiro-cycloalkyl" is optionally substituted with one or more substituents selected from the group consisting of $C_1$-$C_3$ alkyl, halogenated $C_{1-3}$alkyl, $C_1$-$C_3$ alkoxy, deuterated $C_1$-$C_3$ alkoxy, $C_1$-$C_3$ alkylamino, amino, oxo, halogen, hydroxy, cyano, and any combination thereof.

As used herein, the term "$C_{x-y}$ bridged cycloalkyl" (x and y each being an integer), used alone or in combination, refers to a bridged cycloalkyl group containing from x to y carbon atoms. As used herein, the term "$C_{7-11}$ bridged cycloalkyl", used alone or in combination, refers to a bridged cycloalkyl group containing from 7 to 11 carbon atoms. Representative examples of "$C_{7-11}$ bridged cycloalkyl" include, but are not limited to, adamantanyl, noradamantanyl, norbornyl (also named as bicyclo[2.2.1]heptyl by the IUPAC system), and cubanyl. Herein, the term "bridged cycloalkyl" is optionally substituted with 1-10 substituents selected from the group consisting of $C_{1-3}$ alkyl, deuterated $C_{1-3}$ alkyl, $C_{1-3}$ alkoxy, halogen, halogenated $C_{1-3}$ alkyl, $C_{1-3}$ alkylamino, amino, hydroxy, cyano, oxo, or any combination thereof.

As used herein, "*p*-menthane" has the definitions known to those skilled in the art, and its structural formula is e.g., as follows:

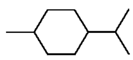.

As used herein, "*p*-menthanyl" refers to a monovalent group of menthane, that is, the group remaining after any hydrogen in p-menthane is removed. Representative examples of "*p*-menthanyl" include, but are not limited to,

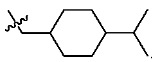, 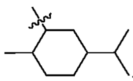, 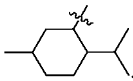, 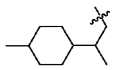,

or

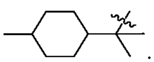.

As used herein, "*m*-menthane" has the definitions known to those skilled in the art, and its structural formula is e.g., as follows:

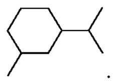.

As used herein, "*m*-menthanyl" refers to a monovalent group of *m*-menthane, that is, the group remaining after any hydrogen in *m*-menthane is removed. Representative examples of "*m*-menthanyl" include, but are not limited to,

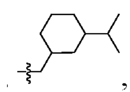, 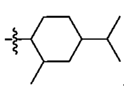, 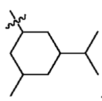, 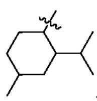, 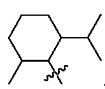,

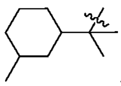, 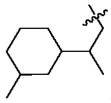, or 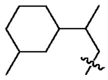.

As used herein, "Quinuclidine" (also known as 1-azabicyclo[2.2.2]octane) has the definitions known to those skilled in the art, and its structural formula is e.g., as follows:

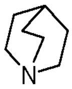.

As used herein, "quinuclidinyl" refers to a monovalent group of Quinuclidine, that is, the group remaining after any hydrogen in Quinuclidine is removed. Representative examples of "quinuclidinyl" include, but are not limited to,

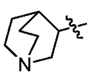, 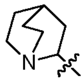, 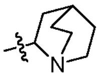, 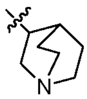, 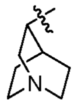, 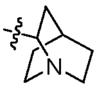, or 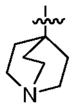.

## Examples

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present disclosure. Although the present disclosure will be described in conjunction with specific embodiments, it should be understood that this is not intended to limit the present disclosure to these embodiments.

The following abbreviations are used throughout the specification and examples:

| | |
|---|---|
| AcOH | acetic acid |
| BnCl | benzyl chloride |
| Bipy | bipyridine |
| BPO | benzoyl peroxide |
| *t*-BuONO | Tert-Butyl nitrite |
| DCE | 1,2-dichloroethane |
| DCM | dichloromethane |
| DIEA or DIPEA | N, N-diisopropylethylamine |
| DMF | N,N-dimethylformamide |
| DMSO | dimethyl sulfoxide |
| equiv | equivalent |
| ESI | electrospray ionization |
| EtOH | ethanol |
| HATU | 2-(7-Azabenzotriazol-1-yl)-N,N,N',N'-tetramethyluronium hexafluorophosphate |
| HOAc or AcOH | acetic acid |
| HPLC | high performance liquid chromatography |
| HRMS | high resolution mass spectrometry |
| LC-MS | liquid chromatography-mass spectrometry |
| LRMS | low resolution mass spectrometry |
| LC | liquid chromatography |
| MeOH | Methanol |
| MsCl | Methanesulfonyl chloride |
| MsO- | Methanesulfonyloxy |
| $^1$H NMR | Proton nuclear magnetic resonance |
| rt | room temperature |
| TEA | triethylamine |
| TFA | trifluoroacetic acid |
| THF | tetrahydrofuran |
| TLC | thin layer chromatography |

| | |
|---|---|
| TMS | tetramethylsilane |
| TsOH | *p*-toluenesulfonic acid |
| TsO- | Tosyloxy |

In the present disclosure, the $^1$H NMR spectrum was recorded on a Bruker-500MHz nuclear magnetic resonance instrument, by using, as a solvent, $CD_3OD$ ($\delta$ = 3.31 ppm) containing 0.1% TMS (as an internal standard); or using, as a solvent, $CDCl_3$ ($\delta$ = 7.26 ppm) containing 0.1% TMS (as an internal standard); or using, as a solvent, DMSO-$d_6$ ($\delta$ = 2.50 ppm) containing 0.03% TMS (as an internal standard). LC-MS spectrum was recorded on a Sciex API 2000 mass spectrometer equipped with an Agilent 1100 binary pump and DAD, as well as an ELSD, or on an Agilent 1260-6125B single quadrupole liquid-mass spectrometer equipped with an Agilent 1260 quadrupole pump, DAD, and ELSD; HPLC preparation was measured on a SHIMADZU LC-20AP type instrument, and HPLC purity was measured on a SHIMADZU LC-30AP or Waters 1525 type instrument. Unless otherwise specified, all reactions were performed in the air atmosphere. The reactions were monitored via TLC or LC-MS. The intermediates were separated and purified by column chromatography using ISCO or Biotage systems, or used directly in the next step without further purification. The final products obtained from the synthesis methods were separated and purified using conventional techniques in the field, such as preparative liquid chromatography with a Waters 2767 system.

Solvents and reagents are processed as follows:

the solvents used in the reactions such as DCM, DMF, anhydrous EtOH, and anhydrous MeOH were all purchased from Sinopharm Group; HPLC preparation uses preparation grade $CH_3CN$ and deionized water; Other reaction substrates, reagents and drugs can be commercially available without special instructions, or can be synthesized using or according to methods known in the art.

**General synthesis methods**

Compounds and/or pharmaceutically acceptable salts thereof of the present disclosure can be synthesized using commercially available raw materials by synthetic techniques known in the art. The synthetic schemes described below illustrate the preparation of most compounds. The starting materials or reagents used in each scheme can be purchased from commercial sources or prepared by methods known to those skilled in the art. One skilled in the art can prepare the salts, racemates, enantiomers, phosphates, sulfates, hydrochlorides and prodrug forms of the compounds of Formula (I) of the present disclosure according to routine techniques in the art.

**Scheme 1:**

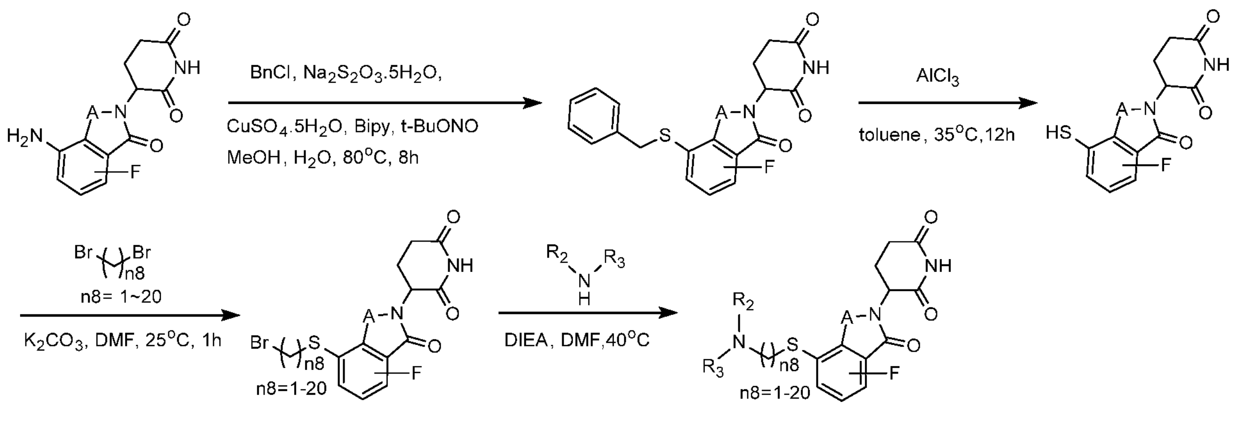


Scheme 1

In Scheme 1, A represents $CH_2$ or C(O); n8 represents an integer of 1 to 20; and $R_2$ and $R_3$ are as defined in the compounds of Formula (I) above and its various sub-embodiments described herein.

**Scheme 2:**

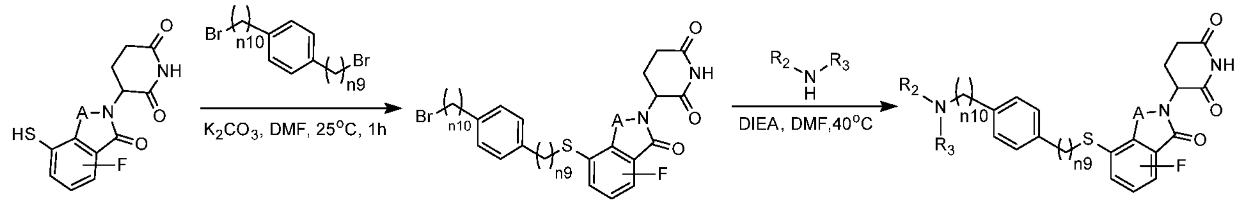


Scheme 2

In Scheme 2, A represents $CH_2$ or C(O); n9 and n10 each independently represent an integer of 1 to 20; and $R_2$ and $R_3$ are as defined in the compounds of Formula (I) above and its various sub-embodiments described herein. In Step 1 of Scheme 2, the phenyl group of the dibromide reaction substrate can also be appropriately modified or replaced, depending on the target compounds, with options such as optionally substituted cycloalkylene, optionally substituted heterocyclylene, or optionally substituted heteroarylene, as defined in the compounds of Formula (I) and its various sub-embodiments in the present disclosure.

**Scheme 3:**

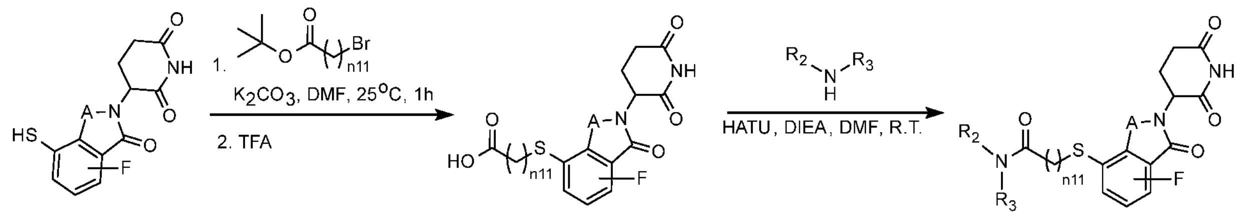


Scheme 3

In Scheme 3, A represents $CH_2$ or C(O); n11 represents an integer of 1 to 20; and $R_2$ and $R_3$ are as defined in the compounds of Formula (I) above and its various sub-embodiments described herein.

**Scheme 4:**

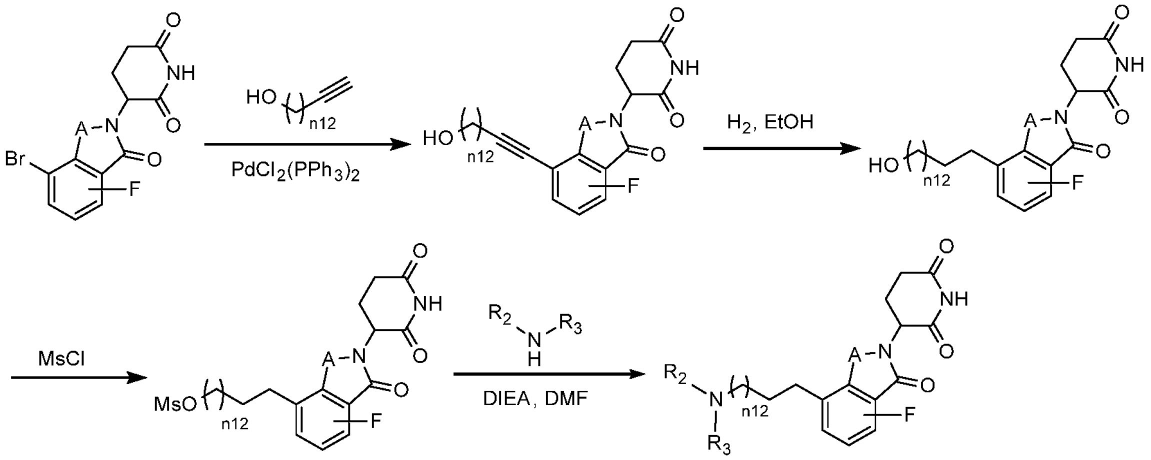


Scheme 4

In Scheme 4, A represents $CH_2$ or C(O); n12 represents an integer of 1 to 20; and $R_2$ and $R_3$ are as defined in the compounds of Formula (I) above and its various sub-embodiments described herein.

**Scheme 5:**

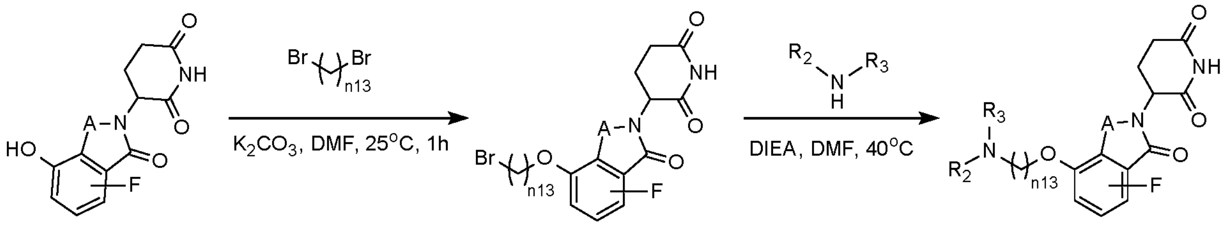


Scheme 5

In Scheme 5, A represents $CH_2$ or C(O); n13 represents an integer of 1 to 20; and $R_2$ and $R_3$ are as defined in the compounds of Formula (I) above and its various sub-embodiments described herein.

**Scheme 6:**

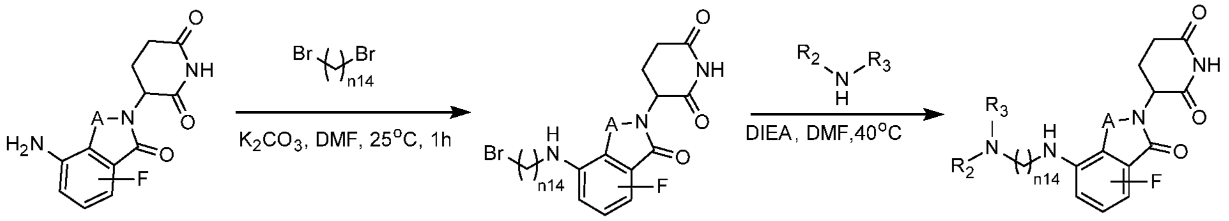


Scheme 6

In Scheme 6, A represents $CH_2$ or C(O); n14 represents an integer of 1 to 20; and $R_2$ and $R_3$ are as defined in the compounds of Formula (I) above and its various sub-embodiments described herein.

**Scheme 7:**

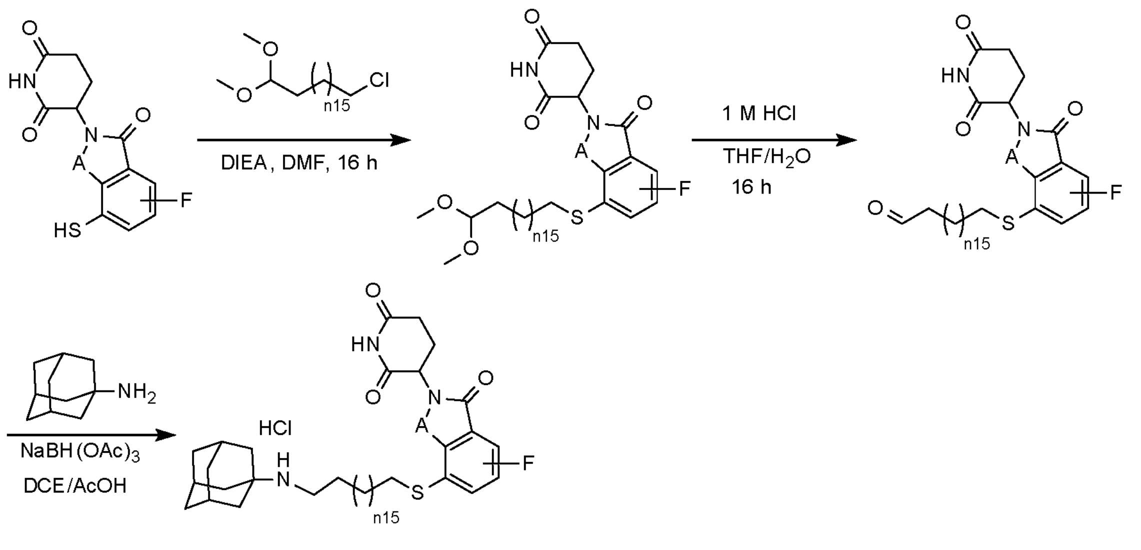


Scheme 7

In Scheme 7, A represents $CH_2$ or C(O), and n15 represents an integer of 0 to 20.

**Scheme 8:**

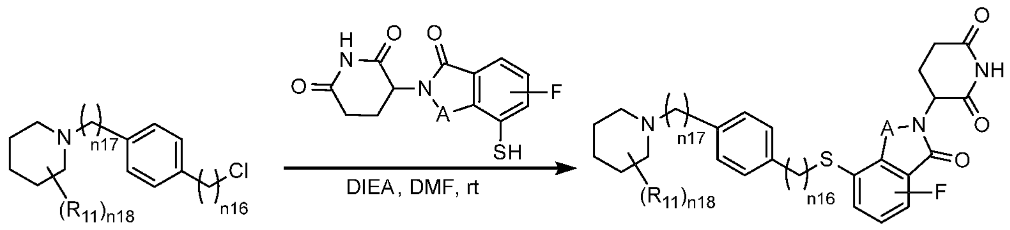


Scheme 8

In Scheme 8, A represents $CH_2$ or C(O); n16 and n17 each independently represent an integer of 1 to 20; $(R_{11})_{n18}$ denotes that the piperidinyl is substituted with n18 $R_{11}$, where n18 represents an integer of 0, 1, 2, 3, 4, 5 or 6; and $R_{11}$ represents fluorine.

**Scheme 9:**

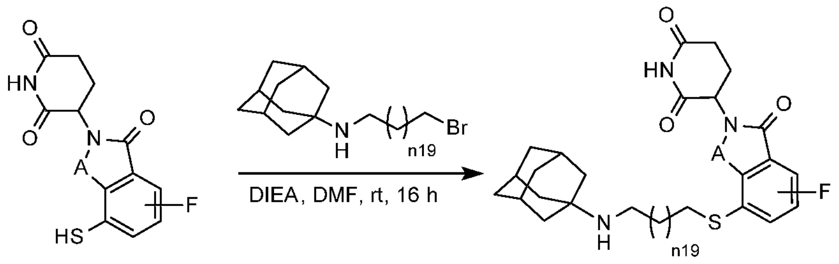


Scheme 9

In Scheme 9, A represents $CH_2$ or C(O), and n19 represents an integer of 0 to 20.

Depending upon the target compounds, the above schemes and their reaction substrates, reaction conditions (including reaction dosage, temperature, duration, etc.), work up, etc. can be appropriately modified and adjusted by techniques and methods well known to those skilled in the art to obtain the desired target compounds. The obtained target compounds can be further modified by the substituents and the like to obtain subsequent target compounds through methods well known to those skilled in the art.

## Examples

### Example 1: preparation of 3-(4-((4-((adamantan-1-yl)amino)butyl)thio)-6-fluoro-1-oxoisoindolin-2-yl)piperidine-2,6-dione (GT-04243)

The compound (GT-04243) was prepared by referring to the method of Scheme 7.

Step 1:

At room temperature, to a 100 mL single-neck flask were sequentially added 3-(6-fluoro-4-mercapto-1-oxoisoindolin-2-yl)piperidine-2,6-dione (300 mg, 1.019 mmol), 4-chloro-1,1-dimethoxybutane (155.58 mg, 1.019 mmol), DMF (5 mL) and DIEA (0.505 mL, 3.058 mmol). The reaction mixture was stirred at 25°C for 16 hours. After the reaction was completed as detected by TLC, the reaction was quenched with water. The reaction mixture was then extracted three times with ethyl acetate. The organic phases were combined, and washed three times with water and once with brine. The organic phase was concentrated under vacuum to yield 3-(4-((4,4-dimethoxybutyl)thio)-6-fluoro-1-oxoisoindolin-2-yl) piperidine-2,6-dione (yellow solid, 410 mg, yield 97.99%). $^1$H NMR (400 MHz, $d_6$-DMSO) δ 11.06 (s, 1H), 7.68 - 7.49 (m, 1H), 7.52 - 7.20 (m, 1H), 5.18 (dd, $J$ = 13.2, 5.2 Hz, 1H), 4.59 - 4.18 (m, 3H), 3.76 - 3.57 (m, 1H), 3.31 - 3.18 (m, 6H), 2.96 (t, $J$ = 12.8 Hz, 1H), 2.77 - 2.37 (m, 6H), 2.12 - 1.99 (m, 1H), 1.77 (d, $J$ = 42.1 Hz, 4H). LCMS (ESI): calcd for $C_{19}H_{23}FN_2O_5S^+$; $[M+Na+CH_3CN]^+$, 474.13; found, 474.2.

Step 2:

At room temperature, to a 100 mL single-neck flask were added 3-(4-((4,4-dimethoxybutyl)thio)-6-fluoro-1-oxoisoindolin-2-yl)piperidine-2,6-dione (410 mg, 0.999 mmol), THF (20 mL) and 1M hydrochloric acid (20 mL). The reaction mixture was stirred at 25°C for 2 hours. After the reaction was completed as detected by TLC, the reaction was quenched with water. The reaction mixture was then extracted four times with ethyl acetate. The organic phases were combined, and dried over anhydrous sodium sulfate, and concentrated under vacuum to give 4-((2-(2,6-dioxopiperidin-3-yl)-6-fluoro-1-oxoisoindolin-4-yl)thio)butanal (white solid, 320 mg, yield 87.92%). $^1$H NMR (400 MHz, $d_6$-DMSO) δ 10.99 (s, 1H), 9.68 (s, 1H), 7.56 (dd, *J* = 10.1, 1.9 Hz, 1H), 7.34 (dd, *J* = 7.4, 1.9 Hz, 1H), 5.12 (dd, *J* = 13.3, 5.2 Hz, 1H), 4.25 (dd, *J*= 57.1, 17.3 Hz, 2H), 3.15 (t, *J*= 7.4 Hz, 2H), 2.96 - 2.81 (m, 1H), 2.69 - 2.54 (m, 2H), 2.46 (dd, *J*= 13.3, 4.3 Hz, 1H), 2.07 - 1.97 (m, 1H), 1.94 - 1.72 (m, 2H), 1.36 (s, 2H).

Step 3:

At room temperature, to a 100 mL single-neck flask were added 4-((2-(2,6-dioxopiperidin-3-yl)-6-fluoro-1-oxoisoindolin-4-yl)thio)butanal (300 mg, 0.823 mmol), adamantan-1-amine (124.52 mg, 0.823 mmol), 1,2-dichloroethane (5 mL), sodium borohydride (436.22 mg, 2.058 mmol), and acetic acid (49.42 mg, 0.823 mmol). Under a nitrogen atmosphere, the reaction mixture was stirred at 25°C for 16 hours. After the reaction was completed as detected by TLC, to the reaction mixture were added dichloromethane and water, followed by extraction three times with dichloromethane. The organic phases were combined, dried over anhydrous sodium sulfate, and concentrated under vacuum to give a crude product. The crude product was separated and purified by preparative HPLC, then treated with water and lyophilized to afford the target compound GT-04243 (white solid, 170 mg, yield 38.53%). $^1$H NMR (400 MHz, $d_6$-DMSO) δ 11.01 (s, 1H), 8.74 (s, 2H), 7.55 (dt, *J* = 13.2, 6.6 Hz, 1H), 7.43 - 7.33 (m, 1H), 5.13 (dd, *J* = 13.3, 5.1 Hz, 1H), 4.38 - 4.15 (m, 2H), 3.21 (t, *J* = 6.8 Hz, 2H), 2.98 - 2.82 (m, 3H), 2.65 - 2.55 (m, 1H), 2.49 - 2.42 (m, 1H), 2.11 (s, 3H), 2.01 (dd, *J* = 10.4, 5.4 Hz, 1H), 1.86 (s, 6H), 1.79 - 1.54 (m, 10H). LCMS (ESI): calcd for $C_{27}H_{34}FN_3O_3S$; $[M+H]^+$; 500.24; found, 500.3.

**Example 2: preparation of 3-(6-fluoro-1-oxo-4-((4-(piperidin-1-ylmethyl)benzyl)thio)isoindolin-2-yl)piperidine-2,6-dione (GT-04117)**

The compound (GT-04117) was prepared by referring to the method of Scheme 8.

At room temperature, to a 100 mL single-neck flask were sequentially added 1-(4-(chloromethyl)benzyl) piperidine (200 mg, 0.894 mmol), 3-(6-fluoro-4-mercapto-1-oxoisoindolin-2-yl)piperidine-2,6-dione (263.07 mg, 0.894 mmol), DMF (5 mL) and DIEA (0.443 mL, 2.682 mmol). The reaction mixture was stirred at 25°C for 2 hours. After the reaction was completed as detected by TLC, the reaction was quenched with water. The reaction mixture was then extracted three times with ethyl acetate. The organic phases were combined, washed three times with water and once with brine, dried over anhydrous sodium sulfate, and concentrated under vacuum. The resulting residue was subjected to normal-phase column chromatography (eluent (v/v): ethyl acetate/petroleum ether = 0 - 80%) for separation and purification, yielding the target compound **GT-04117** (white solid, 371 mg, yield 86.18%). $^1$H NMR (400 MHz, $d_6$-DMSO) δ 11.00 (s, 1H), 10.61 (s, 1H), 7.62 - 7.51 (m, 3H), 7.45 (d, J = 8.1 Hz, 2H), 7.36 (dd, J = 7.5, 2.2 Hz, 1H), 5.10 (dd, J = 13.3, 5.1 Hz, 1H), 4.45 (s, 2H), 4.34 - 4.07 (m, 4H), 3.21 (d, J = 11.0 Hz, 2H), 2.96 - 2.85 (m, 1H), 2.83 - 2.70 (m, 2H), 2.59 (d, J = 16.9 Hz, 1H), 2.41 (dd, J = 13.0, 4.5 Hz, 1H), 2.04 - 1.94 (m, 1H), 1.88 - 1.59 (m, 5H), 1.31 (d, J = 7.3 Hz, 1H). LCMS (ESI): calcd for $[M+H]^+$; 482.19; found, 482.50.

**Example 3: preparation of 3-(4-((4-((4,4-difluoropiperidin-1-yl)methyl)benzyl)thio)-6-fluoro-1-oxoisoindolin-2-yl)piperidine-2,6-dione (GT-04024)**

The compound (GT-04024) was prepared by referring to the method of Scheme 8.

At room temperature, to a 100 mL single-neck flask were sequentially added 1-(4-(chloromethyl)benzyl)-4,4-difluoropiperidine (150 mg, 0.578 mmol), 3-(6-fluoro-4-mercapto-1-oxoisoindolin-2-yl)piperidine-2,6-dione (169.97 mg, 0.578 mmol), DMF (5 mL) and DIEA (0.286 mL, 1.733 mmol). The reaction mixture was stirred at room temperature for 2 hours. After the reaction was completed as detected by TLC, the reaction was quenched with water. The reaction mixture was then extracted three times with ethyl acetate. The organic phases were combined, washed three times with water and once with brine, dried over anhydrous sodium sulfate, and concentrated under vacuum. The resulting residue was subjected to normal-phase column chromatography (eluent (v/v): ethyl acetate/petroleum ether = 0 - 100%) for separation and purification, yielding the target compound **GT-04024** (white solid, 220 mg, yield 68.75%). $^1$H NMR (400 MHz, $d_6$-DMSO) δ 11.54 (s, 1H), 11.00 (s, 1H), 7.68 - 7.51 (m, 3H), 7.46 (d, J = 7.9 Hz, 2H), 7.37 (dd, J = 7.5, 2.1 Hz, 1H), 5.10 (dd, J = 13.3, 5.1 Hz, 1H), 4.45 (s, 2H), 4.40 - 4.18 (m, 3H), 4.12 (d, J = 17.3 Hz, 1H), 3.35 - 3.28 (m, 2H), 3.09 (s, 2H), 2.96 - 2.83 (m, 1H), 2.59 (d, J = 17.5 Hz, 1H), 2.48 - 2.37 (m, 2H), 2.33 (d, J = 18.2 Hz, 2H), 1.99 (dd, J = 8.9, 3.7 Hz, 1H). LCMS (ESI):

calcd for $[M+H]^+$, 518.17, found, 518.30.

**Example 4: preparation of 3-(4-((7-((adamantan-1-yl)amino)heptyl)thio)-6-fluoro-1-oxoisoindolin-2-yl)piperidine-2,6-dione (GT-04333)**

The compound (GT-04333) was prepared by referring to the method of Scheme 9.

At room temperature, to a 100 mL single-neck flask were sequentially added 3-(6-fluoro-4-mercapto-1-oxoisoindolin-2-yl)piperidine-2,6-dione (200 mg, 0.680 mmol), N-(7-bromoheptyl)adamantan-1-amine (223.13 mg, 0.680 mmol), DMF (5 mL) and DIEA (0.337 mL, 2.039 mmol). The reaction mixture was stirred at 25°C for 16 hours. TLC analysis indicated that the starting materials were completely consumed, with a significant amount of impurities generated. The reaction solution was separated by preparative HPLC, then lyophilized to afford the target compound **GT-04333** (white solid, 40 mg, yield 40%). $^1$H NMR (400 MHz, MeOD) δ 7.37 (d, *J* = 8.2 Hz, 1H), 7.29 (d, *J* = 7.3 Hz, 1H), 5.14 (dd, *J* = 13.4, 5.1 Hz, 1H), 4.56 (s, 4H), 4.36 (d, *J* = 9.2 Hz, 2H), 3.11 (dd, *J* = 7.1, 3.9 Hz, 2H), 2.90 (ddd, *J* = 36.5, 25.2, 18.7 Hz, 4H), 2.69 (s, 6H), 2.51 (dd, *J* = 13.1, 4.6 Hz, 1H), 2.19 (s, 4H), 1.90 (s, 6H), 1.84 - 1.59 (m, 10H), 1.46 (d, *J*= 41.5 Hz, 6H). LCMS (ESI): calcd for $C_{30}H_{40}FN_3O_3S^+$, $[M+H]^+$, 542.28; found, 542.3.

**Example 5: preparation of 3-(6-fluoro-4-((8-morpholinooctyl)thio)-1-oxoisoindolin-2-yl)piperidine-2,6-dione (GT-05341)**

Referring to the method of Scheme 1, the target product (GT-05341) was prepared as white solid (10 mg, yield 26.30%). $^1$H NMR (400 MHz, DMSO) δ 11.00 (s, 1H), 10.54 (s, 1H), 7.51 (dd, *J* = 10.2, 2.2 Hz, 1H), 7.34 (dd, *J* = 7.5, 2.2 Hz, 1H), 5.13 (dd, *J* = 13.3, 5.1 Hz, 1H), 4.32 (d, *J* = 17.2 Hz, 1H), 4.17 (d, *J* = 17.1 Hz, 1H), 3.94 (d, *J* = 10.7 Hz, 2H), 3.75 (t, *J* = 11.5 Hz, 2H), 3.39 (s, 2H), 3.15 (t, *J* = 6.6 Hz, 2H), 3.06 - 2.98 (m, 3H), 2.97 - 2.87 (m, 1H), 2.62 (t, *J* = 21.0 Hz, 2H), 2.48 - 2.39 (m, 1H), 2.04 - 1.96 (m, 1H), 1.62 (dt, *J* = 14.7, 7.1 Hz, 4H), 1.41 (s, 2H), 1.28 (s, 6H). LCMS (ESI) calcd for $C_{25}H_{35}FN_3O_4S^+$ $[M+H]^+$: 492.23, found, 492.3.

**Example 6: preparation of 3-(4-((8-(diethylamino)octyl)thio)-6-fluoro-1-oxoisoindolin-2-yl)piperidine-2,6-dione (GT-05342)**

Referring to the method of Scheme 1, the target product (GT-05342) was prepared as white solid (20 mg, yield 37.77%). $^1$H NMR (400 MHz, DMSO) δ 10.92 (s, 1H), 9.71 (s, 1H), 7.43 (dd, *J* = 10.1, 2.1 Hz, 1H), 7.27 (dd, *J* = 7.5, 2.0 Hz, 1H), 5.05 (dd, *J* = 13.3, 5.1 Hz, 1H), 4.25 (d, *J* = 17.3 Hz, 1H), 4.10 (d, *J* = 17.3 Hz, 1H), 3.31 (d, *J* = 8.6 Hz, 2H), 3.21 (s, 1H), 3.08 (t, *J* = 7.0 Hz, 2H), 3.00 (dt, *J* = 11.6, 5.8 Hz, 4H), 2.93 - 2.86 (m, 2H), 2.86 - 2.76 (m, 1H), 2.52 (d, *J* = 17.7 Hz, 2H), 2.38 (dd, *J* = 13.1, 4.3 Hz, 1H), 2.00 - 1.89 (m, 1H), 1.55 (dt, *J* = 14.4, 7.2 Hz, 4H), 1.35 (s, 2H), 1.22 (s, 6H). LCMS (ESI) calcd for $C_{25}H_{37}FN_3O_3S^+$ $[M+H]^+$: 478.25, found, 478.2.

**Example 7: preparation of 3-(6-fluoro-1-oxo-4-((7-oxo-7-(piperidin-1-yl)heptyl)thio)isoindolin-2-yl)piperidine-2,6-dione (GT-05343)**

Referring to the method of Scheme 3, the target product (GT-05343) was prepared as white solid (28 mg, yield 48.46%). $^1$H NMR (400 MHz, DMSO) δ 11.00 (s, 1H), 7.50 (dd, *J* = 10.2, 2.2 Hz, 1H), 7.33 (dd, *J* = 7.5, 2.1 Hz, 1H), 5.12 (dd, *J* = 13.2, 5.1 Hz, 1H), 4.32 (d, *J* = 17.3 Hz, 1H), 4.17 (d, *J* = 17.2 Hz, 1H), 3.40 (d, *J* = 5.6 Hz, 2H), 3.14 (t, *J* = 7.2 Hz, 2H), 2.95 - 2.84 (m, 1H), 2.58 (d, *J* = 17.8 Hz, 2H), 2.45 (dd, *J* = 13.1, 4.5 Hz, 1H), 2.25 (t, *J* = 7.4 Hz, 2H), 2.05 - 1.93 (m, 1H), 1.62 (dd, *J* = 14.4, 7.2 Hz, 2H), 1.55 (dd, *J* = 11.8, 6.6 Hz, 2H), 1.46 (s, 1H), 1.44 (d, *J* = 4.8 Hz, 2H), 1.43 - 1.34 (m, 4H), 1.29 (dt, *J* = 13.6, 6.8 Hz, 2H). LCMS (ESI) calcd for $C_{25}H_{33}FN_3O_4S^+$ $[M+H]^+$: 490.22, found, 490.2.

**Example 8: preparation of 3-(6-fluoro-1-oxo-4-((7-(piperidin-1-yl)heptyl)thio)isoindolin-2-yl)piperidine-2,6-dione (GT-05344)**

Referring to the method of Scheme 1, the target product (GT-05344) was prepared as white solid (5 mg, yield 13.15%). $^1$H NMR (400 MHz, DMSO) δ 11.00 (s, 1H), 9.81 (s, 1H), 7.51 (dd, *J* = 10.1, 2.1 Hz, 1H), 7.34 (dd, *J* = 7.5, 2.1 Hz, 1H), 5.13 (dd, *J* = 13.2, 5.0 Hz, 1H), 4.32 (d, *J* = 17.1 Hz, 1H), 4.17 (d, *J* = 17.3 Hz, 1H), 3.37 (s, 2H), 3.15 (t, *J* = 7.0 Hz, 2H), 2.93 (ddd, *J* = 21.2, 14.0, 6.9 Hz, 3H), 2.85 - 2.74 (m, 2H), 2.59 (d, *J* = 17.4 Hz, 1H), 2.43 (dd, *J* = 13.1, 8.9 Hz, 1H), 2.05 - 1.97 (m, 1H), 1.75 (d, *J* = 10.5 Hz, 3H), 1.70 (s, 2H), 1.67 - 1.58 (m, 4H), 1.45 - 1.38 (m, 2H), 1.36 - 1.21 (m, 5H). LCMS (ESI) calcd for $C_{25}H_{35}FN_3O_3S^+$ $[M+H]^+$: 476.24, found, 476.3.

**Example 9: preparation of 3-(4-((7-(4,4-difluoropiperidin-1-yl)-7-oxoheptyl)thio)-6-fluoro-1-oxoisoindolin-2-yl) piperidine-2,6-dione (GT-05407)**

Referring to the method of Scheme 3, the target product (GT-05407) was prepared as white solid (26 mg, yield 41.80%). $^1$H NMR (400 MHz, DMSO) δ 10.93 (s, 1H), 7.43 (dd, *J* = 10.1, 2.0 Hz, 1H), 7.26 (dd, *J*= 7.5, 1.9 Hz, 1H), 5.05 (dd, *J*= 13.3, 5.0 Hz, 1H), 4.25 (d, *J* = 17.3 Hz, 1H), 4.10 (d, *J* = 17.4 Hz, 1H), 3.50 - 3.44 (m, 4H), 3.07 (t, *J* = 7.2 Hz, 2H), 2.84 (dt, *J* = 12.5, 6.6 Hz, 1H), 2.52 (d, *J* = 17.6 Hz, 1H), 2.41 - 2.32 (m, 1H), 2.27 (t, *J* = 7.4 Hz, 2H), 1.93 (dd, *J* = 12.3, 5.3 Hz, 3H), 1.82 (dd, *J* = 16.2, 10.0 Hz, 2H), 1.59 - 1.51 (m, 2H), 1.42 (dd, *J* = 15.0, 7.4 Hz, 2H), 1.34 (dd, *J* = 15.3, 7.6 Hz, 2H), 1.27 - 1.19 (m, 2H). LCMS (ESI) calcd for $C_{25}H_{31}F_3N_3O_4S^+$ $[M+H]^+$: 526.20, found, 526.2.

**Example 10: preparation of 3-(4-((7-(4,4-difluoropiperidin-1-yl)heptyl)thio)-6-fluoro-1-oxoisoindolin-2-yl)piperidine-2,6-dione (GT-05408)**

Referring to the method of Scheme 1, the target product (GT-05408) was prepared as white solid (7.5 mg, yield 16.10%). $^1$H NMR (400 MHz, DMSO) δ 11.00 (s, 1H), 10.91 (s, 1H), 7.51 (dd, *J* = 10.2, 2.2 Hz, 1H), 7.34 (dd, *J* = 7.5, 2.2 Hz, 1H), 5.13 (dd, *J* = 13.2, 5.1 Hz, 1H), 4.32 (d, *J* = 17.1 Hz, 1H), 4.17 (d, *J* = 17.1 Hz, 1H), 3.56 (s, 2H), 3.15 (t, *J* = 7.1 Hz, 2H), 3.07 (s, 3H), 2.95 - 2.85 (m, 1H), 2.68 - 2.54 (m, 2H), 2.45 (dd, *J* = 13.2, 4.3 Hz, 2H), 2.40 - 2.26 (m, 3H), 2.05 - 1.98 (m, 1H), 1.67 (d, *J* = 8.9 Hz, 2H), 1.61 (dd, *J* = 14.6, 7.5 Hz, 2H), 1.41 (dd, *J* = 14.2, 6.4 Hz, 2H), 1.35 - 1.23 (m, 4H). LCMS (ESI) calcd for $C_{25}H_{33}F_3N_3O_3S+^+$ $[M+H]^+$: 512.22, found, 512.3.

**Example 11: preparation of 3-(4-((7-(cyclohexylamino)heptyl)thio)-6-fluoro-1-oxoisoindolin-2-yl)piperidine-2,6-dione (GT-05409)**

Referring to the method of Scheme 1, the target product (GT-05409) was prepared as white solid (7.3 mg, yield 16.32%). $^1$H NMR (400 MHz, DMSO) δ 11.00 (s, 1H), 8.59 (s, 2H), 7.51 (dd, *J*= 10.1, 2.2 Hz, 1H), 7.34 (dd, *J* = 7.5, 2.1 Hz, 1H), 5.13 (dd, *J* = 13.3, 5.1 Hz, 1H), 4.32 (d, *J* = 17.2 Hz, 1H), 4.17 (d, *J* = 17.2 Hz, 1H), 3.15 (t, *J*= 7.2 Hz, 2H), 3.00 - 2.88 (m, 2H), 2.87 (d, *J* = 5.5 Hz, 2H), 2.61 (s, 1H), 2.47 - 2.39 (m, 1H), 2.08 - 1.95 (m, 3H), 1.75 (d, *J* = 12.1 Hz, 2H), 1.68 - 1.53 (m, 5H), 1.42 (s, 2H), 1.25 (dd, *J* = 25.9, 13.8 Hz, 8H), 1.14 - 0.98 (m, 1H). LCMS (ESI) calcd for $C_{26}H_{37}FN_3O_3S^+$ $[M+H]^+$: 490.25, found, 490.3.

**Example 12: preparation of 3-(4-((7-((adamantan-1-yl)(methyl)amino)heptyl)thio)-6-fluoro-1-oxoisoindolin-2-yl) piperidine-2,6-dione (GT-05421)**

Referring to the method of Scheme 1, the target product (GT-05421) was prepared as white solid (8.5 mg, yield 16.89%). $^1$H NMR (400 MHz, DMSO) δ 11.00 (s, 1H), 9.23 (s, 1H), 7.51 (dd, *J* = 10.2, 2.1 Hz, 1H), 7.34 (dd, *J* = 7.5, 2.1 Hz, 1H), 5.13 (dd, *J* = 13.3, 5.1 Hz, 1H), 4.32 (d, *J* = 17.3 Hz, 1H), 4.17 (d, *J* = 17.4 Hz, 1H), 3.29 - 3.25 (m, 1H), 3.16 (t, *J* = 6.7 Hz, 2H), 2.97 - 2.83 (m, 1H), 2.64 (t, *J* = 9.9 Hz, 4H), 2.57 (s, 1H), 2.49 - 2.39 (m, 2H), 2.15 (s, 3H), 2.06 - 1.99 (m, 1H), 1.95 (d, *J* = 11.3 Hz, 3H), 1.91 - 1.83 (m, 3H), 1.61 (d, *J* = 12.9 Hz, 10H), 1.42 (s, 2H), 1.38 - 1.26 (m, 4H). LCMS (ESI) calcd for $C_{31}H_{43}FN_3O_3S^+$ $[M+H]^+$: 556.30, found, 556.3.

**Example 13: preparation of 3-(6-fluoro-1-oxo-4-((7-(spiro[3.3]heptan-2-ylamino)heptyl)thio)isoindolin-2-yl)piperidine-2,6-dione (GT-05422)**

Referring to the method of Scheme 1, the target product (GT-05422) was prepared as white solid (13 mg, yield 19.02%). $^1$H NMR (400 MHz, DMSO) δ 10.99 (s, 1H), 8.83 (s, 2H), 7.50 (dd, *J* = 10.1, 2.1 Hz, 1H), 7.34 (dd, *J* = 7.5, 2.1 Hz, 1H), 5.12 (dd, *J* = 13.3, 5.1 Hz, 1H), 4.31 (d, *J* = 17.3 Hz, 1H), 4.17 (d, *J* = 17.2 Hz, 1H), 3.49 (s, 1H), 3.15 (t, *J* = 7.1 Hz, 2H), 2.96 - 2.86 (m, 1H), 2.70 (s, 2H), 2.59 (d, *J* = 17.4 Hz, 1H), 2.43 (dd, *J* = 13.0, 8.7 Hz, 1H), 2.28 - 2.23 (m, 2H), 2.15 - 2.08 (m, 2H), 2.00 (dd, *J* = 14.0, 6.7 Hz, 4H), 1.91 (d, *J* = 7.6 Hz, 2H), 1.81 - 1.76 (m, 2H), 1.66 - 1.58 (m, 2H), 1.54 (s, 2H), 1.41 (s, 2H), 1.29 (s, 3H). LCMS (ESI) calcd for $C_{27}H_{37}FN_3O_3S^+$ $[M+H]^+$: 502.25, found, 502.3.

**Example 14: preparation of 3-(4-((4-(((adamantan-1-yl)amino)methyl)benzyl)thio)-6-fluoro-1-oxoisoindolin-2-yl)piperidine-2,6-dione (GT-05423)**

Referring to the method of Scheme 2, the target product (GT-05423) was prepared as white solid (8.8 mg, yield 35.87%). $^1$H NMR (400 MHz, DMSO) δ 11.00 (s, 1H), 8.82 (s, 2H), 7.60 (d, *J* = 9.1 Hz, 1H), 7.48 (d, *J*= 9.2 Hz, 4H), 7.35 (d, *J*= 7.2 Hz, 1H), 5.10 (dd, *J*= 13.1, 5.0 Hz, 1H), 4.51 - 4.35 (m, 2H), 4.28 (d, *J*= 17.5 Hz, 1H), 4.13 (d, *J* = 17.4 Hz, 1H), 4.06 (s, 2H), 2.93 - 2.85 (m, 1H), 2.59 (d, *J* = 17.0 Hz, 2H), 2.46 - 2.35 (m, 1H), 2.14 (s, 3H), 2.01 (s, 1H), 1.94 (s, 5H), 1.64 (dd, *J* = 31.0, 12.0 Hz, 6H). LCMS (ESI) calcd for $C_{31}H_{35}H_3O_3S^+$ $[M+H]^+$: 548.24, found, 548.2.

**Example 15: preparation of 3-(6-fluoro-1-oxo-4-((4-((((1R,2S,4R)-1,7,7-trimethylbicyclo[2.2.1]heptan-2-yl)amino)methyl)benzyl)thio)isoindolin-2-yl)piperidine-2,6-dione (GT-**05424)

Referring to the method of Scheme 2, the target product (GT-05424) was prepared as white solid (13 mg, yield 52.80%). $^1$H NMR (400 MHz, DMSO) δ 11.00 (s, 1H), 9.04 (s, 1H), 8.59 (s, 1H), 7.53 (d, *J* = 8.4 Hz, 3H), 7.47 (d, *J* = 8.1 Hz, 2H), 7.34 (d, *J* = 7.5 Hz, 1H), 5.11 (dd, *J* = 13.4, 4.8 Hz, 1H), 4.47 (s, 2H), 4.28 (d, *J* = 17.3 Hz, 1H), 4.13 (dd, *J* = 16.3, 9.4 Hz, 3H), 3.06 (s, 1H), 2.89 (d, *J* = 12.6 Hz, 1H), 2.64 (d, *J* = 25.3 Hz, 3H), 2.48 - 2.34 (m, 2H), 2.03 - 1.95 (m, 1H), 1.87 (s, 1H), 1.63 (d, *J* = 14.7 Hz, 3H), 1.36 (d, *J* = 7.8 Hz, 2H), 1.08 (d, *J* = 13.3 Hz, 1H), 0.86 - 0.78 (m, 6H), 0.68 (s, 3H). LCMS (ESI) calcd for $C_{31}H_{37}FN_3O_3S^+$ [M+H]$^+$: 550.25, found, 550.3.

**Example 16: preparation of 3-(6-fluoro-1-oxo-4-((7-(((1R,2S,4R)-1,7,7-trimethylbicyclo[2.2.1]heptan-2-yl)amino)heptyl)thio)isoindolin-2-yl)piperidine-2,6-dione (GT-05425)**

Referring to the method of Scheme 1, the target product (GT-05425) was prepared as white solid (16 mg, yield 32.44%). $^1$H NMR (400 MHz, DMSO) δ 11.00 (s, 1H), 8.62 (s, 1H), 8.21 (s, 1H), 7.56 - 7.44 (m, 1H), 7.34 (d, *J* = 7.5 Hz, 1H), 5.13 (dd, *J* = 13.4, 5.0 Hz, 1H), 4.32 (d, *J* = 17.3 Hz, 1H), 4.17 (d, *J* = 17.3 Hz, 1H), 3.17 (dd, *J* = 16.4, 9.2 Hz, 3H), 2.89 (dd, *J* = 12.4, 4.3 Hz, 3H), 2.59 (d, *J* = 17.1 Hz, 2H), 2.49 - 2.38 (m, 2H), 2.19 - 2.11 (m, 1H), 2.05 - 1.96 (m, 1H), 1.64 (dd, *J* = 16.2, 9.1 Hz, 7H), 1.40 (d, *J* = 9.4 Hz, 4H), 1.30 (s, 4H), 1.25 - 1.20 (m, 1H), 0.96 (s, 3H), 0.84 (s, 6H). LCMS (ESI) calcd for $C_{30}H_{43}FN_3O_3S^+$ [M+H]$^+$: 544.30, found, 544.3.

**Example 17: preparation of 3-(4-((4-(2-((adamantan-1-yl)amino)ethyl)benzyl)thio)-6-fluoro-1-oxoisoindolin-2-yl)piperidine-2,6-dione (GT-05431)**

Referring to the method of Scheme 2, the target product (GT-05431) was prepared as white solid (3.3 mg, yield 20.43%). $^1$H NMR (400 MHz, DMSO-d6) δ 11.01 (s, 1H), 8.71 (s, 1H), 7.60 (dd, *J* = 10.0, 2.2 Hz, 1H), 7.36 (dd, *J* = 11.2, 5.1 Hz, 3H), 7.27 - 7.16 (m, 2H), 5.11 (dd, *J* = 13.3, 5.1 Hz, 1H), 4.47 - 4.36 (m, 2H), 4.19 (dd, *J* = 49.7, 17.4 Hz, 2H), 3.05 (s, 2H), 2.95 - 2.80 (m, 3H), 2.58 (d, *J* = 15.8 Hz, 1H), 2.41 (dd, *J* = 12.8, 4.2 Hz, 1H), 2.10 (s, 3H), 2.04 - 1.95 (m, 1H), 1.85 (s, 6H), 1.62 (dd, *J* = 28.1, 11.9 Hz, 6H). LCMS (ESI) calcd for $C_{32}H_{37}FN_3O_3S^+$ [M+H]$^+$: 562.25, found, 562.3.

**Example 18: preparation of 3-(6-fluoro-4-((3-fluoro-4-(piperidin-1-ylmethyl)benzyl)thio)-1-oxoisoindolin-2-yl)piperidine-2,6-dione (GT-05443)**

Referring to the method of Scheme 2, the target product (GT-05443) was prepared as white solid (15 mg, yield 73.64%). $^1$H NMR (400 MHz, DMSO-d6) δ 11.01 (s, 1H), 10.15 (d, *J* = 88.6 Hz, 1H), 7.66 - 7.60 (m, 1H), 7.53 - 7.46 (m, 1H), 7.45 - 7.39 (m, 1H), 7.36 - 7.29 (m, 1H), 5.10 (dd, *J* = 13.3, 5.0 Hz, 1H), 4.44 (s, 1H), 4.35 - 4.17 (m, 3H), 4.10 (dd, *J* = 17.2, 10.0 Hz, 1H), 3.36 (s, 1H), 3.22 (t, *J* = 17.7 Hz, 3H), 2.99 - 2.70 (m, 3H), 2.58 (d, *J* = 17.3 Hz, 1H), 2.46 - 2.31 (m, 1H), 2.02 - 1.90 (m, 1H), 1.76 (s, 3H), 1.69 - 1.64 (m, 1H), 1.33 (d, *J* = 8.3 Hz, 1H). LCMS (ESI) calcd for $C_{26}H_{28}F_2N_3O_3S^+$ [M+H]$^+$: 500.18, found, 500.2.

**Example 19: preparation of 3-(4-((4-((4,4-difluoropiperidin-1-yl)methyl)-3-fluorobenzyl)thio)-6-fluoro-1-oxoisoindolin-2-yl)piperidine-2,6-dione (GT-05444)**

Referring to the method of Scheme 2, the target product (GT-05444) was prepared as white solid (15 mg, yield 69.01%). $^1$H NMR (400 MHz, DMSO-d6) δ 11.17 (s, 1H), 11.01 (s, 1H), 7.73 - 7.58 (m, 1H), 7.55 - 7.44 (m, 1H), 7.40 (ddd, *J* = 12.0, 7.5, 2.1 Hz, 1H), 7.33 (d, *J* = 7.1 Hz, 1H), 5.11 (dd, *J* = 13.3, 5.0 Hz, 1H), 4.46 (d, *J* = 12.7 Hz, 2H), 4.36 (s, 1H), 4.33 - 4.20 (m, 1H), 4.14 - 3.95 (m, 1H), 3.14 (d, *J* = 19.1 Hz, 2H), 2.98 - 2.82 (m, 1H), 2.58 (d, *J* = 16.4 Hz, 1H), 2.47 - 2.36 (m, 2H), 2.33 (s, 2H), 2.06 - 1.87 (m, 1H). LCMS (ESI) calcd for $C_{26}H_{26}F_4N_3O_3S^+$ [M+H]$^+$: 536.16, found, 536.2.

**Example 20: preparation of 3-(4-(8-((adamantan-1-yl)amino)octyl)-6-fluoro-1-oxoisoindolin-2-yl)piperidine-2,6-dione (GT-05617)**

Referring to the method of Scheme 4, the target product (GT-05617) was prepared as white solid (8.8 mg, yield 49.09%). $^1$H NMR (400 MHz, DMSO-d6) δ 11.03 (s, 1H), 8.54 (s, 2H), 7.42 - 7.29 (m, 2H), 5.14 (dd, *J* = 13.3, 5.0 Hz, 1H), 4.45 (d, *J* = 17.1 Hz, 1H), 4.30 (t, *J* = 14.1 Hz, 1H), 2.92 (dd, *J* = 21.6, 9.4 Hz, 1H), 2.81 (s, 2H), 2.69 - 2.59 (m, 3H), 2.47 - 2.34 (m, 1H), 2.11 (s, 3H), 2.05 - 1.97 (m, 1H), 1.84 (s, 6H), 1.66 (d, *J* = 12.5 Hz, 4H), 1.58 (d, *J* = 11.4 Hz, 6H), 1.30 (s, 8H). LCMS (ESI) calcd for $C_{31}H_{43}FN_3O_3$ + [M+H]$^+$: 524.33, found, 524.4.

**Example 21: preparation of 3-(4-((7-((adamantan-1-yl)amino)heptyl)amino)-6-fluoro-1-oxoisoindolin-2-yl)piperidine-2,6-dione (GT-05879)**

Referring to the method of Scheme 6, the target product (GT-05879) was prepared as white solid (3.0 mg, yield 13.35%). $^1H$ NMR (400 MHz, DMSO-d6) δ 8.21 (s, 2H), 6.56 (dd, *J* = 7.7, 2.2 Hz, 1H), 6.51 (dd, *J* = 11.8, 2.2 Hz, 1H), 6.07 - 5.42 (m, 1H), 5.06 (dd, *J* = 13.3, 5.1 Hz, 1H), 4.14 (d, *J* = 17.1 Hz, 1H), 3.99 (d, *J* = 16.8 Hz, 1H), 3.57 (s, 2H), 2.99 - 2.87 (m, 2H), 2.72 (s, 3H), 2.27 - 2.19 (m, 1H), 2.02 (d, *J* = 15.0 Hz, 4H), 1.74 (s, 6H), 1.59 (d, *J* = 12.9 Hz, 3H), 1.51 (d, *J* = 12.0 Hz, 4H), 1.40 - 1.33 (m, 2H), 1.23 (d, *J* = 10.4 Hz, 4H), 1.19 (d, *J* = 6.6 Hz, 3H). LCMS (ESI) calcd for $C_{30}H_{42}FN_4O_3^+$ $[M+H]^+$: 525.32, found, 525.4.

**Biological activity assay**

**Materials:**

| Reagents and materials | Suppliers or Source |
|---|---|
| Human diffuse large B-cell lymphoma cell line: TMD8 | Kyinno Biotechnology Co., Ltd |
| Human multiple myeloma cells: MM.1S | ATCC (American Type Culture Collection) |
| Human mantle cell lymphoma cells: Mino | ATCC |
| Human Burkitt's lymphoma cell line: Daudi | Dalian Meilun Biotech Co., Ltd |
| RPMI-1640 Medium | Dalian Meilun Biotech Co., Ltd |
| Meilun Cell Counting Kit-8 | Dalian Meilun Biotech Co., Ltd |
| Fetal bovine serum (FBS) | Sunrise Science Products Company |
| Penicillin-Streptomycin | Beijing TransGen Biotech Co., Ltd |
| Mycoplasma detection kit | Beijing TransGen Biotech Co., Ltd |
| STR genotype detection | Shanghai Biowing Applied Biotechnology Co. Ltd |

**Methods:**

**Cell cultures**

The tumor cells used herein were cultured in RPMI 1640 medium supplemented with 10% FBS, penicillin to a final concentration of 100 U/mL, and streptomycin to a final concentration of 100 μg/mL, at 37°C in an incubator with 5% $CO_2$. Prior to experimentation, all cells used were correctly identified by STR profiling, and tested negative for mycoplasma through routine screenings.

**I. Determination of half inhibitory concentration ($IC_{50}$) of the compounds against tumor cells:**

$IC_{50}$ values of the compounds of the present disclosure (including the compounds in Table 1, and Examples compounds) were measured using the Meilun Cell Counting Kit-8 kit from Dalian Meilun Biotech Co., Ltd. Assay details are as follows: the tumor cells were seeded into RPMI-1640 medium containing serum at a certain seeding density, wherein the seeding densities of the tumor cells are as follows: TMD8 cells, MM.1S cells, or Mino cells were seeded at a density of 10,000 cells/100 μL/well, and Daudi cells were seeded at a density of 4,000 cells/100 μL/well. After 24h, 100 μL of the inoculated cells were treated with 0.5μL of the compounds of the present disclosure (including the compounds in Table 1, and Examples compounds) in DMSO at 10 different successively decreasing concentrations (starting at a maximum concentration of 10μM; 5-fold serial dilutions). After the cells were treated with the compounds of the present disclosure for 96 hours, cell viability was measured according to the instructions provided with the CCK-8 kit. DMSO served as the negative control, and corresponding commercial immunomodulatory drugs (including lenalidomide (CAS No.: 191732-72-6) and pomalidomide (CAS No.: 19171-19-8)) were used as the positive controls. All controls were treated using the same protocol as the compounds of the present disclosure. The growth inhibition of the compounds of the present disclosure on the cells was plotted using Prism Graphpad software, and the $IC_{50}$ values of the compounds of the present disclosure were calculated. The results were shown in Table 2 below.

Table 2. $IC_{50}$ values (in nM) of the compounds of the present disclosure for inhibiting the proliferation of tumor cells

| Comp. No. | MM.1S cell | Mino cell | Daudi cell | TMD8 cell |
|---|---|---|---|---|
| lenalidomide | D | C | F | D |
| pomalidomide | B | B | D | B |
| GT-04117 | - | A+++ | C | A+ |
| GT-04024 | - | - | - | A++ |
| GT-05408 | - | A | - | - |
| GT-05343 | - | A+ | - | - |
| GT-05407 | A | A+ | - | - |
| GT-05409 | - | A+++ | - | - |
| GT-05421 | A+++ | A+++ | - | - |
| GT-05425 | A | A+++ | C | - |
| GT-05424 | A+ | A+++ | B | - |
| GT-05423 | A+++ | A+++ | - | - |
| GT-05422 | A+++ | A+++ | - | - |
| GT-05617 | - | A | - | - |
| GT-05444 | A+++ | A+++ | A+ | - |
| GT-05431 | A+++ | A+++ | A+++ | - |
| GT-05443 | A+ | A+++ | B | - |
| Note: In Table 2, the symbols A+++, A++, A+, A, B, C, D, and E are defined as follows: 0 nM < A+++ ≤ 5 nM; 5 nM < A++ ≤ 10 nM; 10 nM < A+ ≤ 50 nM; 50 nM < A ≤ 100 nM; 100 nM < B ≤ 500 nM; 500 nM < C ≤ 1000 nM; 1000 nM < D ≤ 5000 nM; 5000 nM < E ≤ 10000 nM; 10000 nM < F. The symbol "-" indicates not tested. | | | | |

The results indicated that the compounds of the present invention (including the compounds listed in Table 1 and the Examples compounds) can inhibit the proliferation of tumor cells (as shown in Table 2), with inhibitory effects superior to or comparable to those of the corresponding positive control drugs, and degradation effects superior to those of the corresponding positive control drugs. Furthermore, the results demonstrated that the compounds of the present invention (including the compounds listed in Table 1 and the Examples compounds) significantly inhibit the proliferation of Mino cells (human mantle cell lymphoma cells), MM.1S cells (human multiple myeloma cells), Daudi cells (human Burkitt's lymphoma cells), and TMD8 cells (human diffuse large B-cell lymphoma cells) (as shown in Table 2), with inhibitory effects superior to those of the corresponding positive control drugs.

**II. Determination of CRBN-binding affinity of compounds:**

The CEREBLON BINDING kits (specification: 10,000 tests; product number: Catalog # 64BDCRBNPEH; CISBIO company) was used to determine the CRBN binding ability of the compounds to be tested through a HTRF method (homogeneous time-resolved fluorescence). The specific methods are as follows:

**1.** According to the instructions of the CEREBLON BINDING kits, the compounds of the present invention to be tested and lenalidomide were serially diluted using diluent #9 (1X) solution to obtain a final concentration of 2 μM for both the tested compounds and lenalidomide solution.
**2.** 2.5 μL of the above 2 μM of the tested compounds and lenalidomide solution, as well as the same volume of diluent #9 (1X) solution (solvent control group, Std0) were added to each well of a 96-well plate, respectively. Then, 2.5 μL of human Cereblon WT GST-tagged protein solution was added to each well. Finally, 5 μL of the thoroughly mixed Thalidomide-Re reagent and GST Eu antibody working solution were added to each of the aforementioned wells. The final concentration of the tested compounds and lenalidomide in each well is 0.5 μM.
**3.** The blank control wells were sequentially added with 2.5 μL of diluent #9 (1X) solution, 2.5 μL of PROTAC binding buffer, and 5 μL of thoroughly mixed Thalidomide-Re reagent and GST Eu antibody working solution.
**4.** After sealing and incubating the solutions in the aforementioned wells at room temperature for 3 hours, the

absorbance values at emission wavelengths of 620 nm and 665 nm were detected by the HTRF method using a Spark microplate reader (V3.1 SP1).

The corresponding CRBN binding inhibition rate was calculated using the following method:

$$R_{compound} = OD\ 665\ nm_{compound} / OD\ 620\ nm_{compound} - OD\ 665\ nm_{control} / OD\ 620\ nm_{control}$$

$R_{Std0}$ = **OD** 665 nm $_{Std0}$ /OD 620 nm $_{Std0}$ - OD 665 nm $_{control}$ /OD 620 nm $_{control}$ inhibition rate (%) = (1 - R $_{compound}$ /$R_{Std0}$) * 100

Note: OD 665 nm $_{compound}$ is the absorbance value of each well of the compounds of the present disclosure to be tested at an emission wavelength of 665 nm.
OD 620 nm $_{compound}$ is the absorbance value of each well of the compounds of the present disclosure to be tested at an emission wavelength of 620 nm.
OD 665 nm $_{control}$ is the absorbance value of the blank control well at an emission wavelength of 665 nm.
OD 620 nm $_{control}$ is the absorbance value of the blank control well at an emission wavelength of 620 nm.
OD 665 nm $_{Std0}$ is the absorbance value of the solvent control well at an emission wavelength of 665 nm.
OD 620 nm $_{Std0}$ is the absorbance value of the solvent control well at an emission wavelength of 620 nm.

The test results were shown in Table 3 below.

Table 3. HTRF inhibitory activity ($IC_{50}$, μM) of the compounds of the present disclosure (including the compounds in Table 1 and the Examples compounds)

| Comp. No. | HTRF $IC_{50}$ (μM) | Comp. No. | HTRF $IC_{50}$ (μM) |
|---|---|---|---|
| lenalidomide | e | GT-05425 | a |
| GT-04117 | a | GT-05424 | a |
| GT-04024 | a | GT-05423 | a |
| GT-04243 | a | GT-05422 | a |
| GT-05341 | a | GT-05617 | a |
| GT-05342 | a | GT-05444 | a |
| GT-05344 | a | GT-05431 | a |
| GT-05408 | a | GT-05443 | a |
| GT-05343 | a | GT-05409 | a |
| GT-05407 | a | GT-05421 | a |
| GT-04333 | a | | |
| Note: In Table 3, the symbols a, b, c, d, e, f, g, and E are defined as follows: 0 μM < a ≤ 0.6 μM; 0.6 μM < b ≤ 1 μM; 1 μM < c ≤ 1.5 μM; 1.5 μM < d ≤ 2.0 μM; 2.0 μM < e < 2.5 μM; 2.5 μM < f ≤ 3 μM; g > 3 μM. | | | |

The results showed that the compounds of the present disclosure (including the compounds listed in Table 1 and the Examples compounds) exhibited strong binding affinity to CRBN.

### III. Western-blot assay

The effect of the compounds of the present disclosure on the expression of target proteins in cells was assessed using conventional Western blot analysis.

After culturing $1.5 \times 10^6$ hPBMC cells for 24 hours, the cells were treated with the compounds of the present disclosure (including the compounds listed in Table 1 and the Examples compounds) at concentrations of 0.1nM, 0.5nM, 10nM, 50nM, 500nM and 1000nM. DMSO was used as the negative control, and commercial immunomodulatory drugs (lenalidomide and pomalidomide) were used as positive controls. All controls were administered to the cells following the same protocol as the compounds of the present disclosure. After 24 hours of treatment with the compounds, the cells were harvested and lysed using RIPA protein lysate supplemented with phosphatase inhibitors. The lysate was incubated on ice

for 30-60 minutes and then centrifuged. The supernatant, containing the total cellular protein, was aspirated. The protein concentration of the supernatant was quantified using the conventional BCA (Bicinchoninic Acid) assay. Subsequently, the supernatant was mixed with 4X SDS sample loading buffer, denatured at 95°C for 5 minutes, and then subjected to SDS-PAGE electrophoresis on a polyacrylamide gel. The separated proteins were then transferred to a nitrocellulose membrane (NC membrane). The membrane was blocked with blocking buffer at room temperature for 1-2 hours, followed by antibody incubation and detection according to the manufacturer's instructions (Cell Signaling Technology).

Half-Maximal Degradation Concentration value (the drug concentration required for degrading 50% of the target protein, abbreviated as $DC_{50}$) was determined as follows: the grayscale values of the Western blotting bands from the drug-treated samples were compared with those from the DMSO control. The $DC_{50}$ value was identified as the drug concentration at which the grayscale value of the Western blotting bands from the drug-treated sample was equal to half of that from the DMSO control.

The $DC_{50}$ value can also be calculated as follows: using ImageJ software to quantify the grayscale values of the Western blotting bands from the drug-treated samples, fitting the concentration-response curve between drug concentrations and grayscale values, and calculating the drug concentration corresponding to half of the grayscale value of the Western blotting band for the DMSO control from the fitted curve.

The results showed that the compounds of the present disclosure (including the compounds in Table 1 and Example compounds) can significantly degrade substrate proteins (e.g., IKZF1/3 proteins, GSPT1 proteins, etc.), particularly the compound GT-05443 of the present disclosure, which can significantly degrade IKZF3 protein at concentrations below 10 nM and IKZF1 protein at concentrations below 0.1 nM. In contrast, lenalidomide does not degrade IKZF1/3 proteins and GSPT1 protein at concentrations of 50 nM and 500 nM, and pomalidomide does not degrade IKZF1/3 proteins and GSPT1 protein at a concentration of 50 nM (Figure 1). Western blotting results showed that, compared with lenalidomide and pomalidomide, the compounds of the present disclosure significantly improve activity in inhibiting tumor cells and inducing substrate protein degradation.

## IV. Pharmacokinetic Study of the compounds of the present disclosure

The pharmacokinetic properties of the compounds of the present disclosure were evaluated using conventional pharmacokinetic experimental methods.

The test compounds of the present disclosure (including the compounds listed in Table 1 and the Examples compounds) were orally administered to experimental animals to assess their pharmacokinetic properties. The experimental animals used can be those commonly used in pharmacokinetic experiments, e.g., adult and healthy rodents (e.g., mice, rats (such as Sprague-Dawley (SD) rats), guinea pigs) or non-rodents (rabbits, dogs, and monkeys). In the present disclosure, mice were used as the experimental animals.

The experimental animals were divided into two groups: a control group (for collecting blank plasma) and an orally administered group (dosed at 10 mg/kg or 30 mg/kg). Blood samples were collected at predetermined sampling time points after administration, such as 0.25 h, 0.5 h, 1 h, 2 h, 4 h, 8 h, and 24 h post-dosing. Blank plasma was collected from the control group.

Plasma samples were preprocessed before analysis: 10 μL of 50% acetonitrile and 200 μL of internal standard solution (containing 50 ng/mL dexamethasone in acetonitrile) were added to 10 μL of plasma samples. For the blank control group, the same volume of acetonitrile was added instead of the internal standard. The processed samples were vortexed, centrifuged, and the supernatant was taken for LC-MS/MS injection analysis.

After preparing standard curves and quality control (QC) samples, the concentrations of the compounds in the plasma samples were determined by LC-MS/MS analysis. Based on the plasma concentration data obtained from LC-MS/MS analysis, the pharmacokinetic parameters of the test compounds were calculated using the non-compartmental model in the pharmacokinetic calculation software WinNonlin v8.1. The results were listed in Table 4 below.

The results showed that the compounds of the present disclosure, administered orally, exhibited good pharmacokinetic (DMPK) properties and can be used as therapeutic agents for cancer patients.

## V. Effects of the compounds of the present disclosure on TNF-α, IL-10, IL-6, IL-1β, IFN-γ, GM-CSF, IL-2, and IL-12p40 release from PBMCs induced by Lipopolysaccharide (LPS) or Phytohemagglutinin (PHA)

The following materials were used in this study:

V.1 Reagents and Consumable materials

| Name | Supplier or Source |
|---|---|
| Human PBMC cell | Shanghai Sai Li |
| Lipopolysaccharide (LPS) | Sigma |
| Phytohemagglutinin (PHA) | MCE |
| RPMI 1640 Medium | Gibco |
| Human TNF-alpha DuoSet ELISA | R&D Systems |
| Human IL-6 DuoSet ELISA | R&D Systems |
| Human IL-10 DuoSet ELISA | R&D Systems |
| Human IFN-gamma DuoSet ELISA | R&D Systems |
| Human IL-2 DuoSet ELISA | R&D Systems |
| Human IL-1 beta ELISA Kit | Abclonal |
| Human GM-CSF ELISA Kit | Abclonal |
| Human IL-12/IL-23 p40 ELISA Kit | Abclonal |

V.2 Methods

V.2.1 Cell recovering and plating

A complete medium was prepared and preheated to 37°C. Cells were retrieved from liquid nitrogen tank, and the frozen vial was held with forceps just below the cap-body junction, immersed in 37°C warm water, and shaken occasionally to speed up thawing. The cell suspension was aspirated and added to a centrifuge tube containing 10 mL of the complete medium, and mixed well. The mixture was centrifuged at 400 g for 10 min at room temperature. The supernatant was discarded. The complete medium was added to the cells, followed by counting the cells and adjusting the cell density. The cells were seeded at 75 μL/well into a 96-well plate to achieve $1\times10^5$ cells per well. The plate was incubated in a high-humidity, 37°C, 5% $CO_2$ incubator for 2 h.

V.2.2 Preparation of Compounds and Addition to Cell Plates

1) 10 mM stock solutions of the test compounds in DMSO were prepared.
2) The test compounds were diluted stepwise 5-fold in DMSO, starting from 1 mM, to obtain nine concentration gradients.
3) The test compounds were diluted in medium to five times the final concentration intended for use.
4) The diluted compounds were added to the corresponding cell wells at 25 μL/well according to the following layout. The final compound concentrations start at 1 μM, with 5-fold dilutions, for a total of nine concentration points.
5) After 1 h, LPS or PHA was added to the cell wells at 25 μL/well to achieve final LPS concentrations of 1 μg/mL (for TNF-α, IL-10, IL-6, GM-CSF, and IL-12p40), 5 μg/mL (for IL-1β), 50 μg/mL (for IFN-γ), or a final PHA concentration of 1 μg/mL (for IL-2).
6) The cell plate was incubated in a high-humidity incubator at 37°C with 5% $CO_2$ for 24 h.

Cell plate layout:

| | 1 | 2 | 3 | 4 | 5 | 6 | 7 | 8 | 9 | 10 | 11 | 12 |
|---|---|---|---|---|---|---|---|---|---|---|---|---|
| A | $H_2O$ | $H_2O$ | $H_2O$ | $H_2O$ | $H_2O$ | $H_2O$ | $H_2O$ | $H_2O$ | $H_2O$ | $H_2O$ | $H_2O$ | $H_2O$ |
| B | $H_2O$ | Cmpd 1# (starting at a maximum concentration of 1 μM, 5-fold serial | | | | | | | | | LPS/PHA | $H_2O$ |
| C | $H_2O$ | dilutions, 9 points) | | | | | | | | | | $H_2O$ |
| D | $H_2O$ | Cmpd 2# (starting at a maximum concentration of 1 μM, 5-fold serial | | | | | | | | | | $H_2O$ |
| E | $H_2O$ | dilutions, 9 points) | | | | | | | | | | $H_2O$ |
| F | $H_2O$ | Cmpd 3# (starting at a maximum concentration of 1 μM, 5-fold serial | | | | | | | | | Blank | $H_2O$ |
| G | $H_2O$ | dilutions, 9 points) | | | | | | | | | | $H_2O$ |
| H | $H_2O$ | $H_2O$ | $H_2O$ | $H_2O$ | $H_2O$ | $H_2O$ | $H_2O$ | $H_2O$ | $H_2O$ | $H_2O$ | $H_2O$ | $H_2O$ |

V.2.3 Detection

(1) The cell plate was centrifuged and the culture supernatant was aspirated. TNF-α, IL-10, IL-6, IL-1β, IFN-γ, GM-CSF, IL-2, and IL-12p40 cytokines in the culture supernatant were detected according to the ELISA kit instructions. The concentrations of TNF-α, IL-10, IL-6, IL-1β, IFN-y, GM-CSF, IL-2, and IL-12p40 in the samples were calculated based on the standard curve.

$$\text{Inhibition rate\%} = [(Ac\text{-}As)/(Ac\text{-}Ab)] \times 100\%$$

As: OA of the samples (cells + LPS/PHA + test compound)
Ac: OA of positive cell control (cells + LPS/PHA + DMSO)
Ab: OA of blank control (cells + culture medium + DMSO)

(2) $IC_{50}$ value was calculated as follows: using GraphPad Prism 6 software and applying the calculation formula XY-analysis/Nonlinear regression (curve fit)/Dose response-Inhibition/log (inhibitor) vs. response-Variable slope (four parameters) to perform $IC_{50}$ curve fitting and determine the $IC_{50}$ values.

The experimental results indicated that the compounds of the present disclosure can modulate the production levels of cytokines associated with autoimmune diseases and may be used for the treatment of autoimmune diseases.

**VI. Efficacy experiment of the compounds of the present disclosure on Psoriasis-like skin lesions induced by Imiquimod in Mice**

The compounds of the present disclosure (test compounds) were administered orally via gavage once daily for 7 consecutive days to mice with psoriasis-like skin lesions induced by imiquimod, to investigate their efficacy on such lesions.

Ninety C57BL/6J female mice (purchased from Shanghai SLAC Laboratory Animal Co., Ltd.) were divided into 9 groups based on their body weights, with 10 mice in each group:

| Group | Drug | Number of Animals | Dose (mg/kg) | Volume (mL/kg) | Concentration (mg/mL) | Route and Duration |
|---|---|---|---|---|---|---|
| 1 | Control: Vehicle | 10 | NA | 10 | NA | ig. qd. 7 days |
| 2 | Model: Vehicle | 10 | NA | 10 | NA | ig. qd. 7 days |

(continued)

| Group | Drug | Number of Animals | Dose (mg/kg) | Volume (mL/kg) | Concentration (mg/mL) | Route and Duration |
|---|---|---|---|---|---|---|
| 3 | Positive Control | 10 | 3 | 10 | 0.3 | sc. qd. 7 days |
| 4 | Low-dose test compounds group | 10 | 3 | 10 | 0.3 | ig. qd. 7 days |
| 5 | Medium-dose test compounds group | 10 | 10 | 10 | 1 | ig. qd. 7 days |
| 6 | High-dose test compounds group | 10 | 30 | 10 | 3 | ig. qd. 7 days |
| Note: ig. = oral gavage; sc. = subcutaneous injection | | | | | | |

After grouping the experimental animals, the backs of all mice were shaved. Except for the Control group, the remaining groups were topically treated with imiquimod cream once daily for 7 consecutive days. Simultaneously, each group was administered with a solvent control, a positive control drug, or the test compounds, according to the above table, once daily for 7 consecutive days.

All clinical symptoms of each animal were observed at the beginning and throughout the experiment. The animals' weights were measured and recorded every 2 days.

Scoring

Gross Observation Scoring of Skin Lesions (PASI Method): during the last 3 days of modeling and drug administration, the changes in skin lesions of mice in each group were observed once daily. PASI Method: the erythema, scaling, and thickening/infiltration of the lesions were scored separately, and the scores were summed to obtain the total score. PASI Scoring Criteria: 0 = none; 1 = mild; 2 = moderate; 3 = severe; 4 = very severe.

The skin lesions were photographed once daily during the last 3 days of modeling and drug administration.

After the experiment, the mice were euthanized by $CO_2$ inhalation. Three different areas of skin from the lesion site on the back were collected using a 6 mm punch, weighed, and recorded, and the average weight was calculated.

The skin tissue from the lesion site was homogenized and the levels of IL-23p19, IL-12p40, IL-17, and TNF-$\alpha$ were determined.

The local lesion tissue was fixed, stained with HE, and subjected to pathological scoring (based on epidermal thickness, degree of epidermal keratinization, thickness of the basal layer, and degree of dermal inflammatory cell infiltration). Scoring Criteria: 0 = none; 1 = mild; 2 = moderate; 3 = severe; 4 = very severe.

Experimental data were presented as Mean $\pm$ SD. Statistical analysis between two groups was performed using SPSS, with $p < 0.05$ considered statistically significant.

The experimental results indicated that the compounds of the present disclosure improve psoriasis-like skin lesions on the back of mice induced by imiquimod and may be used for the treatment of psoriasis.

**VII. Efficacy Experiment of the compounds of the present disclosure on the CIA Model in Rats Induced by Type II Collagen**

The compounds of the present disclosure (test compounds) were administered orally via gavage once daily for 21 consecutive days to Wistar rats with collagen-induced arthritis (CIA) that was induced using bovine type II collagen (Chondrex, Inc.), in order to investigate their efficacy in the rat arthritis model.

Preparation of the Modeling Agent: 10 mg of CII (Immunization Grade Bovine Type II Collagen, purchased from Chondrex, Inc.) was dissolved in 5 mL of 0.05M acetic acid solution, allowing it to be fully dissolved, thereby preparing a solution with a concentration of 2 mg/mL, which was then stored at 4°C in the dark overnight. On the day of the experiment, the 2 mg/mL CII solution was mixed with an equal volume of a 4 mg/mL solution of Complete Freund's Adjuvant (CFA, also purchased from Chondrex, Inc.) on ice, and was thoroughly emulsified into an emulsion.

On day 0, 10 Wistar rats (purchased from Beijing Vital River Laboratory Animal Technology Co., Ltd.) were randomly selected as the blank control group and were not immunized. The remaining 100 Wistar rats were intradermally injected at the base of the tail with an emulsion prepared by mixing equal volumes of CII (2 mg/mL) and CFA (4 mg/mL) for the first immunization. A second booster immunization was administered to them on day 7. Between days 10 and 14, i.e., 3 to 7 days after the second booster immunization, 80 rats with an arthritis index (AI) score ranging from 1 to 2 were selected from the modeled animals and were divided into 8 groups based on their body weight, foot volume, and AI score, with 10

rats per group:

| Group | Drug | Number of Animals | Dose (mg/kg) | Volume (mL/kg) | Concentration (mg/mL) | Route and Duration |
|---|---|---|---|---|---|---|
| 1 | Control: Vehicle | 10 | NA | 10 | NA | ig. qd. 21 days |
| 2 | Model: Vehicle | 10 | NA | 10 | NA | ig. qd. 21 days |
| 3 | Positive Control | 10 | 0.1 | 10 | 0.01 | sc. qd. 21 days |
| 4 | Low-dose test compounds group | 10 | 3 | 10 | 0.3 | ig. qd. 21 days |
| 5 | Medium-dose test compounds group | 10 | 10 | 10 | 1 | ig. qd. 21 days |
| 6 | High-dose test compounds group | 10 | 30 | 10 | 3 | ig. qd. 21 days |
| Note: ig. = oral gavage; sc. = subcutaneous injection | | | | | | |

Between 3 and 7 days after the second booster immunization, when the AI score of the affected feet reached 1 to 2, a solvent control, a positive control drug, or the test compounds were administered orally via gavage (or subcutaneously) to the animals in each group, according to the above table, once daily for 21 consecutive days.

Starting 3 days after the second booster immunization, the volume of both hind paws (or toes) was measured and recorded twice a week.

Foot Volume Measurement: Before the measurement, a line was marked on the rat's ankle joint with a marker pen to indicate the position. After clean water was added to the instrument, the instrument's reading was reset to zero in preparation for the measurement. The hind limb of the rat was placed in the water so that the marked line at the ankle joint was at the surface of the liquid. The reading obtained by pressing the foot pedal at this time was the foot volume of the rat. After the measurement was completed, the foot pedal was pressed again to reset the instrument to zero, preparing it for the measurement of the next rat.

Scoring

Arthritis Index (AI): The foot volume was measured twice a week, and the swelling of the four toes was scored simultaneously. Each foot was scored on a scale from 0 to 4, with a maximum score of 16 for each rat.

Arthritis Index (AI) Scoring Criteria:

| Score | Degree |
|---|---|
| 0 | No swelling, normal appearance |
| 1 | Mild swelling or redness of the ankle, wrist, or finger joints |
| 2 | Moderate swelling or redness of the ankle, wrist, or finger joints |
| 3 | Severe swelling or redness of the ankle, wrist, or finger joints |
| 4 | Very severe swelling or redness of the ankle, wrist, or finger joints |

The day of starting drug administration is recorded as D0. Five days after starting drug administration, i.e., 2 hours after D5 administration, blood was collected from the jugular vein of the experimental animals, allowed to stand for more than 30 minutes, centrifuged to separate the serum, which was stored at -80°C. The levels of TNF-$\alpha$, IL-1$\beta$, and IL-6 in serum were measured by ELISA.

After the experiment, the animals were euthanized by $CO_2$ inhalation. The spleen and thymus of the animals were

collected and weighed, and the organ coefficients were calculated.

Pathological Examination: One side of the foot joint tissue was fixed in 10% neutral formalin solution, followed by decalcification and paraffin embedding to prepare pathological sections. The sections were then stained with hematoxylin and eosin (HE) for pathological observation.

Observation Indices:

1. Infiltration of synovial inflammatory cells (lymphocytes, plasma cells);
2. Synovial tissue hyperplasia;
3. Synovial pannus;
4. Fibrinoid necrosis on the synovial surface.

Scoring and Counting Method: 0, 1, 2, 3, and 4 levels, where "0" indicates no observation; "1" is mild; "2" is moderate; "3" is severe; and "4" is extremely severe.

Experimental data were presented as Mean $\pm$ SEM. Data between two groups were analyzed using Excel-T test, with $p<0.05$ considered statistically significant. Scoring data were analyzed using the nonparametric Mann-Whitney U test in SPSS, with $p<0.05$ considered statistically significant.

The experimental results indicated that the compounds of the present disclosure can regulate the serum levels of inflammatory cytokines in rats with collagen-induced arthritis (CIA) model that was induced using bovine type II collagen, and significantly improve the swelling of rat limbs, and thus might be used for the treatment of arthritis.

The basic principles, main features and advantages of the present disclosure are shown and described above. Those skilled in the art should understand that the present disclosure is not limited by the foregoing embodiments and they can make various changes, substitutions and alterations herein without departing from the spirit and scope of the present disclosure. These changes, substitutions and alterations fall within the scope of the present disclosure. The claimed scope of the present disclosure is defined by the appended claims and their equivalents.

## Claims

**1.** A compound of Formula (I)

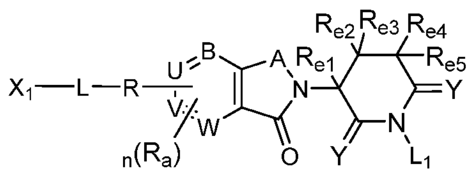

Formula (I)

or salts, enantiomers, diastereoisomer, isotopically enriched analogs, solvates, prodrugs, or polymorphs thereof, wherein

A represents $CH_2$ or C(O);
B, U, V, and W are the same or different and each independently represent CH or N, where B, U, V, and W are not N at the same time;
Y represents O or S;
$L_1$ represents hydrogen or $C_{1-3}$ alkyl;
$R_{e1}$, $R_{e2}$, $R_{e3}$, $R_{e4}$, and $R_{e5}$ are the same or different and each independently represent hydrogen or halogen;
$(R_a)_n$ denotes that the ring containing B, U, V, and W is substituted with n $R_a$, where $R_a$ groups are the same or different and each independently represent deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, optionally substituted $C_{3-6}$ cycloalkyl, $C_{2-6}$ alkenyl, or $C_{2-6}$ alkynyl, and n represents an integer of 1, 2, or 3; and
R represents SH, and L and $X_1$ are absent; or
R represents S, L represents an optionally substituted linear or branched alkyl, and $X_1$ is absent; or

R represents S(O) or $S(O)_2$, L represents an optionally substituted linear or branched alkyl or amino, and $X_1$ is absent; or

R represents S, S(O), $S(O)_2$, O, NH or $CH_2$,

L represents optionally substituted linear or branched alkylene, wherein one or more pairs of adjacent carbon atoms in the backbone carbon chain of the linear or branched alkylene is optionally interrupted by one or more groups selected from the group consisting of: $R_b$, $R_c$, and any combination thereof, wherein $R_b$ groups are each selected from the group consisting of O, C(O), OC(O), S, S(O), $S(O)_2$, $S(O)_2N(R_1)$, $N(R_1)S(O)_2$, $C(O)N(R_1)$, $N(R_1)C(O)$, $N(R_1)$, and $N(R_1)C(O)N(R_1)$, where $R_1$ groups each independently represent H or $C_{1-3}$ alkyl, and when two or more groups $R_b$ are inserted into the backbone carbon chain of the linear or branched alkylene group, the two or more groups $R_b$ are not directly connected to each other; and wherein $R_c$ groups are each selected from the group consisting of optionally substituted cycloalkylene, optionally substituted arylene, optionally substituted heterocyclylene, optionally substituted heteroarylene, and any combination thereof; and

$X_1$ represents $NR_2R_3$, $C(O)NR_4R_5$, $NHC(O)R_6$, $NHC(O)NR_7R_8$, $OR_9$, $SR_{10}$, quaternary ammonium salt group, linear or branched alkyl optionally substituted with one or more fluorine atoms, optionally substituted cycloalkyl, optionally substituted aryl, optionally substituted heterocyclyl or optionally substituted heteroaryl, where $R_2$, $R_3$, $R_4$, $R_5$, $R_7$, $R_8$, $R_9$, and $R_{10}$ each independently represent H, optionally substituted linear or branched alkyl, optionally substituted cycloalkyl, optionally substituted aryl, optionally substituted heterocyclyl or optionally substituted heteroaryl, wherein $R_2$ and $R_3$ are not H at the same time, and wherein $R_6$ represents optionally substituted linear or branched alkyl, optionally substituted cycloalkyl, optionally substituted aryl, optionally substituted heteroaryl or optionally substituted heterocyclyl, or wherein $R_4$ and $R_5$ together with the nitrogen atom to which they are attached form optionally substituted 5-to 8-membered heterocyclyl; or

$X_1$ represents a group of Formula ($G_1$):

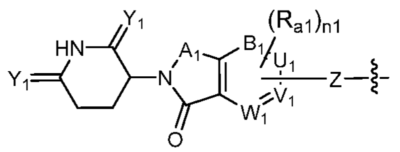

Formula ($G_1$)

wherein $A_1$ represents $CH_2$ or C(O);

$B_1$, $U_1$, $V_1$, and $W_1$ are the same or different and each independently represent CH or N, wherein $B_1$, $U_1$, $V_1$, and $W_1$ are not N at the same time;

$Y_1$ represents O or S; and

Z represents S, S(O), $S(O)_2$, O, NH or $CH_2$; and

$(R_{a1})_{n1}$ denotes that the ring containing $B_1$, $U_1$, $V_1$, and $W_1$ is substituted with n1 $R_{a1}$, where $R_{a1}$ groups are the same or different and each independently represent deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, optionally substituted $C_{3-6}$ cycloalkyl, $C_{2-6}$ alkenyl, or $C_{2-6}$ alkynyl, and n represents an integer of 0, 1, 2, or 3.

**2.** The compound of Formula (I) or salts, enantiomers, diastereoisomer, isotopically enriched analogs, solvates, prodrugs, or polymorphs thereof of claim 1, wherein the compound of Formula (I) is also of Formula (Ia), Formula (Ib), Formula (Ic), Formula (Id), or Formula (Ie):

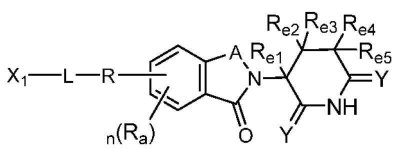

(Ia)

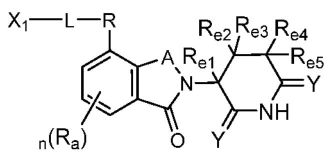

(Ib)

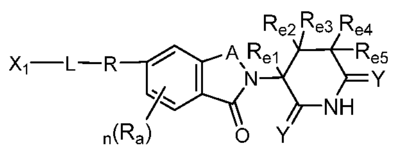

(Ic)

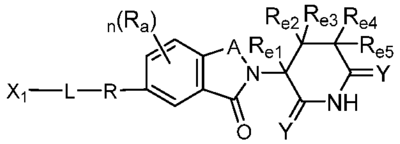

(Id)

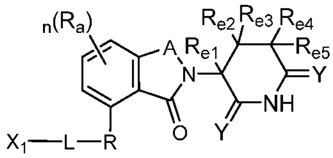

(Ie)

wherein A, $(R_a)_n$, $R_{e1}$, $R_{e2}$, $R_{e3}$, $R_{e4}$, $R_{e5}$, R, L, $X_1$, and Y are as defined in claim 1.

**3.** The compound of Formula (I) or salts, enantiomers, diastereoisomer, isotopically enriched analogs, solvates, prodrugs, or polymorphs thereof of claim 1, wherein Y represents O.

**4.** The compound of Formula (I) or salts, enantiomers, diastereoisomer, isotopically enriched analogs, solvates, prodrugs, or polymorphs thereof of claim 1, wherein A represents C(O).

**5.** The compound of Formula (I) or salts, enantiomers, diastereoisomer, isotopically enriched analogs, solvates, prodrugs, or polymorphs thereof of claim 1, wherein A represents $CH_2$.

**6.** The compound of Formula (I) or salts, enantiomers, diastereoisomer, isotopically enriched analogs, solvates, prodrugs, or polymorphs thereof of any one of claims 1-5, wherein

R represents SH, and L and $X_1$ are absent; or
R represents S, L represents optionally substituted linear or branched $C_{1-40}$alkyl, and $X_1$ is absent; or
R represents S(O) or $S(O)_2$, L represents optionally substituted linear or branched $C_{1-40}$alkyl or amino, and $X_1$ is absent.

**7.** The compound of Formula (I) or salts, enantiomers, diastereoisomer, isotopically enriched analogs, solvates, prodrugs, or polymorphs thereof of any one of claims 1-5, wherein

R represents S, L represents optionally substituted linear or branched $C_{1-30}$ alkyl, and $X_1$ is absent; or
R represents S(O) or $S(O)_2$, L represents optionally substituted linear or branched $C_{1-30}$ alkyl or amino, and $X_1$ is absent.

**8.** The compound of Formula (I) or salts, enantiomers, diastereoisomer, isotopically enriched analogs, solvates, prodrugs, or polymorphs thereof of any one of claims 1-5, wherein

R represents S, L represents the following optionally substituted groups: methyl, ethyl, n-propyl, isopropyl, n-butyl, isobutyl, sec-butyl, tert-butyl, pentyl, isopentyl, neopentyl, tert-amyl, hexyl, heptyl, octyl, nonyl, decyl, undecyl, dodecyl, tridecyl, tetradecyl, or pentadecyl, and $X_1$ is absent; or
R represents S(O) or $S(O)_2$, L represents the following optionally substituted groups: methyl, ethyl, n-propyl, isopropyl, n-butyl, isobutyl, sec-butyl, tert-butyl, pentyl, isopentyl, neopentyl, tert-amyl, hexyl, heptyl, octyl, nonyl, decyl, undecyl, dodecyl, tridecyl, tetradecyl, pentadecyl, or amino, and $X_1$ is absent.

**9.** The compound of Formula (I) or salts, enantiomers, diastereoisomer, isotopically enriched analogs, solvates, prodrugs, or polymorphs thereof of any one of claims 1-5, wherein

R represents S, S(O), $S(O)_2$, O, NH or $CH_2$;
L represents optionally substituted linear or branched $C_{1-40}$ alkylene, wherein one or more pairs of adjacent carbon atoms in the backbone carbon chain of the linear or branched $C_{1-40}$ alkylene is optionally interrupted by one or more groups selected from the group consisting of: $R_b$, $R_c$, and any combination thereof, wherein $R_b$ groups are each selected from the group consisting of O, C(O), OC(O), S, S(O), $S(O)_2$, $S(O)_2N(R_1)$, $N(R_1)S(O)_2$, $C(O)N(R_1)$, $N(R_1)C(O)$, $N(R_1)$, and $N(R_1)C(O)N(R_1)$, wherein $R_1$ groups each independently represent H or $C_{1-3}$ alkyl, and when two or more groups $R_b$ are inserted into the backbone carbon chain of the linear or branched $C_{1-40}$ alkylene group, the two or more groups $R_b$ are not directly connected to each other; and wherein $R_c$ groups are each selected from the group consisting of optionally substituted cycloalkylene, optionally substituted arylene, optionally substituted heterocyclylene, optionally substituted heteroarylene, and any combination thereof; and
$X_1$ represents $NR_2R_3$, $C(O)NR_4R_5$, $NHC(O)R_6$, $NHC(O)NR_7R_8$, $OR_9$, $SR_{10}$, quaternary ammonium salt group,

linear or branched $C_{1-10}$ alkyl optionally substituted with one or more fluorine, optionally substituted cycloalkyl, optionally substituted aryl, optionally substituted heterocyclyl or optionally substituted heteroaryl, where $R_2$, $R_3$, $R_4$, $R_5$, $R_7$, $R_8$, $R_9$, and $R_{10}$ each independently represent H, optionally substituted linear or branched $C_{1-10}$alkyl, optionally substituted cycloalkyl, optionally substituted aryl, optionally substituted heterocyclyl or optionally substituted heteroaryl, wherein $R_2$ and $R_3$ are not H at the same time, and wherein $R_6$ represents optionally substituted linear or branched $C_{1-8}$ alkyl, optionally substituted cycloalkyl, optionally substituted aryl, optionally substituted heteroaryl or optionally substituted heterocyclyl, or wherein $R_4$ and $R_5$ together with the nitrogen atom to which they are attached form optionally substituted 5- to 8-membered heterocyclyl; or

$X_1$ represents a group of Formula ($G_1$):

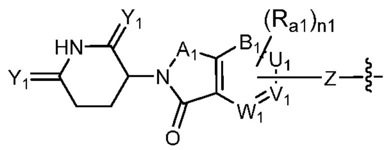

Formula ($G_1$)

wherein $A_1$ represents $CH_2$ or C(O);

$B_1$, $U_1$, $V_1$, and $W_1$ are the same or different and each independently represent CH or N, wherein $B_1$, $U_1$, $V_1$, and $W_1$ are not N at the same time;

$Y_1$ represents O or S; and

Z represents S, S(O) or $S(O)_2$; and

$(R_{a1})_{n1}$ denotes that the ring containing $B_1$, $U_1$, $V_1$, and $W_1$ is substituted with n1 $R_{a1}$, where $R_{a1}$ groups are the same or different and each independently represent deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, optionally substituted $C_{3-6}$ cycloalkyl, $C_{2-6}$ alkenyl, or $C_{2-6}$ alkynyl, and n represents an integer of 0, 1, 2, or 3.

**10.** The compound of Formula (I) or salts, enantiomers, diastereoisomer, isotopically enriched analogs, solvates, prodrugs, or polymorphs thereof of any one of claims 1-5, wherein

R represents S, S(O), $S(O)_2$, O, NH or $CH_2$;

L represents linear or branched $C_{1-40}$ alkylene group optionally substituted with one or more substituents selected from the group consisting of halogen, cyano, hydroxy, amino, mercapto, $C_{1-3}$alkyl, $C_{1-3}$ alkoxy, halogenated $C_{1-3}$ alkyl (e.g., trifluoromethyl), $C_{5-10}$ aryl, $C_{5-10}$ heteroaryl, $C_{3-12}$ cycloalkyl, 3- to 12-membered heterocyclyl, or any combination thereof, wherein one or more pairs of adjacent carbon atoms in the backbone carbon chain of the linear or branched $C_{1-40}$ alkylene is optionally interrupted by one or more groups selected from the group consisting of: $R_b$, $R_c$, and any combination thereof, wherein $R_b$ groups are each selected from the group consisting of O, C(O), OC(O), S, S(O), $S(O)_2$, $S(O)_2N(R_1)$, $N(R_1)S(O)_2$, $C(O)N(R_1)$, $N(R_1)C(O)$, $N(R_1)$, and $N(R_1)C(O)N(R_1)$, where $R_1$ groups each independently represent H or $C_{1-3}$ alkyl, and when two or more groups $R_b$ are inserted into the backbone carbon chain of the linear or branched alkylene group, the two or more groups $R_b$ are not directly connected to each other; and wherein $R_c$ groups are each selected from the group consisting of cycloalkylene (e.g., $C_{3-20}$ cycloalkylene), arylene (e.g., $C_{5-20}$ arylene), heterocyclylene (e.g., 4- to 20-membered heterocyclylene), heteroarylene (e.g., 5- to 20-membered heteroarylene), and any combination thereof, wherein the cycloalkylene and heterocyclylene are each independently optionally substituted with one or more substituents selected from the group consisting of: halogen, cyano, hydroxy, amino, mercapto, oxo, $C_{1-3}$ alkoxy, halogenated $C_{1-3}$ alkyl, methanesulfonyloxy, trifluoromethanesulfonyloxy, p-toluenesulfonyloxy, optionally deuterated $C_{1-6}$ alkyl, optionally deuterated $C_{3-6}$ cycloalkyl, or any combination thereof; and the arylene and heteroarylene are each independently optionally substituted with one or more substituents selected from the group consisting of: halogen, cyano, hydroxy, amino, mercapto, $C_{1-3}$ alkoxy, halogenated $C_{1-3}$ alkyl, methanesulfonyloxy, trifluoromethanesulfonyloxy, p-toluenesulfonyloxy, optionally deuterated $C_{1-6}$ alkyl, optionally deuterated $C_{3-6}$ cycloalkyl, or any combination thereof; and

$X_1$ represents:

$NR_2R_3$, $C(O)NR_4R_5$, $NHC(O)R_6$, $NHC(O)NR_7R_8$, $OR_9$, $SR_{10}$, or quaternary ammonium salt group, where $R_2$, $R_3$, $R_4$, $R_5$, $R_7$, $R_8$, $R_9$, and $R_{10}$ each independently represent H, optionally substituted linear or branched $C_{1-10}$ alkyl, optionally substituted cycloalkyl, optionally substituted aryl, optionally substituted heterocyclyl or optionally substituted heteroaryl, wherein $R_2$ and $R_3$ are not H at the same time, and wherein $R_6$ represents

optionally substituted linear or branched $C_{1-6}$ alkyl, optionally substituted cycloalkyl, optionally substituted aryl, optionally substituted heteroaryl or optionally substituted heterocyclyl, or wherein $R_4$ and $R_5$ together with the nitrogen atom to which they are attached form optionally substituted 5- to 8-membered heterocyclyl; or

linear or branched $C_{1-10}$ alkyl optionally substituted with one or more fluorine; or

$C_3$-$C_{12}$ cycloalkyl, optionally substituted with one or more substituents selected from the group consisting of: halogen, cyano, amino, hydroxy, mercapto, oxo, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more substituents selected from the group consisting of: halogen, cyano, amino, hydroxy, mercapto, oxo, $C_{1-6}$ alkyl, halogenated $C_{1-3}$alkyl, or any combination thereof; and the $C_5$-$C_{20}$ aryl is optionally substituted with one or more substituents selected from the group consisting of: halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more substituents selected from the group consisting of: halogen, cyano, amino, hydroxy, mercapto, oxo, $C_{1-6}$ alkyl, halogenated $C_{1-3}$ alkyl, or any combination thereof; or

$C_5$-$C_{14}$ aryl, optionally substituted with one or more substituents selected from the group consisting of: halogen, cyano, amino, hydroxy, mercapto, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more substituents selected from the group consisting of: halogen, cyano, amino, hydroxy, mercapto, oxo, $C_{1-6}$ alkyl, halogenated $C_{1-3}$ alkyl, or any combination thereof; and the $C_5$-$C_{20}$ aryl is optionally substituted with one or more substituents selected from the group consisting of: halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more substituents selected from the group consisting of: halogen, cyano, amino, hydroxy, mercapto, oxo, $C_{1-6}$ alkyl, halogenated $C_{1-3}$ alkyl, or any combination thereof; or

3- to 12-membered heterocyclyl, optionally substituted with one or more substituents selected from the group consisting of: halogen, cyano, amino, hydroxy, mercapto, oxo, $C_{1-3}$ alkoxy, halogenated $C_{1-3}$ alkoxy, halogenated $C_{1-3}$ alkyl, $C_{1-6}$ alkylsulfonyl, bis($C_{1-6}$ alkyl)phosphon, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more substituents selected from the group consisting of: halogen, cyano, amino, hydroxy, mercapto, oxo, $C_{1-6}$ alkyl, halogenated $C_{1-3}$ alkyl, or any combination thereof; and the $C_5$-$C_{20}$ aryl is optionally substituted with one or more substituents selected from the group consisting of: halogen, cyano, amino, hydroxy, mercapto, $C_{1-6}$ alkyl, halogenated $C_{1-3}$ alkyl, or any combination thereof; and the 5- or 6-membered heteroaryl is optionally substituted with one or more substituents selected from the group consisting of: halogen, cyano, amino, hydroxy, mercapto, oxo, $C_{1-6}$ alkyl, halogenated $C_{1-3}$ alkyl, or any combination thereof; or

5- to 10-membered heteroaryl, optionally substituted with one or more substituents selected from the group consisting of: halogen, cyano, amino, hydroxy, mercapto, $C_{1-3}$ alkoxy, halogenated $C_{1-3}$ alkoxy, halogenated $C_{1-3}$ alkyl, bis($C_{1-6}$ alkyl)phosphon, $C_{1-6}$ alkylsulfonyl, methanesulfonyloxy, trifluoromethanesulfonyloxy, *p*-toluenesulfonyloxy, $C_{1-6}$ alkyl, optionally substituted 5- or 6-membered heterocyclyl, optionally substituted $C_5$-$C_{20}$ aryl, optionally substituted 5- or 6-membered heteroaryl, $C_{1-6}$ alkyl, $C_{1-3}$ alkoxycarbonyl, halogenated $C_{2-4}$ alkenyl, or any combination thereof, wherein the 5- or 6-membered heterocyclyl is optionally substituted with one or more substituents selected from the group consisting of: $C_{1-6}$ alkyl, cyano, halogenated $C_{1-3}$ alkyl, amino, hydroxy, mercapto, halogen, or any combination thereof; and the $C_5$-$C_{20}$ aryl is optionally substituted with one or more substituents selected from the group consisting of: $C_{1-6}$ alkyl, cyano, halogenated $C_{1-3}$alkyl, amino, hydroxy, mercapto, halogen, or any combination thereof; and the 5- or 6-membered heteroaryl is optionally substituted with one or more substituents selected from the group consisting of: $C_{1-6}$ alkyl, halogenated $C_{1-3}$ alkyl, amino, hydroxy, mercapto, halogen, or any combination thereof; or

$X_1$ represents a group of Formula ($G_1$):

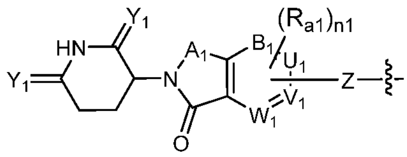

Formula ($G_1$)

wherein $A_1$ represents $CH_2$ or C(O);
$B_1$, $U_1$, $V_1$, and $W_1$ are the same or different and each independently represent CH or N, wherein $B_1$, $U_1$, $V_1$, and $W_1$ are not N at the same time;
$Y_1$ represents O or S; and
Z represents S, S(O) or $S(O)_2$; and
$(R_{a1})_{n1}$ denotes that the ring containing $B_1$, $U_1$, $V_1$, and $W_1$ is substituted with n1 $R_{a1}$, where $R_{a1}$ groups are the same or different and each independently represent deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, optionally substituted $C_{3-6}$ cycloalkyl, $C_{2-6}$ alkenyl, or $C_{2-6}$ alkynyl, and n represents an integer of 0, 1, 2, or 3, wherein the $C_{3-6}$ cycloalkyl is optionally substituted with a substituent selected from the group consisting of: $C_{1-6}$ alkyl, halogenated $C_{1-3}$ alkyl, amino, hydroxy, halogen, or any combination thereof.

**11.** The compound of Formula (I) or salts, enantiomers, diastereoisomer, isotopically enriched analogs, solvates, prodrugs, or polymorphs thereof of claim 9 or 10, wherein

$X_1$ represents:
$NR_2R_3$, $NHC(O)NR_7R_8$, $OR_9$ or $SR_{10}$, wherein $R_2$, $R_3$, $R_7$, $R_8$, $R_9$, and $R_{10}$ each independently represent H, optionally substituted linear or branched $C_{1-10}$ alkyl, optionally substituted $C_3$-$C_{20}$ cycloalkyl, optionally substituted 3- to 20-membered heterocyclyl, optionally substituted $C_5$-$C_{14}$ aryl or optionally substituted 5- to 20-membered heteroaryl, wherein $R_2$ and $R_3$ are not H at the same time,

wherein the optionally substituted linear or branched $C_{1-10}$ alkyl is optionally substituted with one or more substituents selected from the group consisting of: halogen, cyano, hydroxy, amino, mercapto, $C_{1-3}$alkyl, $C_{1-3}$ alkoxy, trifluoromethyl, optionally substituted $C_{5-10}$ aryl, optionally substituted 5- to 10-membered heteroaryl, optionally substituted $C_{3-12}$ cycloalkyl, optionally substituted 3- to 12-membered heterocyclyl, or any combination thereof, wherein the optionally substituted $C_{5-10}$ aryl, optionally substituted 5- to 10-membered heteroaryl, optionally substituted $C_{3-12}$ cycloalkyl and optionally substituted 3- to 12-membered heterocyclyl are each independently optionally substituted with one or more substituents selected from the group consisting of: halogen, cyano, amino, hydroxy, mercapto, $C_{1-6}$ alkyl, $C_{1-3}$ alkoxy, or any combination thereof; the optionally substituted $C_3$-$C_{20}$ cycloalkyl and the optionally substituted 3- to 20-membered heterocyclyl are each independently optionally substituted with one or more substituents selected from the group consisting of: halogen, cyano, amino, hydroxy, mercapto, oxo, $C_{1-6}$ alkyl, $C_{1-3}$ alkoxy, $C_{1-3}$ alkoxycarbonyl, halogenated $C_{2-4}$ alkenyl, halogenated $C_{1-3}$ alkoxy, halogenated $C_{1-3}$alkyl, $C_{1-6}$ alkylsulfonyl, bis($C_{1-6}$ alkyl) phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, or any combination thereof, wherein the 5- or 6-membered heterocyclyl is optionally substituted with a substituent selected from the group consisting of: halogen, cyano, amino, hydroxy, oxo, mercapto, $C_{1-6}$alkyl, halogenated $C_{1-3}$alkyl, or any combination thereof; and the $C_5$-$C_{20}$ aryl is optionally substituted with a substituent selected from the group consisting of: halogen, cyano, amino, hydroxy, mercapto, $C_{1-6}$alkyl, halogenated $C_{1-3}$alkyl, or any combination thereof; and the 5- or 6-membered heteroaryl is optionally substituted with a substituent selected from the group consisting of: halogen, cyano, amino, hydroxy, mercapto, $C_{1-6}$ alkyl, halogenated $C_{1-3}$ alkyl, or any combination thereof;
wherein the optionally substituted $C_5$-$C_{14}$ aryl or optionally substituted 5- to 20-membered heteroaryl is each independently optionally substituted with one or more substituents selected from the group consisting of: halogen, cyano, amino, hydroxy, mercapto, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$alkyl, $C_{1-3}$ alkoxycarbonyl, halogenated $C_{2-4}$ alkenyl, $C(O)NHR_d$, or any combination thereof, wherein $R_d$ represents $C_{1-5}$alkyl or optionally substituted phenyl, and the optionally substituted phenyl is optionally substituted with one or more substituents selected

from the group consisting of: halogen, cyano, amino, hydroxy, mercapto, oxo, $C_{1-6}$alkyl, and any combination thereof; and the 5- or 6-membered heterocyclyl is optionally substituted with a substituent selected from the group consisting of: halogen, cyano, amino, hydroxy, oxo, mercapto, $C_{1-6}$alkyl, halogenated $C_{1-3}$alkyl, or any combination thereof; and the $C_3$-$C_{20}$ aryl is optionally substituted with a substituent selected from the group consisting of: halogen, cyano, amino, hydroxy, mercapto, $C_{1-6}$alkyl, halogenated $C_{1-3}$alkyl, or any combination thereof; and the 5- or 6-membered heteroaryl is optionally substituted with a substituent selected from the group consisting of: halogen, cyano, amino, hydroxy, oxo, mercapto, $C_{1-6}$alkyl, halogenated $C_{1-3}$alkyl, or any combination thereof; or

$X_1$ represents:
$C(O)NR_4R_5$, wherein $R_4$ and $R_5$ each independently represent H, optionally substituted linear or branched $C_{1-10}$alkyl, optionally substituted $C_3$-$C_{20}$cycloalkyl, optionally substituted $C_5$-$C_{14}$ aryl, optionally substituted 3- to 20-membered heterocyclyl or optionally substituted 5- to 20-membered heteroaryl, or wherein $R_4$ and $R_5$ together with the nitrogen atom to which they are attached form optionally substituted 5- to 8-membered heterocyclyl;

wherein the optionally substituted linear or branched $C_{1-10}$alkyl is optionally substituted with one or more substituents selected from the group consisting of: halogen, cyano, hydroxy, amino, mercapto, $C_{1-3}$alkyl, $C_{1-3}$ alkoxy, trifluoromethyl, $C_{5-10}$ aryl, 5- to 10-membered heteroaryl, $C_{3-12}$cycloalkyl, 3- to 12-membered heterocyclyl, or any combination thereof;
the optionally substituted $C_3$-$C_{20}$cycloalkyl and the optionally substituted 3- to 20-membered heterocyclyl are each independently optionally substituted with one or more substituents selected from the group consisting of: halogen, cyano, amino, hydroxy, oxo, mercapto, $C_{1-6}$alkyl, $C_{1-3}$ alkoxy, $C_{1-3}$ alkoxycarbonyl, halogenated $C_{2-4}$ alkenyl, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, or any combination thereof, wherein the 5- or 6-membered heterocyclyl is optionally substituted with a substituent selected from the group consisting of: halogen, cyano, amino, hydroxy, oxo, mercapto, $C_{1-6}$alkyl, halogenated $C_{1-3}$alkyl, or any combination thereof; and the $C_3$-$C_{20}$ aryl is optionally substituted with a substituent selected from the group consisting of: halogen, cyano, amino, hydroxy, mercapto, $C_{1-6}$alkyl, halogenated $C_{1-3}$alkyl, or any combination thereof; and the 5- or 6-membered heteroaryl is optionally substituted with a substituent selected from the group consisting of: halogen, cyano, amino, hydroxy, mercapto, $C_{1-6}$alkyl, halogenated $C_{1-3}$alkyl, or any combination thereof;
the optionally substituted $C_5$-$C_{14}$ aryl or optionally substituted 5- to 20-membered heteroaryl is each independently optionally substituted with one or more substituents selected from the group consisting of: halogen, cyano, amino, hydroxy, mercapto, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$alkyl, $C_{1-3}$ alkoxycarbonyl, halogenated $C_{2-4}$ alkenyl, or any combination thereof, wherein the 5- or 6-membered heterocyclyl is optionally substituted with a substituent selected from the group consisting of: halogen, cyano, amino, hydroxy, oxo, mercapto, $C_{1-6}$alkyl, halogenated $C_{1-3}$alkyl, or any combination thereof; and the $C_5$-$C_{20}$ aryl is optionally substituted with a substituent selected from the group consisting of: halogen, cyano, amino, hydroxy, mercapto, $C_{1-6}$alkyl, halogenated $C_{1-3}$alkyl, or any combination thereof; and the 5- or 6-membered heteroaryl is optionally substituted with a substituent selected from the group consisting of: halogen, cyano, amino, hydroxy, oxo, mercapto, $C_{1-6}$alkyl, halogenated $C_{1-3}$alkyl, or any combination thereof; and
the optionally substituted 5- to 8-membered heterocyclyl is optionally substituted with one or more substituents selected from the group consisting of: halogen, cyano, amino, hydroxy, mercapto, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$alkyl, $C_{1-3}$ alkoxycarbonyl, halogenated $C_{2-4}$ alkenyl, or any combination thereof, wherein the 5- or 6-membered heterocyclyl is optionally substituted with a substituent selected from the group consisting of: halogen, cyano, amino, hydroxy, mercapto, oxo, $C_{1-6}$alkyl, halogenated $C_{1-3}$alkyl, or any combination thereof; and the $C_5$-$C_{20}$ aryl is optionally substituted with a substituent selected from the group consisting of: halogen, cyano, amino, hydroxy, mercapto, $C_{1-6}$alkyl, halogenated $C_{1-3}$alkyl, or any combination thereof; and the 5- or 6-membered heteroaryl is optionally substituted with a substituent selected from the group consisting of: halogen, cyano, amino, hydroxy, mercapto, oxo, $C_{1-6}$alkyl, halogenated $C_{1-3}$alkyl, or any combination thereof; or

$X_1$ represents:
$NHC(O)R_6$, wherein $R_6$ represents optionally substituted linear or branched $C_{1-10}$alkyl, optionally substituted $C_3$-$C_{20}$cycloalkyl, optionally substituted $C_5$-$C_{14}$ aryl, optionally substituted 5- to 20-membered heteroaryl or optionally substituted 3- to 20-membered heterocyclyl;

wherein the optionally substituted linear or branched $C_{1-10}$alkyl is optionally substituted with one or more substituents selected from the group consisting of: halogen, cyano, hydroxy, amino, mercapto, $C_{1-3}$alkyl, $C_{1-3}$ alkoxy, trifluoromethyl, $C_{5-10}$ aryl, 5- to 10-membered heteroaryl, $C_{3-12}$cycloalkyl, 3- to 12-membered heterocyclyl, or any combination thereof;
the optionally substituted $C_3$-$C_{20}$cycloalkyl and the optionally substituted 3- to 20-membered heterocyclyl are each independently optionally substituted with one or more substituents selected from the group consisting of: halogen, cyano, amino, hydroxy, mercapto, oxo, $C_{1-6}$alkyl, $C_{1-3}$ alkoxy, $C_{1-3}$ alkoxycarbonyl, halogenated $C_{2-4}$ alkenyl, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, or any combination thereof, wherein the 5- or 6-membered heterocyclyl is optionally substituted with one or more substituents selected from the group consisting of: halogen, cyano, amino, hydroxy, mercapto, oxo, $C_{1-6}$alkyl, halogenated $C_{1-3}$alkyl, or any combination thereof; and the $C_5$-$C_{20}$ aryl is optionally substituted with one or more substituents selected from the group consisting of: halogen, cyano, amino, hydroxy, mercapto, $C_{1-6}$alkyl, halogenated $C_{1-3}$alkyl, or any combination thereof; and the 5- or 6-membered heteroaryl is optionally substituted with one or more substituents selected from the group consisting of: halogen, cyano, amino, hydroxy, mercapto, $C_{1-6}$alkyl, halogenated $C_{1-3}$alkyl, or any combination thereof; and
the optionally substituted $C_5$-$C_{14}$ aryl or optionally substituted 5- to 20-membered heteroaryl is each independently optionally substituted with one or more substituents selected from the group consisting of: halogen, cyano, amino, hydroxy, mercapto, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$alkyl, $C_{1-3}$ alkoxycarbonyl, halogenated $C_{2-4}$ alkenyl, or any combination thereof, wherein the 5- or 6-membered heterocyclyl is optionally substituted with one or more substituents selected from the group consisting of: halogen, cyano, amino, hydroxy, mercapto, oxo, $C_{1-6}$alkyl, halogenated $C_{1-3}$alkyl, or any combination thereof; and the $C_5$-$C_{20}$ aryl is optionally substituted with one or more substituents selected from the group consisting of: halogen, cyano, amino, hydroxy, mercapto, $C_{1-6}$alkyl, halogenated $C_{1-3}$alkyl, or any combination thereof; and the 5- or 6-membered heteroaryl is optionally substituted with one or more substituents selected from the group consisting of: halogen, cyano, amino, hydroxy, mercapto, oxo, $C_{1-6}$ alkyl, halogenated $C_{1-3}$ alkyl, or any combination thereof; or

$X_1$ represents:
a quaternary ammonium salt group of Formula $N^+R_aR_bR_cX^-$,

wherein $R_a$, $R_b$, and $R_c$ each independently represent $C_{1-20}$alkyl, and $X^-$ represents $F^-$, $Cl^-$, $Br^-$, or $I^-$; or wherein $X^-$ represents $F^-$, $Cl^-$, $Br^-$, or $I^-$, $R_c$ represents $C_{10-20}$alkyl or hydrogen, and $R_a$ and $R_b$ together with the nitrogen atom to which they are attached form a 5- to 8-membered heterocyclyl, wherein the ring atoms of the 5- to 8-membered heterocyclyl optionally further comprise a heteroatom selected from oxygen, nitrogen, and sulfur, and the 5- to 8-membered heterocyclyl is optionally substituted with one or more substituents selected from the group consisting of: halogen, cyano, amino, hydroxy, mercapto, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*- toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$alkyl, $C_{1-3}$ alkoxycarbonyl, halogenated $C_{2-4}$ alkenyl, or any combination thereof, wherein the 5- or 6-membered heterocyclyl is optionally substituted with a substituent selected from the group consisting of: halogen, cyano, amino, hydroxy, mercapto, oxo, $C_{1-6}$alkyl, halogenated $C_{1-3}$alkyl, or any combination thereof; and the $C_5$-$C_{20}$ aryl is optionally substituted with a substituent selected from the group consisting of: halogen, cyano, amino, hydroxy, mercapto, $C_{1-6}$alkyl, halogenated $C_{1-3}$ alkyl, or any combination thereof; and the 5- or 6-membered heteroaryl is optionally substituted with a substituent selected from the group consisting of: halogen, cyano, amino, hydroxy, mercapto, oxo, $C_{1-6}$alkyl, halogenated $C_{1-3}$alkyl, or any combination thereof.

**12.** The compound of Formula (I) or salts, enantiomers, diastereoisomer, isotopically enriched analogs, solvates,

prodrugs, or polymorphs thereof of claim 1 or 10, wherein L represents the structure of the following formula:

linear or branched $C_{1-40}$ alkylene;
*-$(C(R_{a2})(R_{a3}))_{n2}$-$(R_b$-$(C(R_{a4})(R_{a5}))_{n3})_{m1}$-;
*-$(C(R_{a2})(R_{a3}))_{n2}$-$(R_b$-$(C(R_{a4})(R_{a5}))_{n3})_{m1}$-$(R_b$-$(C(R_{a6})(R_{a7}))_{n4})_{m2}$-;
*-$(C(R_{a2})(R_{a3}))_{n2}$-$(R_b$-$(C(R_{a4})(R_{a5}))_{n3})_{m1}$-$(R_b$-$(C(R_{a6})(R_{a7}))_{n4})_{m2}$-$(R_b$-$(C(R_{a8})(R_{a9}))_{n5})_{m3}$-
*-$(C(R_{a2})(R_{a3}))_{n2}$-$(R_c$-$(C(R_{a4})(R_{a5}))_{n3})_{m1}$-;
*-$(C(R_{a2})(R_{a3}))_{n2}$-$(R_c$-$(C(R_{a4})(R_{a5}))_{n3})_{m1}$-$(R_c$-$(C(R_{a6})(R_{a7}))_{n4})_{m2}$-;
*-$(C(R_{a2})(R_{a3}))_{n2}$-$(R_c$-$(C(R_{a4})(R_{a5}))_{n3})_{m1}$-$(R_c$-$(C(R_{a6})(R_{a7}))_{n4})_{m2}$-$(R_c$-$(C(R_{a8})(R_{a9}))_{n5})_{m3}$-;
*-$(C(R_{a2})(R_{a3}))_{n2}$-$(R_b$-$R_c$-$(C(R_{a4})(R_{a5}))_{n3})_{m1}$-;
*-$(C(R_{a2})(R_{a3}))_{n2}$-$(R_b$-$(C(R_{a4})(R_{a5}))_{n3})_{m1}$-$(R_c$-$(C(R_{a6})(R_{a7}))_{n4})_{m2}$-;
*-$(C(R_{a2})(R_{a3}))_{n2}$-$(R_c$-$R_b$-$(C(R_{a4})(R_{a5}))_{n3})_{m1}$-; or
*-$(C(R_{a2})(R_{a3}))_{n2}$-$(R_c$-$(C(R_{a4})(R_{a5}))_{n3})_{m1}$-$(R_b$-$(C(R_{a6})(R_{a7}))_{n4})_{m2}$-;
wherein * indicates the point of attachment to R;
a hydrogen of one or more $CH_2$ groups of the linear or branched $C_{1-40}$ alkylene is optionally replaced with a substituent selected from the group consisting of: halogen, cyano, $C_{1-3}$alkyl, $C_{1-3}$ alkoxy, trifluoromethyl, $C_{5-10}$ aryl, $C_{5-10}$ heteroaryl, $C_{3-12}$cycloalkyl, 3- to 12-membered heterocyclyl, or any combination thereof;
$R_b$ groups are each independently selected from the group consisting of O, C(O), OC(O), S, S(O), $S(O)_2$, $S(O)_2N(R_1)$, $N(R_1)S(O)_2$, $C(O)N(R_1)$, $N(R_1)C(O)$, $N(R_1)$ and $N(R_1)C(O)N(R_1)$, wherein $R_1$ groups each independently represent H or $C_{1-3}$alkyl;
$R_c$ groups are each independently selected from the group consisting of optionally substituted cycloalkylene (e.g., $C_{3-20}$ cycloalkylene), optionally substituted arylene (e.g., $C_{5-20}$ arylene), optionally substituted heterocyclylene (e.g., 4- to 20-membered heterocyclylene), optionally substituted heteroarylene (e.g., 5- to 20-membered heteroarylene), and any combination thereof, wherein the cycloalkylene and heterocyclylene are each independently optionally substituted with a substituent selected from the group consisting of halogen, cyano, hydroxy, amino, mercapto, oxo, $C_{1-3}$ alkoxy, halogenated $C_{1-3}$alkyl, methanesulfonyloxy, trifluoromethanesulfonyloxy, p-toluenesulfonyloxy, optionally deuterated $C_{1-6}$ alkyl, optionally deuterated $C_{3-6}$ cycloalkyl, or any combination thereof; and the arylene and heteroarylene are each independently optionally substituted with a substituent selected from the group consisting of halogen, cyano, hydroxy, amino, mercapto, $C_{1-3}$ alkoxy, halogenated $C_{1-3}$alkyl, methanesulfonyloxy, trifluoromethanesulfonyloxy, *p*-toluenesulfonyloxy, optionally deuterated $C_{1-6}$ alkyl, optionally deuterated $C_{3-6}$ cycloalkyl, or any combination thereof;
$R_{a2}$, $R_{a3}$, $R_{a4}$, $R_{a5}$, $R_{a6}$, $R_{a7}$, $R_{a8}$, and $R_{a9}$ each independently represent H, halogen, cyano, hydroxy, amino, mercapto, $C_{1-3}$alkyl, $C_{1-3}$ alkoxy, trifluoromethyl, $C_{5-10}$ aryl, $C_{5-10}$ heteroaryl, $C_{3-12}$cycloalkyl or 3- to 12-membered heterocyclyl; and
n2, n3, n4, n5, m1, m2, and m3 each independently represent an integer of 1, 2, 3, 4, 5, 6, 7, 8, 9, 10, 11, 12, 13, 14, or 15.

**13.** The compound of Formula (I) or salts, enantiomers, diastereoisomer, isotopically enriched analogs, solvates, prodrugs, or polymorphs thereof of claim 1 or 12, wherein L represents the structure of the following formula:

*-$(C(Ra_2)(Ra_3))_{n2}$-$(C_{5-20}$ arylene-$(C(R_{a4})(R_{a5}))_{n3})_{m1}$-;
*-$(C(R_{a2})(R_{a3}))_{n2}$-$(C_{5-20}$ arylene-$(C(R_{a4})(R_{a5}))_{n3})_{m1}$-$(C_{5-20}$ arylene-$(C(R_{a6})(R_{a7}))_{n4})_{m2}$-;
*-$(C(R_{a2})(R_{a3}))_{n2}$-$(C_{5-20}$arylene-$(C(R_{a4})(R_{a5}))_{n3})_{m1}$-$(C_{5-20}$ arylene-$(C(R_{a6})(R_{a7}))_{n4})_{m2}$-$(C_{5-20}$ arylene-$(C(R_{a8})(R_{a9}))_{n5})_{m3}$-;
*-$(C(R_{a2})(R_{a3}))_{n2}$-(5- to 20-membered heteroarylene-$(C(R_{a4})(R_{a5}))_{n3})_{m1}$-;
*-$(C(R_{a2})(R_{a3}))_{n2}$-(5- to 20-membered heteroarylene-$(C(R_{a4})(R_{a5}))_{n3})_{m1}$-(5- to 20-membered heteroarylene-$(C(R_{a6})(R_{a7}))_{n4})_{m2}$-;
*-$(C(R_{a2})(R_{a3}))_{n2}$-(5- to 20-membered heteroarylene-$(C(R_{a4})(R_{a5}))_{n3})_{m1}$-(5- to 20-membered heteroarylene-$(C(R_{a6})(R_{a7}))_{n4})_{m2}$-(5- to 20-membered heteroarylene-$(C(R_{a8})(R_{a9}))_{n5})_{m3}$-;
*-$(C(R_{a2})(R_{a3}))_{n2}$-(4- to 20-membered heterocyclylene-$(C(R_{a4})(R_{a5}))_{n3})_{m1}$-;
*-$(C(R_{a2})(R_{a3}))_{n2}$-(4- to 20-membered heterocyclylene-$(C(R_{a4})(R_{a5}))_{n3})_{m1}$-(4- to 20-membered heterocyclylene-$(C(R_{a6})(R_{a7}))_{n4})_{m2}$-;
*-$(C(R_{a2})(R_{a3}))_{n2}$-(O-$(C(_{Ra4})(R_{a5}))_{n3})_{m1}$-;
*-$(C(R_{a2})(R_{a3}))_{n2}$-(O-$(C(R_{a4})(R_{a5}))_{n3})_{m1}$-(O-$(C(R_{a6})(R_{a7}))_{n4})_{m2}$-;
*-$(C(R_{a2})(R_{a3}))_{n2}$-(O-$(C(R_{a4})(R_{a5}))_{n3})_{m1}$-(O-$(C(R_{a6})(R_{a7}))_{n4})_{m2}$-(O-$(C(R_{a8})(R_{a9}))_{n5})_{m3}$-;
*-$(C(R_{a2})(R_{a3}))_{n2}$-(S-$(C(R_{a4})(R_{a5}))_{n3})_{m1}$-;
*-$(C(R_{a2})(R_{a3}))_{n2}$-(S-$(C(R_{a4})(R_{a5}))_{n3})_{m1}$-(S-$(C(R_{a6})(R_{a7}))_{n4})_{m2}$-;
*-$(C(R_{a2})(R_{a3}))_{n2}$-(S-$(C(R_{a4})(R_{a5}))_{n3})_{m1}$-(S-$(C(R_{a6})(R_{a7}))_{n4})_{m2}$-(S-$(C(R_{a8})(R_{a9}))_{n5})_{m3}$-;

$*-(C(R_{a2})(R_{a3}))_{n2}-(S(O)-(C(R_{a4})(R_{a5}))_{n3})_{m1}-$;
$*-(C(R_{a2})(R_{a3}))_{n2}-(S(O)_2-(C(R_{a4})(R_{a5}))_{n3})_{m1}-$;
$*-(C(_{Ra2})(R_{a3}))_{n2}-(S-(C(R_{a4})(R_{a5}))_{n3})_{m1}-$;
$*-(C(R_{a2})(R_{a3}))_{n2}-(S(O)_2N(R_1)-(C(R_{a4})(R_{a5}))_{n3})_{m1}-$;
$*-(C(R_{a2})(R_{a3}))_{n2}-(N(R_1)S(O)_2-(C(R_{a4})(R_{a5}))_{n3})_{m1}-$;
$*-(C(R_{a2})(R_{a3}))_{n2}-(C(O)N(R_1)-(C(R_{a4})(R_{a5}))_{n3})_{m1}-$;
$*-(C(R_{a2})(R_{a3}))_{n2}-(N(R_1)C(O)-(C(R_{a4})(R_{a5}))_{n3})_{m1}-$;
$*-(C(R_{a2})(R_{a3}))_{n2}-(N(R_1)-(C(R_{a4})(R_{a5}))_{n3})_{m1}-$;
$*-(C(R_{a2})(R_{a3}))_{n2}-(N(R_1)C(O)N(R_1)-(C(R_{a4})(R_{a5}))_{n3})_{m1}-$;
wherein * indicates the point of attachment to R;
$R_1$ groups each independently represent H or $C_{1-3}$alkyl;
$R_{a2}$, $R_{a8}$, $R_{a4}$, $R_{a5}$, $R_{a6}$, $R_{a7}$, $R_{a8}$, and $R_{a9}$ each independently represent H, halogen, cyano, hydroxy, amino, mercapto, $C_{1-3}$alkyl, $C_{1-3}$ alkoxy, trifluoromethyl, $C_{5-10}$ aryl, $C_{5-10}$ heteroaryl, $C_{3-12}$cycloalkyl, or 3- to 12-membered heterocyclyl;
n2, n3, n4, n5, m1, m2, and m3 each independently represent an integer of 1, 2, 3, 4, 5, 6, 7, 8, 9, 10, 11, 12, 13, 14, or 15; and
the 4- to 20-membered heterocyclylene is optionally substituted with a substituent selected from the group consisting of halogen, cyano, hydroxy, amino, mercapto, oxo, $C_{1-3}$ alkoxy, halogenated $C_{1-3}$alkyl, methanesulfonyloxy, trifluoromethanesulfonyloxy, *p*-toluenesulfonyloxy, optionally deuterated $C_{1-6}$ alkyl, optionally deuterated $C_{3-6}$ cycloalkyl, or any combination thereof; and the $C_{5-20}$ arylene and the 5- to 20-membered heteroarylene are each independently optionally substituted with a substituent selected from the group consisting of halogen, cyano, hydroxy, amino, mercapto, $C_{1-3}$ alkoxy, halogenated $C_{1-3}$alkyl, methanesulfonyloxy, trifluoromethanesulfonyloxy, *p*-toluenesulfonyloxy, optionally deuterated $C_{1-6}$ alkyl, optionally deuterated $C_{3-6}$ cycloalkyl, or any combination thereof.

**14.** The compound of Formula (I) or salts, enantiomers, diastereoisomer, isotopically enriched analogs, solvates, prodrugs, or polymorphs thereof of claim 1, wherein L represents the structure of the following formula:

linear or branched $C_1$-$C_{40}$alkylene, $*-(CH_2)_{n2}-(O-(CH_2)_{n3})_{m1}-$, $*-(CH_2)_{n2}-(O-(CH_2)_{n3})_{m1}-(O-(CH_2)_{n4})_{m2}-$, $*-(CH_2)_{n2}-(O-(CH_2)_{n3})_{m1}-(O-(CH_2)_{n4})_{m2}-(O-(CH_2)_{n5})_{m3}-$, $*-(CH_2)_{n2}-S-(CH_2)_{n3}-$, $*-(CH_2)_{n2}-S(O)-(CH_2)_{n3}-$, $*-(CH_2)_{n2}-S(O)_2-(CH_2)_{n3}-$, $*-(CH_2)_{n2}-N(R_1)S(O)_2-(CH_2)_{n3}-$, $*-(CH_2)_{n2}-S(O)_2N(R_1)-(CH_2)_{n3}-$, $*-(CH_2)_{n2}-N(R_1)-(CH_2)_{n3}-$, $*-(CH_2)_{n2}-N(R_1)C(O)-(CH_2)_{n3}-$, $*-(CH_2)_{n2}-C(O)N(R_1)-(CH_2)_{n3}-$, $*-(CH_2)_{n2}-N(R_1)C(O)N(R_1)-(CH_2)_{n3}-$, $*-(CH_2)_{n2}-(N(R_1)C(O)-(CH_2)_{n3})_{m1}-$, $*-(CH_2)_{n2}$-piperazinylene-$(CH_2)_{n3}-$, $*-(CH_2)_{n2}$-phenylene-$(CH_2)_{n3}-$, $*-(CH_2)_{n2}$-phenylene-$(CH_2)_{n3}$ $-N(R_1)-(CH_2)_{n4}$ -, $*-(CH_2)_{n2}$-furanylene-$(CH_2)_{n3}-$, $*-(CH_2)_{n2}$-furanylene-$(CH_2)_{n3}-N(R_1)-(CH_2)_{n4}-$, $*-(CH_2)_{n2}$-thiazolylene-$(CH_2)_{n3}-$, $*-(CH_2)_{n2}$-thiazolylene-$C(O)N(R_1)-(CH_2)_{n3}-$, $*-(CH_2)_{n2}$-thiazolylene-$(CH_2)_{n3}-N(R_1)-(CH_2)_{n4}-$;
wherein a hydrogen of one or more $CH_2$ groups of the linear or branched $C_{1-40}$ alkylene is optionally replaced with a substituent selected from the group consisting of: halogen, cyano, $C_{1-3}$alkyl, $C_{1-3}$ alkoxy, trifluoromethyl, $C_{5-10}$ aryl, 5- to 10-membered heteroaryl, $C_{3-12}$cycloalkyl, 3- to 12-membered heterocyclyl, or any combination thereof;
* indicates the point of attachment to R;
$R_1$ groups each independently represent H or $C_{1-3}$alkyl;
n2, n3, n4, n5, m1, m2, and m3 each independently represent an integer of 1, 2, 3, 4, 5, 6, 7, 8, 9, 10, 11, 12, 13, 14, or 15;
the piperazinylene is optionally substituted with a substituent selected from the group consisting of halogen, cyano, hydroxy, amino, mercapto, oxo, $C_{1-3}$ alkoxy, halogenated $C_{1-3}$alkyl, methanesulfonyloxy, trifluoromethanesulfonyloxy, *p*-toluenesulfonyloxy, optionally deuterated $C_{1-6}$ alkyl, optionally deuterated $C_{3-6}$ cycloalkyl, or any combination thereof; and the phenylene, the furanylene, and the thiazolylene are each independently optionally substituted with a substituent selected from the group consisting of halogen, cyano, hydroxy, amino, mercapto, $C_{1-3}$ alkoxy, halogenated $C_{1-3}$alkyl, methanesulfonyloxy, trifluoromethanesulfonyloxy, *p*-toluenesulfonyloxy, optionally deuterated $C_{1-6}$ alkyl, optionally deuterated $C_{3-6}$ cycloalkyl, or any combination thereof.

**15.** The compound of Formula (I) or salts, enantiomers, diastereoisomer, isotopically enriched analogs, solvates, prodrugs, or polymorphs thereof of claim 1, wherein L represents the following groups:

$-CH_2-$; $-(CH_2)_2-$; $-(CH_2)_3-$; $-(CH_2)_4-$; $-(CH_2)_5-$; $-(CH_2)_6-$; $-(CH_2)_7-$; $-(CH_2)_8-$; $-(CH_2)_9-$; $-(CH_2)_{10}-$; $-(CH_2)_{11}-$; $-(CH_2)_{12}-$; $-(CH_2)_{13}-$; $-(CH_2)_{14}-$; $-(CH_2)_{15}-$; $-(CH_2)_{16}-$; $-(CH_2)_{17}-$; $-(CH_2)_{18}-$; $-(CH_2)_{19}-$; or $-(CH_2)_{20}-$;
$*-CH_2CH_2O(CH_2)_2-$; $*-(CH_2CH_2O)_2-(CH_2)_2-$; $*-(CH_2CH_2O)_3-(CH_2)_2-$; $*-(CH_2CH_2O)_4-(CH_2)_2-$; $*-(CH_2CH_2O)_5-(CH_2)_2-$; $*-(CH_2CH_2O)_6-(CH_2)_2-$; $*-(CH_2CH_2O)_7-(CH_2)_2-$; $*-(CH_2CH_2O)_8-(CH_2)_2-$;

$*-(CH_2CH_2O)_9(CH_2)_2-$; $*-(CH_2CH_2O)_{10}(CH_2)_2-$; $*-CH_2CH_2OCH_2-$; $*-(CH_2CH_2O)_2-CH_2-$; $*-(CH_2CH_2O)_3-CH_2-$; $*-(CH_2CH_2O)_4-CH_2-$; $*-(CH_2CH_2O)_5-CH_2-$; $*-(CH_2CH_2O)_6-CH_2-$; $*-(CH_2CH_2O)_7-CH_2-$; $*-(CH_2CH_2O)_8-CH_2-$; $*-(CH_2CH_2O)_9-CH_2-$; $*-(CH_2CH_2O)_{10}-CH_2-$; $*-CH_2CH_2O(CH_2)_3-$; $*-(CH_2CH_2O)_2-(CH_2)_3-$; $*-(CH_2CH_2O)_3-(CH_2)_3-$; $*-(CH_2CH_2O)_4-(CH_2)_3-$; $*-(CH_2CH_2O)_5-(CH_2)_3-$; $*-(CH_2CH_2O)_6-(CH_2)_3-$; $*-(CH_2CH_2O)_7-(CH_2)_3-$; $*-(CH_2CH_2O)_8-(CH_2)_3-$; $*-(CH_2CH_2O)_9(CH_2)_3-$; $*-(CH_2CH_2O)_{10}(CH_2)_3-$; $*-CH_2CH_2OCH_2CH_2CH_2OCH_2-$; $*-CH_2CH_2OCH_2CH_2CH_2O-(CH_2)_2-$; $*-CH_2CH_2OCH_2CH_2CH_2O-(CH_2)_3-$; $*-(CH_2CH_2O)_2(CH_2CH_2CH_2O)(CH_2)_3-$; $*-(CH_2CH_2O)_2(CH_2CH_2CH_2O)_2(CH_2)_3-$; $*-(CH_2)_1O(CH_2)_1-$; $*-(CH_2)_1O(CH_2)_2-$; $*-(CH_2)_2O(CH_2)_2-$; $*-(CH_2)_2O(CH_2)_1-$; $*-(CH_2)_2O(CH_2)_3-$; $*-(CH_2)_2O(CH_2)_4-$; $*-(CH_2)_2O(CH_2)_5-$; $*-(CH_2)_2O(CH_2)_6-$; $*-(CH_2)_3O(CH_2)_1-$; $*-(CH_2)_3O(CH_2)_2-$; $*-(CH_2)_3O(CH_2)_3-$; $*-(CH_2)_4O(CH_2)_1-$; $*-(CH_2)_4O(CH_2)_2-$; $*-(CH_2)_4O(CH_2)_3-$; $*-(CH_2)_5O(CH_2)_1-$; $*-(CH_2)_5O(CH_2)_2-$; $*-(CH_2)_5O(CH_2)_3-$; $*-(CH_2)_5O(CH_2)_4-$; or $*-(CH_2)_5O(CH_2)_5-$;

$*-(CH_2)_1-NH-(CH_2)_1-$; $*-(CH_2)_2-NH-(CH_2)_1-$; $*-(CH_2)_2-NH-(CH_2)_2-$; $*-(CH_2)_2-NH-(CH_2)_3-$; $*-(CH_2)_2-NH-(CH_2)_4-$; $*-(CH_2)_2-NH-(CH_2)_5-$; $*-(CH_2)_2-NH-(CH_2)_6-$; $*-(CH_2)_2-NH-(CH_2)_7-$; $*-(CH_2)_2-NH-(CH_2)_8-$; $*-(CH_2)_2-NH-(CH_2)_9-$; $*-(CH_2)_2-NH-(CH_2)_{10}-$; $*-(CH_2)_2-NH-(CH_2)_{11}-$; $*-(CH_2)_2-NH-(CH_2)_{12}-$; $*-(CH_2)_3-NH-(CH_2)_1-$; $*-(CH_2)_3-NH-(CH_2)_2-$; $*-(CH_2)_3-NH-(CH_2)_3-$; $*-(CH_2)_4-NH-(CH_2)_l-$; $*-(CH_2)_4-NH-(CH_2)_2-$; $*-(CH_2)_5-NH-(CH_2)_3-$; $*-(CH_2)_5-NH-(CH_2)_1-$; $*-(CH_2)_5-NH-(CH_2)_2-$; $*-(CH_2)_8-NH-(CH_2)_2-$; $*-(CH_2)_5-NH-(CH_2)_3-$; $*-(CH_2)_5-NH-(CH_2)_4-$; $*-(CH_2)_5-NH-(CH_2)_5-$; $*-(CH_2)_1-N(CH_3)-(CH_2)_8-$; $*-(CH_2)_2-N(CH_3)-(CH_2)_1-$; $*-(CH_2)_3-N(CH_3)-(CH_2)_1-$; $*-(CH_2)_4-N(CH_3)-(CH_2)_1-$; $*-(CH_2)_5-N(CH_3)-(CH_2)_1-$; $*-(CH_2)_6-N(CH_3)-(CH_2)_1-$; $*-(CH_2)_2-N(CH_3)-(CH_2)_2-$; $*-(CH_2)_2-N(CH_3)-(CH_2)_3-$; $*-(CH_2)_2-N(CH_3)-(CH_2)_4-$; $*-(CH_2)_2-N(CH_3)-(CH_2)_5-$; $*-(CH_2)_2-N(CH_3)-(CH_2)_6-$; $*-(CH_2)_2-N(CH_3)-(CH_2)_7-$; $*-(CH_2)_2-N(CH_3)-(CH_2)_8-$; $*-(CH_2)_2-N(CH_3)-(CH_2)_9-$; $*-(CH_2)_2-N(CH_3)-(CH_2)_{10}-$; $*-(CH_2)_2-N(CH_3)-(CH_2)_{11}-$; $*-(CH_2)_2-N(CH_3)-(CH_2)_{12}-$; $*-(CH_2)_2-NHC(O)-CH_2-$; $*-(CH_2)_2-NHC(O)-(CH_2)_2-$; $*-(CH_2)_2-NHC(O)-(CH_2)_3-$; $*-(CH_2)_2-NHC(O)-(CH_2)_4-$; $*-(CH_2)_2-NHC(O)-(CH_2)_5-$; $*-(CH_2)_2-NHC(O)-(CH_2)_6-$; $*-(CH_2)_2-NHC(O)-(CH_2)_7-$; $*-(CH_2)_2-NHC(O)-(CH_2)_8-$; $*-(CH_2)_2-NHC(O)-(CH_2)_9-$; $*-(CH_2)-NHC(O)-(CH_2)_{10}-$; $*-(CH_2)_2-NHC(O)-(CH_2)_{11}-$; $*-(CH_2)_2-NHC(O)-(CH_2)_{12}-$; $*-(CH_2)_2-NHC(O)-(CH_2)_{13}-$; $*-(CH_2)_2-NHC(O)-(CH_2)_{14}-$; $*-(CH_2)_2-NHC(O)-(CH_2)_{15}-$; $*-(CH_2)_3-NHC(O)-CH_2-$; $*-(CH_2)_3-NHC(O)-(CH_2)_2-$; $*-(CH_2)_3-NHC(O)-(CH_2)_3-$; $*-(CH_2)_3-NHC(O)-(CH_2)_4-$; $*-(CH_2)_3-NHC(O)-(CH_2)_5-$; $*-(CH_2)_3-NHC(O)-(CH_2)_6-$; $*-(CH_2)_3-NHC(O)-(CH_2)_7-$; $*-(CH_2)_3-NHC(O)-(CH_2)_8-$; $*-(CH_2)_3-NHC(O)-(CH_2)_9-$; $*-(CH_2)_3-NHC(O)-(CH_2)_{10}-$; $*-(CH_2)_3-NHC(O)-(CH_2)_{11}-$; $*-(CH_2)_3-NHC(O)-(CH_2)_{12}-$; $*-(CH_2)_3-NHC(O)-(CH_2)_{13}-$; $*-(CH_2)_3-NHC(O)-(CH_2)_{14}-$; $*-(CH_2)_3-NHC(O)-(CH_2)_{15}-$; $*-(CH_2)_4NHC(O)(CH_2)_1-$; $*-(CH_2)_4NHC(O)(CH_2)_2-$; $*-(CH_2)_4NHC(O)(CH_2)_3-$; $*-(CH_2)_4NHC(O)(CH_2)_4-$; $*-(CH_2)_4NHC(O)(CH_2)_5-$; $*-(CH_2)_4NHC(O)(CH_2)_6-$; $*-(CH_2)_4NHC(O)(CH_2)_7-$; $*-(CH_2)_4NHC(O)(CH_2)_8-$; $*-(CH_2)_4NHC(O)(CH_2)_9-$; $*-(CH_2)_4NHC(O)(CH_2)_{10}-$; $*-(CH_2)_5NHC(O)(CH_2)_1-$; $*-(CH_2)_8NHC(O)(CH_2)_2-$; $*-(CH_2)_2-N(CH_3)C(O)-CH_2-$; $*-(CH_2)_2-N(CH_3)C(O)-(CH_2)_2-$; $*-(CH_2)_2-N(CH_3)C(O)-(CH_2)_3-$; $*-(CH_2)_2-N(CH_3)C(O)-(CH_2)_4-$; $*-(CH_2)_2-N(CH_3)C(O)-(CH_2)_5-$; $*-(CH_2)_2-N(CH_3)C(O)-(CH_2)_6-$; $*-(CH_2)_2-N(CH_3)C(O)-(CH_2)_7-$; $*-(CH_2)_2-N(CH_3)C(O)-(CH_2)_8-$; $*-(CH_2)_2-N(CH_3)C(O)-(CH_2)_9-$; $* -(CH_2)_2-N(CH_3)C(O)-(CH_2)_{10}-$; $*-(CH_2)_2-N(CH_3)C(O)-(CH_2)_{11}-$; $* -(CH_2)_2-N(CH_3)C(O)-(CH_2)_{12}-$; $*-(CH_2)_2-N(CH_3)C(O)-(CH_2)_{13}-$; $*-(CH_2)_2-N(CH_3)C(O)-(CH_2)_{14}-$; $*-(CH_2)_2-N(CH_3)C(O)-(CH_2)_{15}-$; $*-(CH_2)_2-C(O)NH-CH_2-$; $*-(CH_2)_2-C(O)NH-(CH_2)_2-$; $*-(CH_2)_2-C(O)NH-(CH_2)_3-$; $*-(CH_2)_2-C(O)NH-(CH_2)_4-$; $* -(CH_2)_2-C(O)NH-(CH_2)_5-$; $*-(CH_2)_2-C(O)NH-(CH_2)_6-$; $*-(CH_2)_2-C(O)NH-(CH_2)_7-$; $*-(CH_2)_2-C(O)NH-(CH_2)_8-$; $*-(CH_2)_2-C(O)NH-(CH_2)_9-$; $*-(CH_2)_2-C(O)NH-(CH_2)_{10}-$; $*-(CH_2)_2-C(O)NH-(CH_2)_{11}-$; $*-(CH_2)_2-C(O)NH-(CH_2)_{12}-$; $*-(CH_2)_2-C(O)NH-(CH_2)_{13}-$; $*-(CH_2)_2-C(O)NH-(CH_2)_{14}-$; $*-(CH_2)_2-C(O)NH-(CH_2)_{15}-$; $* -(CH_2)_3-C(O)NH-CH_2-$; $*-(CH_2)_3-C(O)NH-(CH_2)_2-$; $*-(CH_2)_3-C(O)NH-(CH_2)_3-$; $*-(CH_2)_3-C(O)NH-(CH_2)_4-$; $*-(CH_2)_3-C(O)NH-(CH_2)_5-$; $*-(CH_2)_3-C(O)NH-(CH_2)_6-$; $*-(CH_2)_3-C(O)NH-(CH_2)_7-$; $*-(CH_2)_3-C(O)NH-(CH_2)_8-$; $*-(CH_2)_3-C(O)NH-(CH_2)_9-$; $*-(CH_2)_3-C(O)NH-(CH_2)_{10}-$; $*-(CH_2)_3-C(O)NH-(CH_2)_{11}-$; $*-(CH_2)_3-C(O)NH-(CH_2)_{12}-$; $*-(CH_2)_3-C(O)NH-(CH_2)_{13}-$; $*-(CH_2)_3-C(O)NH-(CH_2)_{14}-$; $*-(CH_2)_3-C(O)NH-(CH_2)_{15}-$; $*-(CH_2)_4C(O)NH(CH_2)_1-$; $*-(CH_2)_4C(O)NH(CH_2)_2-$; $*-(CH_2)_4C(O)NH(CH_2)_3-$; $* -(CH_2)_4C(O)NH(CH_2)_4-$; $*-(CH_2)_4C(O)NH(CH_2)_5-$; $*-(CH_2)_4C(O)NH(CH_2)_6-$; $*-(CH_2)_4C(O)NH(CH_2)_7-$; $*-(CH_2)_4C(O)NH(CH_2)_8-$; $*-(CH_2)_4C(O)NH(CH_2)_9-$; $*-(CH_2)_4C(O)NH(CH_2)_{10}-$; $*-(CH_2)_2-C(O)N(CH_3)-CH_2-$; $*-(CH_2)_2-C(O)N(CH_3)-(CH_2)_2-$; $*-(CH_2)_2-C(O)N(CH_3)-(CH_2)_3-$; $*-(CH_2)_2-C(O)N(CH_3)-(CH_2)_4-$; $*-(CH_2)_2-C(O)N(CH_3)-(CH_2)_5-$; $*-(CH_2)_2-C(O)N(CH_3)-(CH_2)_6-$; $*-(CH_2)_2-C(O)N(CH_3)-(CH_2)_7-$; $*-(CH_2)_2-C(O)N(CH_3)-(CH_2)_8-$; $*-(CH_2)_2-C(O)N(CH_3)-(CH_2)_9-$; $*-(CH_2)_2-C(O)N(CH_3)-(CH_2)_{1o}-$; $*-(CH_2)_2-C(O)N(CH_3)-(CH_2)_{11}-$; $*-(CH_2)_2-C(O)N(CH_3)-(CH_2)_{12}-$; $*-(CH_2)_2-C(O)N(CH_3)-(CH_2)_{13}-$; $*-(CH_2)_2-C(O)N(CH_3)-(CH_2)_{14}-$; $*-(CH_2)_2-C(O)N(CH_3)-(CH_2)_{15}-$; $*-(CH_2)_2-NHC(O)NH-(CH_2)_4-$; $*-(CH_2)_4-NHC(O)NH-(CH_2)_2-$; $*-CH_2-NHC(O)NH-(CH_2)_2-$; $*-(CH_2)_2-NHC(O)NH-CH_2-$; $*-(CH_2)_2-NHC(O)NH-(CH_2)_2-$; $*-(CH_2)_2-NHC(O)NH-(CH_2)_3-$; $*-(CH_2)_3-NHC(O)NH-(CH_2)_2-$; $*-CH_2$-piperazinylene-$CH_2-$; $*-(CH_2)_2$-piperazinylene-$(CH_2)_2-$; $*-(CH_2)_2$-piperazinylene-$(CH_2)_3-$; $*-(CH_2)_2$-piperazinylene-$(CH_2)_4-$; $*-(CH_2)_2$-piperazinylene-$(CH_2)_5-$; $*-(CH_2)_3$-piperazinylene-$CH_2-$; $*-(CH_2)_3$-piperazinylene-$(CH_2)_2-$; $*-(CH_2)_3$-piperazinylene-$(CH_2)_3-$; $*-(CH_2)_4$-piperazinylene-$CH_2-$; $-(CH_2)_4$-piperazinylene-$(CH_2)_2-$; $*-(CH_2)_4$-piperazinylene-$(CH_2)_3-$; $*-(CH_2)_8$-piperazinylene-$CH_2-$; $*-(CH_2)_8$-piperazinylene-$(CH_2)_2-$; $*-(CH_2)_8$-piperazinylene-$(CH_2)_3-$; $*-(CH_2)_8$-piperazinylene-$(CH_2)_4-$; $-(CH_2)_8$-piperazinylene-$(CH_2)_5-$; $*-(CH_2)_8-$

piperazinylene-$(CH_2)_6$-; *-$(CH_2)_8$-piperazinylene-$(CH_2)_7$-; *-$(CH_2)_8$-piperazinylene-$(CH_2)_8$-; *-$CH_2$-piperazinylene-$(CH_2)_8$-; *-$(CH_2)_2$-piperazinylene-$(CH_2)_8$-; *-$(CH_2)_3$-piperazinylene-$(CH_2)_8$-; *-$(CH_2)_4$-piperazinylene-$(CH_2)_8$-; -$(CH_2)_5$-piperazinylene-$(CH_2)_8$-; *-$(CH_2)_6$-piperazinylene-$(CH_2)_8$-; *-$(CH_2)_7$-piperazinylene-$(CH_2)_8$-; *-$CH_2$-phenylene-$CH_2$-; *-$(CH_2)_2$-phenylene-$(CH_2)_2$-; *-$(CH_2)_2$-phenylene-$(CH_2)_3$-; *-$(CH_2)_2$-phenylene-$(CH_2)_4$-; *-$(CH_2)_2$-phenylene-$(CH_2)_5$-; *-$(CH_2)_3$-phenylene-$CH_2$-; *-$(CH_2)_3$-phenylene-$(CH_2)_2$-; *-$(CH_2)_3$-phenylene-$(CH_2)_3$-; *-$(CH_2)_4$-phenylene-$CH_2$-; *-$(CH_2)_4$-phenylene-$(CH_2)_2$-; *-$(CH_2)_4$-phenylene-$(CH_2)_3$-; *-$(CH_2)_5$-phenylene-$(CH_2)_3$-; *-$(CH_2)_6$-phenylene-$(CH_2)_3$-; *-$(CH_2)_7$-phenylene-$(CH_2)_3$-; *-$(CH_2)_8$-phenylene-$CH_2$-; *-$(CH_2)_8$-phenylene-$(CH_2)_2$-; *-$(CH_2)_8$-phenylene-$(CH_2)_3$-; *-$(CH_2)_8$-phenylene-$(CH_2)_4$-; *-$(CH_2)_8$-phenylene-$(CH_2)_5$-; *-$(CH_2)_8$-phenylene-$(CH_2)_6$-; *-$(CH_2)_8$-phenylene-$(CH_2)_7$-; *-$(CH_2)_8$-phenylene-$(CH_2)_8$-; *-$CH_2$-phenylene-$(CH_2)_8$-; *-$(CH_2)_2$-phenylene-$(CH_2)_8$-; *-$(CH_2)_3$-phenylene-$(CH_2)_8$-; *-$(CH_2)_4$-phenylene-$(CH_2)_8$-, *-$(CH_2)_5$-phenylene-$(CH_2)_8$-; *-$(CH_2)_6$-phenylene-$(CH_2)_8$-; *-$(CH_2)_7$-phenylene-$(CH_2)_8$-; *-$(CH_2)_1S(CH_2)_1$-; *-$(CH_2)_2S(CH_2)_2$-; *-$(CH_2)_2S(CH_2)_1$; *-$(CH_2)_1S(CH_2)_2$-; *-$(CH_2)_1S(CH_2)_3$-; *-$(CH_2)_1S(CH_2)_4$-; *-$(CH_2)_2S(CH_2)_3$-; *-$(CH_2)_2S(CH_2)_4$-; *-$(CH_2)_2S(CH_2)_5$-; *-$(CH_2)_3S(CH_2)_1$-; *-$(CH_2)_3S(CH_2)_2$-; *-$(CH_2)_3S(CH_2)_3$-; *-$(CH_2)_4S(CH_2)_1$-; *-$(CH_2)_4S(CH_2)_2$-; *-$(CH_2)_4S(CH_2)_3$-; *-$(CH_2)_5S(CH_2)_1$-; *-$(CH_2)_5S(CH_2)_2$-; *-$(CH_2)_5S(CH_2)_3$-; *-$(CH_2)_6S(CH_2)_1$-; *-$(CH_2)_6S(CH_2)_2$-; *-$(CH_2)_6S(CH_2)_3$-; *-$(CH_2)_7S(CH_2)_1$-; *-$(CH_2)_7S(CH_2)_2$-; *-$(CH_2)_7S(CH_2)_3$-; *-$(CH_2)_8S(CH_2)_1$-; *-$(CH_2)_8S(CH_2)_2$-; *-$(CH_2)_8S(CH_2)_3$-; *-$(CH_2)_9S(CH_2)_1$-; * -$(CH_2)_9S(CH_2)_2$-; *-$(CH_2)_9S(CH_2)_3$-; *-$(CH_2)_1S(O)$ $(CH_2)_1$-; *-$(CH_2)_2S(O)(CH_2)_2$-; *-$(CH_2)_2S(O)(CH_2)_1$-; *-$(CH_2)_1S(O)(CH_2)_2$-; *-$(CH_2)_1S(O)(CH_2)_3$-; *-$(CH_2)_1S(O)(CH_2)_4$-; *-$(CH_2)_2S(O)(CH_2)_3$-; *-$(CH_2)_2S(O)(CH_2)_4$-; *-$(CH_2)_2S(O)(CH_2)_5$-; *-$(CH_2)_3S(O)(CH_2)_1$-; + *-$(CH_2)_3S(O)(CH_2)_2$-; *-$(CH_2)_3S(O)$ $(CH_2)_3$-; *-$(CH_2)_4S(O)(CH_2)_1$-; *-$(CH_2)_4S(O)(CH_2)_2$-; *-$(CH_2)_4S(O)(CH_2)_3$-; *-$(CH_2)_5S(O)(CH_2)_1$-; *-$(CH_2)_5S(O)(CH_2)_2$-; *-$(CH_2)_5S(O)(CH_2)_3$-; *-$(CH_2)_6S(O)(CH_2)_1$-; *-$(CH_2)_6S(O)(CH_2)_2$-; *-$(CH_2)_6S(O)(CH_2)_3$-; *-$(CH_2)_7S(O)(CH_2)_1$-; *-$(CH_2)_7S(O)(CH_2)_2$-; *-$(CH_2)_7S(O)(CH_2)_3$-; *-$(CH_2)_8S(O)(CH_2)_1$-; *-$(CH_2)_8S(O)(CH_2)_2$-; *-$(CH_2)_8S(O)(CH_2)_3$-; *-$(CH_2)_9S(O)(CH_2)_1$-; *-$(CH_2)_9S(O)(CH_2)_2$-; $(CH_2)_9S(O)(CH_2)_3$-; *-$(CH_2)_1S(O)_2(CH_2)_1$-; *-$(CH_2)_2S(O)_2(CH_2)_2$-; *-$(CH_2)_2S(O)_2(CH_2)_1$-; *-$(CH_2)_1S(O)_2(CH_2)_2$-; *-$(CH_2)_1S(O)_2(CH_2)_3$-; *-$(CH_2)_1S(O)_2$ $(CH_2)_4$-; *-$(CH_2)_2S(O)_2(CH_2)_3$-; *-$(CH_2)_2S(O)_2(CH_2)_4$-; *-$(CH_2)_2S(O)_2(CH_2)_5$-; *-$(CH_2)_3S(O)_2(CH_2)_1$-; *-$(CH_2)_3S(O)_2(CH_2)_2$-; *-$(CH_2)_3S(O)_2(CH_2)_3$-; *-$(CH_2)_4S(O)_2(CH_2)_1$-; *-$(CH_2)_4S(O)_2(CH_2)_2$-; *-$(CH_2)_4S(O)_2$ $(CH_2)_3$-; *-$(CH_2)_5S(O)_2(CH_2)_1$-; *-$(CH_2)_5S(O)_2(CH_2)_2$-; *-$(CH_2)_5S(O)(CH_2)_3$-; *-$(CH_2)_6S(O)_2(CH_2)_1$-; *-$(CH_2)_6S(O)_2(CH_2)_2$-; *-$(CH_2)_6S(O)_2(CH_2)_3$-; *-$(CH_2)_7S(O)_2(CH_2)_1$-; *-$(CH_2)_7S(O)_2(CH_2)_2$-; *-$(CH_2)_7S(O)_2$ $(CH_2)_3$-; *-$(CH_2)_8S(O)_2(CH_2)_1$-; *-$(CH_2)_8S(O)_2(CH_2)_2$-; *-$(CH_2)_8S(O)_2(CH_2)_3$-; *-$(CH_2)_9S(O)_2(CH_2)_1$-; *-$(CH_2)_9S(O)_2(CH_2)_2$-; or *-$(CH_2)_9S(O)_2(CH_2)_3$-;

*-$(CH_2)_6$-NH-$(CH_2)_1$-; *-$(CH_2)_6$-NH-$(CH_2)_3$-; *-$(CH_2)_6$-NH-$(CH_2)_4$-; *-$(CH_2)_6$-NH-$(CH_2)_5$-; *-$(CH_2)_7$-NH-$(CH_2)_1$-; *-$(CH_2)_7$-NH-$(CH_2)_2$-; *-$(CH_2)_7$-NH-$(CH_2)_3$-; *-$(CH_2)_7$-NH-$(CH_2)_4$-; *-$(CH_2)_6$-NH-CH($CH_3$)-; *-$(CH_2)_5$-NH-CH($CH_3$)-; *-$(CH_2)_4$-NH-CH($CH_3$)-; *-$(CH_2)_3$-NH-CH($CH_3$)-; *-$(CH_2)_2$-NH-CH($CH_3$)-; *-$(CH_2)_7$-NH-CH($CH_3$)-; *-$(CH_2)_8$-NH-CH($CH_3$)-; *-$(CH_2)_7$-NH-CH($CF_3$)-; *-$(CH_2)_6$-NH-CH($CF_3$)-; -$(CH_2)_5$-NH-CH($CF_3$)-; *-$(CH_2)_4$-NH-CH($CF_3$)-; -$(CH_2)_3$-NH-CH($CF_3$)- -$(CH_2)_2$-NH-CH($CF_3$)- -$(CH_2)_8$-NH-CH($CF_3$)-; *-$CH_2$-C(O)NH-$(CH_2)_4$-; *-$CH_2$-C(O)NH-$(CH_2)_2$-; *-$CH_2$-C(O)NH-$(CH_2)_3$-; *-$CH_2$-C(O)NH-$(CH_2)_5$-; *-$(CH_2)_2$-phenylene-$(CH_2)_1$-; *-$(CH_2)_1$-phenylene-$(CH_2)_2$-; *-$(CH_2)_1$-phenylene-$(CH_2)_3$-; *-$(CH_2)_3$-phenylene-$(CH_2)_1$-; *-$(CH_2)_4$-phenylene-$(CH_2)_1$-; *-$(CH_2)_1$-phenylene-$(CH_2)_4$-; *-$(CH_2)_2$-phenylene-$(CH_2)_2$-; *-$CH_2$-phenylene-$CH_2$-NH-CH($CH_3$)-; *-$CH_2$-phenylene-$(CH_2)_2$-NH-CH($CH_3$)-; *-$CH_2$-phenylene-$CH_2$-NH-$CH_2$-; *-$CH_2$-phenylene-$(CH_2)_2$-NH-$CH_2$-; *-$(CH_2)_1$-C(O)NH-$(CH_2)_4$-; *-$(CH_2)_1$-furanylene-$(CH_2)_1$-; *-$(CH_2)_1$-furanylene-$(CH_2)_2$-; *-$(CH_2)_1$-furanylene-$(CH_2)_3$-; *-$(CH_2)_2$-furanylene-$(CH_2)_1$-; *-$(CH_2)_2$-furanylene-$(CH_2)_2$-; *-$(CH_2)_3$-furanylene-$(CH_2)_1$-; *-$(CH_2)_3$-furanylene-$(CH_2)_2$-; *-$(CH_2)_1$-thiazolylene-$(CH_2)_1$-; *-$(CH_2)_1$-thiazolylene-$(CH_2)_2$-; *-$(CH_2)_1$-thiazolylene-$(CH_2)_3$-; *-$(CH_2)_2$-thiazolylene-$(CH_2)_1$-; *-$(CH_2)_2$-thiazolylene-$(CH_2)_2$-; *-$(CH_2)_3$-thiazolylene-$(CH_2)_1$-; *-$(CH_2)_3$-thiazolylene-$(CH_2)_2$-; or *-$CH_2$-thiazolylene-$CH_2$-NH-$CH_2$-;

wherein a hydrogen of one or more $CH_2$ of the groups is optionally replaced with a substituent selected from the group consisting of: halogen, cyano, hydroxy, amino, mercapto, $C_{1-3}$alkyl, $C_{1-3}$ alkoxy, trifluoromethyl, $C_{5-10}$ aryl, 5- to 10-membered heteroaryl, $C_{3-12}$cycloalkyl, 3- to 12-membered heterocyclyl, or any combination thereof;

the piperazinylene is optionally substituted with a substituent selected from the group consisting of halogen, cyano, hydroxy, amino, mercapto, oxo, $C_{1-3}$ alkoxy, halogenated $C_{1-3}$alkyl, methanesulfonyloxy, trifluoromethanesulfonyloxy, p-toluenesulfonyloxy, optionally deuterated $C_{1-6}$ alkyl, optionally deuterated $C_{3-6}$ cycloalkyl, or any combination thereof,

the phenylene, the furanylene, and the thiazolylene are each independently optionally substituted with a substituent selected from the group consisting of halogen, cyano, hydroxy, amino, mercapto, $C_{1-3}$ alkoxy, halogenated $C_{1-3}$alkyl, methanesulfonyloxy, trifluoromethanesulfonyloxy, *p*-toluenesulfonyloxy, optionally deuterated $C_{1-6}$ alkyl, optionally deuterated $C_{3-6}$ cycloalkyl, or any combination thereof; and

* indicates the point of attachment to R.

**16.** The compound of Formula (I) or salts, enantiomers, diastereoisomer, isotopically enriched analogs, solvates,

prodrugs, or polymorphs thereof of claim 14, wherein L represents the following optionally substituted groups:

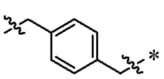 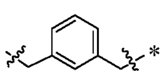 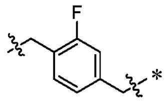 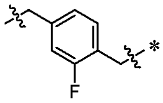 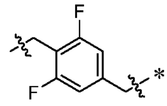 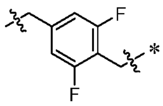 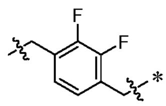

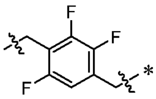 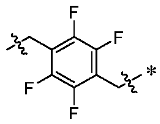 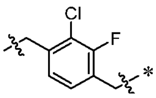 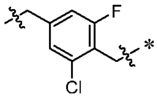 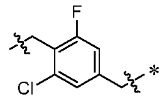 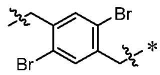 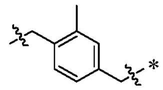

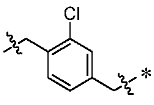 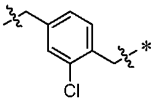 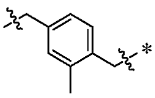 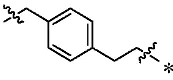 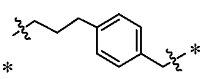 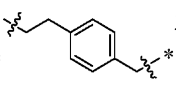 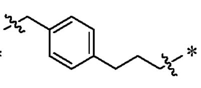

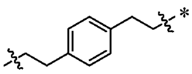 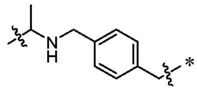 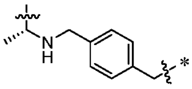 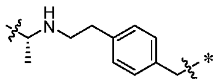 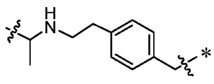 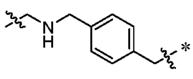

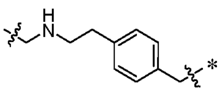 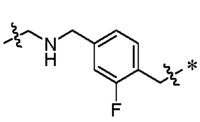 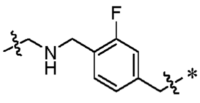 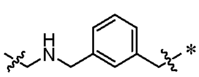 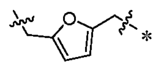 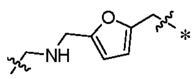

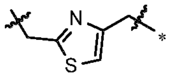 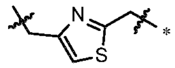 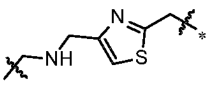 or 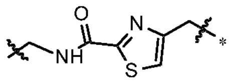 ;

wherein * indicates the point of attachment to R.

**17.** The compound of Formula (I) or salts, enantiomers, diastereoisomer, isotopically enriched analogs, solvates, prodrugs, or polymorphs thereof of any one of claims 9-16, wherein $X_1$ represents:

(i) linear or branched $C_{1-10}$ alkyl optionally substituted with one or more fluorine; or

(ii) cyclopropyl, cyclobutyl, 3,3-difluorocyclobutyl, cyclopentyl, cyclopentenyl, cyclohexyl, cyclohexenyl, cycloheptyl, cyclooctyl, decalinyl, octahydropentalenyl, octahydro-1H-indenyl, 2,3-dihydro-1H-indenyl, 4-cyano-2,3-dihydro-1H-indenyl, spiro[3.3]heptanyl, spiro[2.5]octyl, spiro[3.5]nonyl, spiro[4.4]nonyl, spiro[4.5]decyl, spiro[5.5]undecyl, *p*-menthanyl, *m*-menthanyl, quinuclidinyl, adamantanyl, noradamantanyl, bornyl, 3,5-dimethyladamantan-1-yl, 1,7,7-trimethylbicyclo[2.2.1]heptan-2-yl, 7,7-dimethylbicyclo[2.2.1]heptan-1-yl, 7,7-dimethyl-2-oxobicyclo[2.2.1]heptan-1-yl, bicyclo[2.2.1]heptanyl, 2-oxobicyclo[2.2.1]heptyl, or bicyclo[2.2.1]heptenyl, with each group being optionally substituted with one or more substituents selected from the group consisting of: halogen, cyano, amino, hydroxy, mercapto, oxo, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$alkyl, $C_{1-3}$ alkoxycarbonyl, halogenated $C_{2-4}$ alkenyl, or any combination thereof, wherein the 5- or 6-membered heterocyclyl is optionally substituted with one or more substituents selected from the group consisting of: halogen, cyano, amino, hydroxy, mercapto, oxo, $C_{1-6}$alkyl, halogenated $C_{1-3}$ alkyl, or any combination thereof; and the $C_5$-$C_{20}$ aryl is optionally substituted with one or more substituents selected from the group consisting of: halogen, cyano, amino, hydroxy, mercapto, $C_{1-6}$alkyl, halogenated $C_{1-3}$alkyl, or any combination thereof; and the 5- or 6-membered heteroaryl is optionally substituted with one or more substituents selected from the group consisting of: halogen, cyano, amino, hydroxy, mercapto, oxo, $C_{1-6}$alkyl, halogenated $C_{1-3}$alkyl, or any combination thereof; or

(iii) phenyl or naphthyl, with each group being optionally substituted with one or more substituents selected from the group consisting of halogen, cyano, amino, hydroxy, mercapto, $C_{1-3}$ alkoxy, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesul-

fonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$alkyl, $C_{1-3}$ alkoxycarbonyl, halogenated $C_{2-4}$ alkenyl, or any combination thereof, wherein the 5- or 6-membered heterocyclyl is optionally substituted with one or more substituents selected from the group consisting of: halogen, cyano, amino, hydroxy, mercapto, oxo, $C_{1-6}$alkyl, halogenated $C_{1-3}$alkyl, or any combination thereof; and the $C_5$-$C_{20}$ aryl is optionally substituted with one or more substituents selected from the group consisting of: halogen, cyano, amino, hydroxy, mercapto, $C_{1-6}$alkyl, halogenated $C_{1-3}$alkyl, or any combination thereof; and the 5- or 6-membered heteroaryl is optionally substituted with one or more substituents selected from the group consisting of: halogen, cyano, amino, hydroxy, mercapto, oxo, $C_{1-6}$alkyl, halogenated $C_{1-3}$alkyl, or any combination thereof; or

(iv) azetidinyl, oxetanyl, pyrrolidinyl, imidazolidinyl, pyrazolidinyl, oxazolidinyl, thiazolidinyl, piperidinyl, piperazinyl, morpholinyl, thiomorpholinyl, azacycloheptyl, azacyclooctyl, diazacycloheptanyl, diazacyclooctyl, 3-azabicyclo[3.1.0]hexyl, 3-azabicyclo[3.1.1]heptanyl, 3-azabicyclo[4.1.0]heptanyl, 2-azabicyclo[2.2.1]heptanyl, 6-azabicyclo[3.1.1]heptanyl, 2-azabicyclo[2.2.2]octyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, 2,6-diazaspiro[3.3]heptanyl, 2,7-diazaspiro[3.5]nonyl, 3-azaspiro[5.5]undecyl, 5-azaspiro[2.4]heptanyl, 6-azaspiro[2.5]octyl, 2-oxa-7-azaspiro[3.5]nonyl, 7-azaspiro[3.5]nonyl, hexahydro-1H-isoindol-2(3H)-yl, (3aR,7aS)-octahydro-2H-isoindol-2-yl, octahydropyrrolo[3,4-c]pyrrolyl, or 5,7-dihydro-6H-pyrrolo[3,4-b]pyridyl, with each group being optionally substituted with one or more substituents selected from the group consisting of halogen, cyano, amino, hydroxy, mercapto, oxo, $C_{1-3}$ alkoxy, halogenated $C_{1-3}$ alkoxy, halogenated $C_{1-3}$alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, $C_{1-6}$alkyl, $C_{1-3}$ alkoxycarbonyl, halogenated $C_{2-4}$ alkenyl, or any combination thereof, wherein the 5- or 6-membered heterocyclyl is optionally substituted with one or more substituents selected from the group consisting of: halogen, cyano, amino, hydroxy, mercapto, oxo, $C_{1-6}$alkyl, halogenated $C_{1-3}$alkyl, or any combination thereof; and the $C_5$-$C_{20}$ aryl is optionally substituted with one or more substituents selected from the group consisting of: halogen, cyano, amino, hydroxy, mercapto, $C_{1-6}$alkyl, halogenated $C_{1-3}$alkyl, or any combination thereof; and the 5- or 6-membered heteroaryl is optionally substituted with one or more substituents selected from the group consisting of: halogen, cyano, amino, hydroxy, mercapto, oxo, $C_{1-6}$alkyl, halogenated $C_{1-3}$alkyl, or any combination thereof; or

(v) furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, tetrahydro-2H-pyran-2-yl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl, or imidazo[2,1-b]thiazolyl, with each group being optionally substituted with one or more substituents selected from the group consisting of halogen, cyano, amino, hydroxy, mercapto, $C_{1-3}$ alkoxy, halogenated $C_{1-3}$ alkoxy, halogenated $C_{1-3}$alkyl, bis($C_{1-6}$ alkyl)phosphono, $C_{1-6}$ alkylsulfonyl, methanesulfonyloxy, trifluoromethanesulfonyloxy, *p*-toluenesulfonyloxy, $C_{1-6}$ alkyl, optionally substituted 5-or 6-membered heterocyclyl, optionally substituted $C_5$-$C_{20}$ aryl, optionally substituted 5- or 6-membered heteroaryl, $C_{1-6}$alkyl, $C_{1-3}$ alkoxycarbonyl, halogenated $C_{2-4}$ alkenyl, or any combination thereof, wherein the 5- or 6-membered heterocyclyl is optionally substituted with one or more substituents selected from the group consisting of: $C_{1-6}$ alkyl, cyano, halogenated $C_{1-3}$alkyl, amino, hydroxy, mercapto, halogen, or any combination thereof; and the $C_5$-$C_{20}$ aryl is optionally substituted with one or more substituents selected from the group consisting of: $C_{1-6}$ alkyl, cyano, halogenated $C_{1-3}$alkyl, amino, hydroxy, mercapto, halogen, or any combination thereof; and the 5- or 6-membered heteroaryl is optionally substituted with one or more substituents selected from the group consisting of: $C_{1-6}$alkyl, halogenated $C_{1-3}$alkyl, amino, hydroxy, mercapto, halogen, or any combination thereof; or

(vi) $X_1$ represents the group of Formula ($G_2$):

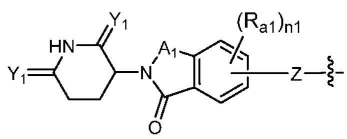

Formula ($G_2$)

wherein $A_1$ represents $CH_2$ or C(O), $Y_1$ represents O or S, and Z represents S, S(O), $S(O)_2$, O, NH or $CH_2$, and $(R_{a1})_{n1}$ denotes that the benzene ring is substituted with n1 $R_{a1}$, where $R_{a1}$ groups are the same or different and each independently represent deuterium, halogen, hydroxy, mercapto, nitro, amino, cyano, optionally deuterated $C_{1-6}$ alkyl, optionally deuterated $C_{1-6}$ alkoxy, halogenated $C_{1-6}$alkyl, optionally substituted $C_{3-6}$ cycloalkyl, $C_{2-6}$ alkenyl, or $C_{2-6}$ alkynyl, and n represents an integer of 0, 1, 2, or 3.

**18.** The compound of Formula (I) or salts, enantiomers, diastereoisomer, isotopically enriched analogs, solvates, prodrugs, or polymorphs thereof of any one of claims 9-16, wherein

(i) $X_1$ represents: morpholinyl, piperidinyl, difluoropiperidinyl, 4,4-dimethylpiperidin-1-yl, 3,5-dimethylpiperidin-1-yl, piperazinyl, 3-hydroxy-2-methylazetidin-1-yl, trifluoromethoxyazetidinyl, azetidinyl, cyanoazetidinyl, hydroxyazetidinyl, 3-hydroxy-3-methylazetidin-1-yl, 3-fluoro-4-hydroxypyrrolidin-1-yl, 3-methyl-4-(trifluoromethyl) pyrrolidin-1-yl, 7-fluoro-5-azaspiro[2.4]heptan-5-yl, 3-azabicyclo[3.1.0]hexan-3-yl, 6-(difluoromethyl)-3-azabicyclo[4.1.0]heptan-3-yl, 7-(3-fluoro-5,7-dihydro-6H-pyrrolo[3,4-b]pyridin-6-yl, 1,1-difluoro-5-azaspiro[2.4]heptan-5-yl, 1,1-difluoro-6-azaspiro[2.5]octan-6-yl, 2-oxa-7-azaspiro[3.5]nonan-7-yl, 4-(methylsulfonyl)piperazin-1-yl, 4-(ethylsulfonyl)piperazin-1-yl, 1,4-diazepan-1-yl, 3,8-diazabicyclo[3.2.1]octan-3-yl, 2-azabicyclo [3.2.1]octanyl, 1,4-diazabicyclo[3.2.1]octanyl, 2,5-diazabicyclo[2.2.2]octan-2-yl, pyrrolidinyl, adamantanyl, noradamantanyl, bornyl, or norbornyl; or

(ii) $X_1$ represents: piperidinyl-C(O)-, difluoropiperidinyl-C(O)-, $-C(O)N(CH(CH_3)_2)_2$, $C(O)NH_2$, N,N-diisopropylcarbamoyl, adamantanyl-NHC(O)-, 3,5-dimethyladamantan-1-yl-NHC(O)-, adamantan-2-yl-NHC(O)-, adamantanyl-C(O)NH-, adamantan-1-yl-C(O)NH-, 3-hydroxyadamantanyl-C(O)NH-, 3-hydroxyadamantan-1-yl-C(O) NH-, 3-chloroadamantan-1-yl-C(O)NH-, 4-chloroadamantan-1-yl-C(O)NH-, 2-chloroadamantan-1-yl-C(O) NH-, adamantan-2-yl-C(O)NH-, $-NHC(O)CH_3$, OH, adamantanyl-O-, adamantan-1-yl-O-, adamantan-2-yl-O-, norbornyl-O-, 1,7,7-trimethyl-bicyclo[2.2.1]heptanyl-O-, -SH, adamantanyl-S-, adamantan-1-yl-S-, 3-hydroxyadamantanyl-S-, 3-hydroxyadamantan-1-yl-S-, 3-chloroadamantan-1-yl-S-, 4-chloroadamantan-1-yl-S-, 2-chloroadamantan-1-yl-S-, adamantan-2-yl-S-, or 2-isopropyl-5-methylcyclohexyl-O-; or

(iii) $X_1$ represents $NR_2R_3$, where $R_2$ and $R_3$ each independently represent H or optionally substituted linear or branched $C_{1-10}$ alkyl, wherein $R_2$ and $R_3$ are not H at the same time, and the optionally substituted linear or branched $C_{1-10}$ alkyl is optionally substituted with one or more substituents selected from the group consisting of: halogen, cyano, hydroxy, amino, mercapto, $C_{1-3}$alkyl, $C_{1-3}$ alkoxy, trifluoromethyl, optionally substituted $C_{5-10}$ aryl, optionally substituted 5- to 10-membered heteroaryl, optionally substituted $C_{3-12}$cycloalkyl, optionally substituted 3- to 12-membered heterocyclyl, or any combination thereof, wherein the optionally substituted $C_{5-10}$ aryl, optionally substituted 5- to 10-membered heteroaryl, optionally substituted $C_{3-12}$ cycloalkyl and optionally substituted 3- to 12-membered heterocyclyl are each independently optionally substituted with one or more substituents selected from the group consisting of: halogen, cyano, amino, hydroxy, mercapto, $C_{1-6}$ alkyl, $C_{1-3}$ alkoxy, or any combination thereof; or

(iii) $X_1$ represents $NR_2R_3$, where $R_2$ represents H or $C_{1-3}$alkyl, and $R_3$ represents optionally substituted $C_3$-$C_{20}$ cycloalkyl, optionally substituted $C_5$-$C_{14}$ aryl, optionally substituted 3- to 20-membered heterocyclyl, or optionally substituted 5- to 20-membered heteroaryl,

wherein the $C_3$-$C_{20}$ cycloalkyl and 3- to 20-membered heterocyclyl are each independently optionally substituted with one or more substituents selected from the group consisting of: halogen, cyano, amino, hydroxy, mercapto, oxo, $C_{1-6}$ alkyl, $C_{1-3}$ alkoxy, $C_{1-3}$ alkoxycarbonyl, halogenated $C_{2-4}$ alkenyl, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl, or any combination thereof, wherein the 5- or 6-membered heterocyclyl is optionally substituted with a substituent selected from the group consisting of: halogen, cyano, amino, hydroxy, oxo, mercapto, $C_{1-6}$ alkyl, halogenated $C_{1-3}$ alkyl, or any combination thereof; and the $C_5$-$C_{20}$ aryl is optionally substituted with a substituent selected from the group consisting of: halogen, cyano, amino, hydroxy, mercapto, $C_{1-6}$ alkyl, halogenated $C_{1-3}$ alkyl, or any combination thereof; and the 5- or 6-membered heteroaryl is optionally substituted with a substituent selected from the group consisting of: halogen, cyano, amino, hydroxy, mercapto, $C_{1-6}$ alkyl, halogenated $C_{1-3}$ alkyl, or any combination thereof; and

the $C_5$-$C_{14}$ aryl and 5- to 20-membered heteroaryl are each independently optionally substituted with one or more substituents selected from the group consisting of: halogen, cyano, amino, hydroxy, mercapto, $C_{1-3}$ alkoxy, halogenated $C_{1-3}$ alkoxy, halogenated $C_{1-3}$ alkyl, $C_{1-6}$ alkylsulfonyl, bis($C_{1-6}$ alkyl)phosphono, methanesulfonyloxy, trifluoromethanesulfonyloxy, *p*-toluenesulfonyloxy, optionally substituted 5- or 6-membered heterocyclyl, optionally substituted $C_5$-$C_{20}$ aryl, optionally substituted 5- or 6-membered heteroaryl,

$C_{1-6}$ alkyl, $C_{1-3}$ alkoxycarbonyl, halogenated $C_{2-4}$ alkenyl, $-C(O)NHR_d$, or any combination thereof, wherein $R_d$ represents $C_{1-5}$ alkyl or optionally substituted phenyl, and the optionally substituted phenyl is optionally substituted with one or more substituents selected from the group consisting of: halogen, cyano, amino, hydroxy, mercapto, oxo, and $C_{1-6}$ alkyl, or any combination thereof; and the 5- or 6-membered heterocyclyl is optionally substituted with a substituent selected from the group consisting of: halogen, cyano, amino, hydroxy, oxo, mercapto, $C_{1-6}$ alkyl, halogenated $C_{1-3}$ alkyl, or any combination thereof; and the $C_5$-$C_{20}$ aryl is optionally substituted with a substituent selected from the group consisting of: halogen, cyano, amino, hydroxy, mercapto, $C_{1-6}$ alkyl, halogenated $C_{1-3}$ alkyl, or any combination thereof; and the 5- or 6-membered heteroaryl is optionally substituted with a substituent selected from the group consisting of: halogen, cyano, amino, hydroxy, oxo, mercapto, $C_{1-6}$ alkyl, halogenated $C_{1-3}$ alkyl, or any combination thereof.

**19.** The compound of Formula (I) or salts, enantiomers, diastereoisomer, isotopically enriched analogs, solvates, prodrugs, or polymorphs thereof of any one of claims 9-16, wherein

(i) $X_1$ represents $CF_2CF_3$, $CF_3$, adamantanyl-NH-, noradamantanyl-NH-, cyclohexyl-NH-, adamantanyl-$N(CH_3)$-, noradamantanyl-$N(CH_3)$-, bornyl-NH-, norbornyl-NH-, spiro[3.3]heptyl-NH-, $N(CH_2CH_3)_2$, phenyl, 4-fluorophenyl, 4-chlorophenyl, 3,5-difluorophenyl, 4-(bromomethyl)-2-fluorophenyl, or 3,4,5-trifluorophenyl; or
(ii) $X_1$ represents $NR_2R_3$, wherein $R_2$ represents H, and $R_3$ represents ethyl substituted with 2,6-dichloro-3-fluorophenyl; or
(iii) $X_1$ represents $NR_2R_3$, wherein

$R_2$ represents H, methyl, ethyl, isopropyl, or cyclohexyl, and
$R_3$ represents the following optionally substituted groups: adamantanyl, noradamantanyl, norbornyl, cyclohexyl, cyclopentyl, cyclopropyl, cyclobutyl, fluorocyclobutyl, difluorocyclobutyl, difluorocyclopentyl, hydroxycyclohexyl, ethyl, isopropyl, *tert*-butyl, methyl, 2,4-dimethylpent-3-yl, dicyclopropylmethyl, spiro[3.3]heptyl, 2-methoxycyclopropyl, hexahydro-1H-isoindol-2(3H)-yl, bicyclo[1.1.1]pentyl, bicyclo[2.2.2]octyl, 4-hydroxybicyclo[2.2.2]octan-1-yl, 4,4-dimethylcyclohexyl, oxetanyl, oxazolyl, 2,3-dihydro-1H-indenyl, quinuclidinyl, 1,7,7-trimethylbicyclo[2.2.1]heptyl, 7,7-dimethylbicyclo[2.2.1]heptyl, bicyclo[2.2.1]heptyl, 1-cyclopropylethyl,

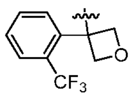 or 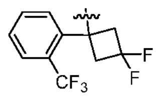 ;

or

(iv) $X_1$ represents $NR_2R_3$, wherein $R_2$ represents H, and $R_3$ represents -thiazolyl-$C(O)NHR_d$, wherein $R_a$ represents $C_{1-5}$ alkyl or 2-chloro-6-methylphenyl; or
(v) $X_1$ represents $NR_2R_3$, wherein $R_2$ represents H, and $R_3$ represents pyrimidin-4-yl, pyridin-4-yl, or quinazolin-4-yl; or
(vi) $X_1$ represents $NR_2R_3$, wherein $R_2$ and $R_3$ represents phenyl; or
(vii) $X_1$ represents $NHCH_3$, $N(CH_3)_2$, $N(CH_2CH_3)_2$ or $N(CH(CH_3)_2)_2$; or
(viii) $X_1$ represents $NHC(O)NR_7R_8$, wherein $R_7$ represents H, and $R_8$ represents adamantanyl, adamantan-1-yl, adamantan-2-yl, 3,5-dimethyladamantan-1-yl, phenyl, or 3-chloro-4-methylphenyl; or
(ix) $X_1$ represents $NR_2R_3$, wherein $R_2$ represents H or methyl, and $R_3$ represents pyrimidinyl, pyridyl, quinazolinyl, 6,7-difluoroquinazolin-4-yl, phenyl, 3-chloro-4-methylphenyl, 4-fluorophenyl, 3,5-difluorophenyl, 3,4,5-trifluorophenyl, 2-(dimethylphosphono)phenyl, 2-(isopropylsulfonyl)phenyl, 4-(pyridin-3-yl)pyrimidin-2-yl, indolyl, 1-methyl-1H-indol-7-yl, benzothienyl, benzo[b]thien-7-yl, benzofuran-7-yl, benzofuranyl, 3-cyano-quinol-4-yl, quinolyl, thiazolyl, adamantanyl, adamantan-1-yl, adamantan-2-yl, 3,5-dimethyladamantan-1-yl, 1,7,7-trimethylbicyclo[2.2.1]heptan-2-yl, 4-(4-methylpiperazin-1-yl)piperidin-1-yl, cyclohexyl, dimethylcyclohexyl, 4,4-dimethylcyclohexyl, spiro-cycloalkyl, spiro[5.5]undec-3-yl, spiro[3.3]heptyl, spiro[3.3]hept-2-yl, pyrrolidinyl, azepanyl, azacyclooctanyl, piperidinyl, dimethylpiperidinyl, or azaspirocycloalkyl.

**20.** The compound of Formula (I) or salts, enantiomers, diastereoisomer, isotopically enriched analogs, solvates, prodrugs, or polymorphs thereof of any one of claims 9-16, wherein

$X_1$ represents a quaternary ammonium salt group of Formula $N^+R_aR_bR_cX^-$, wherein
$R_a$, $R_b$, and $R_c$ each independently represent $C_{1-10}$ alkyl, and $X^-$ represents $Cl^-$; or

$R_a$ and $R_b$ each independently represent methyl, ethyl, propyl, isopropyl or butyl, $R_c$ represents ethyl, and $X^-$ represents $Cl^-$; or

$R_a$, $R_b$, and $R_c$ represent ethyl, and $X^-$ represents $Cl^-$; or

$R_a$, $R_b$, and $R_c$ represent methyl, and $X^-$ represents $Cl^-$; or

$X_1$ represents

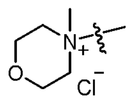 or 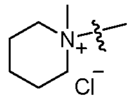 .

**21.** The compound of Formula (I) or salts, enantiomers, diastereoisomer, isotopically enriched analogs, solvates, prodrugs, or polymorphs thereof of any one of claims 9-16, wherein $X_1$ represents:
morpholinyl, piperidinyl, 2-oxopiperidin-1-yl, piperazinyl, N-methylpiperazinyl, 4-(methylsulfonyl)piperazin-1-yl, 4-(ethylsulfonyl)piperazin-1-yl, (N-methylpiperazinyl)piperidinyl, 3,5-dimethylpiperazinyl, 1,4-diazepan-1-yl, 4-methyl-1,4-diazepan-1-yl, 1-methyl-1,4-diazepan-1-yl, 8-methyl-3,8-diazabicyclo[3.2.1]octan-3-yl, 1,4-diazabicyclo[3.2.1]octan-4-yl, 5-methyl-2,5-diazabicyclo[2.2.2]octan-2-yl, 8,8-difluoro-2-azabicyclo[3.2.1]octan-2-yl, 4-(4-fluorophenyl)piperazin-1-yl, 4-(3,4-difluorophenyl)piperazin-1-yl, 2-oxopiperidin-1-yl, 2-oxopyrrolidin-1-yl, 3-fluoro-4-hydroxypyrrolidin-1-yl, 3-methyl-4-(trifluoromethyl)pyrrolidin-1-yl, 2,6-dioxopiperidin-3-yl, 4,4-dimethylpiperidin-1-yl, 3,5-dimethylpiperidin-1-yl, 4,4-difluoropiperidin-1-yl, 4-methylpiperazin-1-yl, tetrahydro-2H-pyran-2-yl, pyridyl, pyrimidinyl, thiazolyl, quinolyl, 6,7-difluoroquinazolin-4-yl, 3-cyano-quinolyl, indolyl, 1-methyl-1H-indol-7-yl, benzothienyl, benzofuranyl, quinazolinyl, 6,7-difluoroquinazolin-4-yl, pyrrolidinyl, pyrrolidin-1-yl, 4-(pyridin-3-yl)pyrimidin-2-yl, 3-cyanoazetidinyl, 3-hydroxyazetidinyl, 3-hydroxy-3-methylazetidinyl, 3-hydroxy-2-methylazetidin-1-yl, 3-(trifluoromethoxy)azetidin-1-yl, azepan-1-yl, azacyclooctan-1-yl, 7-fluoro-5-azaspiro[2.4]heptan-5-yl, 1,1-difluoro-5-azaspiro[2.4]heptan-5-yl, 1,1-difluoro-6-azaspiro[2.5]octan-6-yl, 6-azaspiro[2.5]octan-6-yl, 2-oxa-7-azaspiro[3.5]nonan-7-yl, 3-azabicyclo[3.1.0]hexan-3-yl, 6-(difluoromethyl)-3-azabicyclo[4.1.0]heptan-3-yl, 3-fluoro-5,7-dihydro-6H-pyrrolo[3,4-b]pyridin-6-yl, 3-(trifluoromethyl)-5,7-dihydro-6H-pyrrolo[3,4-b]pyridin-6-yl, isoindol-2-yl, thiomorpholino, [1,4'-dipiperidin]-1'-yl, (2-bromovinyl)phenyl,

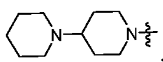, 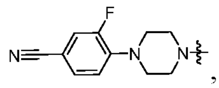, 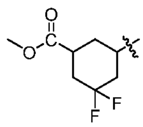,

or 4,4-difluoropiperidinyl, wherein * indicates the point of attachment to R.

**22.** The compound of Formula (I) or salts, enantiomers, diastereoisomer, isotopically enriched analogs, solvates, prodrugs, or polymorphs thereof of any one of claims 9-16, wherein $X_1$ represents $NR_2R_3$, wherein

$R_2$ represents H, methyl, ethyl, isopropyl, or cyclohexyl, and

$R_3$ represents bicyclo[1.1.1]pent-1-yl, 4,4-dimethylcyclohexyl, 2-methoxycyclopropyl, 3-fluorocyclobutyl, 3,3-difluorocyclobutyl, 3,3-difluoro-1-(2-(trifluoromethyl)phenyl)cyclobutyl, 3-(2-(trifluoromethyl)phenyl)oxetan-3-yl, 3,3-difluorocyclopentyl, 3-hydroxycyclohexyl, 3-hydroxycyclohexyl, 3-cyano-bicyclo[1.1.1]pent-l-yl, bicyclo[2.2.2]octan-1-yl, 4-hydroxybicyclo[2.2.2]octan-1-yl, 2-isopropyl-5-methylcyclohexyl, 5-methyloxazol-2-yl, 4-cyano-2,3-dihydro-1H-inden-1-yl, 2,4-dimethylpentan-3-yl, dicyclopropylmethyl, quinuclidin-3-yl, (S)-quinuclidin-3-yl, (R)-quinuclidin-3-yl, adamantan-1-yl, 3-hydroxyadamantanyl, 3-hydroxyadamantan-1-yl, 3-chloroadamantan-1-yl, 4-chloroadamantan-1-yl, 2-chloroadamantan-1-yl, adamantan-2-yl, 1,7,7-trimethylbicyclo[2.2.1]heptan-2-yl, 7,7-dimethylbicyclo[2.2.1]heptan-1-yl, 7,7-dimethyl-2-oxobicyclo[2.2.1]heptan-1-yl, 3,5-dimethyladamantanyl, 3,5-dimethyladamantan-1-yl, hexahydro-2,5-methanopentalen-3a(1H)-yl, bicyclo[2.2.1]heptyl, bicyclo[2.2.1]heptan-2-yl, 1-cyclopropylethyl,

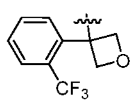, 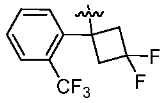, or 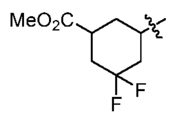.

**23.** The compound of Formula (I) or salts, enantiomers, diastereoisomer, isotopically enriched analogs, solvates, prodrugs, or polymorphs thereof of any one of claims 9-16, wherein $X_1$ represents the group of Formula ($G_2$):

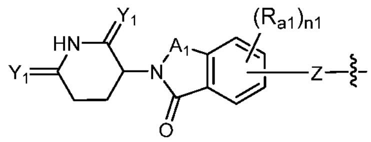

Formula ($G_2$)

wherein $A_1$ represents $CH_2$ or C(O), $Y_1$ represents O, and Z represents S, S(O) or $S(O)_2$, and $(R_{a1})_{n1}$ represents denotes that the benzene ring is substituted with n1 $R_{a1}$, where $R_{a1}$ groups are the same or different and each independently represent deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, optionally substituted $C_{3-6}$ cycloalkyl, $C_{2-6}$ alkenyl, or $C_{2-6}$ alkynyl, and n represents an integer of 0, 1, 2, or 3.

**24.** The compound of Formula (I) or salts, enantiomers, diastereoisomer, isotopically enriched analogs, solvates, prodrugs, or polymorphs thereof of claim 1, which is selected from:

3-(5-fluoro-4-((8-morpholinooctyl)thio)-1-oxoisoindolin-2-yl)piperidine-2,6-dione;
3-(6-fluoro-4-((8-morpholinooctyl)thio)-1-oxoisoindolin-2-yl)piperidine-2,6-dione;
3-(7-fluoro-4-((8-morpholinooctyl)thio)-1-oxoisoindolin-2-yl)piperidine-2,6-dione;
3-(4-((8-(diethylamino)octyl)thio)-5-fluoro-1-oxoisoindolin-2-yl)piperidine-2,6-dione;
3-(4-((8-(diethylamino)octyl)thio)-6-fluoro-1-oxoisoindolin-2-yl)piperidine-2,6-dione;
3-(4-((8-(diethylamino)octyl)thio)-7-fluoro-1-oxoisoindolin-2-yl)piperidine-2,6-dione;
3-(5-fluoro-1-oxo-4-((7-(piperidin-1-yl)heptyl)thio)isoindolin-2-yl)piperidine-2,6-dione;
3-(6-fluoro-1-oxo-4-((7-(piperidin-1-yl)heptyl)thio)isoindolin-2-yl)piperidine-2,6-dione;
3-(7-fluoro-1-oxo-4-((7-(piperidin-1-yl)heptyl)thio)isoindolin-2-yl)piperidine-2,6-dione;
3-(4-((7-(4,4-difluoropiperidin-1-yl)heptyl)thio)-1-oxoisoindolin-2-yl)piperidine-2,6-dione;
3-(4-((7-(4,4-difluoropiperidin-1-yl)heptyl)thio)-5-fluoro-1-oxoisoindolin-2-yl)piperidine-2,6-dione;
3-(4-((7-(4,4-difluoropiperidin-1-yl)heptyl)thio)-6-fluoro-1-oxoisoindolin-2-yl)piperidine-2,6-dione;
3-(4-((7-(4,4-difluoropiperidin-1-yl)heptyl)thio)-7-fluoro-1-oxoisoindolin-2-yl)piperidine-2,6-dione;
3-(5-fluoro-1-oxo-4-((7-oxo-7-(piperidin-1-yl)heptyl)thio)isoindolin-2-yl)piperidine-2,6-dione;
3-(6-fluoro-1-oxo-4-((7-oxo-7-(piperidin-1-yl)heptyl)thio)isoindolin-2-yl)piperidine-2,6-dione;
3-(7-fluoro-1-oxo-4-((7-oxo-7-(piperidin-1-yl)heptyl)thio)isoindolin-2-yl)piperidine-2,6-dione;
3-(4-((7-(4,4-difluoropiperidin-1-yl)-7-oxoheptyl)thio)-5-fluoro-1-oxoisoindolin-2-yl)piperidine-2,6-dione;
3-(4-((7-(4,4-difluoropiperidin-1-yl)-7-oxoheptyl)thio)-6-fluoro-1-oxoisoindolin-2-yl)piperidine-2,6-dione;
3-(4-((7-(4,4-difluoropiperidin-1-yl)-7-oxoheptyl)thio)-7-fluoro-1-oxoisoindolin-2-yl)piperidine-2,6-dione;
7-((2-(2,6-dioxopiperidin-3-yl)-5-fluoro-1-oxoisoindolin-4-yl)thio)-N,N-diisopropylheptanamide;
7-((2-(2,6-dioxopiperidin-3-yl)-6-fluoro-1-oxoisoindolin-4-yl)thio)-N,N-diisopropylheptanamide;
7-((2-(2,6-dioxopiperidin-3-yl)-7-fluoro-1-oxoisoindolin-4-yl)thio)-N,N-diisopropylheptanamide;
3-(4-((7-((adamantan-1-yl)amino)heptyl)thio)-5-fluoro-1-oxoisoindolin-2-yl)piperidine-2,6-dione;
3-(4-((4-((adamantan-1-yl)amino)butyl)thio)-6-fluoro-1-oxoisoindolin-2-yl)piperidine-2,6-dione;
3-(4-((4-((adamantan-1-yl)amino)butyl)thio)-5-fluoro-1-oxoisoindolin-2-yl)piperidine-2,6-dione;
3-(4-((4-((adamantan-1-yl)amino)butyl)thio)-7-fluoro-1-oxoisoindolin-2-yl)piperidine-2,6-dione ;
3-(4-((7-((adamantan-1-yl)amino)heptyl)thio)-6-fluoro-1-oxoisoindolin-2-yl)piperidine-2,6-dione;
3-(4-((7-((adamantan-1-yl)amino)heptyl)thio)-7-fluoro-1-oxoisoindolin-2-yl)piperidine-2,6-dione;
3-(5-fluoro-1-oxo-4-((4-(piperidin-1-ylmethyl)benzyl)thio)isoindolin-2-yl)piperidine-2,6-dione;
3-(6-fluoro-1-oxo-4-((4-(piperidin-1-ylmethyl)benzyl)thio)isoindolin-2-yl)piperidine-2,6-dione;
3-(7-fluoro-1-oxo-4-((4-(piperidin-1-ylmethyl)benzyl)thio)isoindolin-2-yl)piperidine-2,6-dione;
3-(4-((4-((4,4-difluoropiperidin-1-yl)methyl)benzyl)thio)-5-fluoro-1-oxoisoindolin-2-yl)piperidine-2,6-dione;
3-(4-((4-((4,4-difluoropiperidin-1-yl)methyl)benzyl)thio)-6-fluoro-1-oxoisoindolin-2-yl)piperidine-2,6-dione;
3-(4-((4-((4,4-difluoropiperidin-1-yl)methyl)benzyl)thio)-7-fluoro-1-oxoisoindolin-2-yl)piperidine-2,6-dione;
3-(4-((7-(cyclohexylamino)heptyl)thio)-5-fluoro-1-oxoisoindolin-2-yl)piperidine-2,6-dione;
3-(4-((7-(cyclohexylamino)heptyl)thio)-6-fluoro-1-oxoisoindolin-2-yl)piperidine-2,6-dione;
3-(4-((7-(cyclohexylamino)heptyl)thio)-7-fluoro-1-oxoisoindolin-2-yl)piperidine-2,6-dione;
3-(4-((7-((adamantan-1-yl)(methyl)amino)heptyl)thio)-5-fluoro-1-oxoisoindolin-2-yl)piperidine-2,6-dione;

3-(4-((7-((adamantan-1-yl)(methyl)amino)heptyl)thio)-6-fluoro-1-oxoisoindolin-2-yl)piperidine-2,6-dione;
3-(4-((7-((adamantan-1-yl)(methyl)amino)heptyl)thio)-7-fluoro-1-oxoisoindolin-2-yl)piperidine-2,6-dione;
3-(5-fluoro-1-oxo-4-7-((1R,2S,4R)-1,7,7-trimethylbicyclo[2.2.1]heptan-2-yl)amino)heptyl)thio)isoindolin-2-yl)piperidine-2,6-dione;
3-(6-fluoro-1-oxo-4-((7-(((1R,2S,4R)-1,7,7-trimethiylbicyclo[2.2.1]heptan-2-yl)amino)heptyl)thio)isoindolin-2-yl)piperidine-2,6-dione;
3-(7-fluoro-1-oxo-4-((7-(((1R,2S,4R)-1,7,7-trimethylbicyclo[2.2.1]heptan-2-yl)amino)heptyl)thio)isoindolin-2-yl)piperidine-2,6-dione;
3-(5-fluoro-1-oxo-4-((4-((((1R,2S,4R)-1,7,7-trimethylbicyclo[2.2.1]heptan-2-yl)amino)methyl)benzyl)thio)isoindolin-2-yl)piperidine-2,6-dione;
3-(6-fluoro-1-oxo-4-((4-((((1R,2S,4R)-1,7,7-trimethylbicyclo[2.2.1]heptan-2-yl)amino)methyl)benzyl)thio)isoindolin-2-yl)piperidine-2,6-dione;
3-(7-fluoro-1-oxo-4-((4-((((1R,2S,4R)-1,7,7-trimethylbicyclo[2.2.1]heptan-2-yl)amino)methyl)benzyl)thio)isoindolin-2-yl)piperidine-2,6-dione;
3-(4-((4-(((adamantan-1-yl)amino)methyl)benzyl)thio)-5-fluoro-1-oxoisoindolin-2-yl)piperidine-2,6-dione;
3-(4-((4-(((adamantan-1-yl)amino)methyl)benzyl)thio)-6-fluoro-1-oxoisoindolin-2-yl)piperidine-2,6-dione;
3-(4-((4-(((adamantan-1-yl)amino)methyl)benzyl)thio)-7-fluoro-1-oxoisoindolin-2-yl)piperidine-2,6-dione;
3-(5-fluoro-1-oxo-4-((7-(spiro[3.3]heptan-2-ylamino)heptyl)thio)isoindolin-2-yl)piperidine-2,6-dione;
3-(6-fluoro-1-oxo-4-((7-(spiro[3.3]heptan-2-ylamino)heptyl)thio)isoindolin-2-yl)piperidine-2,6-dione;
3-(7-fluoro-1-oxo-4-((7-(spiro[3.3]heptan-2-ylamino)heptyl)thio)isoindolin-2-yl)piperidine-2,6-dione;
3-(4-(8-((adamantan-1-yl)amino)octyl)-5-fluoro-1-oxoisoindolin-2-yl)piperidine-2,6-dione;
3-(4-(8-((adamantan-1-yl)amino)octyl)-6-fluoro-1-oxoisoindolin-2-yl)piperidine-2,6-dione;
3-(4-(8-((adamantan-1-yl)amino)octyl)-7-fluoro-1-oxoisoindolin-2-yl)piperidine-2,6-dione;
3-(4-((7-((yl)amino)heptyl)oxy)-5-fluoro-1-oxoisoindolin-2-yl)piperidine-2,6-dione;
3-(4-((7-((adamantan-1-yl)amino)heptyl)oxy)-6-fluoro-1-oxoisoindolin-2-yl)piperidine-2,6-dione;
3-(4-((7-((adamantan-1-yl)amino)heptyl)oxy)-7-fluoro-1-oxoisoindolin-2-yl)piperidine-2,6-dione;
3-(4-((7-((adamantan-1-yl)amino)heptyl)amino)-5-fluoro-1-oxoisoindolin-2-yl)piperidine-2,6-dione;
3-(4-((7-((adamantan-1-yl)amino)heptyl)amino)-6-fluoro-1-oxoisoindolin-2-yl)piperidine-2,6-dione;
3-(4-((7-((adamantan-1-yl)amino)heptyl)amino)-7-fluoro-1-oxoisoindolin-2-yl)piperidine-2,6-dione;
3-(5-fluoro-4-((3-fluoro-4-(piperidin-1-ylmethyl)benzyl)thio)-1-oxoisoindolin-2-yl)piperidine-2,6-dione;
3-(6-fluoro-4-((3-fluoro-4-(piperidin-1-ylmethyl)benzyl)thio)-1-oxoisoindolin-2-yl)piperidine-2,6-dione;
3-(7-fluoro-4-((3-fluoro-4-(piperidin-1-ylmethyl)benzyl)thio)-1-oxoisoindolin-2-yl)piperidine-2,6-dione;
3-(4-((4-((4,4-difluoropiperidin-1-yl)methyl)-3-fluorobenzyl)thio)-1-oxoisoindolin-2-yl)piperidine-2,6-dione;
3-(4-((4-((4,4-difluoropiperidin-1-yl)methyl)-3-fluorobenzyl)thio)-5-fluoro-1-oxoisoindolin-2-yl)piperidine-2,6-dione;
3-(4-((4-((4,4-difluoropiperidin-1-yl)methyl)-3-fluorobenzyl)thio)-6-fluoro-1-oxoisoindolin-2-yl)piperidine-2,6-dione;
3-(4-((4-((4,4-difluoropiperidin-1-yl)methyl)-3-fluorobenzyl)thio)-7-fluoro-1-oxoisoindolin-2-yl)piperidine-2,6-dione;
3-(4-((4-(2-((adamantan-1-yl)amino)ethyl)benzyl)thio)-5-fluoro-1-oxoisoindolin-2-yl)piperidine-2,6-dione;
3-(4-((4-(2-((adamantan-1-yl)amino)ethyl)benzyl)thio)-6-fluoro-1-oxoisoindolin-2-yl)piperidine-2,6-dione;
3-(4-((4-(2-((adamantan-1-yl)amino)ethyl)benzyl)thio)-7-fluoro-1-oxoisoindolin-2-yl)piperidine-2,6-dione;
3-(4-(((4-(((adamantan-1-yl)amino)methyl)thiazol-2-yl)methyl)thio)-5-fluoro-1-oxoisoindolin-2-yl)piperidine-2,6-dione;
3-(4-(((4-(((adamantan-1-yl)amino)methyl)thiazol-2-yl)methyl)thio)-6-fluoro-1-oxoisoindolin-2-yl)piperidine-2,6-dione;
3-(4-(((4-(((adamantan-1-yl)amino)methyl)thiazol-2-yl)methyl)thio)-7-fluoro-1-oxoisoindolin-2-yl)piperidine-2,6-dione;
2-(2,6-dioxopiperidin-3-yl)-5-fluoro-4-((8-morpholinooctyl)thio)isoindoline-1,3-dione;
2-(2,6-dioxopiperidin-3-yl)-6-fluoro-4-((8-morpholinooctyl)thio)isoindoline-1,3-dione;
2-(2,6-dioxopiperidin-3-yl)-4-fluoro-7-((8-morpholinooctyl)thio)isoindoline-1,3-dione;
4-((8-(diethylamino)octyl)thio)-2-(2,6-dioxopiperidin-3-yl)-3-fluoroisoindoline-1,3-dione;
4-((8-(diethylamino)octyl)thio)-2-(2,6-dioxopiperidin-3-yl)-6-fluoroisoindoline-1,3-dione;
4-((8-(diethylamino)octyl)thio)-2-(2,6-dioxopiperidin-3-yl)-7-fluoroisoindoline-1,3-dione;
2-(2,6-dioxopiperidin-3-yl)-5-fluoro-4-((7-(piperidin-1-yl)heptyl)thio)isoindoline-1,3-dione;
2-(2,6-dioxopiperidin-3-yl)-6-fluoro-4-((7-(piperidin-1-yl)heptyl)thio)isoindoline-1,3-dione;
2-(2,6-dioxopiperidin-3-yl)-4-fluoro-7-((7-(piperidin-1-yl)heptyl)thio)isoindoline-1,3-dione;
4-((7-(4,4-difluoropiperidin-1-yl)heptyl)thio)-2-(2,6-dioxopiperidin-3-yl)isoindoline-1,3-dione;

4-((7-(4,4-difluoropiperidin-1-yl)heptyl)thio)-2-(2,6-dioxopiperidin-3-yl)-5-fluoroisoindoline-1,3-dione;
4-((7-(4,4-difluoropiperidin-1-yl)heptyl)thio)-2-(2,6-dioxopiperidin-3-yl)-6-fluoroisoindoline-1,3-dione;
4-((7-(4,4-difluoropiperidin-1-yl)heptyl)thio)-2-(2,6-dioxopiperidin-3-yl)-7-fluoroisoindoline-1,3-dione;
2-(2,6-dioxopiperidin-3-yl)-5-fluoro-4-((7-oxo-7-(piperidin-1-yl)heptyl)thio)isoindoline-1,3-dione;
2-(2,6-dioxopiperidin-3-yl)-6-fluoro-4-((7-oxo-7-(piperidin-1-yl)heptyl)thio)isoindoline-1,3-dione;
2-(2,6-dioxopiperidin-3-yl)-4-fluoro-7-((7-oxo-7-(piperidin-1-yl)heptyl)thio)isoindoline-1,3-dione;
4-((7-(4,4-difluoropiperidin-1-yl)-7-oxoheptyl)thio)-2-(2,6-dioxopiperidin-3-yl)-5-fluoroisoindoline-1,3-dione;
4-((7-(4,4-difluoropiperidin-1-yl)-7-oxoheptyl)thio)-2-(2,6-dioxopiperidin-3-yl)-6-fluoroisoindoline-1,3-dione;
4-((7-(4,4-difluoropiperidin-1-yl)-7-oxoheptyl)thio)-2-(2,6-dioxopiperidin-3-yl)-7-fluoroisoindoline-1,3-dione;
7-((2-(2,6-dioxopiperidin-3-yl)-5-fluoro-1,3-dioxoisoindolin-4-yl)thio)-N,N-diisopropylheptanamide;
7-((2-(2,6-dioxopiperidin-3-yl)-6-fluoro-1,3-dioxoisoindolin-4-yl)thio)-N,N-diisopropylheptanamide;
7-((2-(2,6-dioxopiperidin-3-yl)-7-fluoro-1,3-dioxoisoindolin-4-yl)thio)-N,N-diisopropylheptanamide;
4-((7-((adamantan-1-yl)amino)heptyl)thio)-2-(2,6-dioxopiperidin-3-yl)-5-fluoroisoindoline-1,3-dione;
4-((7-((adamantan-1-yl)amino)heptyl)thio)-2-(2,6-dioxopiperidin-3-yl)-6-fluoroisoindoline-1,3-dione;
4-((7-((adamantan-1-yl)amino)heptyl)thio)-2-(2,6-dioxopiperidin-3-yl)-7-fluoroisoindoline-1,3-dione;
2-(2,6-dioxopiperidin-3-yl)-5-fluoro-4-((4-(piperidin-1-ylmethyl)benzyl)thio)isoindoline-1,3-dione;
2-(2,6-dioxopiperidin-3-yl)-6-fluoro-4-((4-(piperidin-1-ylmethyl)benzyl)thio)isoindoline-1,3-dione;
2-(2,6-dioxopiperidin-3-yl)-4-fluoro-7-((4-(piperidin-1-ylmethyl)benzyl)thio)isoindoline-1,3-dione;
4-((4-((4,4-difluoropiperidin-1-yl)methyl)benzyl)thio)-2-(2,6-dioxopiperidin-3-yl)isoindoline-1,3-dione;
4-((4-((4,4-difluoropiperidin-1-yl)methyl)benzyl)thio)-2-(2,6-dioxopiperidin-3-yl)-5-fluoroisoindoline-1,3-dione;
4-((4-((4,4-difluoropiperidin-1-yl)methyl)benzyl)thio)-2-(2,6-dioxopiperidin-3-yl)-6-fluoroisoindoline-1,3-dione;
4-((4-((4,4-difluoropiperidin-1-yl)methyl)benzyl)thio)-2-(2,6-dioxopiperidin-3-yl)-7-fluoroisoindoline-1,3-dione;
4-((7-(cyclohexylamino)heptyl)thio)-2-(2,6-dioxopiperidin-3-yl)-5-fluoroisoindoline-1,3-dione;
4-((7-(cyclohexylamino)heptyl)thio)-2-(2,6-dioxopiperidin-3-yl)-6-fluoroisoindoline-1,3-dione;
4-((7-(cyclohexylamino)heptyl)thio)-2-(2,6-dioxopiperidin-3-yl)-7-fluoroisoindoline-1,3-dione;
4-((7-((adamantan-1-yl)(methyl)amino)heptyl)thio)-2-(2,6-dioxopiperidin-3-yl)-5-fluoroisoindoline-1,3-dione;
4-((7-((adamantan-1-yl)(methyl)amino)heptyl)thio)-2-(2,6-dioxopiperidin-3-yl)-6-fluoroisoindoline-1,3-dione;
4-((7-((adamantan-1-yl)(methyl)amino)heptyl)thio)-2-(2,6-dioxopiperidin-3-yl)-7-fluoroisoindoline-1,3-dione;
2-(2,6-dioxopiperidin-3-yl)-5-fluoro-4-((7-(((1R,2S,4R)-1,7,7-trimethylbicyclo[2.2.1]heptan-2-yl)amino)heptyl) thio)isoindoline-1,3-dione;
2-(2,6-dioxopiperidin-3-yl)-6-fluoro-4-((7-(((1R,2S,4R)-1,7,7-trimethylbicyclo[2.2.1]heptan-2-yl)amino)heptyl) thio)isoindoline-1,3-dione;
2-(2,6-dioxopiperidin-3-yl)-4-fluoro-7-((7-(((1R,2S,4R)-1,7,7-trimethylbicyclo[2.2.1]heptan-2-yl)amino)heptyl) thio)isoindoline-1,3-dione;
2-(2,6-dioxopiperidin-3-yl)-5-fluoro-4-((4-((((1R,2S,4R)-1,7,7-trimethylbicyclo[2.2.1]heptan-2-yl)amino)methyl) benzyl)thio)isoindoline-1,3-dione;
2-(2,6-dioxopiperidin-3-yl)-6-fluoro-4-((4-((((1R,2S,4R)-1,7,7-trimethylbicyclo[2.2.1]heptan-2-yl)amino)methyl) benzyl)thio)isoindoline-1,3-dione;
2-(2,6-dioxopiperidin-3-yl)-4-fluoro-7-((4-((((1R,2S,4R)-1,7,7-trimethylbicyclo[2.2.1]heptan-2-yl)amino)methyl) benzyl)thio)isoindoline-1,3-dione;
4-((4-(((adamantan-1-yl)amino)methyl)benzyl)thio)-2-(2,6-dioxopiperidin-3-yl)-5-fluoroisoindoline-1,3-dione;
4-((4-(((adamantan-1-yl)amino)methyl)benzyl)thio)-2-(2,6-dioxopiperidin-3-yl)-6-fluoroisoindoline-1,3-dione;
4-((4-(((adamantan-1-yl)amino)methyl)benzyl)thio)-2-(2,6-dioxopiperidin-3-yl)-7-fluoroisoindoline-1,3-dione;
2-(2,6-dioxopiperidin-3-yl)-5-fluoro-4-((7-(spiro[3.3]heptan-2-ylamino)heptyl)thio)isoindoline-1,3-dione;
2-(2,6-dioxopiperidin-3-yl)-6-fluoro-4-((7-(spiro[3.3]heptan-2-ylamino)heptyl)thio)isoindoline-1,3-dione;
2-(2,6-dioxopiperidin-3-yl)-4-fluoro-7-((7-(spiro[3.3]heptan-2-ylamino)heptyl)thio)isoindoline-1,3-dione;
4-(8-((adamantan-1-yl)amino)octyl)-2-(2,6-dioxopiperidin-3-yl)-5-fluoroisoindoline-1,3-dione;
4-(8-((adamantan-1-yl)amino)octyl)-2-(2,6-dioxopiperidin-3-yl)-6-fluoroisoindoline-1,3-dione;
4-(8-((adamantan-1-yl)amino)octyl)-2-(2,6-dioxopiperidin-3-yl)-7-fluoroisoindoline-1,3-dione;
4-((7-((adamantan-1-yl)amino)heptyl)oxy)-2-(2,6-dioxopiperidin-3-yl)-5-fluoroisoindoline-1,3-dione;
4-((7-((adamantan-1-yl)amino)heptyl)oxy)-2-(2,6-dioxopiperidin-3-yl)-6-fluoroisoindoline-1,3-dione;
4-((7-((adamantan-1-yl)amino)heptyl)oxy)-2-(2,6-dioxopiperidin-3-yl)-7-fluoroisoindoline-1,3-dione;
4-((7-((adamantan-1-yl)amino)heptyl)amino)-2-(2,6-dioxopiperidin-3-yl)-5-fluoroisoindoline-1,3-dione;
4-((7-((adamantan-1-yl)amino)heptyl)amino)-2-(2,6-dioxopiperidin-3-yl)-6-fluoroisoindoline-1,3-dione;
4-((7-((adamantan-1-yl)amino)heptyl)amino)-2-(2,6-dioxopiperidin-3-yl)-7-fluoroisoindoline-1,3-dione;
2-(2,6-dioxopiperidin-3-yl)-5-fluoro-4-((3-fluoro-4-(piperidin-1-ylmethyl)benzyl)thio)isoindoline-1,3-dione;
2-(2,6-dioxopiperidin-3-yl)-6-fluoro-4-((3-fluoro-4-(piperidin-1-ylmethyl)benzyl)thio)isoindoline-1,3-dione;
2-(2,6-dioxopiperidin-3-yl)-4-fluoro-7-((3-fluoro-4-(piperidin-1-ylmethyl)benzyl)thio)isoindoline-1,3-dione;

4-((4-((4,4-difluoropiperidin-1-yl)methyl)-3-fluorobenzyl)thio)-2-(2,6-dioxopiperidin-3-yl)isoindoline-1,3-dione;
4-((4-((4,4-difluoropiperidin-1-yl)methyl)-3-fluorobenzyl)thio)-2-(2,6-dioxopiperidin-3-yl)-5-fluoroisoindoline-1,3-dione;
4-((4-((4,4-difluoropiperidin-1-yl)methyl)-3-fluorobenzyl)thio)-2-(2,6-dioxopiperidin-3-yl)-6-fluoroisoindoline-1,3-dione;
4-((4-((4,4-difluoropiperidin-1-yl)methyl)-3-fluorobenzyl)thio)-2-(2,6-dioxopiperidin-3-yl)-7-fluoroisoindoline-1,3-dione;
4-((4-(2-((adamantan-1-yl)amino)ethyl)benzyl)thio)-2-(2,6-dioxopiperidin-3-yl)-5-fluoroisoindoline-1,3-dione;
4-((4-(2-((adamantan-1-yl)amino)ethyl)benzyl)thio)-2-(2,6-dioxopiperidin-3-yl)-6-fluoroisoindoline-1,3-dione;
4-((4-(2-((adamantan-1-yl)amino)ethyl)benzyl)thio)-2-(2,6-dioxopiperidin-3-yl)-7-fluoroisoindoline-1,3-dione;
4-(((4-(((adamantan-1-yl)amino)methyl)thiazol-2-yl)methyl)thio)-2-(2,6-dioxopiperidin-3-yl)-5-fluoroisoindoline-1,3-dione;
4-(((4-(((adamantan-1-yl)amino)methyl)thiazol-2-yl)methyl)thio)-2-(2,6-dioxopiperidin-3-yl)-6-fluoroisoindoline-1,3-dione; and
4-(((4-(((adamantan-1-yl)amino)methyl)thiazol-2-yl)methyl)thio)-2-(2,6-dioxopiperidin-3-yl)-7-fluoroisoindoline-1,3-dione.

**25.** A pharmaceutical composition comprising the compound of Formula (I) of any one of claims 1-24 or a pharmaceutically acceptable salt thereof, and at least one pharmaceutically acceptable carrier.

**26.** The pharmaceutical composition of claim 25, further comprising at least one additional therapeutic agent.

**27.** The pharmaceutical composition of claim 26, wherein said at least one additional therapeutic agent is used for treating or preventing cancer or tumor.

**28.** The compound of Formula (I) of any one of claims 1-24 or a pharmaceutically acceptable salt thereof, for use as a medicament.

**29.** The compound of Formula (I) of any one of claims 1-24 or a pharmaceutically acceptable salt thereof, for use in the prevention and/or treatment of a disease or disorder associated with cereblon protein.

**30.** The compound of Formula (I) or pharmaceutically acceptable salt thereof of claim 29, wherein the disease or disorder associated with cereblon protein is selected from the group consisting of: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes.

**31.** The compound of Formula (I) or pharmaceutically acceptable salt thereof of claim 29, wherein the disease or disorder associated with cereblon protein is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; Burkitt's lymphoma; thyroid cancer; melanoma; lung cancer, including lung adenocarcinoma, lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; neuroglioma; Peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

**32.** Use of the compound of Formula (I) of any one of claims 1-24 or a pharmaceutically acceptable salt thereof for the manufacture of a medicament for the prevention and/or treatment of a disease or disorder associated with cereblon protein.

**33.** A method for treating or preventing a disease or disorder associated with cereblon protein in a subject, comprising administering to the subject a therapeutically effective amount of the compound of Formula (I) of any one of claims 1-24 or a pharmaceutically acceptable salt thereof, or the pharmaceutical composition of any one of claims 25-27.

**34.** The use of claim 32 or the method of claim 33, wherein the disease or disorder associated with cereblon protein is selected from the group consisting of: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes.

**35.** The use of claim 32 or the method of claim 33, wherein the disease or disorder associated with cereblon protein is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; Burkitt's lymphoma; thyroid cancer; melanoma; lung cancer, including lung adenocarcinoma, lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; neuroglioma; Peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock;

or acute liver failure.

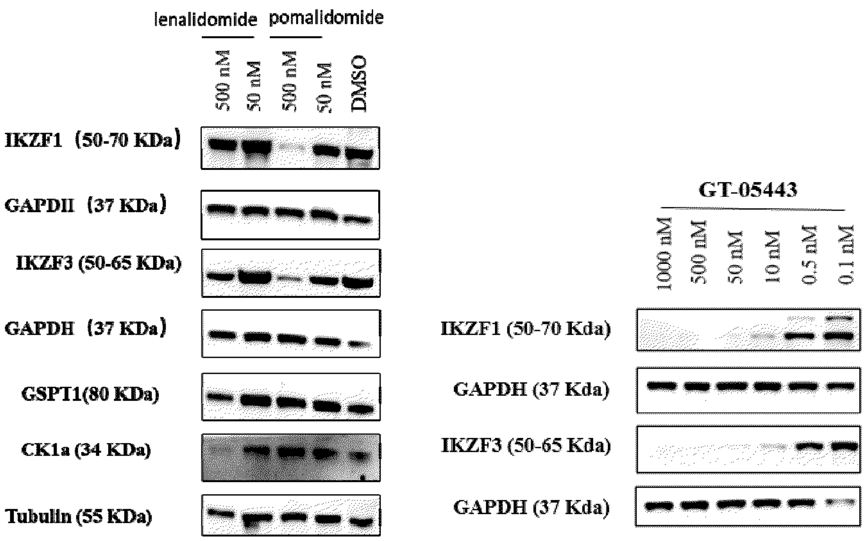
lenalidomide
pomalidomide
500 nM
50 nM
500 nM
50 nM
DMSO
IKZF1 (50-70 KDa)
GAPDH (37 KDa)
IKZF3 (50-65 KDa)
GAPDH (37 KDa)
GSPT1(80 KDa)
CK1a (34 KDa)
Tubulin (55 KDa)
GT-05443
1000 nM
500 nM
50 nM
10 nM
0.5 nM
0.1 nM
IKZF1 (50-70 Kda)
GAPDH (37 Kda)
IKZF3 (50-65 Kda)
GAPDH (37 Kda)

Figure 1

**INTERNATIONAL SEARCH REPORT**

International application No.
**PCT/CN2023/117399**

**A. CLASSIFICATION OF SUBJECT MATTER**

C07D 401/04(2006.01)i; C07D 401/14(2006.01)i; C07D 405/14(2006.01)i; C07D 471/04(2006.01)i; C07D 491/107(2006.01)i; C07D 487/08(2006.01)i; C07D 413/14(2006.01)i; C07D 471/08(2006.01)i; C07D 409/14(2006.01)i; C07D 417/14(2006.01)i; A61K 31/454(2006.01)i; A61K 31/496(2006.01)i; A61K 31/541(2006.01)i; A61K 31/55(2006.01)i; A61K 31/506(2006.01)i; A61K 31/517(2006.01)i; A61K 31/675(2006.01)i; A61P 35/00(2006.01)i; A61P 35/02(2006.01)i; A61P 7/06(2006.01)i

According to International Patent Classification (IPC) or to both national classification and IPC

**B. FIELDS SEARCHED**

Minimum documentation searched (classification system followed by classification symbols)

IPC:C07D A61K A61P

Documentation searched other than minimum documentation to the extent that such documents are included in the fields searched

Electronic data base consulted during the international search (name of data base and, where practicable, search terms used)

CNTXT; VEN; WOTXT; USTXT; EPTXT; CNKI; 万方, WANFANG; STN: 标新生物医药, 杨小宝, 孙仁红, cereblon, CRBN, 沙利度胺, 来那度胺, 泊马度胺, 邻苯二甲酰亚胺, 免疫调节, 多发性骨髓瘤, 肿瘤, 癌症, thalidomid+, lenalidomid+, pomalidomid+, phthalimid+, immunomodulat+, multiple myelom, tumor, cancer, 结构式, structural formula

**C. DOCUMENTS CONSIDERED TO BE RELEVANT**

| Category* | Citation of document, with indication, where appropriate, of the relevant passages | Relevant to claim No. |
|---|---|---|
| X | CN 110963994 A (SHANGHAI INSTITUTE OF MATERIA MEDICA, CHINESE ACADEMY OF SCIENCES) 07 April 2020 (2020-04-07)<br>claims 1-18, and description, paragraphs [0416]-[0419] | 1-3, 5, 9, 10, 12, 14, 15, 17, 21, 25, 26, 28-32, 34 (in part), 35 (in part) |
| Y | CN 110963994 A (SHANGHAI INSTITUTE OF MATERIA MEDICA, CHINESE ACADEMY OF SCIENCES) 07 April 2020 (2020-04-07)<br>claims 1-18, and description, paragraphs [0416]-[0419] | 1-32, 34 (in part), 35 (in part) |
| X | CN 105566290 A (KANGPU BIOMEDICAL TECHNOLOGY (SHANGHAI) CO., LTD.) 11 May 2016 (2016-05-11)<br>claims 1-13, and description, paragraphs [0046], [0047] and [0052]-[0054] | 1-3, 5, 9, 10, 12-18, 21, 25-32, 34 (in part), 35 (in part) |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 November 2023** | **26 December 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/117399**

**C. DOCUMENTS CONSIDERED TO BE RELEVANT**

| Category* | Citation of document, with indication, where appropriate, of the relevant passages | Relevant to claim No. |
|---|---|---|
| X | CN 111285850 A (SHANGHAI INSTITUTE OF MATERIA MEDICA, CHINESE ACADEMY OF SCIENCES) 16 June 2020 (2020-06-16) claims 1-15 | 1-3, 5, 9, 10, 12-15, 17, 25-32, 34 (in part), 35 (in part) |
| X | WO 2020118098 A1 (VIVIDION THERAPEUTICS INC.) 11 June 2020 (2020-06-11) description, pages 37 and 38; and claims 1-44 | 1-3, 5, 9, 10, 12, 14, 15, 17, 25, 28-32, 34 (in part), 35 (in part) |
| X | CN 110612294 A (ARVINAS OPERATIONS, INC.) 24 December 2019 (2019-12-24) description, paragraph [2364], table 1, and claims 1-33 | 1-3, 5, 9-12, 14, 15, 25-32, 34 (in part), 35 (in part) |
| X | WO 2022061348 A1 (BIOTHERYX INC.) 24 March 2022 (2022-03-24) description, paragraphs [00426], [00457], [00486], [00493] and [00533] | 1-3, 5, 9-12, 14, 15, 17, 18, 21 |
| X | CN 111247138 A (BIOTHERYX INC.) 05 June 2020 (2020-06-05) description, paragraphs [0394]-[0404] | 1-3, 5, 9, 10, 12, 14, 15, 17 |
| Y | CN 111606883 A (SHANGHAITECH UNIVERSITY) 01 September 2020 (2020-09-01) description, paragraphs [0003]-[0006]; claims 1-73; and description, paragraph [0124], and table 1 | 1-32, 34 (in part), 35 (in part) |
| Y | CN 109311900 A (THE REGENTS OF THE UNIVERSITY OF MICHIGAN) 05 February 2019 (2019-02-05) claims 1-81 | 1-32, 34 (in part), 35 (in part) |
| Y | CN 113582980 A (SHANGHAITECH UNIVERSITY) 02 November 2021 (2021-11-02) claims 1-31, and description, paragraph [0051], and table 1 | 1-32, 34 (in part), 35 (in part) |
| A | CN 112321566 A (SHANGHAITECH UNIVERSITY) 05 February 2021 (2021-02-05) entire document | 1-32, 34 (in part), 35 (in part) |
| A | CN 112707900 A (SHANGHAITECH UNIVERSITY et al.) 27 April 2021 (2021-04-27) entire document | 1-32, 34 (in part), 35 (in part) |

Form PCT/ISA/210 (second sheet) (July 2022)

**INTERNATIONAL SEARCH REPORT**

International application No.
**PCT/CN2023/117399**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **33, 34 (in part), 35 (in part)**
because they relate to subject matter not required to be searched by this Authority, namely:

The method for treatment or prevention of a cereblon protein-related disease or condition in a subject that is set forth in claim 33, and the technical solutions of claims 34 and 35, which refer to claim 33, fall within methods for treatment of a living human or animal body, that is, fall within the cases set out in PCT Rule 39.1(iv) for which an international search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/117399**

| Patent document cited in search report | Publication date (day/month/year) | Patent family member(s) | Publication date (day/month/year) |
|---|---|---|---|
| CN 110963994 A | 07 April 2020 | BR 112021006458 A2 | 06 July 2021 |
| | | KR 20210069085 A | 10 June 2021 |
| | | CA 3121667 A1 | 02 April 2020 |
| | | AU 2019348094 A1 | 27 May 2021 |
| | | AU 2019348094 B2 | 22 December 2022 |
| | | WO 2020064002 A1 | 02 April 2020 |
| | | EP 3862348 A1 | 11 August 2021 |
| | | EP 3862348 A4 | 22 June 2022 |
| | | US 2022041576 A1 | 10 February 2022 |
| | | JP 2022503942 A | 12 January 2022 |
| | | JP 7168773 B2 | 09 November 2022 |
| | | HK 40052595 A0 | 21 January 2022 |
| | | CN 110963994 B | 08 February 2022 |
| | | CN 114085212 A | 25 February 2022 |
| | | RU 2021111871 A | 31 October 2022 |
| | | CN 114085212 B | 02 May 2023 |
| CN 105566290 A | 11 May 2016 | KR 20170078740 A | 07 July 2017 |
| | | KR 102191256 B1 | 15 December 2020 |
| | | AU 2015341301 A1 | 01 June 2017 |
| | | AU 2015341301 B2 | 16 May 2019 |
| | | CA 2966038 A1 | 06 May 2016 |
| | | CA 2966038 C | 21 April 2020 |
| | | RU 2017118453 A | 04 December 2018 |
| | | RU 2695521 C2 | 23 July 2019 |
| | | RU 2695521 C9 | 19 August 2019 |
| | | JP 2017533955 A | 16 November 2017 |
| | | JP 6546997 B2 | 17 July 2019 |
| | | US 2017313676 A1 | 02 November 2017 |
| | | US 10017492 B2 | 10 July 2018 |
| | | NZ 731789 A | 26 April 2019 |
| | | EP 3214081 A1 | 06 September 2017 |
| | | EP 3214081 A4 | 15 August 2018 |
| | | EP 3214081 B1 | 08 July 2020 |
| | | ES 2812877 T3 | 18 March 2021 |
| | | ES 2901509 T3 | 22 March 2022 |
| | | EP 3643709 A1 | 29 April 2020 |
| | | EP 3643709 B1 | 20 October 2021 |
| | | WO 2016065980 A1 | 06 May 2016 |
| | | HK 1235401 A0 | 09 March 2018 |
| | | ZA 201703036 A | 28 March 2018 |
| | | CN 105566290 B | 22 May 2020 |
| | | CN 111205268 A | 29 May 2020 |
| | | HK 40018274 A0 | 30 September 2020 |
| | | CN 111205268 B | 26 January 2021 |
| | | HK 1235401 A1 | 29 January 2021 |
| | | HK 40018274 A1 | 18 February 2022 |
| CN 111285850 A | 16 June 2020 | CA 3122317 A1 | 11 June 2020 |
| | | AU 2019392231 A1 | 29 July 2021 |
| | | AU 2019392231 B2 | 20 October 2022 |
| | | WO 2020114482 A1 | 11 June 2020 |

Form PCT/ISA/210 (patent family annex) (July 2022)

**INTERNATIONAL SEARCH REPORT**
**Information on patent family members**

International application No.
**PCT/CN2023/117399**

| Patent document cited in search report | Publication date (day/month/year) | Patent family member(s) | Publication date (day/month/year) |
|---|---|---|---|
| | | EP 3896062 A1 | 20 October 2021 |
| | | EP 3896062 A4 | 15 June 2022 |
| | | US 2022041578 A1 | 10 February 2022 |
| | | HK 40053022 A0 | 28 January 2022 |
| | | CN 111285850 B | 22 April 2022 |
| WO 2020118098 A1 | 11 June 2020 | EP 3891128 A1 | 13 October 2021 |
| | | EP 3891128 A4 | 17 August 2022 |
| | | US 2023045737 A1 | 09 February 2023 |
| CN 110612294 A | 24 December 2019 | KR 20190116315 A | 14 October 2019 |
| | | JP 2020506922 A | 05 March 2020 |
| | | BR 112019015484 A2 | 28 April 2020 |
| | | AU 2022221386 A1 | 15 September 2022 |
| | | EP 3577109 A1 | 11 December 2019 |
| | | EP 3577109 A4 | 18 November 2020 |
| | | CA 3050309 A1 | 09 August 2018 |
| | | RU 2019123462 A | 27 January 2021 |
| | | MX 2019009046 A | 30 October 2019 |
| | | AU 2018215212 A1 | 11 July 2019 |
| | | AU 2018215212 B2 | 02 June 2022 |
| | | WO 2018144649 A1 | 09 August 2018 |
| | | WO 2018144649 A8 | 22 August 2019 |
| | | US 2018215731 A1 | 02 August 2018 |
| | | IL 268069 A | 29 August 2019 |
| | | IN 201937034098 A | 22 November 2019 |
| | | HK 40009838 A0 | 26 June 2020 |
| | | RU 2019123462 A | 27 January 2021 |
| | | CN 115974840 A | 18 April 2023 |
| | | RU 2795146 C2 | 28 April 2023 |
| | | IN 202138038142 A | 10 September 2021 |
| WO 2022061348 A1 | 24 March 2022 | TW 202227427 A | 16 July 2022 |
| CN 111247138 A | 05 June 2020 | TW 201919639 A | 01 June 2019 |
| | | EP 3672955 A1 | 01 July 2020 |
| | | WO 2019040274 A1 | 28 February 2019 |
| | | US 2019062320 A1 | 28 February 2019 |
| | | US 10513515 B2 | 24 December 2019 |
| | | TW 202222794 A | 16 June 2022 |
| | | JP 2020531481 A | 05 November 2020 |
| | | CA 3072543 A1 | 28 February 2019 |
| | | IN 202017005715 A | 06 March 2020 |
| | | TW 742303 B1 | 11 October 2021 |
| CN 111606883 A | 01 September 2020 | AU 2020228803 A1 | 30 September 2021 |
| | | AU 2020228803 B2 | 18 May 2023 |
| | | WO 2020173426 A1 | 03 September 2020 |
| | | JP 2022521746 A | 12 April 2022 |
| | | CA 3132308 A1 | 03 September 2020 |
| | | EP 3932922 A1 | 05 January 2022 |
| | | EP 3932922 A4 | 11 May 2022 |
| | | KR 20210142114 A | 24 November 2021 |
| | | US 2022143002 A1 | 12 May 2022 |

Form PCT/ISA/210 (patent family annex) (July 2022)

**INTERNATIONAL SEARCH REPORT**
**Information on patent family members**

International application No.
**PCT/CN2023/117399**

| Patent document cited in search report | Publication date (day/month/year) | Patent family member(s) | Publication date (day/month/year) |
|---|---|---|---|
| | | IN 202117038488 A | 10 December 2021 |
| | | HK 40057665 A0 | 14 April 2022 |
| | | CN 111606883 B | 09 May 2023 |
| CN 109311900 A | 05 February 2019 | WO 2017176958 A1 | 12 October 2017 |
| | | US 2019119289 A1 | 25 April 2019 |
| | | US 10759808 B2 | 01 September 2020 |
| | | JP 2022050469 A | 30 March 2022 |
| | | CA 3020281 A1 | 12 October 2017 |
| | | US 2021002289 A1 | 07 January 2021 |
| | | AU 2017246453 A1 | 08 November 2018 |
| | | EP 3440082 A1 | 13 February 2019 |
| | | AU 2021232732 A1 | 14 October 2021 |
| | | JP 2019513746 A | 30 May 2019 |
| | | JP 7001614 B2 | 03 February 2022 |
| | | HK 40004606 A0 | 29 April 2020 |
| CN 113582980 A | 02 November 2021 | CA 3177252 A1 | 04 November 2021 |
| | | US 2023203022 A1 | 29 June 2023 |
| | | AU 2021262334 A1 | 22 December 2022 |
| | | WO 2021219078 A1 | 04 November 2021 |
| | | EP 4144728 A1 | 08 March 2023 |
| | | HK 40061821 A0 | 10 June 2022 |
| CN 112321566 A | 05 February 2021 | WO 2021023233 A1 | 11 February 2021 |
| | | US 2022313829 A1 | 06 October 2022 |
| | | HK 40041926 A0 | 27 August 2021 |
| | | CN 112321566 B | 10 June 2022 |
| | | HK 40041926 A1 | 16 September 2022 |
| CN 112707900 A | 27 April 2021 | WO 2021078301 A1 | 29 April 2021 |
| | | CN 112707900 B | 10 June 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Clinical Pharmacology. People's Public Health Press, 2008